# EUROPEAN PATENT APPLICATION

(11) **EP 2 930 244 A1**
(43) Date of publication of application: **14.10.2015**
(21) Application number: 14163770.2
(22) Date of filing: 07.04.2014
(51) Int. Cl.: C12P 19/26, C12P 7/42, C12P 7/52, C12P 7/62

(54) **Microorganisms and methods for producing acrylate and other products from homoserine**

(71) Applicant: The Procter & Gamble Company, Cincinnati, OH 45202 (US)
(72) Inventor: Xu, Jun, Mason, OH 45040 (US); Saunders, Charles, Fairfield, OH 45014 (US); Green, Phillip, Wyoming, OH 45231 (US); Velasquez, Juan, Cincinnati, OH 45202 (US)
(74) Representative: Boubel, Thomas

(57) **Abstract**

The invention relates to a micro-organism that is able to convert homoserine to 3-hydroxypropionaldehyde, wherein the micro-organism expresses a protein capable of converting homoserine to 2-keto-4-hydroxybutyrate wherein the protein is selected from the group consisting of: aminotransferases, an amino acid oxidases, and amino acid dehydrogenases.

SEQUENCE LISTING

<110>  The Procter & Gamble Company
       Xu, Jun
       Saunders, Charles W
       Green, Phillip R
       Velasquez, Juan E

<120>  Microorganisms And Methods For Producing Acrylate And Other
       Products From Homoserine

<130>  CM4068F

<160>  127

<170>  PatentIn version 3.5

<210>  1
<211>  2001
<212>  DNA
<213>  Sus scrofa

<400>  1
gagcggctcc gggcgcgagg tgaaagctcc cggccgactc ctgctctcta gctatggcac      60

ctccatcagt ctttgccgag gttcctcagg cccagccggt ccttgtcttt aagctcattg     120

ctgacttccg ggaggatccg gacccccgca aggtcaacct aggagtggga gcttatcgca     180

ccgatgattg ccagccttgg gttttgccag tcgtgaggaa ggtggagcag aggattgcta     240

atgacagcag cctaaaccac gagtacctgc ccatcctggg cctggcagag ttccggacct     300

gtgcttcccg ccttgccctt ggagatgaca gcccagctct tcaggagaag cgggtgggag     360

gggtgcagtc tttgggggga acgggtgcac ttcgaattgg agctgagttc ttagcacgat     420

ggtacaatgg aacgaacaac aaagacacgc ctgtctacgt atcctcacca acctgggaaa     480

atcacaatgg agtcttcact actgctggat tcaaagacat tcggtcctat cgctattggg     540

ataccgagaa gagaggactt gatctccagg gtttcctgag tgatctggag aacgctcctg     600

agttctccat ctttgtcctc cacgcctgtg cccacaaccc gacagggacc gacccaactc     660

cggagcaatg gaagcagatc gcctctgtca tgaagcgccg gtttctgttc cccttctttg     720

actcagccta tcagggcttc gcatctggca acctagaaaa agacgcctgg gccattcgct     780

attttgtgtc tgaagggttc gagctcttct gtgcccagtc cttctccaag aacttcgggc     840

tctacaatga gcgcgtgggg aacctgaccg tggttgcaaa agaacccgat agcatcctgc     900

gagtcctttc ccagatggag aagatcgtgc gagtgacgtg gtccaatccc cctgctcagg     960

gagcgagaat cgtggcccgt acgctctctg accctgagct ctttcatgaa tggacaggta    1020

acgtgaagac aatggctgac cgcattctga gcatgagatc tgagcttagg gcacgattag 1080

aagccctcaa gacccctgga acctggaacc acatcacgga ccagattgga atgttcagct 1140

tcactgggtt gaaccccaag caggttgaat atctgatcaa cgaaaagcac atctatctgc 1200

taccaagtgg tcggatcaac atgtgtggct taaccaccaa aaatctagat tatgtggcca 1260

cctccatcca tgaagctgtc accaaaatcc agtgaagcaa caccacccaa gccagcgcca 1320

cccaagcggt cctctgtctc gtgtgttccc tgcctgcaca aacctggttc tatacatcac 1380

aactgtatta gaggctaccg agggacagaa aaggctgctc tggtgaggta gctgctattt 1440

aaattggccc catgggaaga gaacatctct tgaaaagaaa tgggggccag ggaatagagc 1500

ccttttggag gccagagcaa attcaggctt ttatttgaaa agaataaaaa ggtcctttga 1560

tcatgagatg tagatgtctt gccccctcac tagaagcagg agtattgcct gtgtcactca 1620

cgtgctcctg tgtgttttac tctgtacaaa gtctagtccc aaagatcaag ttgtctgaag 1680

agcaaagtgt gattgtgggt attggctgtg tcattaacag ttgtcctctg gacccagagt 1740

gtctgtctcc ctgctctttc tgcatggctc tgtccctagc cctaagcttg agttctttag 1800

ggtggtcaag gtaggaaata tatttatatt ttacccacac gttaactgaa ataaaagttt 1860

cacagagtca aatttaccct tactatgtgg agtacattct ggtattttct tttctattct 1920

attctattct attctattct attctattct attctattct gttgtttcac taaagaaata 1980

aaagtgctga ttgagaccca t 2001

<210> 2
<211> 413
<212> PRT
<213> Sus scrofa

<400> 2

Met Ala Pro Pro Ser Val Phe Ala Glu Val Pro Gln Ala Gln Pro Val
1               5                   10                  15

Leu Val Phe Lys Leu Ile Ala Asp Phe Arg Glu Asp Pro Asp Pro Arg
            20                  25                  30

Lys Val Asn Leu Gly Val Gly Ala Tyr Arg Thr Asp Asp Cys Gln Pro
        35                  40                  45

Trp Val Leu Pro Val Val Arg Lys Val Glu Gln Arg Ile Ala Asn Asp
    50                  55                  60

Ser Ser Leu Asn His Glu Tyr Leu Pro Ile Leu Gly Leu Ala Glu Phe
65                  70                  75                  80

Arg Thr Cys Ala Ser Arg Leu Ala Leu Gly Asp Asp Ser Pro Ala Leu

                85                  90                  95

Gln Glu Lys Arg Val Gly Gly Val Gln Ser Leu Gly Gly Thr Gly Ala
            100                 105                 110

Leu Arg Ile Gly Ala Glu Phe Leu Ala Arg Trp Tyr Asn Gly Thr Asn
        115                 120                 125

Asn Lys Asp Thr Pro Val Tyr Val Ser Ser Pro Thr Trp Glu Asn His
    130                 135                 140

Asn Gly Val Phe Thr Thr Ala Gly Phe Lys Asp Ile Arg Ser Tyr Arg
145                 150                 155                 160

Tyr Trp Asp Thr Glu Lys Arg Gly Leu Asp Leu Gln Gly Phe Leu Ser
                165                 170                 175

Asp Leu Glu Asn Ala Pro Glu Phe Ser Ile Phe Val Leu His Ala Cys
            180                 185                 190

Ala His Asn Pro Thr Gly Thr Asp Pro Thr Pro Glu Gln Trp Lys Gln
        195                 200                 205

Ile Ala Ser Val Met Lys Arg Arg Phe Leu Phe Pro Phe Phe Asp Ser
    210                 215                 220

Ala Tyr Gln Gly Phe Ala Ser Gly Asn Leu Glu Lys Asp Ala Trp Ala
225                 230                 235                 240

Ile Arg Tyr Phe Val Ser Glu Gly Phe Glu Leu Phe Cys Ala Gln Ser
                245                 250                 255

Phe Ser Lys Asn Phe Gly Leu Tyr Asn Glu Arg Val Gly Asn Leu Thr
            260                 265                 270

Val Val Ala Lys Glu Pro Asp Ser Ile Leu Arg Val Leu Ser Gln Met
        275                 280                 285

Glu Lys Ile Val Arg Val Thr Trp Ser Asn Pro Pro Ala Gln Gly Ala
    290                 295                 300

Arg Ile Val Ala Arg Thr Leu Ser Asp Pro Glu Leu Phe His Glu Trp
305                 310                 315                 320

Thr Gly Asn Val Lys Thr Met Ala Asp Arg Ile Leu Ser Met Arg Ser
                325                 330                 335

Glu Leu Arg Ala Arg Leu Glu Ala Leu Lys Thr Pro Gly Thr Trp Asn
            340                 345                 350

His Ile Thr Asp Gln Ile Gly Met Phe Ser Phe Thr Gly Leu Asn Pro
        355                 360                 365

Lys Gln Val Glu Tyr Leu Ile Asn Glu Lys His Ile Tyr Leu Leu Pro
    370                 375                 380

Ser Gly Arg Ile Asn Met Cys Gly Leu Thr Thr Lys Asn Leu Asp Tyr
385                 390                 395                 400

Val Ala Thr Ser Ile His Glu Ala Val Thr Lys Ile Gln
                405                 410

<210>  3
<211>  1744
<212>  DNA
<213>  Rattus norvegicus

<400>  3
ctgcagtgtg aagggtgttg tcttacctct cgcagacttt cccattccca gccctgattt      60

ccccactgga cccttctcct tctgaaccag catcctgcct ggtttgagca gtcatggcct     120

cacgggtgaa tgatcaaagc caggcttcaa ggaatgggct gaagggaaag gtgctaactc     180

tggacactat gaacccatgt gtgcggaggg tggagtatgc agttcgagga cccattgtgc     240

agcgtgcctt ggagctggag caggagctgc gtcagggtgt gaagaagccg tttactgagg     300

tcatccgtgc caacattggg gatgcacaag ccatggggca gagacccatc accttcttcc     360

gccaggtcct ggccctctgt gtctacccca atcttctgag cagtcctgac ttcccagagg     420

atgccaagag aagggcagaa cgcatcttgc aggcctgcgg gggccacagc ctgggtgcct     480

atagcattag ctctggaatc cagccgatcc gggaggatgt ggcgcaatac attgagagaa     540

gagacggagg catccccgca gacccgaaca acatatttct atccacaggg gccagcgatg     600

ccatcgtgac aatgctcaag ctgctggtat ctggcgaggg ccgtgcacga acaggtgtac     660

tcattcccat tcctcagtac ccactgtact cagccgcgct ggctgaactg gacgccgtgc     720

aagtggacta ctacctggac gaagagcgcg cctgggctct ggacatcgca gagctgcggc     780

gcgctctgtg ccaggcacgt gaccgttgct gccctcgagt actgtgcgtc atcaaccccg     840

gcaaccccac tgggcaggtg cagacccgtg agtgcatcga ggccgtaatc cgctttgctt     900

tcaaagaagg actcttcttg atggctgatg aggtatacca ggacaacgtg tatgccgagg     960

gctctcagtt ccattcattc aagaaggtgc tcatggagat ggggccaccg tattccacgc    1020

agcaggagct tgcttctttc cactcagtct ctaagggcta catgggcgag tgcgggtttc    1080

gtggtggcta tgtggaggtg gtaaacatgg atgctgaggt gcagaaacag atggggaagc    1140

tgatgagtgt gcggctgtgt ccaccagtgc caggccaggc cttgatggac atggtggtca 1200

gtccgccaac accctccgag ccgtccttca agcagtttca agcagagaga caggaggtgc 1260

tggctgaact ggcagccaag gctaagctca cggagcaggt cttcaatgag gctcccggga 1320

tccgctgcaa cccagtgcag ggcgccatgt attccttccc tcaagtgcag ctgcccttga 1380

aagcggtgca gcgtgctcag gaactgggcc tggcccctga catgttcttc tgcctgtgcc 1440

tcctggaaga gactggcatc tgcgttgtgc ccgggagtgg ctttgggcag caggagggca 1500

cctatcattt ccggatgacc attctgcccc ccatggagaa actgcggctg ctgctggaaa 1560

aactcagtca cttccatgcc aagttcaccc atgagtactc ctgaagccac tgctagggcc 1620

acactggaca gtctctgacg caacaaaccg agggtcctta ggaaccctca gtatttctga 1680

ttttgtctag ggtctcggta actgtcctgc gggtccctaa taaatctgat gtcagcctga 1740

aaaa 1744

<210> 4
<211> 496
<212> PRT
<213> Rattus norvegicus

<400> 4

Met Ala Ser Arg Val Asn Asp Gln Ser Gln Ala Ser Arg Asn Gly Leu
1               5                   10                  15

Lys Gly Lys Val Leu Thr Leu Asp Thr Met Asn Pro Cys Val Arg Arg
            20                  25                  30

Val Glu Tyr Ala Val Arg Gly Pro Ile Val Gln Arg Ala Leu Glu Leu
        35                  40                  45

Glu Gln Glu Leu Arg Gln Gly Val Lys Lys Pro Phe Thr Glu Val Ile
    50                  55                  60

Arg Ala Asn Ile Gly Asp Ala Gln Ala Met Gly Gln Arg Pro Ile Thr
65                  70                  75                  80

Phe Phe Arg Gln Val Leu Ala Leu Cys Val Tyr Pro Asn Leu Leu Ser
                85                  90                  95

Ser Pro Asp Phe Pro Glu Asp Ala Lys Arg Arg Ala Glu Arg Ile Leu
            100                 105                 110

Gln Ala Cys Gly Gly His Ser Leu Gly Ala Tyr Ser Ile Ser Ser Gly
        115                 120                 125

Ile Gln Pro Ile Arg Glu Asp Val Ala Gln Tyr Ile Glu Arg Arg Asp
    130                 135                 140

Gly Gly Ile Pro Ala Asp Pro Asn Asn Ile Phe Leu Ser Thr Gly Ala
145                 150                 155                 160

Ser Asp Ala Ile Val Thr Met Leu Lys Leu Leu Val Ser Gly Glu Gly
                165                 170                 175

Arg Ala Arg Thr Gly Val Leu Ile Pro Ile Pro Gln Tyr Pro Leu Tyr
            180                 185                 190

Ser Ala Ala Leu Ala Glu Leu Asp Ala Val Gln Val Asp Tyr Tyr Leu
        195                 200                 205

Asp Glu Glu Arg Ala Trp Ala Leu Asp Ile Ala Glu Leu Arg Arg Ala
    210                 215                 220

Leu Cys Gln Ala Arg Asp Arg Cys Cys Pro Arg Val Leu Cys Val Ile
225                 230                 235                 240

Asn Pro Gly Asn Pro Thr Gly Gln Val Gln Thr Arg Glu Cys Ile Glu
                245                 250                 255

Ala Val Ile Arg Phe Ala Phe Lys Glu Gly Leu Phe Leu Met Ala Asp
            260                 265                 270

Glu Val Tyr Gln Asp Asn Val Tyr Ala Glu Gly Ser Gln Phe His Ser
        275                 280                 285

Phe Lys Lys Val Leu Met Glu Met Gly Pro Pro Tyr Ser Thr Gln Gln
    290                 295                 300

Glu Leu Ala Ser Phe His Ser Val Ser Lys Gly Tyr Met Gly Glu Cys
305                 310                 315                 320

Gly Phe Arg Gly Gly Tyr Val Glu Val Val Asn Met Asp Ala Glu Val
                325                 330                 335

Gln Lys Gln Met Gly Lys Leu Met Ser Val Arg Leu Cys Pro Pro Val
            340                 345                 350

Pro Gly Gln Ala Leu Met Asp Met Val Val Ser Pro Pro Thr Pro Ser
        355                 360                 365

Glu Pro Ser Phe Lys Gln Phe Gln Ala Glu Arg Gln Glu Val Leu Ala
    370                 375                 380

Glu Leu Ala Ala Lys Ala Lys Leu Thr Glu Gln Val Phe Asn Glu Ala
385                 390                 395                 400

Pro Gly Ile Arg Cys Asn Pro Val Gln Gly Ala Met Tyr Ser Phe Pro
                405                 410                 415

Gln Val Gln Leu Pro Leu Lys Ala Val Gln Arg Ala Gln Glu Leu Gly
            420                 425                 430

Leu Ala Pro Asp Met Phe Phe Cys Leu Cys Leu Leu Glu Glu Thr Gly
        435                 440                 445

Ile Cys Val Val Pro Gly Ser Gly Phe Gly Gln Gln Glu Gly Thr Tyr
    450                 455                 460

His Phe Arg Met Thr Ile Leu Pro Pro Met Glu Lys Leu Arg Leu Leu
465                 470                 475                 480

Leu Glu Lys Leu Ser His Phe His Ala Lys Phe Thr His Glu Tyr Ser
                485                 490                 495

<210>  5
<211>  1182
<212>  DNA
<213>  Saccharomyces cerevisiae

<400>  5
atgttgcaga gacattcctt gaagttgggg aaattctcca tcagaacact cgctactggt      60

gccccattag atgcatccaa actaaaaatt actagaaacc caaatccatc caagccaaga     120

ccaaatgaag aattagtgtt cggccagaca ttcaccgatc atatgttgac cattccttgg     180

tcagccaaag aagggtgggg cactccacac atcaagcctt acggtaatct ttctcttgac     240

ccatctgctt gtgtattcca ttatgcattt gaattatttg aaggtttgaa agcctacaga     300

actcctcaaa atactatcac catgttccgt ccggataaga acatggcccg tatgaacaag     360

tctgccgcta gaatttgttt gccaactttc gaatctgaag aattgatcaa acttaccggg     420

aaattgatcg aacaagataa acacttggtt cctcaaggta atggttactc attatacatc     480

agaccaacaa tgattggtac atccaagggt ttaggtgttg gcactccctc cgaggctctt     540

ctttatgtta ttacttctcc agtcggtcct tattataaga ctggtttcaa agccgtacgt     600

cttgaagcaa cagactatgc tacaagagct tggccaggtg gtgttggcga caaaaaattg     660

ggtgctaact atgccccatg catcttacct caactacaag ctgccaaaag agggtaccaa     720

caaaatctat ggttgttcgg cccagaaaag aacatcactg aggttggtac tatgaacgtg     780

ttcttcgttt tcctcaacaa agtcactggc aagaaggaat tggttaccgc tccattagat     840

ggtaccattt tagaaggtgt taccagagac tctgttttaa cattggctcg tgacaaacta 900

gatcctcaag aatgggacat caacgagcgt tattacacta ttactgaagt cgccactaga 960

gcaaaacaag gtgaactatt agaagccttc ggttctggta ctgctgctgt cgtttcacct 1020

atcaaggaaa ttggctggaa caacgaagat attcatgttc cactattgcc tggtgaacaa 1080

tgtggtgcat tgaccaagca agttgctcaa tggattgctg atatccaata cggtagagtc 1140

aattatggta actggtcaaa aactgttgcc gacttgaact aa 1182

<210> 6
<211> 393
<212> PRT
<213> Saccharomyces cerevisiae

<400> 6

Met Leu Gln Arg His Ser Leu Lys Leu Gly Lys Phe Ser Ile Arg Thr
1               5                   10                  15

Leu Ala Thr Gly Ala Pro Leu Asp Ala Ser Lys Leu Lys Ile Thr Arg
            20                  25                  30

Asn Pro Asn Pro Ser Lys Pro Arg Pro Asn Glu Glu Leu Val Phe Gly
        35                  40                  45

Gln Thr Phe Thr Asp His Met Leu Thr Ile Pro Trp Ser Ala Lys Glu
    50                  55                  60

Gly Trp Gly Thr Pro His Ile Lys Pro Tyr Gly Asn Leu Ser Leu Asp
65                  70                  75                  80

Pro Ser Ala Cys Val Phe His Tyr Ala Phe Glu Leu Phe Glu Gly Leu
                85                  90                  95

Lys Ala Tyr Arg Thr Pro Gln Asn Thr Ile Thr Met Phe Arg Pro Asp
            100                 105                 110

Lys Asn Met Ala Arg Met Asn Lys Ser Ala Ala Arg Ile Cys Leu Pro
        115                 120                 125

Thr Phe Glu Ser Glu Glu Leu Ile Lys Leu Thr Gly Lys Leu Ile Glu
    130                 135                 140

Gln Asp Lys His Leu Val Pro Gln Gly Asn Gly Tyr Ser Leu Tyr Ile
145                 150                 155                 160

Arg Pro Thr Met Ile Gly Thr Ser Lys Gly Leu Gly Val Gly Thr Pro
                165                 170                 175

Ser Glu Ala Leu Leu Tyr Val Ile Thr Ser Pro Val Gly Pro Tyr Tyr
            180                 185                 190

Lys Thr Gly Phe Lys Ala Val Arg Leu Glu Ala Thr Asp Tyr Ala Thr
        195                 200                 205

Arg Ala Trp Pro Gly Gly Val Gly Asp Lys Lys Leu Gly Ala Asn Tyr
    210                 215                 220

Ala Pro Cys Ile Leu Pro Gln Leu Gln Ala Ala Lys Arg Gly Tyr Gln
225                 230                 235                 240

Gln Asn Leu Trp Leu Phe Gly Pro Glu Lys Asn Ile Thr Glu Val Gly
                245                 250                 255

Thr Met Asn Val Phe Phe Val Phe Leu Asn Lys Val Thr Gly Lys Lys
            260                 265                 270

Glu Leu Val Thr Ala Pro Leu Asp Gly Thr Ile Leu Glu Gly Val Thr
        275                 280                 285

Arg Asp Ser Val Leu Thr Leu Ala Arg Asp Lys Leu Asp Pro Gln Glu
    290                 295                 300

Trp Asp Ile Asn Glu Arg Tyr Tyr Thr Ile Thr Glu Val Ala Thr Arg
305                 310                 315                 320

Ala Lys Gln Gly Glu Leu Leu Glu Ala Phe Gly Ser Gly Thr Ala Ala
                325                 330                 335

Val Val Ser Pro Ile Lys Glu Ile Gly Trp Asn Asn Glu Asp Ile His
            340                 345                 350

Val Pro Leu Leu Pro Gly Glu Gln Cys Gly Ala Leu Thr Lys Gln Val
        355                 360                 365

Ala Gln Trp Ile Ala Asp Ile Gln Tyr Gly Arg Val Asn Tyr Gly Asn
    370                 375                 380

Trp Ser Lys Thr Val Ala Asp Leu Asn
385                 390

<210>  7
<211>  1020
<212>  DNA
<213>  Pseudomonas fluorescens

<400>  7

atgggtaacg aaagcatcaa ttgggacaag ctgggttttg actacatcaa gaccgacaag 60

cggtttctcc aggtctggaa aaacggcgaa tggcaagctg gcaccctgac cgacgacaac 120

gtgctgcaca tcagcgaggg ctccaccgcc ctgcactatg gccagcaatg ctttgaaggc 180

ctcaaggcct accgctgcaa ggacggctcg atcaacctgt tccgcccgga ccagaacgcc 240

gcccgcatgc agcgcagctg cgcgcgcctg ctgatgccgc atgtgtcgac cgaagacttc 300

atcgacgcct gcaaacaagt ggtcaaggcc aacgagcgct tcatcccgcc gtatggcagt 360

ggcggcgcgc tgtacctgcg cccgttcgtg atcggcaccg gtgacaacat cggtgtgcgt 420

accgcgccgg agttcatctt ctcggtgttc gccatcccgg ttggcgccta cttcaaaggc 480

ggcctggtac cacacaactt ccagatctcc accttcgacc gcgccgcacc ccagggcacc 540

ggtgccgcca aggtcggtgg caactacgcc gccagcctga tgccgggcgc cgaagcgaag 600

aaatccggtt tcgccgatgc gatctacctg gacccgatga ctcactcgaa aatcgaagaa 660

gtcggctcgg ccaacttctt cgggatcacc cacgacaata agttcatcac accgaagtcg 720

ccttcggtgc tgccaggcat cacccgtctg tcgctgatcg aactggccaa gacccgcctg 780

ggcctggaag tggtcgaggg cgaagtgttc atcgacaaac tggaccagtt caaggaagcc 840

ggtgcctgcg gtaccgccgc ggtgatctcg ccgatcggcg gtatccagta caacggcaag 900

ctgcacgtgt tccacagcga gaccgaagtc ggcccgatca cccagaagct ctacaaagag 960

ctgaccggcg tgcagaccgg cgacgttgaa gcgccagcgg gctggatcgt caaggtctaa 1020

<210> 8
<211> 339
<212> PRT
<213> Pseudomonas fluorescens

<400> 8

Met Gly Asn Glu Ser Ile Asn Trp Asp Lys Leu Gly Phe Asp Tyr Ile
1               5                   10                  15

Lys Thr Asp Lys Arg Phe Leu Gln Val Trp Lys Asn Gly Glu Trp Gln
            20                  25                  30

Ala Gly Thr Leu Thr Asp Asp Asn Val Leu His Ile Ser Glu Gly Ser
        35                  40                  45

Thr Ala Leu His Tyr Gly Gln Gln Cys Phe Glu Gly Leu Lys Ala Tyr
    50                  55                  60

Arg Cys Lys Asp Gly Ser Ile Asn Leu Phe Arg Pro Asp Gln Asn Ala
65                  70                  75                  80

Ala Arg Met Gln Arg Ser Cys Ala Arg Leu Leu Met Pro His Val Ser

85                  90                  95

Thr Glu Asp Phe Ile Asp Ala Cys Lys Gln Val Val Lys Ala Asn Glu
            100                 105                 110

Arg Phe Ile Pro Pro Tyr Gly Ser Gly Gly Ala Leu Tyr Leu Arg Pro
        115                 120                 125

Phe Val Ile Gly Thr Gly Asp Asn Ile Gly Val Arg Thr Ala Pro Glu
    130                 135                 140

Phe Ile Phe Ser Val Phe Ala Ile Pro Val Gly Ala Tyr Phe Lys Gly
145                 150                 155                 160

Gly Leu Val Pro His Asn Phe Gln Ile Ser Thr Phe Asp Arg Ala Ala
                165                 170                 175

Pro Gln Gly Thr Gly Ala Ala Lys Val Gly Gly Asn Tyr Ala Ala Ser
            180                 185                 190

Leu Met Pro Gly Ala Glu Ala Lys Lys Ser Gly Phe Ala Asp Ala Ile
        195                 200                 205

Tyr Leu Asp Pro Met Thr His Ser Lys Ile Glu Glu Val Gly Ser Ala
    210                 215                 220

Asn Phe Phe Gly Ile Thr His Asp Asn Lys Phe Ile Thr Pro Lys Ser
225                 230                 235                 240

Pro Ser Val Leu Pro Gly Ile Thr Arg Leu Ser Leu Ile Glu Leu Ala
                245                 250                 255

Lys Thr Arg Leu Gly Leu Glu Val Val Glu Gly Glu Val Phe Ile Asp
            260                 265                 270

Lys Leu Asp Gln Phe Lys Glu Ala Gly Ala Cys Gly Thr Ala Ala Val
        275                 280                 285

Ile Ser Pro Ile Gly Gly Ile Gln Tyr Asn Gly Lys Leu His Val Phe
    290                 295                 300

His Ser Glu Thr Glu Val Gly Pro Ile Thr Gln Lys Leu Tyr Lys Glu
305                 310                 315                 320

Leu Thr Gly Val Gln Thr Gly Asp Val Glu Ala Pro Ala Gly Trp Ile
                325                 330                 335

Val Lys Val

<210> 9
<211> 1218
<212> DNA
<213> Escherichia coli

<400> 9
atgtccccca ttgaaaaatc cagcaaatta gagaatgtct gttatgacat ccgtggtccg 60

gtgctgaaag aagcaaaacg cctggaagaa gaaggtaaca aggtactgaa actgaacatc 120

ggcaacccag ccccgttcgg ttttgacgcg ccagatgaaa tcctcgttga cgtgatacgc 180

aacctgccta ccgctcaagg gtattgcgat tccaaaggtc tttactccgc gcgtaaagcc 240

atcatgcagc actaccaggc tcgtggcatg cgtgatgtta ccgtggaaga tatttacatc 300

ggcaatggtg tatcggagct tatcgttcag gcaatgcagg cattgctgaa cagcggggac 360

gaaatgttgg ttcctgcacc agattaccca ctctggaccg cggcggtttc gctttccagc 420

ggtaaagcgg tgcattatct ttgcgatgaa tcctctgact ggttcccgga cctcgatgat 480

attcgcgcta aaattacgcc tcgtacgcgt gggatcgtta ttatcaaccc aaataaccca 540

accggcgcgg tatattccaa agagctttta atggagattg tggagattgc acgtcagcat 600

aatctcatta tcttcgccga tgaaatttat gacaaaattc tctacgacga cgctgagcat 660

cactcaattg cgccgctggc acctgacctg ctgaccatta cctttaacgg actgtcgaaa 720

acgtaccgcg ttgcaggctt ccgtcagggg tggatggtgt tgaacgggcc gaaaaaacac 780

gccaaaggct acatcgaagg tctggaaatg ctggcttcaa tgcgcctgtg tgctaacgtt 840

cctgcgcaac acgccattca gaccgcgcta ggtggttatc agagcatcag tgaatttatt 900

acccctggcg gtcgtcttta tgagcagcgt aaccgcgcgt gggaactgat caacgatatt 960

ccgggcgttt cctgcgtgaa acctcgtggt gcgctgtata tgttcccgaa aatcgacgcc 1020

aaacgcttta acattcacga cgatcagaaa atggtgttgg atttcctgtt gcaggaaaaa 1080

gttctgttgg tgcaagggac ggcattcaac tggccgtggc cggatcactt ccgcattgtc 1140

acgctaccgc gtgtcgatga tatcgagctg tctttgagca agttcgcgcg tttcctttct 1200

ggttatcatc agctgtaa 1218

<210> 10
<211> 405
<212> PRT
<213> Escherichia coli

<400> 10

Met Ser Pro Ile Glu Lys Ser Ser Lys Leu Glu Asn Val Cys Tyr Asp
1               5                   10                  15

Ile Arg Gly Pro Val Leu Lys Glu Ala Lys Arg Leu Glu Glu Glu Gly
        20                  25                  30

Asn Lys Val Leu Lys Leu Asn Ile Gly Asn Pro Ala Pro Phe Gly Phe
    35                  40                  45

Asp Ala Pro Asp Glu Ile Leu Val Asp Val Ile Arg Asn Leu Pro Thr
    50                  55                  60

Ala Gln Gly Tyr Cys Asp Ser Lys Gly Leu Tyr Ser Ala Arg Lys Ala
65                  70                  75                  80

Ile Met Gln His Tyr Gln Ala Arg Gly Met Arg Asp Val Thr Val Glu
                85                  90                  95

Asp Ile Tyr Ile Gly Asn Gly Val Ser Glu Leu Ile Val Gln Ala Met
        100                 105                 110

Gln Ala Leu Leu Asn Ser Gly Asp Glu Met Leu Val Pro Ala Pro Asp
    115                 120                 125

Tyr Pro Leu Trp Thr Ala Ala Val Ser Leu Ser Ser Gly Lys Ala Val
    130                 135                 140

His Tyr Leu Cys Asp Glu Ser Ser Asp Trp Phe Pro Asp Leu Asp Asp
145                 150                 155                 160

Ile Arg Ala Lys Ile Thr Pro Arg Thr Arg Gly Ile Val Ile Ile Asn
                165                 170                 175

Pro Asn Asn Pro Thr Gly Ala Val Tyr Ser Lys Glu Leu Leu Met Glu
        180                 185                 190

Ile Val Glu Ile Ala Arg Gln His Asn Leu Ile Ile Phe Ala Asp Glu
    195                 200                 205

Ile Tyr Asp Lys Ile Leu Tyr Asp Asp Ala Glu His His Ser Ile Ala
    210                 215                 220

Pro Leu Ala Pro Asp Leu Leu Thr Ile Thr Phe Asn Gly Leu Ser Lys
225                 230                 235                 240

Thr Tyr Arg Val Ala Gly Phe Arg Gln Gly Trp Met Val Leu Asn Gly
                245                 250                 255

Pro Lys Lys His Ala Lys Gly Tyr Ile Glu Gly Leu Glu Met Leu Ala
        260                 265                 270

Ser Met Arg Leu Cys Ala Asn Val Pro Ala Gln His Ala Ile Gln Thr
        275                 280                 285

Ala Leu Gly Gly Tyr Gln Ser Ile Ser Glu Phe Ile Thr Pro Gly Gly
    290                 295                 300

Arg Leu Tyr Glu Gln Arg Asn Arg Ala Trp Glu Leu Ile Asn Asp Ile
305                 310                 315                 320

Pro Gly Val Ser Cys Val Lys Pro Arg Gly Ala Leu Tyr Met Phe Pro
                325                 330                 335

Lys Ile Asp Ala Lys Arg Phe Asn Ile His Asp Asp Gln Lys Met Val
            340                 345                 350

Leu Asp Phe Leu Leu Gln Glu Lys Val Leu Leu Val Gln Gly Thr Ala
        355                 360                 365

Phe Asn Trp Pro Trp Pro Asp His Phe Arg Ile Val Thr Leu Pro Arg
    370                 375                 380

Val Asp Asp Ile Glu Leu Ser Leu Ser Lys Phe Ala Arg Phe Leu Ser
385                 390                 395                 400

Gly Tyr His Gln Leu
                405

<210>  11
<211>  1191
<212>  DNA
<213>  Escherichia coli

<400>  11
   atgtttgaga acattaccgc cgctcctgcc gacccgattc tgggcctggc cgatctgttt      60
   cgtgccgatg aacgtcccgg caaaattaac ctcgggattg gtgtctataa agatgagacg     120
   ggcaaaaccc cggtactgac cagcgtgaaa aaggctgaac agtatctgct cgaaaatgaa     180
   accaccaaaa attacctcgg cattgacggc atccctgaat ttggtcgctg cactcaggaa     240
   ctgctgtttg gtaaaggtag cgccctgatc aatgacaaac gtgctcgcac ggcacagact     300
   ccggggggca ctggcgcact acgcgtggct gccgatttcc tggcaaaaaa taccagcgtt     360
   aagcgtgtgt gggtgagcaa cccaagctgg ccgaaccata agagcgtctt taactctgca     420
   ggtctggaag ttcgtgaata cgcttattat gatgcggaaa atcacactct tgacttcgat     480
   gcactgatta acagcctgaa tgaagctcag gctggcgacg tagtgctgtt ccatggctgc     540
   tgccataacc caaccggtat cgaccctacg ctggaacaat ggcaaacact ggcacaactc     600
   tccgttgaga aaggctggtt accgctgttt gacttcgctt accagggttt tgcccgtggt 660
   ctggaagaag atgctgaagg actgcgcgct ttcgcggcta tgcataaaga gctgattgtt 720
   gccagttcct actctaaaaa ctttggcctg tacaacgagc gtgttggcgc ttgtactctg 780
   gttgctgccg acagtgaaac cgttgatcgc gcattcagcc aaatgaaagc ggcgattcgc 840
   gctaactact ctaacccacc agcacacggc gcttctgttg ttgccaccat cctgagcaac 900
   gatgcgttac gtgcgatttg ggaacaagag ctgactgata tgcgccagcg tattcagcgt 960
   atgcgtcagt tgttcgtcaa tacgctgcag gaaaaaggcg caaaccgcga cttcagcttt 1020
   atcatcaaac agaacggcat gttctccttc agtggcctga caaaagaaca agtgctgcgt 1080
   ctgcgcgaag agtttggcgt atatgcggtt gcttctggtc gcgtaaatgt ggccgggatg 1140
   acaccagata acatggctcc gctgtgcgaa gcgattgtgg cagtgctgta a 1191

<210> 12
<211> 396
<212> PRT
<213> Escherichia coli

<400> 12

Met Phe Glu Asn Ile Thr Ala Ala Pro Ala Asp Pro Ile Leu Gly Leu
1               5                   10                  15

Ala Asp Leu Phe Arg Ala Asp Glu Arg Pro Gly Lys Ile Asn Leu Gly
            20                  25                  30

Ile Gly Val Tyr Lys Asp Glu Thr Gly Lys Thr Pro Val Leu Thr Ser
        35                  40                  45

Val Lys Lys Ala Glu Gln Tyr Leu Leu Glu Asn Glu Thr Thr Lys Asn
    50                  55                  60

Tyr Leu Gly Ile Asp Gly Ile Pro Glu Phe Gly Arg Cys Thr Gln Glu
65                  70                  75                  80

Leu Leu Phe Gly Lys Gly Ser Ala Leu Ile Asn Asp Lys Arg Ala Arg
                85                  90                  95

Thr Ala Gln Thr Pro Gly Gly Thr Gly Ala Leu Arg Val Ala Ala Asp
            100                 105                 110

Phe Leu Ala Lys Asn Thr Ser Val Lys Arg Val Trp Val Ser Asn Pro
        115                 120                 125

Ser Trp Pro Asn His Lys Ser Val Phe Asn Ser Ala Gly Leu Glu Val
    130                 135                 140

Arg Glu Tyr Ala Tyr Tyr Asp Ala Glu Asn His Thr Leu Asp Phe Asp
145                 150                 155                 160

Ala Leu Ile Asn Ser Leu Asn Glu Ala Gln Ala Gly Asp Val Val Leu
                165                 170                 175

Phe His Gly Cys Cys His Asn Pro Thr Gly Ile Asp Pro Thr Leu Glu
            180                 185                 190

Gln Trp Gln Thr Leu Ala Gln Leu Ser Val Glu Lys Gly Trp Leu Pro
        195                 200                 205

Leu Phe Asp Phe Ala Tyr Gln Gly Phe Ala Arg Gly Leu Glu Glu Asp
    210                 215                 220

Ala Glu Gly Leu Arg Ala Phe Ala Ala Met His Lys Glu Leu Ile Val
225                 230                 235                 240

Ala Ser Ser Tyr Ser Lys Asn Phe Gly Leu Tyr Asn Glu Arg Val Gly
                245                 250                 255

Ala Cys Thr Leu Val Ala Ala Asp Ser Glu Thr Val Asp Arg Ala Phe
            260                 265                 270

Ser Gln Met Lys Ala Ala Ile Arg Ala Asn Tyr Ser Asn Pro Pro Ala
        275                 280                 285

His Gly Ala Ser Val Val Ala Thr Ile Leu Ser Asn Asp Ala Leu Arg
    290                 295                 300

Ala Ile Trp Glu Gln Glu Leu Thr Asp Met Arg Gln Arg Ile Gln Arg
305                 310                 315                 320

Met Arg Gln Leu Phe Val Asn Thr Leu Gln Glu Lys Gly Ala Asn Arg
                325                 330                 335

Asp Phe Ser Phe Ile Ile Lys Gln Asn Gly Met Phe Ser Phe Ser Gly
            340                 345                 350

Leu Thr Lys Glu Gln Val Leu Arg Leu Arg Glu Glu Phe Gly Val Tyr
        355                 360                 365

Ala Val Ala Ser Gly Arg Val Asn Val Ala Gly Met Thr Pro Asp Asn
    370                 375                 380

Met Ala Pro Leu Cys Glu Ala Ile Val Ala Val Leu

385                 390                 395

<210>  13
<211>  2533
<212>  DNA
<213>  Calloselasma rhodostoma

<400>  13
ggcacgagct ttgcttagca tcagtaactt ttcttccaag cattgccatc cacagacttc      60

aaaccaataa gatgaatgtc ttctttatgt tctcgctgct gttcttggct gccttgggaa     120

gctgtgcaga tgacagaaac cctctagcgg aatgcttcca agaaaatgac tatgaagaat     180

ttctagagat cgccagaaat ggtctgaaag cgacatcaaa cccaaaacat gttgtgattg     240

taggtgcagg aatggctggg cttagtgcag cctatgttct tgcaggggct ggacatcagg     300

tgacagttct tgaagccagt gaacgtccgg gaggacgagt gagaacttat cgaaatgagg     360

aagcaggctg gtatgccaat ctcgggccca tgcgtttacc tgagaaacac aggattgtcc     420

gggaatatat cagaaagttt gatctccggt tgaatgaatt ttctcaggaa aatgacaatg     480

cctggtattt tatcaaaaac atcaggaaga aagttgggga agtcaagaaa gaccctggcc     540

tcttgaaata tcccgtgaag ccttcagaag caggcaaaag tgctggacag ctatatgaag     600

agtccctcgg aaaggttgta gaagaattaa aaaggactaa ctgcagctac atactaaata     660

aatatgacac ctactcaacg aaggagtatc taattaaaga aggagatctg agtcctggag     720

ctgtagatat gattggagac ctactgaatg aagattctgg ctattatgtg tcttttattg     780

agagcctgaa acatgatgat atcttcgctt atgaaaaaag atttgatgaa attgttgatg     840

gaatggataa gttgcctaca gccatgtatc gagacattca ggataaggtg catttcaatg     900

cccaagtaat caagatacag caaaatgacc agaaagtcac agtggtatat gaaaccttat     960

caaaggagac gccatctgtg acagctgatt atgtcattgt gtgcactacg tcaagggccg    1020

tccgtctcat caaatttaat ccaccccttt tgccaaagaa agcgcatgct ttgcggtctg    1080

tccactatag aagtggcacc aagatcttcc tcacttgcac tacgaaattt tgggaggatg    1140

atggcattca tggtgggaag tccacaactg atcttccatc ccgattcatc tactacccta    1200

accataactt tactaatgga gttggggtta ttatagccta tggcattggt gatgatgcca    1260

atttctttca agctcttgat ttcaaggact gtgctgatat tgtctttaat gacctttcat    1320

tgatccatca gctgccgaag aaagatatcc agtccttctg ttatccctca gtgattcaaa    1380

aatggagcct ggataagtat gctatgggtg gtataaccac cttcactccc taccagtttc    1440

aacattttag tgacccactc actgcatctc aaggcagaat ctactttgca ggggagtata    1500

cggcccaagc tcatggttgg attgacagca caattaagtc agggctgaga gcagcaagag    1560

atgtgaatct tgcttctgag aatccatcag gaatccacct gagcaatgac aatgaacttt    1620

aagaaggagg tcagcaatgt ttcagactaa aattcccaga ataccagttg gtaattctaa 1680

gagctatagt cccaggaggt tggagccaga aggaaggggg gaagtaagct gatttggcct 1740

ggctaaatta tcaaaagtga tctatcagcc aaaacatgtc aaaatatatc agagcatttt 1800

agaggaaaca aaaataaacc acagaatgaa aaggattctt agcatgccac cccaatttgg 1860

aaaggaagtc ctctcaagac aatctatata tactactaaa actctctttt ctgtaacatt 1920

ttactggaca aaacagtgca ccatagggca agaatgtttg aaccaaaata cctgagctga 1980

tgttttattg tgctgtacag ttcgccatgg gctattttca aggcagatac atctgtgaat 2040

gtatcccaac ttttcagtca agatagcaca ttatttttgc tctgccgttg tttaaatgat 2100

tgaggaatct ggtaaagaat tatctctgaa gggatgacca gaaagtcatg gattgtctag 2160

tgctgcaata aagataggat atttctgttg cccattccaa atagtaatgg aaagtaatgg 2220

aaaagaaatg tgcaattgtt gttaaactga ctttgtgagt ttattttaat gtatgactat 2280

atttctttgg gacatcaagc atctagtggt cctaagctat gattctatat ttcctacaac 2340

catcaaaaat tgtcagtgct acagtgtcac tgatggagaa gaaaacacat tatggcatca 2400

agcaggcctg tagacaacct taatttgtaa tttctccagt ttgccaccct cctctgacta 2460

gtgttctgaa aaactgggga tacagataat tataataaaa gcacatatta tcaaaaaaaa 2520

aaaaaaaaaa aaa 2533

<210> 14
<211> 516
<212> PRT
<213> Calloselasma rhodostoma

<400> 14

Met Asn Val Phe Phe Met Phe Ser Leu Leu Phe Leu Ala Ala Leu Gly
1               5                   10                  15

Ser Cys Ala Asp Asp Arg Asn Pro Leu Ala Glu Cys Phe Gln Glu Asn
            20                  25                  30

Asp Tyr Glu Glu Phe Leu Glu Ile Ala Arg Asn Gly Leu Lys Ala Thr
        35                  40                  45

Ser Asn Pro Lys His Val Val Ile Val Gly Ala Gly Met Ala Gly Leu
    50                  55                  60

Ser Ala Ala Tyr Val Leu Ala Gly Ala Gly His Gln Val Thr Val Leu
65                  70                  75                  80

Glu Ala Ser Glu Arg Pro Gly Gly Arg Val Arg Thr Tyr Arg Asn Glu
                85                  90                  95

Glu Ala Gly Trp Tyr Ala Asn Leu Gly Pro Met Arg Leu Pro Glu Lys
            100                 105                 110

His Arg Ile Val Arg Glu Tyr Ile Arg Lys Phe Asp Leu Arg Leu Asn
        115                 120                 125

Glu Phe Ser Gln Glu Asn Asp Asn Ala Trp Tyr Phe Ile Lys Asn Ile
    130                 135                 140

Arg Lys Lys Val Gly Glu Val Lys Lys Asp Pro Gly Leu Leu Lys Tyr
145                 150                 155                 160

Pro Val Lys Pro Ser Glu Ala Gly Lys Ser Ala Gly Gln Leu Tyr Glu
                165                 170                 175

Glu Ser Leu Gly Lys Val Val Glu Glu Leu Lys Arg Thr Asn Cys Ser
            180                 185                 190

Tyr Ile Leu Asn Lys Tyr Asp Thr Tyr Ser Thr Lys Glu Tyr Leu Ile
        195                 200                 205

Lys Glu Gly Asp Leu Ser Pro Gly Ala Val Asp Met Ile Gly Asp Leu
    210                 215                 220

Leu Asn Glu Asp Ser Gly Tyr Tyr Val Ser Phe Ile Glu Ser Leu Lys
225                 230                 235                 240

His Asp Asp Ile Phe Ala Tyr Glu Lys Arg Phe Asp Glu Ile Val Asp
                245                 250                 255

Gly Met Asp Lys Leu Pro Thr Ala Met Tyr Arg Asp Ile Gln Asp Lys
            260                 265                 270

Val His Phe Asn Ala Gln Val Ile Lys Ile Gln Gln Asn Asp Gln Lys
        275                 280                 285

Val Thr Val Val Tyr Glu Thr Leu Ser Lys Glu Thr Pro Ser Val Thr
    290                 295                 300

Ala Asp Tyr Val Ile Val Cys Thr Thr Ser Arg Ala Val Arg Leu Ile
305                 310                 315                 320

Lys Phe Asn Pro Pro Leu Leu Pro Lys Lys Ala His Ala Leu Arg Ser
                325                 330                 335

Val His Tyr Arg Ser Gly Thr Lys Ile Phe Leu Thr Cys Thr Thr Lys
            340                 345                 350

Phe Trp Glu Asp Asp Gly Ile His Gly Gly Lys Ser Thr Thr Asp Leu
        355                 360                 365

Pro Ser Arg Phe Ile Tyr Tyr Pro Asn His Asn Phe Thr Asn Gly Val
    370                 375                 380

Gly Val Ile Ile Ala Tyr Gly Ile Gly Asp Asp Ala Asn Phe Phe Gln
385                 390                 395                 400

Ala Leu Asp Phe Lys Asp Cys Ala Asp Ile Val Phe Asn Asp Leu Ser
                405                 410                 415

Leu Ile His Gln Leu Pro Lys Lys Asp Ile Gln Ser Phe Cys Tyr Pro
            420                 425                 430

Ser Val Ile Gln Lys Trp Ser Leu Asp Lys Tyr Ala Met Gly Gly Ile
        435                 440                 445

Thr Thr Phe Thr Pro Tyr Gln Phe Gln His Phe Ser Asp Pro Leu Thr
    450                 455                 460

Ala Ser Gln Gly Arg Ile Tyr Phe Ala Gly Glu Tyr Thr Ala Gln Ala
465                 470                 475                 480

His Gly Trp Ile Asp Ser Thr Ile Lys Ser Gly Leu Arg Ala Ala Arg
                485                 490                 495

Asp Val Asn Leu Ala Ser Glu Asn Pro Ser Gly Ile His Leu Ser Asn
            500                 505                 510

Asp Asn Glu Leu
        515

<210> 15
<211> 1605
<212> DNA
<213> Rhodococcus opacus

<400> 15
ttggcattca cacgtagatc tttcatgaag ggcctcgggg ccaccggcgg cgcaggcctc 60

gcgtacggcg cgatgtcgac gctcgggctc gcaccgtcga ccgctgcgcc cgcccgcacc 120

ttccagccgc tcgcggccgg cgacctgatc ggcaaggtga agggcagcca ttccgtggtc 180

gtgctcggcg gcggccccgc cggtctgtgt tcggcattcg aactgcagaa ggccgggtac 240

aaggtgacgg tcctcgaggc ccgcacccgg cccggtggcc gcgtctggac cgcacggggc 300

ggcagcgagg agaccgacct gagcggcgag acgcagaagt gcacgttctc ggagggccac 360

ttctacaacg tcggcgccac ccgcatcccg cagagccaca tcacgctcga ctactgccgc 420

gaactcggcg tcgagatcca gggattcgga aaccagaacg ccaacacgtt cgtgaactac 480

cagagcgaca cgtcgctgtc tggccagtcc gtcacctacc gggccgcgaa ggccgacacg 540

ttcggctaca tgtcggaact gctgaagaag gccaccgatc agggtgccct ggatcaggta 600

ctgagccggg aggacaagga tgcgctgtcg gagttcctca gcgacttcgg tgacctgtcc 660

gacgacggcc gctacctcgg atcctcgcgt cgcggttacg attccgagcc cggagccggc 720

ctgaacttcg gcaccgagaa gaagccgttc gcgatgcagg aagtgatccg cagcggcatc 780

ggccgcaact tcagcttcga cttcggctac gaccaggcga tgatgatgtt caccccggtc 840

ggcggcatgg accggatcta ctacgcgttc caggacagga tcggcaccga caacatcgtc 900

ttcggcgccg aggtgacgtc gatgaagaac gtgtccgagg gcgtcaccgt cgaatacacc 960

gccggcggct cgaagaagtc gatcaccgcc gactacgcga tctgcacgat cccgccgcac 1020

ctcgtcggac gactgcagaa caatctgccc ggcgacgtgc tcaccgcgct gaaggcggcc 1080

aagccgtcgt cgtccggaaa gctcggcatc gagtactcgc gccggtggtg ggagacggag 1140

gaccgcatct acggcggcgc gtccaacacc gacaaggaca tctcgcagat catgttcccg 1200

tacgaccact acaactccga tcgcggtgtg gtcgtcgcct actacagcag cggcaagcgt 1260

caggaggcgt tcgagtccct cacgcaccgc cagcggctcg ccaaggcgat cgcggagggc 1320

tcggagatcc acggcgagaa gtacacccgc gacatctcgt cgtcgttctc gggcagctgg 1380

cggcgcacca agtactccga gagtgcctgg gccaactggg cgggcagtgg cggatcgcac 1440

ggcggggcgg ccactcccga gtacgagaag ctgctcgaac ccgtcgacaa gatctatttc 1500

gccggcgacc acctgtccaa cgccatcgcc tggcagcacg gcgccctgac gtccgcccgc 1560

gacgtcgtca cccacatcca cgagcgcgtg gcccaggaag cctga 1605

<210> 16
<211> 534
<212> PRT
<213> Rhodococcus opacus

<400> 16

Leu Ala Phe Thr Arg Arg Ser Phe Met Lys Gly Leu Gly Ala Thr Gly
1               5                   10                  15

Gly Ala Gly Leu Ala Tyr Gly Ala Met Ser Thr Leu Gly Leu Ala Pro
            20                  25                  30

Ser Thr Ala Ala Pro Ala Arg Thr Phe Gln Pro Leu Ala Ala Gly Asp
        35                  40                  45

Leu Ile Gly Lys Val Lys Gly Ser His Ser Val Val Val Leu Gly Gly
    50                  55                  60

Gly Pro Ala Gly Leu Cys Ser Ala Phe Glu Leu Gln Lys Ala Gly Tyr
65                  70                  75                  80

Lys Val Thr Val Leu Glu Ala Arg Thr Arg Pro Gly Gly Arg Val Trp
                85                  90                  95

Thr Ala Arg Gly Gly Ser Glu Glu Thr Asp Leu Ser Gly Glu Thr Gln
            100                 105                 110

Lys Cys Thr Phe Ser Glu Gly His Phe Tyr Asn Val Gly Ala Thr Arg
        115                 120                 125

Ile Pro Gln Ser His Ile Thr Leu Asp Tyr Cys Arg Glu Leu Gly Val
    130                 135                 140

Glu Ile Gln Gly Phe Gly Asn Gln Asn Ala Asn Thr Phe Val Asn Tyr
145                 150                 155                 160

Gln Ser Asp Thr Ser Leu Ser Gly Gln Ser Val Thr Tyr Arg Ala Ala
                165                 170                 175

Lys Ala Asp Thr Phe Gly Tyr Met Ser Glu Leu Leu Lys Lys Ala Thr
            180                 185                 190

Asp Gln Gly Ala Leu Asp Gln Val Leu Ser Arg Glu Asp Lys Asp Ala
        195                 200                 205

Leu Ser Glu Phe Leu Ser Asp Phe Gly Asp Leu Ser Asp Asp Gly Arg
    210                 215                 220

Tyr Leu Gly Ser Ser Arg Arg Gly Tyr Asp Ser Glu Pro Gly Ala Gly
225                 230                 235                 240

Leu Asn Phe Gly Thr Glu Lys Lys Pro Phe Ala Met Gln Glu Val Ile
                245                 250                 255

Arg Ser Gly Ile Gly Arg Asn Phe Ser Phe Asp Phe Gly Tyr Asp Gln
            260                 265                 270

Ala Met Met Met Phe Thr Pro Val Gly Gly Met Asp Arg Ile Tyr Tyr
        275                 280                 285

Ala Phe Gln Asp Arg Ile Gly Thr Asp Asn Ile Val Phe Gly Ala Glu
    290                 295                 300

Val Thr Ser Met Lys Asn Val Ser Glu Gly Val Thr Val Glu Tyr Thr
305                 310                 315                 320

Ala Gly Gly Ser Lys Lys Ser Ile Thr Ala Asp Tyr Ala Ile Cys Thr
                325                 330                 335

Ile Pro Pro His Leu Val Gly Arg Leu Gln Asn Asn Leu Pro Gly Asp
            340                 345                 350

Val Leu Thr Ala Leu Lys Ala Ala Lys Pro Ser Ser Ser Gly Lys Leu
        355                 360                 365

Gly Ile Glu Tyr Ser Arg Arg Trp Trp Glu Thr Glu Asp Arg Ile Tyr
    370                 375                 380

Gly Gly Ala Ser Asn Thr Asp Lys Asp Ile Ser Gln Ile Met Phe Pro
385                 390                 395                 400

Tyr Asp His Tyr Asn Ser Asp Arg Gly Val Val Val Ala Tyr Tyr Ser
                405                 410                 415

Ser Gly Lys Arg Gln Glu Ala Phe Glu Ser Leu Thr His Arg Gln Arg
            420                 425                 430

Leu Ala Lys Ala Ile Ala Glu Gly Ser Glu Ile His Gly Glu Lys Tyr
        435                 440                 445

Thr Arg Asp Ile Ser Ser Ser Phe Ser Gly Ser Trp Arg Arg Thr Lys
    450                 455                 460

Tyr Ser Glu Ser Ala Trp Ala Asn Trp Ala Gly Ser Gly Gly Ser His
465                 470                 475                 480

Gly Gly Ala Ala Thr Pro Glu Tyr Glu Lys Leu Leu Glu Pro Val Asp
                485                 490                 495

Lys Ile Tyr Phe Ala Gly Asp His Leu Ser Asn Ala Ile Ala Trp Gln
            500                 505                 510

His Gly Ala Leu Thr Ser Ala Arg Asp Val Val Thr His Ile His Glu
        515                 520                 525

Arg Val Ala Gln Glu Ala
    530

<210>  17
<211>  1134

<212>  DNA
<213>  Rhizobium leguminosarum

<400>  17
atgagaggaa cgcgcatgcg tgtcggttgc ccgaaggaaa tcaagaatca tgaatatcgc      60

gtcggcctga cgccggcttc ggtgcgcgaa tatgttgccc acggccacga ggtctgggtg     120

gagaccaagg cgggcgtcgg tatcggcgct gatgatgccg cctatgccgc ggctggcgcc     180

aagatcgccg cctccgccaa ggatatcttc gaaaagtgcg acatgatcgt caaggtgaag     240

gagccgcagc ctgccgaatg ggcgcagctc cgcgacggtc agcttctcta cacctatctg     300

catctggcgc cggatcccga acagaccaaa ggcctcatcg cctccggcgt caccgcgatc     360

gcctatgaga cggtgaccga cgagcgcggc ggcctgccgt tgctggcgcc gatgtcggag     420

gtcgccggtc gcctgtcgat ccaggcagga gcgaccgccc tgcagaaggc caatggcggc     480

ctcggcgtcc tcctcggcgg cgtgcccggc gtgctgccgg ccaaggtcgc agtcatcggc     540

ggcggcgtcg tcggcctgca tgcggccagg atggccgccg gccttggcgc cgatgtcagc     600

atccttgaca agtcgctgcc gcgtctgcgc cagctcgacg atatctttgc aggccgcatc     660

cacacccgtt attccagcat ccaggcgctg gaggaggaag tcttctcggc cgatctcatt     720

atcggcgccg tgctgatccc gggcgctgcc gccccgaagc tcgtcacccg cgagatgctg     780

tccggcatga agaagggctc cgtcatcgtc gacgttgcca tcgaccaggg cggctgcttc     840

gagacctcgc atgcgacgac ccattccgat ccgacctatg aagtcgatgg cgtcgtgcat     900

tattgcgttg ccaacatgcc gggcgccgtg ccggtcacct cggcacacgc gctgaacaat     960

gccacattgg ttcatggcct ggcgcttgcc gatcgcggcc tgcgcgccat cgccgaagac    1020

aggcatctga ggaatggcct caacgtccac aagggccgca tcaccagcaa gccggtcgcc    1080

gaagcgctgg gctacgaggc cttcgcgccg gaaagcgtgc tgaacgtagc gtaa          1134

<210>  18
<211>  377
<212>  PRT
<213>  Rhizobium leguminosarum

<400>  18

Met Arg Gly Thr Arg Met Arg Val Gly Cys Pro Lys Glu Ile Lys Asn
1               5                   10                  15

His Glu Tyr Arg Val Gly Leu Thr Pro Ala Ser Val Arg Glu Tyr Val
            20                  25                  30

Ala His Gly His Glu Val Trp Val Glu Thr Lys Ala Gly Val Gly Ile
        35                  40                  45

Gly Ala Asp Asp Ala Ala Tyr Ala Ala Ala Gly Ala Lys Ile Ala Ala

50                  55                  60

Ser Ala Lys Asp Ile Phe Glu Lys Cys Asp Met Ile Val Lys Val Lys
65                  70                  75                  80

Glu Pro Gln Pro Ala Glu Trp Ala Gln Leu Arg Asp Gly Gln Leu Leu
                85                  90                  95

Tyr Thr Tyr Leu His Leu Ala Pro Asp Pro Glu Gln Thr Lys Gly Leu
            100                 105                 110

Ile Ala Ser Gly Val Thr Ala Ile Ala Tyr Glu Thr Val Thr Asp Glu
        115                 120                 125

Arg Gly Gly Leu Pro Leu Leu Ala Pro Met Ser Glu Val Ala Gly Arg
    130                 135                 140

Leu Ser Ile Gln Ala Gly Ala Thr Ala Leu Gln Lys Ala Asn Gly Gly
145                 150                 155                 160

Leu Gly Val Leu Leu Gly Gly Val Pro Gly Val Leu Pro Ala Lys Val
                165                 170                 175

Ala Val Ile Gly Gly Gly Val Val Gly Leu His Ala Ala Arg Met Ala
            180                 185                 190

Ala Gly Leu Gly Ala Asp Val Ser Ile Leu Asp Lys Ser Leu Pro Arg
        195                 200                 205

Leu Arg Gln Leu Asp Asp Ile Phe Ala Gly Arg Ile His Thr Arg Tyr
    210                 215                 220

Ser Ser Ile Gln Ala Leu Glu Glu Glu Val Phe Ser Ala Asp Leu Ile
225                 230                 235                 240

Ile Gly Ala Val Leu Ile Pro Gly Ala Ala Ala Pro Lys Leu Val Thr
                245                 250                 255

Arg Glu Met Leu Ser Gly Met Lys Lys Gly Ser Val Ile Val Asp Val
            260                 265                 270

Ala Ile Asp Gln Gly Gly Cys Phe Glu Thr Ser His Ala Thr Thr His
        275                 280                 285

Ser Asp Pro Thr Tyr Glu Val Asp Gly Val Val His Tyr Cys Val Ala
    290                 295                 300

Asn Met Pro Gly Ala Val Pro Val Thr Ser Ala His Ala Leu Asn Asn
305                 310                 315                 320

Ala Thr Leu Val His Gly Leu Ala Leu Ala Asp Arg Gly Leu Arg Ala
                325                 330                 335

Ile Ala Glu Asp Arg His Leu Arg Asn Gly Leu Asn Val His Lys Gly
            340                 345                 350

Arg Ile Thr Ser Lys Pro Val Ala Glu Ala Leu Gly Tyr Glu Ala Phe
        355                 360                 365

Ala Pro Glu Ser Val Leu Asn Val Ala
    370                 375

<210>  19
<211>  1116
<212>  DNA
<213>  Streptomyces coelicolor

<400>  19
gtgaaggtcg gcatcccccg cgaggtcaag aacaacgagt tccgggtggc catcaccccc      60

gccggcgtgc acgagctggt gcgccacggt caccaggtcg tcgtcgagcg caacgccggc     120

gtcggctcct cgatccccga cgaggagtac gtcacggccg gtgcgcggat cctcgacacc     180

gccgacgagg tctgggccac cgcggacctg ctcctgaagg tcaaggagcc gatcgcggag     240

gagtaccacc gcctgcgcaa ggaccagacg ctcttcacct acctgcacct ggccgcctcc     300

aaggagtgca cggacgcgct catcgagtcc cgcaccaccg ccatcgcgta cgagacggtc     360

gagctgccca gccgcgcgct gccgctgctg gccccgatgt ccgaggtcgc gggccgcctc     420

gccccccagg tcggcgccta ccacctgatg gccgccaacg gcgggcgcgg tgtgctgccc     480

ggcggtgtcc ccggtgtgct cgcgggccgc gccgtcgtca tcggcggcgg tgtctccggc     540

tggaacgcgg cgcagatcgc catcggcctg ggcttccacg tcaccctgct cgacaaggac     600

atcaccaagc tcagggaagc cgacaagatc ttcggcacga agatccagac cgtcgtctcc     660

aacgccttcg agctggagaa ggcctgcctg gaggccgacc tcgtgatcgg cgccgtgctc     720

atcccgggcg ccaaggcacc gaagctggtc accaacgagc tggtgtcccg gatgaagccc     780

ggaagtgtcc ttgtcgacat cgcgatcgac cagggcggct gcttcgagga ctcccacccg     840

accacccacg ccgagccgac cttcccggtc cacaactcgg tcttctactg cgtcgccaac     900

atgcccggcg cggtgcccaa cacctccacc tacgcgctga ccaacgccac gctgccgtac     960

atcgtggagc tggccgaccg cggctgggcc gaggcgctgc gccgcgaccc cgcgctggcc    1020

aagggtctca acacccatga cggcaaggtc gtttaccggg aggtcgccga ggcacacggc    1080

ctggagcacg tggagctcgc ctcgctgctc gcctaa                              1116

<210>  20
<211>  371
<212>  PRT
<213>  Streptomyces coelicolor

<400>  20

Val Lys Val Gly Ile Pro Arg Glu Val Lys Asn Asn Glu Phe Arg Val
1               5                   10                  15

Ala Ile Thr Pro Ala Gly Val His Glu Leu Val Arg His Gly His Gln
            20                  25                  30

Val Val Val Glu Arg Asn Ala Gly Val Gly Ser Ser Ile Pro Asp Glu
        35                  40                  45

Glu Tyr Val Thr Ala Gly Ala Arg Ile Leu Asp Thr Ala Asp Glu Val
    50                  55                  60

Trp Ala Thr Ala Asp Leu Leu Leu Lys Val Lys Glu Pro Ile Ala Glu
65                  70                  75                  80

Glu Tyr His Arg Leu Arg Lys Asp Gln Thr Leu Phe Thr Tyr Leu His
                85                  90                  95

Leu Ala Ala Ser Lys Glu Cys Thr Asp Ala Leu Ile Glu Ser Arg Thr
            100                 105                 110

Thr Ala Ile Ala Tyr Glu Thr Val Glu Leu Pro Ser Arg Ala Leu Pro
        115                 120                 125

Leu Leu Ala Pro Met Ser Glu Val Ala Gly Arg Leu Ala Pro Gln Val
    130                 135                 140

Gly Ala Tyr His Leu Met Ala Ala Asn Gly Gly Arg Gly Val Leu Pro
145                 150                 155                 160

Gly Gly Val Pro Gly Val Leu Ala Gly Arg Ala Val Val Ile Gly Gly
                165                 170                 175

Gly Val Ser Gly Trp Asn Ala Ala Gln Ile Ala Ile Gly Leu Gly Phe
            180                 185                 190

His Val Thr Leu Leu Asp Lys Asp Ile Thr Lys Leu Arg Glu Ala Asp
        195                 200                 205

Lys Ile Phe Gly Thr Lys Ile Gln Thr Val Val Ser Asn Ala Phe Glu
    210                 215                 220

Leu Glu Lys Ala Cys Leu Glu Ala Asp Leu Val Ile Gly Ala Val Leu
225                 230                 235                 240

Ile Pro Gly Ala Lys Ala Pro Lys Leu Val Thr Asn Glu Leu Val Ser
                245                 250                 255

Arg Met Lys Pro Gly Ser Val Leu Val Asp Ile Ala Ile Asp Gln Gly
            260                 265                 270

Gly Cys Phe Glu Asp Ser His Pro Thr Thr His Ala Glu Pro Thr Phe
        275                 280                 285

Pro Val His Asn Ser Val Phe Tyr Cys Val Ala Asn Met Pro Gly Ala
    290                 295                 300

Val Pro Asn Thr Ser Thr Tyr Ala Leu Thr Asn Ala Thr Leu Pro Tyr
305                 310                 315                 320

Ile Val Glu Leu Ala Asp Arg Gly Trp Ala Glu Ala Leu Arg Arg Asp
                325                 330                 335

Pro Ala Leu Ala Lys Gly Leu Asn Thr His Asp Gly Lys Val Val Tyr
            340                 345                 350

Arg Glu Val Ala Glu Ala His Gly Leu Glu His Val Glu Leu Ala Ser
        355                 360                 365

Leu Leu Ala
    370

<210>  21
<211>  993
<212>  DNA
<213>  Bacillus subtilis

<400>  21
atgagtacaa accgacatca agcactaggg ctgactgatc aggaagccgt tgatatgtat      60

agaaccatgc tgttagcaag aaaaatcgat gaaagaatgt ggctgttaaa ccgttctggc     120

aaaattccat ttgtaatctc ttgtcaagga caggaagcag cacaggtagg agcggctttc     180

gcacttgacc gtgaaatgga ttatgtattg ccgtactaca gagacatggg tgtcgtgctc     240

gcgtttggca tgacagcaaa ggacttaatg atgtccgggt ttgcaaaagc agcagatccg     300

aactcaggag gccgccagat gccgggacat ttcggacaaa agaaaaaccg cattgtgacg     360

ggatcatctc cggttacaac gcaagtgccg cacgcagtcg gtattgcgct tgcgggacgt     420

atggagaaaa aggatatcgc agcctttgtt acattcgggg aagggtcttc aaaccaaggc     480

gatttccatg aaggggcaaa ctttgccgct gtccataagc tgccggttat tttcatgtgt 540

gaaaacaaca aatacgcaat ctcagtgcct tacgataagc aagtcgcatg tgagaacatt 600

tccgaccgtg ccataggcta tgggatgcct ggcgtaactg tgaatggaaa tgatccgctg 660

gaagtttatc aagcggttaa agaagcacgc gaaagggcac gcagaggaga aggcccgaca 720

ttaattgaaa cgatttctta ccgccttaca ccacattcca gtgatgacga tgacagcagc 780

tacagaggcc gtgaagaagt agaggaagcg aaaaaaagtg atcccctgct tacttatcaa 840

gcttacttaa aggaaacagg cctgctgtcc gatgagatag aacaaaccat gctggatgaa 900

attatggcaa tcgtaaatga agcgacggat gaagcggaga acgccccata tgcagctcct 960

gagtcagcgc ttgattatgt ttatgcgaag tag 993

<210> 22
<211> 330
<212> PRT
<213> Bacillus subtilis

<400> 22

Met Ser Thr Asn Arg His Gln Ala Leu Gly Leu Thr Asp Gln Glu Ala
1               5                   10                  15

Val Asp Met Tyr Arg Thr Met Leu Leu Ala Arg Lys Ile Asp Glu Arg
            20                  25                  30

Met Trp Leu Leu Asn Arg Ser Gly Lys Ile Pro Phe Val Ile Ser Cys
        35                  40                  45

Gln Gly Gln Glu Ala Ala Gln Val Gly Ala Ala Phe Ala Leu Asp Arg
    50                  55                  60

Glu Met Asp Tyr Val Leu Pro Tyr Tyr Arg Asp Met Gly Val Val Leu
65                  70                  75                  80

Ala Phe Gly Met Thr Ala Lys Asp Leu Met Met Ser Gly Phe Ala Lys
                85                  90                  95

Ala Ala Asp Pro Asn Ser Gly Gly Arg Gln Met Pro Gly His Phe Gly
            100                 105                 110

Gln Lys Lys Asn Arg Ile Val Thr Gly Ser Ser Pro Val Thr Thr Gln
        115                 120                 125

Val Pro His Ala Val Gly Ile Ala Leu Ala Gly Arg Met Glu Lys Lys
    130                 135                 140

Asp Ile Ala Ala Phe Val Thr Phe Gly Glu Gly Ser Ser Asn Gln Gly
145                 150                 155                 160

Asp Phe His Glu Gly Ala Asn Phe Ala Ala Val His Lys Leu Pro Val
                165                 170                 175

Ile Phe Met Cys Glu Asn Asn Lys Tyr Ala Ile Ser Val Pro Tyr Asp
            180                 185                 190

Lys Gln Val Ala Cys Glu Asn Ile Ser Asp Arg Ala Ile Gly Tyr Gly
        195                 200                 205

Met Pro Gly Val Thr Val Asn Gly Asn Asp Pro Leu Glu Val Tyr Gln
    210                 215                 220

Ala Val Lys Glu Ala Arg Glu Arg Ala Arg Arg Gly Glu Gly Pro Thr
225                 230                 235                 240

Leu Ile Glu Thr Ile Ser Tyr Arg Leu Thr Pro His Ser Ser Asp Asp
                245                 250                 255

Asp Asp Ser Ser Tyr Arg Gly Arg Glu Glu Val Glu Glu Ala Lys Lys
            260                 265                 270

Ser Asp Pro Leu Leu Thr Tyr Gln Ala Tyr Leu Lys Glu Thr Gly Leu
        275                 280                 285

Leu Ser Asp Glu Ile Glu Gln Thr Met Leu Asp Glu Ile Met Ala Ile
    290                 295                 300

Val Asn Glu Ala Thr Asp Glu Ala Glu Asn Ala Pro Tyr Ala Ala Pro
305                 310                 315                 320

Glu Ser Ala Leu Asp Tyr Val Tyr Ala Lys
                325                 330

<210>  23
<211>  984
<212>  DNA
<213>  Bacillus subtilis

<400>  23
atgtcagtaa tgtcatatat tgatgcaatc aatttggcga tgaaagaaga aatggaacga      60

gattctcgcg ttttcgtcct tggggaagat gtaggaagaa aaggcggtgt gtttaaagcg     120

acagcgggac tctatgaaca atttggggaa gagcgcgtta tggatacgcc gcttgctgaa     180

tctgcaatcg caggagtcgg tatcggagcg gcaatgtacg gaatgagacc gattgctgaa     240

atgcagtttg ctgatttcat tatgccggca gtcaaccaaa ttatttctga agcggctaaa     300

atccgctacc gcagcaacaa tgactggagc tgtccgattg tcgtcagagc gccatacggc 360

ggaggcgtgc acggagccct gtatcattct caatcagtcg aagcaatttt cgccaaccag 420

cccggactga aaattgtcat gccatcaaca ccatatgacg cgaaagggct cttaaaagcc 480

gcagttcgtg acgaagaccc cgtgctgttt tttgagcaca agcgggcata ccgtctgata 540

aagggcgagg ttccggctga tgattatgtc ctgccaatcg gcaaggcgga cgtaaaaagg 600

gaaggcgacg acatcacagt gatcacatac ggcctgtgtg tccacttcgc cttacaagct 660

gcagaacgtc tcgaaaaaga tggcatttca gcgcatgtgg tggatttaag aacagtttac 720

ccgcttgata aagaagccat catcgaagct gcgtccaaaa ctggaaaggt tcttttggtc 780

acagaagata caaaagaagg cagcatcatg agcgaagtag ccgcaattat atccgagcat 840

tgtctgttcg acttagacgc gccgatcaaa cggcttgcag gtcctgatat tccggctatg 900

ccttatgcgc cgacaatgga aaaatacttt atggtcaacc ctgataaagt ggaagcggcg 960

atgagagaat tagcggagtt ttaa 984

<210> 24
<211> 327
<212> PRT
<213> Bacillus subtilis

<400> 24

Met Ser Val Met Ser Tyr Ile Asp Ala Ile Asn Leu Ala Met Lys Glu
1               5                   10                  15

Glu Met Glu Arg Asp Ser Arg Val Phe Val Leu Gly Glu Asp Val Gly
            20                  25                  30

Arg Lys Gly Gly Val Phe Lys Ala Thr Ala Gly Leu Tyr Glu Gln Phe
        35                  40                  45

Gly Glu Glu Arg Val Met Asp Thr Pro Leu Ala Glu Ser Ala Ile Ala
    50                  55                  60

Gly Val Gly Ile Gly Ala Ala Met Tyr Gly Met Arg Pro Ile Ala Glu
65                  70                  75                  80

Met Gln Phe Ala Asp Phe Ile Met Pro Ala Val Asn Gln Ile Ile Ser
                85                  90                  95

Glu Ala Ala Lys Ile Arg Tyr Arg Ser Asn Asn Asp Trp Ser Cys Pro
            100                 105                 110

Ile Val Val Arg Ala Pro Tyr Gly Gly Gly Val His Gly Ala Leu Tyr
        115                 120                 125

His Ser Gln Ser Val Glu Ala Ile Phe Ala Asn Gln Pro Gly Leu Lys
    130                 135                 140

Ile Val Met Pro Ser Thr Pro Tyr Asp Ala Lys Gly Leu Leu Lys Ala
145                 150                 155                 160

Ala Val Arg Asp Glu Asp Pro Val Leu Phe Phe Glu His Lys Arg Ala
                165                 170                 175

Tyr Arg Leu Ile Lys Gly Glu Val Pro Ala Asp Asp Tyr Val Leu Pro
            180                 185                 190

Ile Gly Lys Ala Asp Val Lys Arg Glu Gly Asp Asp Ile Thr Val Ile
        195                 200                 205

Thr Tyr Gly Leu Cys Val His Phe Ala Leu Gln Ala Ala Glu Arg Leu
    210                 215                 220

Glu Lys Asp Gly Ile Ser Ala His Val Val Asp Leu Arg Thr Val Tyr
225                 230                 235                 240

Pro Leu Asp Lys Glu Ala Ile Ile Glu Ala Ala Ser Lys Thr Gly Lys
                245                 250                 255

Val Leu Leu Val Thr Glu Asp Thr Lys Glu Gly Ser Ile Met Ser Glu
            260                 265                 270

Val Ala Ala Ile Ile Ser Glu His Cys Leu Phe Asp Leu Asp Ala Pro
        275                 280                 285

Ile Lys Arg Leu Ala Gly Pro Asp Ile Pro Ala Met Pro Tyr Ala Pro
    290                 295                 300

Thr Met Glu Lys Tyr Phe Met Val Asn Pro Asp Lys Val Glu Ala Ala
305                 310                 315                 320

Met Arg Glu Leu Ala Glu Phe
                325

<210>  25
<211>  1275
<212>  DNA
<213>  Bacillus subtilis

<400>  25
atggcaattg aacaaatgac gatgccgcag cttggagaaa gcgtaacaga ggggacgatc      60

agcaaatggc ttgtcgcccc cggtgataaa gtgaacaaat acgatccgat cgcggaagtc     120

atgacagata aggtaaatgc agaggttccg tcttctttta ctggtacgat aacagagctt 180

gtgggagaag aaggccaaac cctgcaagtc ggagaaatga tttgcaaaat tgaaacagaa 240

ggcgcgaatc cggctgaaca aaaacaagaa cagccagcag catcagaagc cgctgagaac 300

cctgttgcaa aaagtgctgg agcagccgat cagcccaata aaaagcgcta ctcgccagct 360

gttctccgtt tggccggaga gcacggcatt gacctcgatc aagtgacagg aactggtgcc 420

ggcgggcgca tcacacgaaa agatattcag cgcttaattg aaacaggcgg cgtgcaagaa 480

cagaatcctg aggagctgaa aacagcagct cctgcaccga agtctgcatc aaaacctgag 540

ccaaaagaag agacgtcata tcctgcgtct gcagccggtg ataaagaaat ccctgtcaca 600

ggtgtaagaa aagcaattgc ttccaatatg aagcgaagca aaacagaaat tccgcatgct 660

tggacgatga tggaagtcga cgtcacaaat atggttgcat atcgcaacag tataaaagat 720

tcttttaaga agacagaagg ctttaattta acgttcttcg ccttttttgt aaaagcggtc 780

gctcaggcgt taaaagaatt cccgcaaatg aatagcatgt gggcggggga caaaattatt 840

cagaaaaagg atatcaatat ttcaattgca gttgccacag aggattcttt atttgttccg 900

gtgattaaaa acgctgatga aaaaacaatt aaaggcattg cgaaagacat taccggccta 960

gctaaaaaag taagagacgg aaaactcact gcagatgaca tgcagggagg cacgtttacc 1020

gtcaacaaca caggttcgtt cgggtctgtt cagtcgatgg gcattatcaa ctaccctcag 1080

gctgcgattc ttcaagtaga atccatcgtc aaacgcccgg ttgtcatgga caatggcatg 1140

attgctgtca gagacatggt taatctgtgc ctgtcattag atcacagagt gcttgacggt 1200

ctcgtgtgcg gacgattcct cggacgagtg aaacaaattt tagaatcgat tgacgagaag 1260

acatctgttt actaa 1275

<210> 26
<211> 424
<212> PRT
<213> Bacillus subtilis

<400> 26

Met Ala Ile Glu Gln Met Thr Met Pro Gln Leu Gly Glu Ser Val Thr
1               5                   10                  15

Glu Gly Thr Ile Ser Lys Trp Leu Val Ala Pro Gly Asp Lys Val Asn
            20                  25                  30

Lys Tyr Asp Pro Ile Ala Glu Val Met Thr Asp Lys Val Asn Ala Glu
        35                  40                  45

Val Pro Ser Ser Phe Thr Gly Thr Ile Thr Glu Leu Val Gly Glu Glu
    50                  55                  60

Gly Gln Thr Leu Gln Val Gly Glu Met Ile Cys Lys Ile Glu Thr Glu
65                  70                  75                  80

Gly Ala Asn Pro Ala Glu Gln Lys Gln Glu Gln Pro Ala Ala Ser Glu
                85                  90                  95

Ala Ala Glu Asn Pro Val Ala Lys Ser Ala Gly Ala Ala Asp Gln Pro
            100                 105                 110

Asn Lys Lys Arg Tyr Ser Pro Ala Val Leu Arg Leu Ala Gly Glu His
        115                 120                 125

Gly Ile Asp Leu Asp Gln Val Thr Gly Thr Gly Ala Gly Gly Arg Ile
    130                 135                 140

Thr Arg Lys Asp Ile Gln Arg Leu Ile Glu Thr Gly Gly Val Gln Glu
145                 150                 155                 160

Gln Asn Pro Glu Glu Leu Lys Thr Ala Ala Pro Ala Pro Lys Ser Ala
                165                 170                 175

Ser Lys Pro Glu Pro Lys Glu Glu Thr Ser Tyr Pro Ala Ser Ala Ala
            180                 185                 190

Gly Asp Lys Glu Ile Pro Val Thr Gly Val Arg Lys Ala Ile Ala Ser
        195                 200                 205

Asn Met Lys Arg Ser Lys Thr Glu Ile Pro His Ala Trp Thr Met Met
    210                 215                 220

Glu Val Asp Val Thr Asn Met Val Ala Tyr Arg Asn Ser Ile Lys Asp
225                 230                 235                 240

Ser Phe Lys Lys Thr Glu Gly Phe Asn Leu Thr Phe Phe Ala Phe Phe
                245                 250                 255

Val Lys Ala Val Ala Gln Ala Leu Lys Glu Phe Pro Gln Met Asn Ser
            260                 265                 270

Met Trp Ala Gly Asp Lys Ile Ile Gln Lys Lys Asp Ile Asn Ile Ser
        275                 280                 285

Ile Ala Val Ala Thr Glu Asp Ser Leu Phe Val Pro Val Ile Lys Asn
    290                 295                 300

Ala Asp Glu Lys Thr Ile Lys Gly Ile Ala Lys Asp Ile Thr Gly Leu

305                 310                 315                 320

Ala Lys Lys Val Arg Asp Gly Lys Leu Thr Ala Asp Asp Met Gln Gly
                325                 330                 335

Gly Thr Phe Thr Val Asn Asn Thr Gly Ser Phe Gly Ser Val Gln Ser
            340                 345                 350

Met Gly Ile Ile Asn Tyr Pro Gln Ala Ala Ile Leu Gln Val Glu Ser
        355                 360                 365

Ile Val Lys Arg Pro Val Val Met Asp Asn Gly Met Ile Ala Val Arg
    370                 375                 380

Asp Met Val Asn Leu Cys Leu Ser Leu Asp His Arg Val Leu Asp Gly
385                 390                 395                 400

Leu Val Cys Gly Arg Phe Leu Gly Arg Val Lys Gln Ile Leu Glu Ser
                405                 410                 415

Ile Asp Glu Lys Thr Ser Val Tyr
            420

<210>  27
<211>  1413
<212>  DNA
<213>  Bacillus subtilis

<400>  27
atggtagtag gagatttccc tattgaaaca gatactcttg taattggtgc gggacctggc      60

ggctatgtag ctgccatccg cgctgcacag cttggacaaa aagtaacagt cgttgaaaaa     120

gcaactcttg gaggcgtttg tctgaacgtt ggatgtatcc cttcaaaagc gctgatcaat     180

gcaggtcacc gttatgagaa tgcaaaacat tctgatgaca tgggaatcac tgctgagaat     240

gtaacagttg atttcacaaa agttcaagaa tggaaagctt ctgttgtcaa caagcttact     300

ggcggtgtag caggtcttct taaaggcaac aaagtagatg ttgtaaaagg tgaagcttac     360

tttgtagaca gcaattcagt tcgtgttatg gatgagaact ctgctcaaac atacacgttt     420

aaaaacgcaa tcattgctac tggttctcgt cctatcgaat tgccaaactt caaatatagt     480

gagcgtgtcc tgaattcaac tggcgctttg gctcttaaag aaattcctaa aaagctcgtt     540

gttatcggcg gcggatacat cggaactgaa cttggaactg cgtatgctaa cttcggtact     600

gaacttgtta ttcttgaagg cggagatgaa attcttcctg gcttcgaaaa acaaatgagt     660

tctctcgtta cacgcagact gaagaaaaaa ggcaacgttg aaatccatac aaacgcgatg     720

gctaaaggcg ttgaagaaag accagacggc gtaacagtta ctttcgaagt aaaaggcgaa     780

gaaaaaactg ttgatgctga ttacgtattg attacagtag gacgccgtcc aaacactgat 840

gagcttggtc ttgagcaagt cggtatcgaa atgacggacc gcggtatcgt gaaaactgac 900

aaacagtgcc gcacaaacgt acctaacatt tatgcaatcg gtgatatcat cgaaggaccg 960

ccgcttgctc ataaagcatc ttacgaaggt aaaatcgctg cagaagctat cgctggagag 1020

cctgcagaaa tcgattacct tggtattcct gcggttgttt tctctgagcc tgaacttgca 1080

tcagttggtt acactgaagc acaggcgaaa gaagaaggtc ttgacattgt tgctgctaaa 1140

ttcccatttg cagcaaacgg ccgcgcgctt tctcttaacg aaacagacgg cttcatgaag 1200

ctgatcactc gtaaagagga cggtcttgtg atcggtgcgc aaatcgccgg agcaagtgct 1260

tctgatatga tttctgaatt aagcttagcg attgaaggcg gcatgactgc tgaagatatc 1320

gcaatgacaa ttcacgctca cccaacattg ggcgaaatca caatggaagc tgctgaagtg 1380

gcaatcggaa gtccgattca catcgtaaaa taa 1413

<210> 28
<211> 470
<212> PRT
<213> Bacillus subtilis

<400> 28

Met Val Val Gly Asp Phe Pro Ile Glu Thr Asp Thr Leu Val Ile Gly
1               5                   10                  15

Ala Gly Pro Gly Gly Tyr Val Ala Ala Ile Arg Ala Ala Gln Leu Gly
            20                  25                  30

Gln Lys Val Thr Val Val Glu Lys Ala Thr Leu Gly Gly Val Cys Leu
        35                  40                  45

Asn Val Gly Cys Ile Pro Ser Lys Ala Leu Ile Asn Ala Gly His Arg
    50                  55                  60

Tyr Glu Asn Ala Lys His Ser Asp Asp Met Gly Ile Thr Ala Glu Asn
65                  70                  75                  80

Val Thr Val Asp Phe Thr Lys Val Gln Glu Trp Lys Ala Ser Val Val
                85                  90                  95

Asn Lys Leu Thr Gly Gly Val Ala Gly Leu Leu Lys Gly Asn Lys Val
            100                 105                 110

Asp Val Val Lys Gly Glu Ala Tyr Phe Val Asp Ser Asn Ser Val Arg
        115                 120                 125

Val Met Asp Glu Asn Ser Ala Gln Thr Tyr Thr Phe Lys Asn Ala Ile

130                 135                 140

Ile Ala Thr Gly Ser Arg Pro Ile Glu Leu Pro Asn Phe Lys Tyr Ser
145                 150                 155                 160

Glu Arg Val Leu Asn Ser Thr Gly Ala Leu Ala Leu Lys Glu Ile Pro
                165                 170                 175

Lys Lys Leu Val Val Ile Gly Gly Gly Tyr Ile Gly Thr Glu Leu Gly
            180                 185                 190

Thr Ala Tyr Ala Asn Phe Gly Thr Glu Leu Val Ile Leu Glu Gly Gly
        195                 200                 205

Asp Glu Ile Leu Pro Gly Phe Glu Lys Gln Met Ser Ser Leu Val Thr
    210                 215                 220

Arg Arg Leu Lys Lys Lys Gly Asn Val Glu Ile His Thr Asn Ala Met
225                 230                 235                 240

Ala Lys Gly Val Glu Glu Arg Pro Asp Gly Val Thr Val Thr Phe Glu
                245                 250                 255

Val Lys Gly Glu Glu Lys Thr Val Asp Ala Asp Tyr Val Leu Ile Thr
            260                 265                 270

Val Gly Arg Arg Pro Asn Thr Asp Glu Leu Gly Leu Glu Gln Val Gly
        275                 280                 285

Ile Glu Met Thr Asp Arg Gly Ile Val Lys Thr Asp Lys Gln Cys Arg
    290                 295                 300

Thr Asn Val Pro Asn Ile Tyr Ala Ile Gly Asp Ile Ile Glu Gly Pro
305                 310                 315                 320

Pro Leu Ala His Lys Ala Ser Tyr Glu Gly Lys Ile Ala Ala Glu Ala
                325                 330                 335

Ile Ala Gly Glu Pro Ala Glu Ile Asp Tyr Leu Gly Ile Pro Ala Val
            340                 345                 350

Val Phe Ser Glu Pro Glu Leu Ala Ser Val Gly Tyr Thr Glu Ala Gln
        355                 360                 365

Ala Lys Glu Glu Gly Leu Asp Ile Val Ala Ala Lys Phe Pro Phe Ala
    370                 375                 380

Ala Asn Gly Arg Ala Leu Ser Leu Asn Glu Thr Asp Gly Phe Met Lys
385                 390                 395                 400

Leu Ile Thr Arg Lys Glu Asp Gly Leu Val Ile Gly Ala Gln Ile Ala
                405                 410                 415

Gly Ala Ser Ala Ser Asp Met Ile Ser Glu Leu Ser Leu Ala Ile Glu
            420                 425                 430

Gly Gly Met Thr Ala Glu Asp Ile Ala Met Thr Ile His Ala His Pro
        435                 440                 445

Thr Leu Gly Glu Ile Thr Met Glu Ala Ala Glu Val Ala Ile Gly Ser
    450                 455                 460

Pro Ile His Ile Val Lys
465                 470

<210>  29
<211>  2664
<212>  DNA
<213>  Escherichia coli

<400>  29
atgtcagaac gtttcccaaa tgacgtggat ccgatcgaaa ctcgcgactg gctccaggcg      60

atcgaatcgg tcatccgtga agaaggtgtt gagcgtgctc agtatctgat cgaccaactg     120

cttgctgaag cccgcaaagg cggtgtaaac gtagccgcag gcacaggtat cagcaactac     180

atcaacacca tccccgttga agaacaaccg gagtatccgg gtaatctgga actggaacgc     240

cgtattcgtt cagctatccg ctggaacgcc atcatgacgg tgctgcgtgc gtcgaaaaaa     300

gacctcgaac tgggcggcca tatggcgtcc ttccagtctt ccgcaaccat ttatgatgtg     360

tgctttaacc acttcttccg tgcacgcaac gagcaggatg gcggcgacct ggtttacttc     420

cagggccaca tctccccggg cgtgtacgct cgtgctttcc tggaaggtcg tctgactcag     480

gagcagctgg ataacttccg tcaggaagtt cacggcaatg gcctctcttc ctatccgcac     540

ccgaaactga tgccggaatt ctggcagttc ccgaccgtat ctatgggtct gggtccgatt     600

ggtgctattt accaggctaa attcctgaaa tatctggaac accgtggcct gaaagatacc     660

tctaaacaaa ccgtttacgc gttcctcggt gacggtgaaa tggacgaacc ggaatccaaa     720

ggtgcgatca ccatcgctac ccgtgaaaaa ctggataacc tggtcttcgt tatcaactgt     780

aacctgcagc gtcttgacgg cccggtcacc ggtaacggca agatcatcaa cgaactggaa     840

ggcatcttcg aaggtgctgg ctggaacgtg atcaaagtga tgtggggtag ccgttgggat     900

gaactgctgc gtaaggatac cagcggtaaa ctgatccagc tgatgaacga aaccgttgac     960

ggcgactacc agaccttcaa atcgaaagat ggtgcgtacg ttcgtgaaca cttcttcggt    1020

aaatatcctg aaaccgcagc actggttgca gactggactg acgagcagat ctgggcactg 1080

aaccgtggtg gtcacgatcc gaagaaaatc tacgctgcat tcaagaaagc gcaggaaacc 1140

aaaggcaaag cgacagtaat ccttgctcat accattaaag gttacggcat gggcgacgcg 1200

gctgaaggta aaaacatcgc gcaccaggtt aagaaaatga acatggacgg tgtgcgtcat 1260

atccgcgacc gtttcaatgt gccggtgtct gatgcagata tcgaaaaact gccgtacatc 1320

accttcccgg aaggttctga agagcatacc tatctgcacg ctcagcgtca gaaactgcac 1380

ggttatctgc caagccgtca gccgaacttc accgagaagc ttgagctgcc gagcctgcaa 1440

gacttcggcg cgctgttgga agagcagagc aaagagatct ctaccactat cgctttcgtt 1500

cgtgctctga acgtgatgct gaagaacaag tcgatcaaag atcgtctggt accgatcatc 1560

gccgacgaag cgcgtacttt cggtatggaa ggtctgttcc gtcagattgg tatttacagc 1620

ccgaacggtc agcagtacac cccgcaggac cgcgagcagg ttgcttacta taaagaagac 1680

gagaaaggtc agattctgca ggaagggatc aacgagctgg gcgcaggttg ttcctggctg 1740

gcagcggcga cctcttacag caccaacaat ctgccgatga tcccgttcta catctattac 1800

tcgatgttcg gcttccagcg tattggcgat ctgtgctggg cggctggcga ccagcaagcg 1860

cgtggcttcc tgatcggcgg tacttccggt cgtaccaccc tgaacggcga aggtctgcag 1920

cacgaagatg gtcacagcca cattcagtcg ctgactatcc cgaactgtat ctcttacgac 1980

ccggcttacg cttacgaagt tgctgtcatc atgcatgacg gtctggagcg tatgtacggt 2040

gaaaaacaag agaacgttta ctactacatc actacgctga acgaaaacta ccacatgccg 2100

gcaatgccgg aaggtgctga ggaaggtatc cgtaaaggta tctacaaact cgaaactatt 2160

gaaggtagca aaggtaaagt tcagctgctc ggctccggtt ctatcctgcg tcacgtccgt 2220

gaagcagctg agatcctggc gaaagattac ggcgtaggtt ctgacgttta tagcgtgacc 2280

tccttcaccg agctggcgcg tgatggtcag gattgtgaac gctggaacat gctgcacccg 2340

ctggaaactc cgcgcgttcc gtatatcgct caggtgatga acgacgctcc ggcagtggca 2400

tctaccgact atatgaaact gttcgctgag caggtccgta cttacgtacc ggctgacgac 2460

taccgcgtac tgggtactga tggcttcggt cgttccgaca gccgtgagaa cctgcgtcac 2520

cacttcgaag ttgatgcttc ttatgtcgtg gttgcggcgc tgggcgaact ggctaaacgt 2580

ggcgaaatcg ataagaaagt ggttgctgac gcaatcgcca aattcaacat cgatgcagat 2640

aaagttaacc cgcgtctggc gtaa 2664

<210> 30
<211> 887
<212> PRT
<213> Escherichia coli

<400>  30

Met Ser Glu Arg Phe Pro Asn Asp Val Asp Pro Ile Glu Thr Arg Asp
1               5                   10                  15

Trp Leu Gln Ala Ile Glu Ser Val Ile Arg Glu Glu Gly Val Glu Arg
            20                  25                  30

Ala Gln Tyr Leu Ile Asp Gln Leu Leu Ala Glu Ala Arg Lys Gly Gly
        35                  40                  45

Val Asn Val Ala Ala Gly Thr Gly Ile Ser Asn Tyr Ile Asn Thr Ile
    50                  55                  60

Pro Val Glu Glu Gln Pro Glu Tyr Pro Gly Asn Leu Glu Leu Glu Arg
65                  70                  75                  80

Arg Ile Arg Ser Ala Ile Arg Trp Asn Ala Ile Met Thr Val Leu Arg
                85                  90                  95

Ala Ser Lys Lys Asp Leu Glu Leu Gly Gly His Met Ala Ser Phe Gln
            100                 105                 110

Ser Ser Ala Thr Ile Tyr Asp Val Cys Phe Asn His Phe Phe Arg Ala
        115                 120                 125

Arg Asn Glu Gln Asp Gly Gly Asp Leu Val Tyr Phe Gln Gly His Ile
    130                 135                 140

Ser Pro Gly Val Tyr Ala Arg Ala Phe Leu Glu Gly Arg Leu Thr Gln
145                 150                 155                 160

Glu Gln Leu Asp Asn Phe Arg Gln Glu Val His Gly Asn Gly Leu Ser
                165                 170                 175

Ser Tyr Pro His Pro Lys Leu Met Pro Glu Phe Trp Gln Phe Pro Thr
            180                 185                 190

Val Ser Met Gly Leu Gly Pro Ile Gly Ala Ile Tyr Gln Ala Lys Phe
        195                 200                 205

Leu Lys Tyr Leu Glu His Arg Gly Leu Lys Asp Thr Ser Lys Gln Thr
    210                 215                 220

Val Tyr Ala Phe Leu Gly Asp Gly Glu Met Asp Glu Pro Glu Ser Lys
225                 230                 235                 240

Gly Ala Ile Thr Ile Ala Thr Arg Glu Lys Leu Asp Asn Leu Val Phe

                245                 250                 255

Val Ile Asn Cys Asn Leu Gln Arg Leu Asp Gly Pro Val Thr Gly Asn
            260                 265                 270

Gly Lys Ile Ile Asn Glu Leu Glu Gly Ile Phe Glu Gly Ala Gly Trp
        275                 280                 285

Asn Val Ile Lys Val Met Trp Gly Ser Arg Trp Asp Glu Leu Leu Arg
    290                 295                 300

Lys Asp Thr Ser Gly Lys Leu Ile Gln Leu Met Asn Glu Thr Val Asp
305                 310                 315                 320

Gly Asp Tyr Gln Thr Phe Lys Ser Lys Asp Gly Ala Tyr Val Arg Glu
                325                 330                 335

His Phe Phe Gly Lys Tyr Pro Glu Thr Ala Ala Leu Val Ala Asp Trp
            340                 345                 350

Thr Asp Glu Gln Ile Trp Ala Leu Asn Arg Gly Gly His Asp Pro Lys
        355                 360                 365

Lys Ile Tyr Ala Ala Phe Lys Lys Ala Gln Glu Thr Lys Gly Lys Ala
    370                 375                 380

Thr Val Ile Leu Ala His Thr Ile Lys Gly Tyr Gly Met Gly Asp Ala
385                 390                 395                 400

Ala Glu Gly Lys Asn Ile Ala His Gln Val Lys Lys Met Asn Met Asp
                405                 410                 415

Gly Val Arg His Ile Arg Asp Arg Phe Asn Val Pro Val Ser Asp Ala
            420                 425                 430

Asp Ile Glu Lys Leu Pro Tyr Ile Thr Phe Pro Glu Gly Ser Glu Glu
        435                 440                 445

His Thr Tyr Leu His Ala Gln Arg Gln Lys Leu His Gly Tyr Leu Pro
    450                 455                 460

Ser Arg Gln Pro Asn Phe Thr Glu Lys Leu Glu Leu Pro Ser Leu Gln
465                 470                 475                 480

Asp Phe Gly Ala Leu Leu Glu Glu Gln Ser Lys Glu Ile Ser Thr Thr
                485                 490                 495

Ile Ala Phe Val Arg Ala Leu Asn Val Met Leu Lys Asn Lys Ser Ile
            500                 505                 510

Lys Asp Arg Leu Val Pro Ile Ile Ala Asp Glu Ala Arg Thr Phe Gly
        515                 520                 525

Met Glu Gly Leu Phe Arg Gln Ile Gly Ile Tyr Ser Pro Asn Gly Gln
    530                 535                 540

Gln Tyr Thr Pro Gln Asp Arg Glu Gln Val Ala Tyr Tyr Lys Glu Asp
545                 550                 555                 560

Glu Lys Gly Gln Ile Leu Gln Glu Gly Ile Asn Glu Leu Gly Ala Gly
                565                 570                 575

Cys Ser Trp Leu Ala Ala Ala Thr Ser Tyr Ser Thr Asn Asn Leu Pro
            580                 585                 590

Met Ile Pro Phe Tyr Ile Tyr Tyr Ser Met Phe Gly Phe Gln Arg Ile
        595                 600                 605

Gly Asp Leu Cys Trp Ala Ala Gly Asp Gln Gln Ala Arg Gly Phe Leu
    610                 615                 620

Ile Gly Gly Thr Ser Gly Arg Thr Thr Leu Asn Gly Glu Gly Leu Gln
625                 630                 635                 640

His Glu Asp Gly His Ser His Ile Gln Ser Leu Thr Ile Pro Asn Cys
                645                 650                 655

Ile Ser Tyr Asp Pro Ala Tyr Ala Tyr Glu Val Ala Val Ile Met His
            660                 665                 670

Asp Gly Leu Glu Arg Met Tyr Gly Glu Lys Gln Glu Asn Val Tyr Tyr
        675                 680                 685

Tyr Ile Thr Thr Leu Asn Glu Asn Tyr His Met Pro Ala Met Pro Glu
    690                 695                 700

Gly Ala Glu Glu Gly Ile Arg Lys Gly Ile Tyr Lys Leu Glu Thr Ile
705                 710                 715                 720

Glu Gly Ser Lys Gly Lys Val Gln Leu Leu Gly Ser Gly Ser Ile Leu
                725                 730                 735

Arg His Val Arg Glu Ala Ala Glu Ile Leu Ala Lys Asp Tyr Gly Val
            740                 745                 750

Gly Ser Asp Val Tyr Ser Val Thr Ser Phe Thr Glu Leu Ala Arg Asp
        755                 760                 765

Gly Gln Asp Cys Glu Arg Trp Asn Met Leu His Pro Leu Glu Thr Pro
    770                 775                 780

Arg Val Pro Tyr Ile Ala Gln Val Met Asn Asp Ala Pro Ala Val Ala
785                 790                 795                 800

Ser Thr Asp Tyr Met Lys Leu Phe Ala Glu Gln Val Arg Thr Tyr Val
                805                 810                 815

Pro Ala Asp Asp Tyr Arg Val Leu Gly Thr Asp Gly Phe Gly Arg Ser
            820                 825                 830

Asp Ser Arg Glu Asn Leu Arg His His Phe Glu Val Asp Ala Ser Tyr
        835                 840                 845

Val Val Val Ala Ala Leu Gly Glu Leu Ala Lys Arg Gly Glu Ile Asp
    850                 855                 860

Lys Lys Val Val Ala Asp Ala Ile Ala Lys Phe Asn Ile Asp Ala Asp
865                 870                 875                 880

Lys Val Asn Pro Arg Leu Ala
                885

<210>  31
<211>  1893
<212>  DNA
<213>  Escherichia coli

<400>  31
atggctatcg aaatcaaagt accggacatc ggggctgatg aagttgaaat caccgagatc      60

ctggtcaaag tgggcgacaa agttgaagcc gaacagtcgc tgatcaccgt agaaggcgac     120

aaagcctcta tggaagttcc gtctccgcag gcgggtatcg ttaaagagat caaagtctct     180

gttggcgata aaacccagac cggcgcactg attatgattt tcgattccgc cgacggtgca     240

gcagacgctg cacctgctca ggcagaagag aagaaagaag cagctccggc agcagcacca     300

gcggctgcgg cggcaaaaga cgttaacgtt ccggatatcg gcagcgacga agttgaagtg     360

accgaaatcc tggtgaaagt tggcgataaa gttgaagctg aacagtcgct gatcaccgta     420

gaaggcgaca aggcttctat ggaagttccg gctccgtttg ctggcaccgt gaaagagatc     480

aaagtgaacg tgggtgacaa agtgtctacc ggctcgctga ttatggtctt cgaagtcgcg     540

ggtgaagcag gcgcggcagc tccggccgct aaacaggaag cagctccggc agcggcccct     600

gcaccagcgg ctggcgtgaa agaagttaac gttccggata tcggcggtga cgaagttgaa 660

gtgactgaag tgatggtgaa agtgggcgac aaagttgccg ctgaacagtc actgatcacc 720

gtagaaggcg acaaagcttc tatggaagtt ccggcgccgt ttgcaggcgt cgtgaaggaa 780

ctgaaagtca acgttggcga taaagtgaaa actggctcgc tgattatgat cttcgaagtt 840

gaaggcgcag cgcctgcggc agctcctgcg aaacaggaag cggcagcgcc ggcaccggca 900

gcaaaagctg aagccccggc agcagcacca gctgcgaaag cggaaggcaa atctgaattt 960

gctgaaaacg acgcttatgt tcacgcgact ccgctgatcc gccgtctggc acgcgagttt 1020

ggtgttaacc ttgcgaaagt gaagggcact ggccgtaaag gtcgtatcct gcgcgaagac 1080

gttcaggctt acgtgaaaga agctatcaaa cgtgcagaag cagctccggc agcgactggc 1140

ggtggtatcc ctggcatgct gccgtggccg aaggtggact tcagcaagtt tggtgaaatc 1200

gaagaagtgg aactgggccg catccagaaa atctctggtg cgaacctgag ccgtaactgg 1260

gtaatgatcc cgcatgttac tcacttcgac aaaaccgata tcaccgagtt ggaagcgttc 1320

cgtaaacagc agaacgaaga agcggcgaaa cgtaagctgg atgtgaagat caccccggtt 1380

gtcttcatca tgaaagccgt tgctgcagct cttgagcaga tgcctcgctt caatagttcg 1440

ctgtcggaag acggtcagcg tctgaccctg aagaaataca tcaacatcgg tgtggcggtg 1500

gataccccga acggtctggt tgttccggta ttcaaagacg tcaacaagaa aggcatcatc 1560

gagctgtctc gcgagctgat gactatttct aagaaagcgc gtgacggtaa gctgactgcg 1620

ggcgaaatgc agggcggttg cttcaccatc tccagcatcg gcggcctggg tactacccac 1680

ttcgcgccga ttgtgaacgc gccggaagtg gctatcctcg gcgtttccaa gtccgcgatg 1740

gagccggtgt ggaatggtaa agagttcgtg ccgcgtctga tgctgccgat ttctctctcc 1800

ttcgaccacc gcgtgatcga cggtgctgat ggtgcccgtt tcattaccat cattaacaac 1860

acgctgtctg acattcgccg tctggtgatg taa 1893

<210> 32
<211> 630
<212> PRT
<213> Escherichia coli

<400> 32

Met Ala Ile Glu Ile Lys Val Pro Asp Ile Gly Ala Asp Glu Val Glu
1               5                   10                  15

Ile Thr Glu Ile Leu Val Lys Val Gly Asp Lys Val Glu Ala Glu Gln
            20                  25                  30

Ser Leu Ile Thr Val Glu Gly Asp Lys Ala Ser Met Glu Val Pro Ser
        35                  40                  45

Pro Gln Ala Gly Ile Val Lys Glu Ile Lys Val Ser Val Gly Asp Lys
    50                  55                  60

Thr Gln Thr Gly Ala Leu Ile Met Ile Phe Asp Ser Ala Asp Gly Ala
65                  70                  75                  80

Ala Asp Ala Ala Pro Ala Gln Ala Glu Glu Lys Lys Glu Ala Ala Pro
                85                  90                  95

Ala Ala Ala Pro Ala Ala Ala Ala Ala Lys Asp Val Asn Val Pro Asp
            100                 105                 110

Ile Gly Ser Asp Glu Val Glu Val Thr Glu Ile Leu Val Lys Val Gly
        115                 120                 125

Asp Lys Val Glu Ala Glu Gln Ser Leu Ile Thr Val Glu Gly Asp Lys
    130                 135                 140

Ala Ser Met Glu Val Pro Ala Pro Phe Ala Gly Thr Val Lys Glu Ile
145                 150                 155                 160

Lys Val Asn Val Gly Asp Lys Val Ser Thr Gly Ser Leu Ile Met Val
                165                 170                 175

Phe Glu Val Ala Gly Glu Ala Gly Ala Ala Ala Pro Ala Ala Lys Gln
            180                 185                 190

Glu Ala Ala Pro Ala Ala Ala Pro Ala Pro Ala Ala Gly Val Lys Glu
        195                 200                 205

Val Asn Val Pro Asp Ile Gly Gly Asp Glu Val Glu Val Thr Glu Val
    210                 215                 220

Met Val Lys Val Gly Asp Lys Val Ala Ala Glu Gln Ser Leu Ile Thr
225                 230                 235                 240

Val Glu Gly Asp Lys Ala Ser Met Glu Val Pro Ala Pro Phe Ala Gly
                245                 250                 255

Val Val Lys Glu Leu Lys Val Asn Val Gly Asp Lys Val Lys Thr Gly
            260                 265                 270

Ser Leu Ile Met Ile Phe Glu Val Glu Gly Ala Ala Pro Ala Ala Ala
        275                 280                 285

Pro Ala Lys Gln Glu Ala Ala Ala Pro Ala Pro Ala Ala Lys Ala Glu
    290                 295                 300

Ala Pro Ala Ala Ala Pro Ala Ala Lys Ala Glu Gly Lys Ser Glu Phe
305                 310                 315                 320

Ala Glu Asn Asp Ala Tyr Val His Ala Thr Pro Leu Ile Arg Arg Leu
                325                 330                 335

Ala Arg Glu Phe Gly Val Asn Leu Ala Lys Val Lys Gly Thr Gly Arg
            340                 345                 350

Lys Gly Arg Ile Leu Arg Glu Asp Val Gln Ala Tyr Val Lys Glu Ala
        355                 360                 365

Ile Lys Arg Ala Glu Ala Ala Pro Ala Ala Thr Gly Gly Gly Ile Pro
    370                 375                 380

Gly Met Leu Pro Trp Pro Lys Val Asp Phe Ser Lys Phe Gly Glu Ile
385                 390                 395                 400

Glu Glu Val Glu Leu Gly Arg Ile Gln Lys Ile Ser Gly Ala Asn Leu
                405                 410                 415

Ser Arg Asn Trp Val Met Ile Pro His Val Thr His Phe Asp Lys Thr
            420                 425                 430

Asp Ile Thr Glu Leu Glu Ala Phe Arg Lys Gln Gln Asn Glu Glu Ala
        435                 440                 445

Ala Lys Arg Lys Leu Asp Val Lys Ile Thr Pro Val Val Phe Ile Met
    450                 455                 460

Lys Ala Val Ala Ala Ala Leu Glu Gln Met Pro Arg Phe Asn Ser Ser
465                 470                 475                 480

Leu Ser Glu Asp Gly Gln Arg Leu Thr Leu Lys Lys Tyr Ile Asn Ile
                485                 490                 495

Gly Val Ala Val Asp Thr Pro Asn Gly Leu Val Val Pro Val Phe Lys
            500                 505                 510

Asp Val Asn Lys Lys Gly Ile Ile Glu Leu Ser Arg Glu Leu Met Thr
        515                 520                 525

Ile Ser Lys Lys Ala Arg Asp Gly Lys Leu Thr Ala Gly Glu Met Gln
    530                 535                 540

Gly Gly Cys Phe Thr Ile Ser Ser Ile Gly Gly Leu Gly Thr Thr His

545                 550                 555                 560

Phe Ala Pro Ile Val Asn Ala Pro Glu Val Ala Ile Leu Gly Val Ser
                565                 570                 575

Lys Ser Ala Met Glu Pro Val Trp Asn Gly Lys Glu Phe Val Pro Arg
            580                 585                 590

Leu Met Leu Pro Ile Ser Leu Ser Phe Asp His Arg Val Ile Asp Gly
        595                 600                 605

Ala Asp Gly Ala Arg Phe Ile Thr Ile Ile Asn Asn Thr Leu Ser Asp
    610                 615                 620

Ile Arg Arg Leu Val Met
625                 630

<210>  33
<211>  1425
<212>  DNA
<213>  Escherichia coli

<400>  33
atgagtactg aaatcaaaac tcaggtcgtg gtacttgggg caggccccgc aggttactcc      60

gctgccttcc gttgcgctga tttaggtctg gaaaccgtaa tcgtagaacg ttacaacacc     120

cttggcggtg tttgcctgaa cgtcggctgt atcccttcta aagcactgct gcacgtagca     180

aaagttatcg aagaagccaa agcgctggct gaacacggta tcgtcttcgg cgaaccgaaa     240

accgatatcg acaagattcg tacctggaaa gagaaagtga tcaatcagct gaccggtggt     300

ctggctggta tggcgaaagg ccgcaaagtc aaagtggtca acggtctggg taaattcacc     360

ggggctaaca ccctggaagt tgaaggtgag aacggcaaaa ccgtgatcaa cttcgacaac     420

gcgatcattg cagcgggttc tcgcccgatc caactgccgt ttattccgca tgaagatccg     480

cgtatctggg actccactga cgcgctggaa ctgaaagaag taccagaacg cctgctggta     540

atgggtggcg gtatcatcgg tctggaaatg ggcaccgttt accacgcgct gggttcacag     600

attgacgtgg ttgaaatgtt cgaccaggtt atcccggcag ctgacaaaga catcgttaaa     660

gtcttcacca agcgtatcag caagaaattc aacctgatgc tggaaaccaa agttaccgcc     720

gttgaagcga aagaagacgg catttatgtg acgatggaag gcaaaaaagc acccgctgaa     780

ccgcagcgtt acgacgccgt gctggtagcg attggtcgtg tgccgaacgg taaaaacctc     840

gacgcaggca aagcaggcgt ggaagttgac gaccgtggtt tcatccgcgt tgacaaacag     900

ctgcgtacca acgtaccgca catctttgct atcggcgata tcgtcggtca accgatgctg     960

gcacacaaag gtgttcacga aggtcacgtt gccgctgaag ttatcgccgg taagaaacac    1020

tacttcgatc cgaaagttat cccgtccatc gcctataccg aaccagaagt tgcatgggtg 1080

ggtctgactg agaaagaagc gaaagagaaa ggcatcagct atgaaaccgc caccttcccg 1140

tgggctgctt ctggtcgtgc tatcgcttcc gactgcgcag acggtatgac caagctgatt 1200

ttcgacaaag aatctcaccg tgtgatcggt ggtgcgattg tcggtactaa cggcggcgag 1260

ctgctgggtg aaatcggcct ggcaatcgaa atgggttgtg atgctgaaga catcgcactg 1320

accatccacg cgcacccgac tctgcacgag tctgtgggcc tggcggcaga agtgttcgaa 1380

ggtagcatta ccgacctgcc gaacccgaaa gcgaagaaga agtaa 1425

<210> 34
<211> 474
<212> PRT
<213> Escherichia coli

<400> 34

<210>  35
<211>  2802
<212>  DNA
<213>  Escherichia coli

<400>  35
atgcagaaca gcgctttgaa agcctggttg gactcttctt acctctctgg cgcaaaccag      60

agctggatag aacagctcta tgaagacttc ttaaccgatc ctgactcggt tgacgctaac     120

tggcgttcga cgttccagca gttacctggt acgggagtca aaccggatca attccactct     180

caaacgcgtg aatatttccg ccgcctggcg aaagacgctt cacgttactc ttcaacgatc     240

tccgaccctg acaccaatgt gaagcaggtt aaagtcctgc agctcattaa cgcataccgc     300

ttccgtggtc accagcatgc gaatctcgat ccgctgggac tgtggcagca agataaagtg     360

gccgatctgg atccgtcttt ccacgatctg accgaagcag acttccagga gaccttcaac     420

gtcggttcat ttgccagcgg caaagaaacc atgaaactcg gcgagctgct ggaagccctc     480

aagcaaacct actgcggccc gattggtgcc gagtatatgc acattaccag caccgaagaa     540

aaacgctgga tccaacagcg tatcgagtct ggtcgcgcga ctttcaatag cgaagagaaa     600

aaacgcttct taagcgaact gaccgccgct gaaggtcttg aacgttacct cggcgcaaaa     660

ttccctggcg caaaacgctt ctcgctggaa ggcggtgacg cgttaatccc gatgcttaaa     720

gagatgatcc gccacgctgg caacagcggc acccgcgaag tggttctcgg gatggcgcac     780

cgtggtcgtc tgaacgtgct ggtgaacgtg ctgggtaaaa aaccgcaaga cttgttcgac     840

gagttcgccg gtaaacataa agaacacctc ggcacgggtg acgtgaaata ccacatgggc     900

ttctcgtctg acttccagac cgatggcggc ctggtgcacc tggcgctggc gtttaacccg     960

tctcaccttg agattgtaag cccggtagtt atcggttctg ttcgtgcccg tctggacaga    1020

cttgatgagc cgagcagcaa caaagtgctg ccaatcacca tccacggtga cgccgcagtg    1080

accgggcagg gcgtggttca ggaaaccctg aacatgtcga aagcgcgtgg ttatgaagtt    1140

ggcggtacgg tacgtatcgt tatcaacaac caggttggtt tcaccacctc taatccgctg    1200

gatgcccgtt ctacgccgta ctgtactgat atcggtaaga tggttcaggc cccgattttc    1260

cacgttaacg cggacgatcc ggaagccgtt gcctttgtga cccgtctggc gctcgatttc 1320

cgtaacacct ttaaacgtga tgtcttcatc gacctggtgt gctaccgccg tcacggccac 1380

aacgaagccg acgagccgag cgcaacccag ccgctgatgt atcagaaaat caaaaaacat 1440

ccgacaccgc gcaaaatcta cgctgacaag ctggagcagg aaaaagtggc gacgctggaa 1500

gatgccaccg agatggttaa cctgtaccgc gatgcgctgg atgctggcga ttgcgtagtg 1560

gcagagtggc gtccgatgaa catgcactct ttcacctggt cgccgtacct caaccacgaa 1620

tgggacgaag agtacccgaa caaagttgag atgaagcgcc tgcaggagct ggcgaaacgc 1680

atcagcacgg tgccggaagc agttgaaatg cagtctcgcg ttgccaagat ttatggcgat 1740

cgccaggcga tggctgccgg tgagaaactg ttcgactggg gcggtgcgga aaacctcgct 1800

tacgccacgc tggttgatga aggcattccg gttcgcctgt cgggtgaaga ctccggtcgc 1860

ggtaccttct tccaccgcca cgcggtgatc cacaaccagt ctaacggttc cacttacacg 1920

ccgctgcaac atatccataa cgggcagggc gcgttccgtg tctgggactc cgtactgtct 1980

gaagaagcag tgctggcgtt tgaatatggt tatgccaccg cagaaccacg cactctgacc 2040

atctgggaag cgcagttcgg tgacttcgcc aacggtgcgc aggtggttat cgaccagttc 2100

atctcctctg gcgaacagaa atggggccgg atgtgtggtc tggtgatgtt gctgccgcac 2160

ggttacgaag ggcaggggcc ggagcactcc tccgcgcgtc tggaacgtta tctgcaactt 2220

tgtgctgagc aaaacatgca ggtttgcgta ccgtctaccc cggcacaggt ttaccacatg 2280

ctgcgtcgtc aggcgctgcg cgggatgcgt cgtccgctgg tcgtgatgtc gccgaaatcc 2340

ctgctgcgtc atccgctggc ggtttccagc ctcgaagaac tggcgaacgg caccttcctg 2400

ccagccatcg gtgaaatcga cgagcttgat ccgaagggcg tgaagcgcgt agtgatgtgt 2460

tctggtaagg tttattacga cctgctggaa cagcgtcgta agaacaatca acacgatgtc 2520

gccattgtgc gtatcgagca actctacccg ttcccgcata aagcgatgca ggaagtgttg 2580

cagcagtttg ctcacgtcaa ggattttgtc tggtgccagg aagagccgct caaccagggc 2640

gcatggtact gcagccagca tcatttccgt gaagtgattc cgtttggggc ttctctgcgt 2700

tatgcaggcc gcccggcctc cgcctctccg gcggtagggt atatgtccgt tcaccagaaa 2760

cagcaacaag atctggttaa tgacgcgctg aacgtcgaat aa 2802

<210> 36
<211> 933
<212> PRT
<213> Escherichia coli

<400> 36

Met Gln Asn Ser Ala Leu Lys Ala Trp Leu Asp Ser Ser Tyr Leu Ser
1               5                   10                  15

Gly Ala Asn Gln Ser Trp Ile Glu Gln Leu Tyr Glu Asp Phe Leu Thr
            20                  25                  30

Asp Pro Asp Ser Val Asp Ala Asn Trp Arg Ser Thr Phe Gln Gln Leu
        35                  40                  45

Pro Gly Thr Gly Val Lys Pro Asp Gln Phe His Ser Gln Thr Arg Glu
    50                  55                  60

Tyr Phe Arg Arg Leu Ala Lys Asp Ala Ser Arg Tyr Ser Ser Thr Ile
65                  70                  75                  80

Ser Asp Pro Asp Thr Asn Val Lys Gln Val Lys Val Leu Gln Leu Ile
                85                  90                  95

Asn Ala Tyr Arg Phe Arg Gly His Gln His Ala Asn Leu Asp Pro Leu
            100                 105                 110

Gly Leu Trp Gln Gln Asp Lys Val Ala Asp Leu Asp Pro Ser Phe His
        115                 120                 125

Asp Leu Thr Glu Ala Asp Phe Gln Glu Thr Phe Asn Val Gly Ser Phe
    130                 135                 140

Ala Ser Gly Lys Glu Thr Met Lys Leu Gly Glu Leu Leu Glu Ala Leu
145                 150                 155                 160

Lys Gln Thr Tyr Cys Gly Pro Ile Gly Ala Glu Tyr Met His Ile Thr
                165                 170                 175

Ser Thr Glu Glu Lys Arg Trp Ile Gln Gln Arg Ile Glu Ser Gly Arg
            180                 185                 190

Ala Thr Phe Asn Ser Glu Glu Lys Lys Arg Phe Leu Ser Glu Leu Thr
        195                 200                 205

Ala Ala Glu Gly Leu Glu Arg Tyr Leu Gly Ala Lys Phe Pro Gly Ala
    210                 215                 220

Lys Arg Phe Ser Leu Glu Gly Gly Asp Ala Leu Ile Pro Met Leu Lys
225                 230                 235                 240

Glu Met Ile Arg His Ala Gly Asn Ser Gly Thr Arg Glu Val Val Leu
                245                 250                 255

Gly Met Ala His Arg Gly Arg Leu Asn Val Leu Val Asn Val Leu Gly

            260                 265                 270

Lys Lys Pro Gln Asp Leu Phe Asp Glu Phe Ala Gly Lys His Lys Glu
        275                 280                 285

His Leu Gly Thr Gly Asp Val Lys Tyr His Met Gly Phe Ser Ser Asp
    290                 295                 300

Phe Gln Thr Asp Gly Gly Leu Val His Leu Ala Leu Ala Phe Asn Pro
305                 310                 315                 320

Ser His Leu Glu Ile Val Ser Pro Val Val Ile Gly Ser Val Arg Ala
                325                 330                 335

Arg Leu Asp Arg Leu Asp Glu Pro Ser Ser Asn Lys Val Leu Pro Ile
            340                 345                 350

Thr Ile His Gly Asp Ala Ala Val Thr Gly Gln Gly Val Val Gln Glu
        355                 360                 365

Thr Leu Asn Met Ser Lys Ala Arg Gly Tyr Glu Val Gly Gly Thr Val
    370                 375                 380

Arg Ile Val Ile Asn Asn Gln Val Gly Phe Thr Thr Ser Asn Pro Leu
385                 390                 395                 400

Asp Ala Arg Ser Thr Pro Tyr Cys Thr Asp Ile Gly Lys Met Val Gln
                405                 410                 415

Ala Pro Ile Phe His Val Asn Ala Asp Asp Pro Glu Ala Val Ala Phe
            420                 425                 430

Val Thr Arg Leu Ala Leu Asp Phe Arg Asn Thr Phe Lys Arg Asp Val
        435                 440                 445

Phe Ile Asp Leu Val Cys Tyr Arg Arg His Gly His Asn Glu Ala Asp
    450                 455                 460

Glu Pro Ser Ala Thr Gln Pro Leu Met Tyr Gln Lys Ile Lys Lys His
465                 470                 475                 480

Pro Thr Pro Arg Lys Ile Tyr Ala Asp Lys Leu Glu Gln Glu Lys Val
                485                 490                 495

Ala Thr Leu Glu Asp Ala Thr Glu Met Val Asn Leu Tyr Arg Asp Ala
            500                 505                 510

Leu Asp Ala Gly Asp Cys Val Val Ala Glu Trp Arg Pro Met Asn Met
        515                 520                 525

His Ser Phe Thr Trp Ser Pro Tyr Leu Asn His Glu Trp Asp Glu Glu
    530                 535                 540

Tyr Pro Asn Lys Val Glu Met Lys Arg Leu Gln Glu Leu Ala Lys Arg
545                 550                 555                 560

Ile Ser Thr Val Pro Glu Ala Val Glu Met Gln Ser Arg Val Ala Lys
                565                 570                 575

Ile Tyr Gly Asp Arg Gln Ala Met Ala Ala Gly Glu Lys Leu Phe Asp
            580                 585                 590

Trp Gly Gly Ala Glu Asn Leu Ala Tyr Ala Thr Leu Val Asp Glu Gly
        595                 600                 605

Ile Pro Val Arg Leu Ser Gly Glu Asp Ser Gly Arg Gly Thr Phe Phe
    610                 615                 620

His Arg His Ala Val Ile His Asn Gln Ser Asn Gly Ser Thr Tyr Thr
625                 630                 635                 640

Pro Leu Gln His Ile His Asn Gly Gln Gly Ala Phe Arg Val Trp Asp
                645                 650                 655

Ser Val Leu Ser Glu Glu Ala Val Leu Ala Phe Glu Tyr Gly Tyr Ala
            660                 665                 670

Thr Ala Glu Pro Arg Thr Leu Thr Ile Trp Glu Ala Gln Phe Gly Asp
        675                 680                 685

Phe Ala Asn Gly Ala Gln Val Val Ile Asp Gln Phe Ile Ser Ser Gly
    690                 695                 700

Glu Gln Lys Trp Gly Arg Met Cys Gly Leu Val Met Leu Leu Pro His
705                 710                 715                 720

Gly Tyr Glu Gly Gln Gly Pro Glu His Ser Ser Ala Arg Leu Glu Arg
                725                 730                 735

Tyr Leu Gln Leu Cys Ala Glu Gln Asn Met Gln Val Cys Val Pro Ser
            740                 745                 750

Thr Pro Ala Gln Val Tyr His Met Leu Arg Arg Gln Ala Leu Arg Gly
        755                 760                 765

Met Arg Arg Pro Leu Val Val Met Ser Pro Lys Ser Leu Leu Arg His
    770                 775                 780

Pro Leu Ala Val Ser Ser Leu Glu Glu Leu Ala Asn Gly Thr Phe Leu
785                 790                 795                 800

Pro Ala Ile Gly Glu Ile Asp Glu Leu Asp Pro Lys Gly Val Lys Arg
                805                 810                 815

Val Val Met Cys Ser Gly Lys Val Tyr Tyr Asp Leu Leu Glu Gln Arg
            820                 825                 830

Arg Lys Asn Asn Gln His Asp Val Ala Ile Val Arg Ile Glu Gln Leu
        835                 840                 845

Tyr Pro Phe Pro His Lys Ala Met Gln Glu Val Leu Gln Gln Phe Ala
    850                 855                 860

His Val Lys Asp Phe Val Trp Cys Gln Glu Glu Pro Leu Asn Gln Gly
865                 870                 875                 880

Ala Trp Tyr Cys Ser Gln His His Phe Arg Glu Val Ile Pro Phe Gly
                885                 890                 895

Ala Ser Leu Arg Tyr Ala Gly Arg Pro Ala Ser Ala Ser Pro Ala Val
            900                 905                 910

Gly Tyr Met Ser Val His Gln Lys Gln Gln Gln Asp Leu Val Asn Asp
        915                 920                 925

Ala Leu Asn Val Glu
    930

<210>  37
<211>  1503
<212>  DNA
<213>  Escherichia coli

<400>  37
tcgaataaat aaaggataca caatgagtag cgtagatatt ctggtccctg acctgcctga      60

atccgtagcc gatgccaccg tcgcaacctg gcataaaaaa cccggcgacg cagtcgtacg     120

tgatgaagtg ctggtagaaa tcgaaactga caaagtggta ctggaagtac cggcatcagc     180

agacggcatt ctggatgcgg ttctggaaga tgaaggtaca acggtaacgt ctcgtcagat     240

ccttggtcgc ctgcgtgaag gcaacagcgc cggtaaagaa accagcgcca aatctgaaga     300

gaaagcgtcc actccggcgc aacgccagca ggcgtctctg gaagagcaaa acaacgatgc     360

gttaagcccg gcgatccgtc gcctgctggc tgaacacaat ctcgacgcca gcgccattaa 420

aggcaccggt gtgggtggtc gtctgactcg tgaagatgtg gaaaaacatc tggcgaaagc 480

cccggcgaaa gagtctgctc cggcagcggc tgctccggcg gcgcaaccgg ctctggctgc 540

acgtagtgaa aaacgtgtcc cgatgactcg cctgcgtaag cgtgtggcag agcgtctgct 600

ggaagcgaaa aactccaccg ccatgctgac cacgttcaac gaagtcaaca tgaagccgat 660

tatggatctg cgtaagcagt acggtgaagc gtttgaaaaa cgccacggca tccgtctggg 720

ctttatgtcc ttctacgtga aagcggtggt tgaagccctg aaacgttacc cggaagtgaa 780

cgcttctatc gacggcgatg acgtggttta ccacaactat ttcgacgtca gcatggcggt 840

ttctacgccg cgcggcctgg tgacgccggt tctgcgtgat gtcgataccc tcggcatggc 900

agacatcgag aagaaaatca aagagctggc agtcaaaggc cgtgacggca agctgaccgt 960

tgaagatctg accggtggta acttcaccat caccaacggt ggtgtgttcg gttccctgat 1020

gtctacgccg atcatcaacc cgccgcagag cgcaattctg ggtatgcacg ctatcaaaga 1080

tcgtccgatg gcggtgaatg gtcaggttga gatcctgccg atgatgtacc tggcgctgtc 1140

ctacgatcac cgtctgatcg atggtcgcga atccgtgggc ttcctggtaa cgatcaaaga 1200

gttgctggaa gatccgacgc gtctgctgct ggacgtgtag tagtttaagt ttcacctgca 1260

ctgtagaccg gataaggcat tatcgccttc tccggcaatt gaagcctgat gcgacgctga 1320

cgcgtcttat caggcctacg ggaccaccaa tgtaggtcgg ataaggcgca acgccgcatc 1380

cgacaagcga tgcctgatgt gacgtttaac gtgtcttatc aggcctacgg gtgaccgaca 1440

atgcccggaa gcgatacgaa atattcggtc tacggtttaa aagataacga ttactgaagg 1500

atg 1503

<210> 38
<211> 405
<212> PRT
<213> Escherichia coli

<400> 38

Met Ser Ser Val Asp Ile Leu Val Pro Asp Leu Pro Glu Ser Val Ala
1               5                   10                  15

Asp Ala Thr Val Ala Thr Trp His Lys Lys Pro Gly Asp Ala Val Val
            20                  25                  30

Arg Asp Glu Val Leu Val Glu Ile Glu Thr Asp Lys Val Val Leu Glu
        35                  40                  45

Val Pro Ala Ser Ala Asp Gly Ile Leu Asp Ala Val Leu Glu Asp Glu
    50                  55                  60

Gly Thr Thr Val Thr Ser Arg Gln Ile Leu Gly Arg Leu Arg Glu Gly
65                  70                  75                  80

Asn Ser Ala Gly Lys Glu Thr Ser Ala Lys Ser Glu Glu Lys Ala Ser
                85                  90                  95

Thr Pro Ala Gln Arg Gln Gln Ala Ser Leu Glu Glu Gln Asn Asn Asp
            100                 105                 110

Ala Leu Ser Pro Ala Ile Arg Arg Leu Leu Ala Glu His Asn Leu Asp
        115                 120                 125

Ala Ser Ala Ile Lys Gly Thr Gly Val Gly Gly Arg Leu Thr Arg Glu
    130                 135                 140

Asp Val Glu Lys His Leu Ala Lys Ala Pro Ala Lys Glu Ser Ala Pro
145                 150                 155                 160

Ala Ala Ala Ala Pro Ala Ala Gln Pro Ala Leu Ala Ala Arg Ser Glu
                165                 170                 175

Lys Arg Val Pro Met Thr Arg Leu Arg Lys Arg Val Ala Glu Arg Leu
            180                 185                 190

Leu Glu Ala Lys Asn Ser Thr Ala Met Leu Thr Thr Phe Asn Glu Val
        195                 200                 205

Asn Met Lys Pro Ile Met Asp Leu Arg Lys Gln Tyr Gly Glu Ala Phe
    210                 215                 220

Glu Lys Arg His Gly Ile Arg Leu Gly Phe Met Ser Phe Tyr Val Lys
225                 230                 235                 240

Ala Val Val Glu Ala Leu Lys Arg Tyr Pro Glu Val Asn Ala Ser Ile
                245                 250                 255

Asp Gly Asp Asp Val Val Tyr His Asn Tyr Phe Asp Val Ser Met Ala
            260                 265                 270

Val Ser Thr Pro Arg Gly Leu Val Thr Pro Val Leu Arg Asp Val Asp
        275                 280                 285

Thr Leu Gly Met Ala Asp Ile Glu Lys Lys Ile Lys Glu Leu Ala Val
    290                 295                 300

Lys Gly Arg Asp Gly Lys Leu Thr Val Glu Asp Leu Thr Gly Gly Asn
305                 310                 315                 320

Phe Thr Ile Thr Asn Gly Gly Val Phe Gly Ser Leu Met Ser Thr Pro
                325                 330                 335

Ile Ile Asn Pro Pro Gln Ser Ala Ile Leu Gly Met His Ala Ile Lys
            340                 345                 350

Asp Arg Pro Met Ala Val Asn Gly Gln Val Glu Ile Leu Pro Met Met
        355                 360                 365

Tyr Leu Ala Leu Ser Tyr Asp His Arg Leu Ile Asp Gly Arg Glu Ser
    370                 375                 380

Val Gly Phe Leu Val Thr Ile Lys Glu Leu Leu Glu Asp Pro Thr Arg
385                 390                 395                 400

Leu Leu Leu Asp Val
                405

<210>  39
<211>  1425
<212>  DNA
<213>  Escherichia coli

<400>  39
atgagtactg aaatcaaaac tcaggtcgtg gtacttgggg caggccccgc aggttactcc      60

gctgccttcc gttgcgctga tttaggtctg gaaaccgtaa tcgtagaacg ttacaacacc     120

cttggcggtg tttgcctgaa cgtcggctgt atcccttcta aagcactgct gcacgtagca     180

aaagttatcg aagaagccaa agcgctggct gaacacggta tcgtcttcgg cgaaccgaaa     240

accgatatcg acaagattcg tacctggaaa gagaaagtga tcaatcagct gaccggtggt     300

ctggctggta tggcgaaagg ccgcaaagtc aaagtggtca acggtctggg taaattcacc     360

ggggctaaca ccctggaagt tgaaggtgag aacggcaaaa ccgtgatcaa cttcgacaac     420

gcgatcattg cagcgggttc tcgcccgatc caactgccgt ttattccgca tgaagatccg     480

cgtatctggg actccactga cgcgctggaa ctgaaagaag taccagaacg cctgctggta     540

atgggtggcg gtatcatcgg tctggaaatg ggcaccgttt accacgcgct gggttcacag     600

attgacgtgg ttgaaatgtt cgaccaggtt atcccggcag ctgacaaaga catcgttaaa     660

gtcttcacca agcgtatcag caagaaattc aacctgatgc tggaaaccaa agttaccgcc     720

gttgaagcga aagaagacgg catttatgtg acgatggaag gcaaaaaagc acccgctgaa     780

ccgcagcgtt acgacgccgt gctggtagcg attggtcgtg tgccgaacgg taaaaacctc     840

gacgcaggca aagcaggcgt ggaagttgac gaccgtggtt tcatccgcgt tgacaaacag     900

ctgcgtacca acgtaccgca catctttgct atcggcgata tcgtcggtca accgatgctg     960

gcacacaaag gtgttcacga aggtcacgtt gccgctgaag ttatcgccgg taagaaacac     1020

tacttcgatc cgaaagttat cccgtccatc gcctataccg aaccagaagt tgcatgggtg     1080

ggtctgactg agaaagaagc gaaagagaaa ggcatcagct atgaaaccgc caccttcccg     1140

tgggctgctt ctggtcgtgc tatcgcttcc gactgcgcag acggtatgac caagctgatt     1200

ttcgacaaag aatctcaccg tgtgatcggt ggtgcgattg tcggtactaa cggcggcgag     1260

ctgctgggtg aaatcggcct ggcaatcgaa atgggttgtg atgctgaaga catcgcactg     1320

accatccacg cgcacccgac tctgcacgag tctgtgggcc tggcggcaga agtgttcgaa     1380

ggtagcatta ccgacctgcc gaacccgaaa gcgaagaaga agtaa                     1425

<210>  40
<211>  474
<212>  PRT
<213>  Escherichia coli

<400>  40

<210>  41
<211>  1770
<212>  DNA
<213>  Ralstonia eutropha

<400>  41
atggcgaccg gcaaaggcgc ggcagcttcc acgcaggaag gcaagtccca accattcaag      60

gtcacgccgg ggccattcga tccagccaca tggctggaat ggtcccgcca gtggcagggc     120

actgaaggca acggccacgc ggccgcgtcc ggcattccgg gcctggatgc gctggcaggc     180

gtcaagatcg cgccggcgca gctgggtgat atccagcagc gctacatgaa ggacttctca     240

gcgctgtggc aggccatggc cgagggcaag gccgaggcca ccggtccgct gcacgaccgg     300

cgcttcgccg gcgacgcatg gcgcaccaac ctcccatatc gcttcgctgc cgcgttctac     360

ctgctcaatg cgcgcgcctt gaccgagctg gccgatgccg tcgaggccga tgccaagacc     420

cgccagcgca tccgcttcgc gatctcgcaa tgggtcgatg cgatgtcgcc cgccaacttc     480

cttgccacca atcccgaggc gcagcgcctg ctgatcgagt cgggcggcga atcgctgcgt     540

gccggcgtgc gcaacatgat ggaagacctg acacgcggca agatctcgca gaccgacgag     600

agcgcgtttg aggtcggccg caatgtcgcg gtgaccgaag gcgccgtggt cttcgagaac     660

gagtacttcc agctgttgca gtacaagccg ctgaccgaca aggtgcacgc gcgcccgctg     720

ctgatggtgc cgccgtgcat caacaagtac tacatcctgg acctgcagcc ggagagctcg     780

ctggtgcgcc atgtggtgga gcagggacat acggtgtttc tggtgtcgtg gcgcaatccg     840

gacgccagca tggccggcag cacctgggac gactacatcg agcacgcggc catccgcgcc     900

atcgaagtcg cgcgcgacat cagcggccag gacaagatca acgtgctcgg cttctgcgtg     960

ggcggcacca ttgtctcgac cgcgctggcg gtgctggccg cgcgcggcga gcacccggcc    1020

gccagcgtca cgctgctgac cacgctgctg gactttgccg acacgggcat cctcgacgtc    1080

tttgtcgacg agggccatgt gcagttgcgc gaggccacgc tgggcggcgg cgccggcgcg    1140

ccgtgcgcgc tgctgcgcgg ccttgagctg gccaatacct tctcgttctt gcgcccgaac 1200

gacctggtgt ggaactacgt ggtcgacaac tacctgaagg gcaacacgcc ggtgccgttc 1260

gacctgctgt tctggaacgg cgacgccacc aacctgccgg ggccgtggta ctgctggtac 1320

ctgcgccaca cctacctgca gaacgagctc aaggtaccgg gcaagctgac cgtgtgcggc 1380

gtgccggtgg acctggccag catcgacgtg ccgacctata tctacggctc gcgcgaagac 1440

catatcgtgc cgtggaccgc ggcctatgcc tcgaccgcgc tgctggcgaa caagctgcgc 1500

ttcgtgctgg gtgcgtcggg ccatatcgcc ggtgtgatca acccgccggc caagaacaag 1560

cgcagccact ggactaacga tgcgctgccg gagtcgccgc agcaatggct ggccggcgcc 1620

atcgagcatc acggcagctg gtggccggac tggaccgcat ggctggccgg gcaggccggc 1680

gcgaaacgcg ccgcgcccgc caactatggc aatgcgcgct atcgcgcaat cgaacccgcg 1740

cctgggcgat acgtcaaagc caaggcatga 1770

<210> 42
<211> 589
<212> PRT
<213> Ralstonia eutropha

<400> 42

Met Ala Thr Gly Lys Gly Ala Ala Ala Ser Thr Gln Glu Gly Lys Ser
1               5                   10                  15

Gln Pro Phe Lys Val Thr Pro Gly Pro Phe Asp Pro Ala Thr Trp Leu
            20                  25                  30

Glu Trp Ser Arg Gln Trp Gln Gly Thr Glu Gly Asn Gly His Ala Ala
        35                  40                  45

Ala Ser Gly Ile Pro Gly Leu Asp Ala Leu Ala Gly Val Lys Ile Ala
    50                  55                  60

Pro Ala Gln Leu Gly Asp Ile Gln Gln Arg Tyr Met Lys Asp Phe Ser
65                  70                  75                  80

Ala Leu Trp Gln Ala Met Ala Glu Gly Lys Ala Glu Ala Thr Gly Pro
                85                  90                  95

Leu His Asp Arg Arg Phe Ala Gly Asp Ala Trp Arg Thr Asn Leu Pro
            100                 105                 110

Tyr Arg Phe Ala Ala Ala Phe Tyr Leu Leu Asn Ala Arg Ala Leu Thr
        115                 120                 125

Glu Leu Ala Asp Ala Val Glu Ala Asp Ala Lys Thr Arg Gln Arg Ile

130                 135                 140

Arg Phe Ala Ile Ser Gln Trp Val Asp Ala Met Ser Pro Ala Asn Phe
145                 150                 155                 160

Leu Ala Thr Asn Pro Glu Ala Gln Arg Leu Leu Ile Glu Ser Gly Gly
                165                 170                 175

Glu Ser Leu Arg Ala Gly Val Arg Asn Met Met Glu Asp Leu Thr Arg
            180                 185                 190

Gly Lys Ile Ser Gln Thr Asp Glu Ser Ala Phe Glu Val Gly Arg Asn
        195                 200                 205

Val Ala Val Thr Glu Gly Ala Val Val Phe Glu Asn Glu Tyr Phe Gln
    210                 215                 220

Leu Leu Gln Tyr Lys Pro Leu Thr Asp Lys Val His Ala Arg Pro Leu
225                 230                 235                 240

Leu Met Val Pro Pro Cys Ile Asn Lys Tyr Tyr Ile Leu Asp Leu Gln
                245                 250                 255

Pro Glu Ser Ser Leu Val Arg His Val Val Glu Gln Gly His Thr Val
            260                 265                 270

Phe Leu Val Ser Trp Arg Asn Pro Asp Ala Ser Met Ala Gly Ser Thr
        275                 280                 285

Trp Asp Asp Tyr Ile Glu His Ala Ala Ile Arg Ala Ile Glu Val Ala
    290                 295                 300

Arg Asp Ile Ser Gly Gln Asp Lys Ile Asn Val Leu Gly Phe Cys Val
305                 310                 315                 320

Gly Gly Thr Ile Val Ser Thr Ala Leu Ala Val Leu Ala Ala Arg Gly
                325                 330                 335

Glu His Pro Ala Ala Ser Val Thr Leu Leu Thr Thr Leu Leu Asp Phe
            340                 345                 350

Ala Asp Thr Gly Ile Leu Asp Val Phe Val Asp Glu Gly His Val Gln
        355                 360                 365

Leu Arg Glu Ala Thr Leu Gly Gly Gly Ala Gly Ala Pro Cys Ala Leu
    370                 375                 380

Leu Arg Gly Leu Glu Leu Ala Asn Thr Phe Ser Phe Leu Arg Pro Asn
385                 390                 395                 400

Asp Leu Val Trp Asn Tyr Val Val Asp Asn Tyr Leu Lys Gly Asn Thr
                405                 410                 415

Pro Val Pro Phe Asp Leu Leu Phe Trp Asn Gly Asp Ala Thr Asn Leu
            420                 425                 430

Pro Gly Pro Trp Tyr Cys Trp Tyr Leu Arg His Thr Tyr Leu Gln Asn
        435                 440                 445

Glu Leu Lys Val Pro Gly Lys Leu Thr Val Cys Gly Val Pro Val Asp
    450                 455                 460

Leu Ala Ser Ile Asp Val Pro Thr Tyr Ile Tyr Gly Ser Arg Glu Asp
465                 470                 475                 480

His Ile Val Pro Trp Thr Ala Ala Tyr Ala Ser Thr Ala Leu Leu Ala
                485                 490                 495

Asn Lys Leu Arg Phe Val Leu Gly Ala Ser Gly His Ile Ala Gly Val
            500                 505                 510

Ile Asn Pro Pro Ala Lys Asn Lys Arg Ser His Trp Thr Asn Asp Ala
        515                 520                 525

Leu Pro Glu Ser Pro Gln Gln Trp Leu Ala Gly Ala Ile Glu His His
    530                 535                 540

Gly Ser Trp Trp Pro Asp Trp Thr Ala Trp Leu Ala Gly Gln Ala Gly
545                 550                 555                 560

Ala Lys Arg Ala Ala Pro Ala Asn Tyr Gly Asn Ala Arg Tyr Arg Ala
                565                 570                 575

Ile Glu Pro Ala Pro Gly Arg Tyr Val Lys Ala Lys Ala
            580                 585

<210>  43
<211>  642
<212>  DNA
<213>  Escherichia coli

<400>  43
atgaaaaact ggaaaacaag tgcagaatca atcctgacca ccggcccggt tgtaccggtt      60

atcgtggtaa aaaaactgga acacgcggtg ccgatggcaa aagcgttggt tgctggtggg     120

gtgcgcgttc tggaagtgac tctgcgtacc gagtgtgcag ttgacgctat ccgtgctatc     180

gccaaagaag tgcctgaagc gattgtgggt gccggtacgg tgctgaatcc acagcagctg 240

gcagaagtca ctgaagcggg tgcacagttc gcaattagcc cgggtctgac cgagccgctg 300

ctgaaagctg ctaccgaagg gactattcct ctgattccgg ggatcagcac tgtttccgaa 360

ctgatgctgg gtatggacta cggtttgaaa gagttcaaat tcttcccggc tgaagctaac 420

ggcggcgtga aagccctgca ggcgatcgcg ggtccgttct cccaggtccg tttctgcccg 480

acgggtggta tttctccggc taactaccgt gactacctgg cgctgaaaag cgtgctgtgc 540

atcggtggtt cctggctggt tccggcagat gcgctggaag cgggcgatta cgaccgcatt 600

actaagctgg cgcgtgaagc tgtagaaggc gctaagctgt aa 642

<210> 44
<211> 213
<212> PRT
<213> Escherichia coli

<400> 44

Met Lys Asn Trp Lys Thr Ser Ala Glu Ser Ile Leu Thr Thr Gly Pro
1               5                   10                  15

Val Val Pro Val Ile Val Val Lys Lys Leu Glu His Ala Val Pro Met
            20                  25                  30

Ala Lys Ala Leu Val Ala Gly Gly Val Arg Val Leu Glu Val Thr Leu
        35                  40                  45

Arg Thr Glu Cys Ala Val Asp Ala Ile Arg Ala Ile Ala Lys Glu Val
    50                  55                  60

Pro Glu Ala Ile Val Gly Ala Gly Thr Val Leu Asn Pro Gln Gln Leu
65                  70                  75                  80

Ala Glu Val Thr Glu Ala Gly Ala Gln Phe Ala Ile Ser Pro Gly Leu
                85                  90                  95

Thr Glu Pro Leu Leu Lys Ala Ala Thr Glu Gly Thr Ile Pro Leu Ile
            100                 105                 110

Pro Gly Ile Ser Thr Val Ser Glu Leu Met Leu Gly Met Asp Tyr Gly
        115                 120                 125

Leu Lys Glu Phe Lys Phe Phe Pro Ala Glu Ala Asn Gly Gly Val Lys
    130                 135                 140

Ala Leu Gln Ala Ile Ala Gly Pro Phe Ser Gln Val Arg Phe Cys Pro
145                 150                 155                 160

Thr Gly Gly Ile Ser Pro Ala Asn Tyr Arg Asp Tyr Leu Ala Leu Lys
                165                 170                 175

Ser Val Leu Cys Ile Gly Gly Ser Trp Leu Val Pro Ala Asp Ala Leu
            180                 185                 190

Glu Ala Gly Asp Tyr Asp Arg Ile Thr Lys Leu Ala Arg Glu Ala Val
        195                 200                 205

Glu Gly Ala Lys Leu
    210

<210>  45
<211>  1575
<212>  DNA
<213>  Clostridium propionicum

<400>  45
atgagaaagg ttcccattat taccgcagat gaggctgcaa agcttattaa agacggtgat      60

acagttacaa caagtggttt cgttggaaat gcaatccctg aggctcttga tagagctgta     120

gaaaaaagat tcttagaaac aggcgaaccc aaaaacatta catatgttta ttgtggttct     180

caaggtaaca gagacggaag aggtgctgag cactttgctc atgaaggcct tttaaaacgt     240

tacatcgctg gtcactgggc tacagttcct gctttgggta aaatggctat ggaaaataaa     300

atggaagcat ataatgtatc tcagggtgca ttgtgtcatt tgttccgtga tatagcttct     360

cataagccag gcgtatttac aaaggtaggt atcggtactt tcattgaccc cagaaatggc     420

ggcggtaaag taaatgatat taccaaagaa gatattgttg aattggtaga gattaagggt     480

caggaatatt tattctaccc tgcttttcct attcatgtag ctcttattcg tggtacttac     540

gctgatgaaa gcggaaatat cacatttgag aaagaagttg ctcctctgga aggaacttca     600

gtatgccagg ctgttaaaaa cagtggcggt atcgttgtag ttcaggttga aagagtagta     660

aaagctggta ctcttgaccc tcgtcatgta aaagttccag gaatttatgt tgactatgtt     720

gttgttgctg acccagaaga tcatcagcaa tctttagatt gtgaatatga tcctgcatta     780

tcaggcgagc atagaagacc tgaagttgtt ggagaaccac ttcctttgag tgcaaagaaa     840

gttattggtc gtcgtggtgc cattgaatta gaaaaagatg ttgctgtaaa tttaggtgtt     900

ggtgcgcctg aatatgtagc aagtgttgct gatgaagaag gtatcgttga ttttatgact     960

ttaactgctg aaagtggtgc tattggtggt gttcctgctg gtggcgttcg ctttggtgct    1020

tcttataatg cggatgcatt gatcgatcaa ggttatcaat tcgattacta tgatggcggc    1080

ggcttagacc tttgctattt aggcttagct gaatgcgatg aaaaaggcaa tatcaacgtt    1140

tcaagatttg gccctcgtat cgctggttgt ggtggtttca tcaacattac acagaataca    1200

cctaaggtat tcttctgtgg tactttcaca gcaggtggct taaaggttaa aattgaagat 1260

ggcaaggtta ttattgttca agaaggcaag cagaaaaaat tcttgaaagc tgttgagcag 1320

attacattca atggtgacgt tgcacttgct aataagcaac aagtaactta tattacagaa 1380

agatgcgtat tccttttgaa ggaagatggt ttgcacttat ctgaaattgc acctggtatt 1440

gatttgcaga cacagattct tgacgttatg gattttgcac ctattattga cagagatgca 1500

aacggccaaa tcaaattgat ggacgctgct ttgtttgcag aaggcttaat gggtctgaag 1560

gaaatgaagt cctga 1575

<210> 46
<211> 524
<212> PRT
<213> Clostridium propionicum

<400> 46

Met Arg Lys Val Pro Ile Ile Thr Ala Asp Glu Ala Ala Lys Leu Ile
1               5                   10                  15

Lys Asp Gly Asp Thr Val Thr Thr Ser Gly Phe Val Gly Asn Ala Ile
            20                  25                  30

Pro Glu Ala Leu Asp Arg Ala Val Glu Lys Arg Phe Leu Glu Thr Gly
        35                  40                  45

Glu Pro Lys Asn Ile Thr Tyr Val Tyr Cys Gly Ser Gln Gly Asn Arg
    50                  55                  60

Asp Gly Arg Gly Ala Glu His Phe Ala His Glu Gly Leu Leu Lys Arg
65                  70                  75                  80

Tyr Ile Ala Gly His Trp Ala Thr Val Pro Ala Leu Gly Lys Met Ala
                85                  90                  95

Met Glu Asn Lys Met Glu Ala Tyr Asn Val Ser Gln Gly Ala Leu Cys
            100                 105                 110

His Leu Phe Arg Asp Ile Ala Ser His Lys Pro Gly Val Phe Thr Lys
        115                 120                 125

Val Gly Ile Gly Thr Phe Ile Asp Pro Arg Asn Gly Gly Gly Lys Val
    130                 135                 140

Asn Asp Ile Thr Lys Glu Asp Ile Val Glu Leu Val Glu Ile Lys Gly
145                 150                 155                 160

Gln Glu Tyr Leu Phe Tyr Pro Ala Phe Pro Ile His Val Ala Leu Ile
                165                 170                 175

Arg Gly Thr Tyr Ala Asp Glu Ser Gly Asn Ile Thr Phe Glu Lys Glu
            180                 185                 190

Val Ala Pro Leu Glu Gly Thr Ser Val Cys Gln Ala Val Lys Asn Ser
        195                 200                 205

Gly Gly Ile Val Val Val Gln Val Glu Arg Val Val Lys Ala Gly Thr
    210                 215                 220

Leu Asp Pro Arg His Val Lys Val Pro Gly Ile Tyr Val Asp Tyr Val
225                 230                 235                 240

Val Val Ala Asp Pro Glu Asp His Gln Gln Ser Leu Asp Cys Glu Tyr
                245                 250                 255

Asp Pro Ala Leu Ser Gly Glu His Arg Arg Pro Glu Val Val Gly Glu
            260                 265                 270

Pro Leu Pro Leu Ser Ala Lys Lys Val Ile Gly Arg Arg Gly Ala Ile
        275                 280                 285

Glu Leu Glu Lys Asp Val Ala Val Asn Leu Gly Val Gly Ala Pro Glu
    290                 295                 300

Tyr Val Ala Ser Val Ala Asp Glu Glu Gly Ile Val Asp Phe Met Thr
305                 310                 315                 320

Leu Thr Ala Glu Ser Gly Ala Ile Gly Gly Val Pro Ala Gly Gly Val
                325                 330                 335

Arg Phe Gly Ala Ser Tyr Asn Ala Asp Ala Leu Ile Asp Gln Gly Tyr
            340                 345                 350

Gln Phe Asp Tyr Tyr Asp Gly Gly Gly Leu Asp Leu Cys Tyr Leu Gly
        355                 360                 365

Leu Ala Glu Cys Asp Glu Lys Gly Asn Ile Asn Val Ser Arg Phe Gly
    370                 375                 380

Pro Arg Ile Ala Gly Cys Gly Gly Phe Ile Asn Ile Thr Gln Asn Thr
385                 390                 395                 400

Pro Lys Val Phe Phe Cys Gly Thr Phe Thr Ala Gly Gly Leu Lys Val
                405                 410                 415

Lys Ile Glu Asp Gly Lys Val Ile Ile Val Gln Glu Gly Lys Gln Lys
            420                 425                 430

Lys Phe Leu Lys Ala Val Glu Gln Ile Thr Phe Asn Gly Asp Val Ala
        435                 440                 445

Leu Ala Asn Lys Gln Gln Val Thr Tyr Ile Thr Glu Arg Cys Val Phe
    450                 455                 460

Leu Leu Lys Glu Asp Gly Leu His Leu Ser Glu Ile Ala Pro Gly Ile
465                 470                 475                 480

Asp Leu Gln Thr Gln Ile Leu Asp Val Met Asp Phe Ala Pro Ile Ile
                485                 490                 495

Asp Arg Asp Ala Asn Gly Gln Ile Lys Leu Met Asp Ala Ala Leu Phe
            500                 505                 510

Ala Glu Gly Leu Met Gly Leu Lys Glu Met Lys Ser
        515                 520

<210>  47
<211>  780
<212>  DNA
<213>  Metallosphaera sedula

<400>  47
atggaatttg aaacaataga aactaaaaaa gaaggaaact tgttctggat tacgttaaat      60

agacccgata aactaaacgc actaaacgct aaattacttg aggagttaga tagggcagtc     120

tctcaggcag agtctgaccc agagattagg gttatcatca ttacagggaa aggaaaggcc     180

ttctgcgcag gggctgacat aacccagttt aaccagttaa ccccagcaga agcctggaaa     240

ttctctaaga aaggaagaga gatcatggac aagatagagg cactgagcaa acccaccatt     300

gccatgatca atggatatgc ccttgggggt ggactagagc tagccttagc ctgtgatata     360

aggatcgcag cggaggaggc ccaactaggc cttccagaga taaacctagg gatatatccg     420

gggtatgggg ggactcagag gttaaccaga gttataggaa agggaagagc cctggagatg     480

atgatgacgg gcgatcgtat tcctggtaag gatgctgaga aatatggtct cgtgaatagg     540

gttgtccccc tagctaactt ggagcaagag acaaggaagc tggcagaaaa gatagccaag     600

aagtctccta tctctctcgc cttaatcaag gaagttgtaa acaggggact agactctccc     660

ctactgtcag gtctagcgtt ggaaagcgta ggatggggag tcgtgttttc tacggaggac     720

aagaaggagg gggtaagtgc cttcctggag aagagagagc ctacgtttaa gggaaaatag     780

<210>  48

<211>  259
<212>  PRT
<213>  Metallosphaera sedula

<400>  48

Met Glu Phe Glu Thr Ile Glu Thr Lys Lys Glu Gly Asn Leu Phe Trp
1               5                   10                  15

Ile Thr Leu Asn Arg Pro Asp Lys Leu Asn Ala Leu Asn Ala Lys Leu
            20                  25                  30

Leu Glu Glu Leu Asp Arg Ala Val Ser Gln Ala Glu Ser Asp Pro Glu
        35                  40                  45

Ile Arg Val Ile Ile Ile Thr Gly Lys Gly Lys Ala Phe Cys Ala Gly
    50                  55                  60

Ala Asp Ile Thr Gln Phe Asn Gln Leu Thr Pro Ala Glu Ala Trp Lys
65                  70                  75                  80

Phe Ser Lys Lys Gly Arg Glu Ile Met Asp Lys Ile Glu Ala Leu Ser
                85                  90                  95

Lys Pro Thr Ile Ala Met Ile Asn Gly Tyr Ala Leu Gly Gly Gly Leu
            100                 105                 110

Glu Leu Ala Leu Ala Cys Asp Ile Arg Ile Ala Ala Glu Glu Ala Gln
        115                 120                 125

Leu Gly Leu Pro Glu Ile Asn Leu Gly Ile Tyr Pro Gly Tyr Gly Gly
    130                 135                 140

Thr Gln Arg Leu Thr Arg Val Ile Gly Lys Gly Arg Ala Leu Glu Met
145                 150                 155                 160

Met Met Thr Gly Asp Arg Ile Pro Gly Lys Asp Ala Glu Lys Tyr Gly
                165                 170                 175

Leu Val Asn Arg Val Val Pro Leu Ala Asn Leu Glu Gln Glu Thr Arg
            180                 185                 190

Lys Leu Ala Glu Lys Ile Ala Lys Lys Ser Pro Ile Ser Leu Ala Leu
        195                 200                 205

Ile Lys Glu Val Val Asn Arg Gly Leu Asp Ser Pro Leu Leu Ser Gly
    210                 215                 220

Leu Ala Leu Glu Ser Val Gly Trp Gly Val Val Phe Ser Thr Glu Asp

225                 230                 235                 240

Lys Lys Glu Gly Val Ser Ala Phe Leu Glu Lys Arg Glu Pro Thr Phe
                245                 250                 255

Lys Gly Lys

<210>  49
<211>  2145
<212>  DNA
<213>  Escherichia coli

<400>  49
gtgtcccgta ttattatgct gatccctacc ggaaccagcg tcggtctgac cagcgtcagc      60

cttggcgtga tccgtgcaat ggaacgcaaa ggcgttcgtc tgagcgtttt caaacctatc     120

gctcagccgc gtaccggtgg cgatgcgccc gatcagacta cgactatcgt gcgtgcgaac     180

tcttccacca cgacggccgc tgaaccgctg aaaatgagct acgttgaagg tctgctttcc     240

agcaatcaga aagatgtgct gatggaagag atcgtcgcaa actaccacgc taacaccaaa     300

gacgctgaag tcgttctggt tgaaggtctg gtcccgacac gtaagcacca gtttgcccag     360

tctctgaact acgaaatcgc taaaacgctg aatgcggaaa tcgtcttcgt tatgtctcag     420

ggcactgaca ccccggaaca gctgaaagag cgtatcgaac tgacccgcaa cagcttcggc     480

ggtgccaaaa acaccaacat caccggcgtt atcgttaaca aactgaacgc accggttgat     540

gaacagggtc gtactcgccc ggatctgtcc gagattttcg acgactcttc caaagctaaa     600

gtaaacaatg ttgatccggc gaagctgcaa gaatccagcc cgctgccggt tctcggcgct     660

gtgccgtgga gctttgacct gatcgcgact cgtgcgatcg atatggctcg ccacctgaat     720

gcgaccatca tcaacgaagg cgacatcaat actcgccgcg ttaaatccgt cactttctgc     780

gcacgcagca ttccgcacat gctggagcac ttccgtgccg gttctctgct ggtgacttcc     840

gcagaccgtc ctgacgtgct ggtggccgct tgcctggcag ccatgaacgg cgtagaaatc     900

ggtgccctgc tgctgactgg cggttacgaa atggacgcgc gcatttctaa actgtgcgaa     960

cgtgctttcg ctaccggcct gccggtattt atggtgaaca ccaacacctg gcagacctct    1020

ctgagcctgc agagcttcaa cctggaagtt ccggttgacg atcacgaacg tatcgagaaa    1080

gttcaggaat acgttgctaa ctacatcaac gctgactgga tcgaatctct gactgccact    1140

tctgagcgca gccgtcgtct gtctccgcct gcgttccgtt atcagctgac tgaacttgcg    1200

cgcaaagcgg gcaaacgtat cgtactgccg gaaggtgacg aaccgcgtac cgttaaagca    1260

gccgctatct gtgctgaacg tggtatcgca acttgcgtac tgctgggtaa tccggcagag    1320

atcaaccgtg ttgcagcgtc tcagggtgta gaactgggtg cagggattga aatcgttgat    1380

ccagaagtgg ttcgcgaaag ctatgttggt cgtctggtcg aactgcgtaa gaacaaaggc 1440

atgaccgaaa ccgttgcccg cgaacagctg gaagacaacg tggtgctcgg tacgctgatg 1500

ctggaacagg atgaagttga tggtctggtt tccggtgctg ttcacactac cgcaaacacc 1560

atccgtccgc cgctgcagct gatcaaaact gcaccgggca gctccctggt atcttccgtg 1620

ttcttcatgc tgctgccgga acaggtttac gtttacggtg actgtgcgat caacccggat 1680

ccgaccgctg aacagctggc agaaatcgcg attcagtccg ctgattccgc tgcggccttc 1740

ggtatcgaac cgcgcgttgc tatgctctcc tactccaccg gtacttctgg tgcaggtagc 1800

gacgtagaaa aagttcgcga agcaactcgt ctggcgcagg aaaaacgtcc tgacctgatg 1860

atcgacggtc cgctgcagta cgacgctgcg gtaatggctg acgttgcgaa atccaaagcg 1920

ccgaactctc cggttgcagg tcgcgctacc gtgttcatct tcccggatct gaacaccggt 1980

aacaccacct acaaagcggt acagcgttct gccgacctga tctccatcgg gccgatgctg 2040

cagggtatgc gcaagccggt taacgacctg tcccgtggcg cactggttga cgatatcgtc 2100

tacaccatcg cgctgactgc gattcagtct gcacagcagc agtaa 2145

<210> 50
<211> 714
<212> PRT
<213> Escherichia coli

<400> 50

Val Ser Arg Ile Ile Met Leu Ile Pro Thr Gly Thr Ser Val Gly Leu
1               5                   10                  15

Thr Ser Val Ser Leu Gly Val Ile Arg Ala Met Glu Arg Lys Gly Val
            20                  25                  30

Arg Leu Ser Val Phe Lys Pro Ile Ala Gln Pro Arg Thr Gly Gly Asp
        35                  40                  45

Ala Pro Asp Gln Thr Thr Thr Ile Val Arg Ala Asn Ser Ser Thr Thr
    50                  55                  60

Thr Ala Ala Glu Pro Leu Lys Met Ser Tyr Val Glu Gly Leu Leu Ser
65                  70                  75                  80

Ser Asn Gln Lys Asp Val Leu Met Glu Glu Ile Val Ala Asn Tyr His
                85                  90                  95

Ala Asn Thr Lys Asp Ala Glu Val Val Leu Val Glu Gly Leu Val Pro
            100                 105                 110

Thr Arg Lys His Gln Phe Ala Gln Ser Leu Asn Tyr Glu Ile Ala Lys

115                 120                 125

Thr Leu Asn Ala Glu Ile Val Phe Val Met Ser Gln Gly Thr Asp Thr
    130                 135                 140

Pro Glu Gln Leu Lys Glu Arg Ile Glu Leu Thr Arg Asn Ser Phe Gly
145                 150                 155                 160

Gly Ala Lys Asn Thr Asn Ile Thr Gly Val Ile Val Asn Lys Leu Asn
                165                 170                 175

Ala Pro Val Asp Glu Gln Gly Arg Thr Arg Pro Asp Leu Ser Glu Ile
            180                 185                 190

Phe Asp Asp Ser Ser Lys Ala Lys Val Asn Asn Val Asp Pro Ala Lys
        195                 200                 205

Leu Gln Glu Ser Ser Pro Leu Pro Val Leu Gly Ala Val Pro Trp Ser
    210                 215                 220

Phe Asp Leu Ile Ala Thr Arg Ala Ile Asp Met Ala Arg His Leu Asn
225                 230                 235                 240

Ala Thr Ile Ile Asn Glu Gly Asp Ile Asn Thr Arg Arg Val Lys Ser
                245                 250                 255

Val Thr Phe Cys Ala Arg Ser Ile Pro His Met Leu Glu His Phe Arg
            260                 265                 270

Ala Gly Ser Leu Leu Val Thr Ser Ala Asp Arg Pro Asp Val Leu Val
        275                 280                 285

Ala Ala Cys Leu Ala Ala Met Asn Gly Val Glu Ile Gly Ala Leu Leu
    290                 295                 300

Leu Thr Gly Gly Tyr Glu Met Asp Ala Arg Ile Ser Lys Leu Cys Glu
305                 310                 315                 320

Arg Ala Phe Ala Thr Gly Leu Pro Val Phe Met Val Asn Thr Asn Thr
                325                 330                 335

Trp Gln Thr Ser Leu Ser Leu Gln Ser Phe Asn Leu Glu Val Pro Val
            340                 345                 350

Asp Asp His Glu Arg Ile Glu Lys Val Gln Glu Tyr Val Ala Asn Tyr
        355                 360                 365

Ile Asn Ala Asp Trp Ile Glu Ser Leu Thr Ala Thr Ser Glu Arg Ser
    370                 375                 380

Arg Arg Leu Ser Pro Pro Ala Phe Arg Tyr Gln Leu Thr Glu Leu Ala
385                 390                 395                 400

Arg Lys Ala Gly Lys Arg Ile Val Leu Pro Glu Gly Asp Glu Pro Arg
                405                 410                 415

Thr Val Lys Ala Ala Ala Ile Cys Ala Glu Arg Gly Ile Ala Thr Cys
            420                 425                 430

Val Leu Leu Gly Asn Pro Ala Glu Ile Asn Arg Val Ala Ala Ser Gln
        435                 440                 445

Gly Val Glu Leu Gly Ala Gly Ile Glu Ile Val Asp Pro Glu Val Val
    450                 455                 460

Arg Glu Ser Tyr Val Gly Arg Leu Val Glu Leu Arg Lys Asn Lys Gly
465                 470                 475                 480

Met Thr Glu Thr Val Ala Arg Glu Gln Leu Glu Asp Asn Val Val Leu
                485                 490                 495

Gly Thr Leu Met Leu Glu Gln Asp Glu Val Asp Gly Leu Val Ser Gly
            500                 505                 510

Ala Val His Thr Thr Ala Asn Thr Ile Arg Pro Pro Leu Gln Leu Ile
        515                 520                 525

Lys Thr Ala Pro Gly Ser Ser Leu Val Ser Ser Val Phe Phe Met Leu
    530                 535                 540

Leu Pro Glu Gln Val Tyr Val Tyr Gly Asp Cys Ala Ile Asn Pro Asp
545                 550                 555                 560

Pro Thr Ala Glu Gln Leu Ala Glu Ile Ala Ile Gln Ser Ala Asp Ser
                565                 570                 575

Ala Ala Ala Phe Gly Ile Glu Pro Arg Val Ala Met Leu Ser Tyr Ser
            580                 585                 590

Thr Gly Thr Ser Gly Ala Gly Ser Asp Val Glu Lys Val Arg Glu Ala
        595                 600                 605

Thr Arg Leu Ala Gln Glu Lys Arg Pro Asp Leu Met Ile Asp Gly Pro
    610                 615                 620

Leu Gln Tyr Asp Ala Ala Val Met Ala Asp Val Ala Lys Ser Lys Ala
625                 630                 635                 640

Pro Asn Ser Pro Val Ala Gly Arg Ala Thr Val Phe Ile Phe Pro Asp
                645                 650                 655

Leu Asn Thr Gly Asn Thr Thr Tyr Lys Ala Val Gln Arg Ser Ala Asp
            660                 665                 670

Leu Ile Ser Ile Gly Pro Met Leu Gln Gly Met Arg Lys Pro Val Asn
        675                 680                 685

Asp Leu Ser Arg Gly Ala Leu Val Asp Asp Ile Val Tyr Thr Ile Ala
    690                 695                 700

Leu Thr Ala Ile Gln Ser Ala Gln Gln Gln
705                 710

<210>  51
<211>  1209
<212>  DNA
<213>  Escherichia coli

<400>  51
atgaatgaat ttccggttgt tttggttatt aactgtggtt cgtcttcgat taagttttcc      60

gtgctcgatg ccagcgactg tgaagtatta atgtcaggta ttgccgacgg tattaactcg     120

gaaaatgcat tcttatccgt aaatggggga gagccagcac cgctggctca ccacagctac     180

gaaggtgcat tgaaggcaat tgcatttgaa ctggaaaaac ggaatttaaa tgacagtgtg     240

gccttaattg gccaccgcat cgctcacggc ggcagtattt ttaccgagtc cgccattatt     300

accgatgaag tcattgataa tatccgtcgc gtttctccac tggcacccct gcataattac     360

gccaatttaa gtggtattga atcggcgcag caattatttc cgggcgtaac tcaggtggcg     420

gtatttgata ccagtttcca ccagacgatg gctccggaag cttatttata cggcctgccg     480

tggaaatatt atgaagagtt aggtgtacgc cgttatggtt tccacggcac gtcgcaccgc     540

tatgtttccc agcgcgcaca ttcgctgctg aatctggcgg aagatgactc cggcctggtt     600

gtggcgcatc ttggcaatgg cgcgtcaatc tgcgcggttc gcaacggtca gagtgttgat     660

acctcaatgg gaatgacgcc gctggaaggc ttgatgatgg gtacccgcag tggcgatgtc     720

gactttggtg cgatgtcctg ggtcgccagc caaaccaacc agagcctggg tgacctggaa     780

cgcgtagtga ataaagagtc gggattatta ggtatttccg gtctttcttc ggatttacgt     840

gttctggaaa aagcctggca tgaaggtcac gaacgcgcgc aactggcaat taaaaccttt     900

gttcaccgaa ttgcccgtca tattgccgga cacgcagctt cattacgtcg cctggatgga     960

attatattca ccggcggaat aggagagaat tcaagcttaa ttcgtcgtct ggtcatggaa 1020

catttggctg tattaggctt agagattgat acagaaatga ataatcgctc taactcctgt 1080

ggtgagcgaa ttgtttccag tgaaaatgcg cgtgtcattt gtgccgttat tccgactaac 1140

gaagaaaaaa tgattgcttt ggatgccatt catttaggca aagttaacgc gcccgcagaa 1200

tttgcataa 1209

<210> 52
<211> 402
<212> PRT
<213> Escherichia coli

<400> 52

Met Asn Glu Phe Pro Val Val Leu Val Ile Asn Cys Gly Ser Ser Ser
1               5                   10                  15

Ile Lys Phe Ser Val Leu Asp Ala Ser Asp Cys Glu Val Leu Met Ser
            20                  25                  30

Gly Ile Ala Asp Gly Ile Asn Ser Glu Asn Ala Phe Leu Ser Val Asn
        35                  40                  45

Gly Gly Glu Pro Ala Pro Leu Ala His His Ser Tyr Glu Gly Ala Leu
    50                  55                  60

Lys Ala Ile Ala Phe Glu Leu Glu Lys Arg Asn Leu Asn Asp Ser Val
65                  70                  75                  80

Ala Leu Ile Gly His Arg Ile Ala His Gly Gly Ser Ile Phe Thr Glu
                85                  90                  95

Ser Ala Ile Ile Thr Asp Glu Val Ile Asp Asn Ile Arg Arg Val Ser
            100                 105                 110

Pro Leu Ala Pro Leu His Asn Tyr Ala Asn Leu Ser Gly Ile Glu Ser
        115                 120                 125

Ala Gln Gln Leu Phe Pro Gly Val Thr Gln Val Ala Val Phe Asp Thr
    130                 135                 140

Ser Phe His Gln Thr Met Ala Pro Glu Ala Tyr Leu Tyr Gly Leu Pro
145                 150                 155                 160

Trp Lys Tyr Tyr Glu Glu Leu Gly Val Arg Arg Tyr Gly Phe His Gly
                165                 170                 175

Thr Ser His Arg Tyr Val Ser Gln Arg Ala His Ser Leu Leu Asn Leu

180                 185                 190

Ala Glu Asp Asp Ser Gly Leu Val Val Ala His Leu Gly Asn Gly Ala
        195                 200                 205

Ser Ile Cys Ala Val Arg Asn Gly Gln Ser Val Asp Thr Ser Met Gly
    210                 215                 220

Met Thr Pro Leu Glu Gly Leu Met Met Gly Thr Arg Ser Gly Asp Val
225                 230                 235                 240

Asp Phe Gly Ala Met Ser Trp Val Ala Ser Gln Thr Asn Gln Ser Leu
                245                 250                 255

Gly Asp Leu Glu Arg Val Val Asn Lys Glu Ser Gly Leu Leu Gly Ile
            260                 265                 270

Ser Gly Leu Ser Ser Asp Leu Arg Val Leu Glu Lys Ala Trp His Glu
        275                 280                 285

Gly His Glu Arg Ala Gln Leu Ala Ile Lys Thr Phe Val His Arg Ile
    290                 295                 300

Ala Arg His Ile Ala Gly His Ala Ala Ser Leu Arg Arg Leu Asp Gly
305                 310                 315                 320

Ile Ile Phe Thr Gly Gly Ile Gly Glu Asn Ser Ser Leu Ile Arg Arg
                325                 330                 335

Leu Val Met Glu His Leu Ala Val Leu Gly Leu Glu Ile Asp Thr Glu
            340                 345                 350

Met Asn Asn Arg Ser Asn Ser Cys Gly Glu Arg Ile Val Ser Ser Glu
        355                 360                 365

Asn Ala Arg Val Ile Cys Ala Val Ile Pro Thr Asn Glu Glu Lys Met
    370                 375                 380

Ile Ala Leu Asp Ala Ile His Leu Gly Lys Val Asn Ala Pro Ala Glu
385                 390                 395                 400

Phe Ala

<210>  53
<211>  1644
<212>  DNA
<213>  Lactococcus lactis

<400>  53
atgtatacag taggagatta cctgttagac cgattacacg agttgggaat tgaagaaatt      60

tttggagttc ctggtgacta taacttacaa tttttagatc aaattatttc acgcgaagat     120

atgaaatgga ttggaaatgc taatgaatta aatgcttctt atatggctga tggttatgct     180

cgtactaaaa aagctgccgc atttctcacc acatttggag tcggcgaatt gagtgcgatc     240

aatggactgg caggaagtta tgccgaaaat ttaccagtag tagaaattgt tggttcacca     300

acttcaaaag tacaaaatga cggaaaattt gtccatcata cactagcaga tggtgatttt     360

aaacacttta tgaagatgca tgaacctgtt acagcagcgc ggactttact gacagcagaa     420

aatgccacat atgaaattga ccgagtactt tctcaattac taaaagaaag aaaaccagtc     480

tatattaact taccagtcga tgttgctgca gcaaaagcag agaagcctgc attatcttta     540

gaaaaagaaa gctctacaac aaatacaact gaacaagtga ttttgagtaa gattgaagaa     600

agtttgaaaa atgcccaaaa accagtagtg attgcaggac acgaagtaat tagttttggt     660

ttagaaaaaa cggtaactca gtttgtttca gaaacaaaac taccgattac gacactaaat     720

tttggtaaaa gtgctgttga tgaatctttg ccctcatttt taggaatata taacggaaaa     780

ctttcagaaa tcagtcttaa aaattttgtg gagtccgcag actttatcct aatgcttgga     840

gtgaagctta cggactcctc aacaggtgca ttcacacatc atttagatga aaataaaatg     900

atttcactaa acatagatga aggaataatt ttcaataaag tggtagaaga ttttgatttt     960

agagcagtgg tttcttcttt atcagaatta aaaggaatag aatatgaagg acaatatatt    1020

gataagcaat atgaagaatt tattccatca agtgctccct tatcacaaga ccgtctatgg    1080

caggcagttg aaagtttgac tcaaagcaat gaaacaatcg ttgctgaaca aggaacctca    1140

ttttttggag cttcaacaat tttcttaaaa tcaaatagtc gttttattgg acaaccttta    1200

tggggttcta ttggatatac ttttccagcg gctttaggaa gccaaattgc ggataaagag    1260

agcagacacc ttttatttat tggtgatggt tcacttcaac ttaccgtaca agaattagga    1320

ctatcaatca gagaaaaact caatccaatt tgttttatca taaataatga tggttataca    1380

gttgaaagag aaatccacgg acctactcaa agttataacg acattccaat gtggaattac    1440

tcgaaattac cagaaacatt tggagcaaca gaagatcgtg tagtatcaaa aattgttaga    1500

acagagaatg aatttgtgtc tgtcatgaaa gaagcccaag cagatgtcaa tagaatgtat    1560

tggatagaac tagttttgga aaaagaagat gcgccaaaat tactgaaaaa aatgggtaaa    1620

ttatttgctg agcaaaataa atag                                           1644

<210>  54
<211>  547
<212>  PRT
<213>  Lactococcus lactis

<400>  54

Met Tyr Thr Val Gly Asp Tyr Leu Leu Asp Arg Leu His Glu Leu Gly
1               5                   10                  15

Ile Glu Glu Ile Phe Gly Val Pro Gly Asp Tyr Asn Leu Gln Phe Leu
            20                  25                  30

Asp Gln Ile Ile Ser Arg Glu Asp Met Lys Trp Ile Gly Asn Ala Asn
        35                  40                  45

Glu Leu Asn Ala Ser Tyr Met Ala Asp Gly Tyr Ala Arg Thr Lys Lys
    50                  55                  60

Ala Ala Ala Phe Leu Thr Thr Phe Gly Val Gly Glu Leu Ser Ala Ile
65                  70                  75                  80

Asn Gly Leu Ala Gly Ser Tyr Ala Glu Asn Leu Pro Val Val Glu Ile
                85                  90                  95

Val Gly Ser Pro Thr Ser Lys Val Gln Asn Asp Gly Lys Phe Val His
            100                 105                 110

His Thr Leu Ala Asp Gly Asp Phe Lys His Phe Met Lys Met His Glu
        115                 120                 125

Pro Val Thr Ala Ala Arg Thr Leu Leu Thr Ala Glu Asn Ala Thr Tyr
    130                 135                 140

Glu Ile Asp Arg Val Leu Ser Gln Leu Leu Lys Glu Arg Lys Pro Val
145                 150                 155                 160

Tyr Ile Asn Leu Pro Val Asp Val Ala Ala Ala Lys Ala Glu Lys Pro
                165                 170                 175

Ala Leu Ser Leu Glu Lys Glu Ser Ser Thr Thr Asn Thr Thr Glu Gln
            180                 185                 190

Val Ile Leu Ser Lys Ile Glu Glu Ser Leu Lys Asn Ala Gln Lys Pro
        195                 200                 205

Val Val Ile Ala Gly His Glu Val Ile Ser Phe Gly Leu Glu Lys Thr
    210                 215                 220

Val Thr Gln Phe Val Ser Glu Thr Lys Leu Pro Ile Thr Thr Leu Asn
225                 230                 235                 240

Phe Gly Lys Ser Ala Val Asp Glu Ser Leu Pro Ser Phe Leu Gly Ile
                245                 250                 255

Tyr Asn Gly Lys Leu Ser Glu Ile Ser Leu Lys Asn Phe Val Glu Ser
            260                 265                 270

Ala Asp Phe Ile Leu Met Leu Gly Val Lys Leu Thr Asp Ser Ser Thr
        275                 280                 285

Gly Ala Phe Thr His His Leu Asp Glu Asn Lys Met Ile Ser Leu Asn
    290                 295                 300

Ile Asp Glu Gly Ile Ile Phe Asn Lys Val Val Glu Asp Phe Asp Phe
305                 310                 315                 320

Arg Ala Val Val Ser Ser Leu Ser Glu Leu Lys Gly Ile Glu Tyr Glu
                325                 330                 335

Gly Gln Tyr Ile Asp Lys Gln Tyr Glu Glu Phe Ile Pro Ser Ser Ala
            340                 345                 350

Pro Leu Ser Gln Asp Arg Leu Trp Gln Ala Val Glu Ser Leu Thr Gln
        355                 360                 365

Ser Asn Glu Thr Ile Val Ala Glu Gln Gly Thr Ser Phe Phe Gly Ala
    370                 375                 380

Ser Thr Ile Phe Leu Lys Ser Asn Ser Arg Phe Ile Gly Gln Pro Leu
385                 390                 395                 400

Trp Gly Ser Ile Gly Tyr Thr Phe Pro Ala Ala Leu Gly Ser Gln Ile
                405                 410                 415

Ala Asp Lys Glu Ser Arg His Leu Leu Phe Ile Gly Asp Gly Ser Leu
            420                 425                 430

Gln Leu Thr Val Gln Glu Leu Gly Leu Ser Ile Arg Glu Lys Leu Asn
        435                 440                 445

Pro Ile Cys Phe Ile Ile Asn Asn Asp Gly Tyr Thr Val Glu Arg Glu
    450                 455                 460

Ile His Gly Pro Thr Gln Ser Tyr Asn Asp Ile Pro Met Trp Asn Tyr
465                 470                 475                 480

Ser Lys Leu Pro Glu Thr Phe Gly Ala Thr Glu Asp Arg Val Val Ser
                485                 490                 495

Lys Ile Val Arg Thr Glu Asn Glu Phe Val Ser Val Met Lys Glu Ala
            500                 505                 510

Gln Ala Asp Val Asn Arg Met Tyr Trp Ile Glu Leu Val Leu Glu Lys
        515                 520                 525

Glu Asp Ala Pro Lys Leu Leu Lys Lys Met Gly Lys Leu Phe Ala Glu
    530                 535                 540

Gln Asn Lys
545

<210>  55
<211>  1488
<212>  DNA
<213>  Escherichia coli

<400>  55
atgaattttc atcatctggc ttactggcag gataaagcgt taagtctcgc cattgaaaac      60

cgcttattta ttaacggtga atatactgct gcggcggaaa atgaaacctt tgaaaccgtt     120

gatccggtca cccaggcacc gctggcgaaa attgcccgcg gcaagagcgt cgatatcgac     180

cgtgcgatga gcgcagcacg cggcgtattt gaacgcggcg actggtcact ctcttctccg     240

gctaaacgta aagcggtact gaataaactc gccgatttaa tggaagccca cgccgaagag     300

ctggcactgc tggaaactct cgacaccggc aaaccgattc gtcacagtct gcgtgatgat     360

attcccggcg cggcgcgcgc cattcgctgg tacgccgaag cgatcgacaa agtgtatggc     420

gaagtggcga ccaccagtag ccatgagctg gcgatgatcg tgcgtgaacc ggtcggcgtg     480

attgccgcca tcgtgccgtg gaacttcccg ctgttgctga cttgctggaa actcggcccg     540

gcgctggcgg cgggaaacag cgtgattcta aaaccgtctg aaaaatcacc gctcagtgcg     600

attcgtctcg cggggctggc gaaagaagca ggcttgccgg atggtgtgtt gaacgtggtg     660

acgggttttg gtcatgaagc cgggcaggcg ctgtcgcgtc ataacgatat cgacgccatt     720

gcctttaccg gttcaacccg taccgggaaa cagctgctga aagatgcggg cgacagcaac     780

atgaaacgcg tctggctgga agcgggcggc aaaagcgcca acatcgtttt cgctgactgc     840

ccggatttgc aacaggcggc aagcgccacc gcagcaggca ttttctacaa ccagggacag     900

gtgtgcatcg ccggaacgcg cctgttgctg gaagagagca tcgccgatga attcttagcc     960

ctgttaaaac agcaggcgca aaactggcaa ccgggccatc cacttgatcc cgcaaccacc    1020

atgggcacct taatcgactg cgcccacgcc gactcggtcc atagctttat tcgggaaggc    1080

gaaagcaaag ggcaactgtt gttggatggc cgtaacgccg ggctggctgc cgccatcggc    1140

ccgaccatct ttgtggatgt ggacccgaat gcgtccttaa gtcgcgaaga gattttcggt     1200

ccggtgctgg tggtcacgcg tttcacatca gaagaacagg cgctacagct tgccaacgac 1260

agccagtacg gccttggcgc ggcggtatgg acgcgcgacc tctcccgcgc gcaccgcatg 1320

agccgacgcc tgaaagccgg ttccgtcttc gtcaataact acaacgacgg cgatatgacc 1380

gtgccgtttg gcggctataa gcagagcggc aacggtcgcg acaaatccct gcatgccctt 1440

gaaaaattca ctgaactgaa aaccatctgg ataagcctgg aggcctga 1488

<210> 56
<211> 495
<212> PRT
<213> Escherichia coli

<400> 56

Met Asn Phe His His Leu Ala Tyr Trp Gln Asp Lys Ala Leu Ser Leu
1               5                   10                  15

Ala Ile Glu Asn Arg Leu Phe Ile Asn Gly Glu Tyr Thr Ala Ala Ala
            20                  25                  30

Glu Asn Glu Thr Phe Glu Thr Val Asp Pro Val Thr Gln Ala Pro Leu
        35                  40                  45

Ala Lys Ile Ala Arg Gly Lys Ser Val Asp Ile Asp Arg Ala Met Ser
    50                  55                  60

Ala Ala Arg Gly Val Phe Glu Arg Gly Asp Trp Ser Leu Ser Ser Pro
65                  70                  75                  80

Ala Lys Arg Lys Ala Val Leu Asn Lys Leu Ala Asp Leu Met Glu Ala
                85                  90                  95

His Ala Glu Glu Leu Ala Leu Leu Glu Thr Leu Asp Thr Gly Lys Pro
            100                 105                 110

Ile Arg His Ser Leu Arg Asp Asp Ile Pro Gly Ala Ala Arg Ala Ile
        115                 120                 125

Arg Trp Tyr Ala Glu Ala Ile Asp Lys Val Tyr Gly Glu Val Ala Thr
    130                 135                 140

Thr Ser Ser His Glu Leu Ala Met Ile Val Arg Glu Pro Val Gly Val
145                 150                 155                 160

Ile Ala Ala Ile Val Pro Trp Asn Phe Pro Leu Leu Leu Thr Cys Trp
                165                 170                 175

Lys Leu Gly Pro Ala Leu Ala Ala Gly Asn Ser Val Ile Leu Lys Pro

180                 185                 190

Ser Glu Lys Ser Pro Leu Ser Ala Ile Arg Leu Ala Gly Leu Ala Lys
        195                 200                 205

Glu Ala Gly Leu Pro Asp Gly Val Leu Asn Val Val Thr Gly Phe Gly
    210                 215                 220

His Glu Ala Gly Gln Ala Leu Ser Arg His Asn Asp Ile Asp Ala Ile
225                 230                 235                 240

Ala Phe Thr Gly Ser Thr Arg Thr Gly Lys Gln Leu Leu Lys Asp Ala
                245                 250                 255

Gly Asp Ser Asn Met Lys Arg Val Trp Leu Glu Ala Gly Gly Lys Ser
            260                 265                 270

Ala Asn Ile Val Phe Ala Asp Cys Pro Asp Leu Gln Gln Ala Ala Ser
        275                 280                 285

Ala Thr Ala Ala Gly Ile Phe Tyr Asn Gln Gly Gln Val Cys Ile Ala
    290                 295                 300

Gly Thr Arg Leu Leu Leu Glu Glu Ser Ile Ala Asp Glu Phe Leu Ala
305                 310                 315                 320

Leu Leu Lys Gln Gln Ala Gln Asn Trp Gln Pro Gly His Pro Leu Asp
                325                 330                 335

Pro Ala Thr Thr Met Gly Thr Leu Ile Asp Cys Ala His Ala Asp Ser
            340                 345                 350

Val His Ser Phe Ile Arg Glu Gly Glu Ser Lys Gly Gln Leu Leu Leu
        355                 360                 365

Asp Gly Arg Asn Ala Gly Leu Ala Ala Ala Ile Gly Pro Thr Ile Phe
    370                 375                 380

Val Asp Val Asp Pro Asn Ala Ser Leu Ser Arg Glu Glu Ile Phe Gly
385                 390                 395                 400

Pro Val Leu Val Val Thr Arg Phe Thr Ser Glu Glu Gln Ala Leu Gln
                405                 410                 415

Leu Ala Asn Asp Ser Gln Tyr Gly Leu Gly Ala Ala Val Trp Thr Arg
            420                 425                 430

Asp Leu Ser Arg Ala His Arg Met Ser Arg Arg Leu Lys Ala Gly Ser
        435                 440                 445

Val Phe Val Asn Asn Tyr Asn Asp Gly Asp Met Thr Val Pro Phe Gly
    450                 455                 460

Gly Tyr Lys Gln Ser Gly Asn Gly Arg Asp Lys Ser Leu His Ala Leu
465                 470                 475                 480

Glu Lys Phe Thr Glu Leu Lys Thr Ile Trp Ile Ser Leu Glu Ala
                485                 490                 495

<210>  57
<211>  1491
<212>  DNA
<213>  Klebsiella pneumoniae

<400>  57
atgatgaatt ttcagcacct ggcttactgg caggaaaaag cgaaaaacct ggccattgaa      60

acgcgcttat ttattaacgg cgaatattgc gccgcggccg ataataccac ctttgagact     120

atcgaccccg ccgcgcagca gacattagcc caggtcgccc gcggtaaaaa agccgacgtc     180

gaacgggcgg tgaaagccgc gcgccaggct tttgataacg gcgactggtc gcaggcctcc     240

cccgcacagc gtaaagcgat cctcactcgc tttgctaatc tgatggaggc ccatcgtgaa     300

gagctggcgc tgctggaaac gctggatacc ggcaagccga ttcgccacag cctgcgcgac     360

gatattcccg gcgccgcccg cgccattcgc tggtatgccg aagcgctgga taaagtctat     420

ggcgaagtgg cccccaccgg cagcaacgag ctggcgatga tcgttcgcga accaattggc     480

gtgatcgccg cggtggtgcc gtggaacttc ccgctgctgc tggcctgctg gaaactcggc     540

ccggcgctgg cggcaggcaa tagcgtaatc ctcaaaccct cggaaaaatc gccgcttacc     600

gccctgcgtc tggccgggct ggcgaaagag gccggcctgc cggacggcgt gttgaacgtg     660

gtcagcggct ttggccacga ggccgggcag gcgctggccc tgcatcctga tgttgaagtc     720

atcaccttca ccggctccac ccgcaccggc aagcagctgc tgaaagacgc cggcgacagc     780

aatatgaagc gcgtgtggct ggaagcgggc ggcaagagcg ccaacattgt cttcgccgat     840

tgcccggatc tgcaacaagc ggttcgcgcc accgccggcg gcatcttcta caaccaggga     900

caggtgtgca tcgccgggac ccgtctgctg ctcgaggaga gcatcgctga cgagttcctg     960

gcgcggctga aagctgaggc gcaacactgg cagccgggca acccgctcga tccggacacc    1020

accatgggca tgctgattga caatacccat gccgacaacg tgcatagctt tattcgcggc    1080

ggcgaaagcc aaagcaccct gttcctcgac ggacggaaaa acccgtggcc tgccgccgtt    1140

ggcccgacca ttttcgttga cgtcgacccg gcatcaaccc tcagccggga agagatcttc    1200

ggcccggtgc tggtggtgac ccgcttcaaa agcgaagaag aggcgctaaa gctcgccaat    1260

gacagcgact acggcttggg cgccgcggtg tggacccgcg atctctcccg cgcccaccgc     1320

atgagccgcc gcctgaaggc cggctcggtc ttcgtcaaca actataacga tggtgatatg     1380

accgttccgt tcggcggcta caagcagagc ggcaacgggc gcgataaatc gctgcacgcg     1440

ctggaaaaat tcaccgaact gaaaaccatc tggattgccc tggagtcttg a              1491

<210>  58
<211>  496
<212>  PRT
<213>  Klebsiella pneumoniae

<400>  58

Met Met Asn Phe Gln His Leu Ala Tyr Trp Gln Glu Lys Ala Lys Asn
1               5                   10                  15

Leu Ala Ile Glu Thr Arg Leu Phe Ile Asn Gly Glu Tyr Cys Ala Ala
            20                  25                  30

Ala Asp Asn Thr Thr Phe Glu Thr Ile Asp Pro Ala Ala Gln Gln Thr
        35                  40                  45

Leu Ala Gln Val Ala Arg Gly Lys Lys Ala Asp Val Glu Arg Ala Val
    50                  55                  60

Lys Ala Ala Arg Gln Ala Phe Asp Asn Gly Asp Trp Ser Gln Ala Ser
65                  70                  75                  80

Pro Ala Gln Arg Lys Ala Ile Leu Thr Arg Phe Ala Asn Leu Met Glu
                85                  90                  95

Ala His Arg Glu Glu Leu Ala Leu Leu Glu Thr Leu Asp Thr Gly Lys
            100                 105                 110

Pro Ile Arg His Ser Leu Arg Asp Asp Ile Pro Gly Ala Ala Arg Ala
        115                 120                 125

Ile Arg Trp Tyr Ala Glu Ala Leu Asp Lys Val Tyr Gly Glu Val Ala
    130                 135                 140

Pro Thr Gly Ser Asn Glu Leu Ala Met Ile Val Arg Glu Pro Ile Gly
145                 150                 155                 160

Val Ile Ala Ala Val Val Pro Trp Asn Phe Pro Leu Leu Leu Ala Cys
                165                 170                 175

Trp Lys Leu Gly Pro Ala Leu Ala Ala Gly Asn Ser Val Ile Leu Lys
            180                 185                 190

Pro Ser Glu Lys Ser Pro Leu Thr Ala Leu Arg Leu Ala Gly Leu Ala
        195                 200                 205

Lys Glu Ala Gly Leu Pro Asp Gly Val Leu Asn Val Val Ser Gly Phe
    210                 215                 220

Gly His Glu Ala Gly Gln Ala Leu Ala Leu His Pro Asp Val Glu Val
225                 230                 235                 240

Ile Thr Phe Thr Gly Ser Thr Arg Thr Gly Lys Gln Leu Leu Lys Asp
                245                 250                 255

Ala Gly Asp Ser Asn Met Lys Arg Val Trp Leu Glu Ala Gly Gly Lys
            260                 265                 270

Ser Ala Asn Ile Val Phe Ala Asp Cys Pro Asp Leu Gln Gln Ala Val
        275                 280                 285

Arg Ala Thr Ala Gly Gly Ile Phe Tyr Asn Gln Gly Gln Val Cys Ile
    290                 295                 300

Ala Gly Thr Arg Leu Leu Leu Glu Glu Ser Ile Ala Asp Glu Phe Leu
305                 310                 315                 320

Ala Arg Leu Lys Ala Glu Ala Gln His Trp Gln Pro Gly Asn Pro Leu
                325                 330                 335

Asp Pro Asp Thr Thr Met Gly Met Leu Ile Asp Asn Thr His Ala Asp
            340                 345                 350

Asn Val His Ser Phe Ile Arg Gly Gly Glu Ser Gln Ser Thr Leu Phe
        355                 360                 365

Leu Asp Gly Arg Lys Asn Pro Trp Pro Ala Ala Val Gly Pro Thr Ile
    370                 375                 380

Phe Val Asp Val Asp Pro Ala Ser Thr Leu Ser Arg Glu Glu Ile Phe
385                 390                 395                 400

Gly Pro Val Leu Val Val Thr Arg Phe Lys Ser Glu Glu Glu Ala Leu
                405                 410                 415

Lys Leu Ala Asn Asp Ser Asp Tyr Gly Leu Gly Ala Ala Val Trp Thr
            420                 425                 430

Arg Asp Leu Ser Arg Ala His Arg Met Ser Arg Arg Leu Lys Ala Gly

435                 440                 445

Ser Val Phe Val Asn Asn Tyr Asn Asp Gly Asp Met Thr Val Pro Phe
    450                 455                 460

Gly Gly Tyr Lys Gln Ser Gly Asn Gly Arg Asp Lys Ser Leu His Ala
465                 470                 475                 480

Leu Glu Lys Phe Thr Glu Leu Lys Thr Ile Trp Ile Ala Leu Glu Ser
                485                 490                 495

<210>  59
<211>  1395
<212>  DNA
<213>  Salmonella enterica

<400>  59
atgaatactt ctgaactcga aaccctgatt cgcaccattc ttagcgagca attaaccacg      60

ccggcgcaaa cgccggtcca gcctcagggc aaagggattt tccagtccgt gagcgaggcc     120

atcgacgccg cgcaccaggc gttcttacgt tatcagcagt gcccgctaaa aacccgcagc     180

gccattatca gcgcgatgcg tcaggagctg acgccgctgc tggcgcccct ggcggaagag     240

agcgccaatg aaacggggat gggcaacaaa gaagataaat ttctcaaaaa caaggctgcg     300

ctggacaaca cgccgggcgt agaagatctc accaccaccg cgctgaccgg cgacggcggc     360

atggtgctgt ttgaatactc accgtttggc gttatcggtt cggtcgcccc aagcaccaac     420

ccgacggaaa ccatcatcaa caacagtatc agcatgctgg cggcgggcaa cagtatctac     480

tttagcccgc atccgggagc gaaaaaggtc tctctgaagc tgattagcct gattgaagag     540

attgccttcc gctgctgcgg catccgcaat ctggtggtga ccgtggcgga acccaccttc     600

gaagcgaccc agcagatgat ggcccacccg cgaatcgcag tactggccat taccggcggc     660

ccgggcattg tggcaatggg catgaagagc ggtaagaagg tgattggcgc tggcgcgggt     720

aacccgccct gcatcgttga tgaaacggcg gacctggtga aagcggcgga agatatcatc     780

aacggcgcgt cattcgatta caacctgccc tgcattgccg agaagagcct gatcgtagtg     840

gagagtgtcg ccgaacgtct ggtgcagcaa atgcaaacct tcggcgcgct gctgttaagc     900

cctgccgata ccgacaaact ccgcgccgtc tgcctgcctg aaggccaggc gaataaaaaa     960

ctggtcggca agagcccatc ggccatgctg gaagccgccg ggatcgctgt ccctgcaaaa    1020

gcgccgcgtc tgctgattgc gctggttaac gctgacgatc cgtgggtcac cagcgaacag    1080

ttgatgccga tgctgccagt ggtaaaagtc agcgatttcg atagcgcgct ggcgctggcc    1140

ctgaaggttg aagaggggct gcatcatacc gccattatgc actcgcagaa cgtgtcacgc    1200

ctgaacctcg cggcccgcac gctgcaaacc tcgatattcg tcaaaaacgg cccctcttat    1260

gccgggatcg gcgtcggcgg cgaaggcttt accaccttca ctatcgccac accaaccggt 1320

gaagggacca cgtcagcgcg tacttttgcc cgttcccggc gctgcgtact gaccaacggc 1380

ttttctattc gctaa 1395

<210> 60
<211> 464
<212> PRT
<213> Salmonella enterica

<400> 60

Met Asn Thr Ser Glu Leu Glu Thr Leu Ile Arg Thr Ile Leu Ser Glu
1               5                   10                  15

Gln Leu Thr Thr Pro Ala Gln Thr Pro Val Gln Pro Gln Gly Lys Gly
            20                  25                  30

Ile Phe Gln Ser Val Ser Glu Ala Ile Asp Ala Ala His Gln Ala Phe
        35                  40                  45

Leu Arg Tyr Gln Gln Cys Pro Leu Lys Thr Arg Ser Ala Ile Ile Ser
    50                  55                  60

Ala Met Arg Gln Glu Leu Thr Pro Leu Leu Ala Pro Leu Ala Glu Glu
65                  70                  75                  80

Ser Ala Asn Glu Thr Gly Met Gly Asn Lys Glu Asp Lys Phe Leu Lys
                85                  90                  95

Asn Lys Ala Ala Leu Asp Asn Thr Pro Gly Val Glu Asp Leu Thr Thr
            100                 105                 110

Thr Ala Leu Thr Gly Asp Gly Gly Met Val Leu Phe Glu Tyr Ser Pro
        115                 120                 125

Phe Gly Val Ile Gly Ser Val Ala Pro Ser Thr Asn Pro Thr Glu Thr
    130                 135                 140

Ile Ile Asn Asn Ser Ile Ser Met Leu Ala Ala Gly Asn Ser Ile Tyr
145                 150                 155                 160

Phe Ser Pro His Pro Gly Ala Lys Lys Val Ser Leu Lys Leu Ile Ser
                165                 170                 175

Leu Ile Glu Glu Ile Ala Phe Arg Cys Cys Gly Ile Arg Asn Leu Val
            180                 185                 190

Val Thr Val Ala Glu Pro Thr Phe Glu Ala Thr Gln Gln Met Met Ala

195                 200                 205

His Pro Arg Ile Ala Val Leu Ala Ile Thr Gly Gly Pro Gly Ile Val
    210                 215                 220

Ala Met Gly Met Lys Ser Gly Lys Lys Val Ile Gly Ala Gly Ala Gly
225                 230                 235                 240

Asn Pro Pro Cys Ile Val Asp Glu Thr Ala Asp Leu Val Lys Ala Ala
                245                 250                 255

Glu Asp Ile Ile Asn Gly Ala Ser Phe Asp Tyr Asn Leu Pro Cys Ile
            260                 265                 270

Ala Glu Lys Ser Leu Ile Val Val Glu Ser Val Ala Glu Arg Leu Val
        275                 280                 285

Gln Gln Met Gln Thr Phe Gly Ala Leu Leu Leu Ser Pro Ala Asp Thr
    290                 295                 300

Asp Lys Leu Arg Ala Val Cys Leu Pro Glu Gly Gln Ala Asn Lys Lys
305                 310                 315                 320

Leu Val Gly Lys Ser Pro Ser Ala Met Leu Glu Ala Ala Gly Ile Ala
                325                 330                 335

Val Pro Ala Lys Ala Pro Arg Leu Leu Ile Ala Leu Val Asn Ala Asp
            340                 345                 350

Asp Pro Trp Val Thr Ser Glu Gln Leu Met Pro Met Leu Pro Val Val
        355                 360                 365

Lys Val Ser Asp Phe Asp Ser Ala Leu Ala Leu Ala Leu Lys Val Glu
    370                 375                 380

Glu Gly Leu His His Thr Ala Ile Met His Ser Gln Asn Val Ser Arg
385                 390                 395                 400

Leu Asn Leu Ala Ala Arg Thr Leu Gln Thr Ser Ile Phe Val Lys Asn
                405                 410                 415

Gly Pro Ser Tyr Ala Gly Ile Gly Val Gly Gly Glu Gly Phe Thr Thr
            420                 425                 430

Phe Thr Ile Ala Thr Pro Thr Gly Glu Gly Thr Thr Ser Ala Arg Thr
        435                 440                 445

Phe Ala Arg Ser Arg Arg Cys Val Leu Thr Asn Gly Phe Ser Ile Arg
    450                 455                 460

<210>  61
<211>  1386
<212>  DNA
<213>  Klebsiella pneumoniae

<400>  61
atgaatacag cagaactgga aacccttatc cgcaccattc tcagcgaaaa gctcgcgccc      60

gcccccgttt ctcaggaaca gcagggcatt taccgcgacg tcggcagcgc catcgacgcc     120

gcccatcagg cttttctccg ctatcagcag tgtccgctaa aaacccgcag cgccattatc     180

agcgccctgc gggagacgct ggcccccgag ctggcgacgc tggcggagga gagcgccacg     240

gagaccggca tgggcaacaa agaagataaa tatctgaaaa ataaagctgc ccttgagaac     300

acaccgggca ttgaggatct caccaccagc gccctcaccg gcgatggcgg gatggtgctg     360

tttgagtact cgccgttcgg ggttattggc gccgtggcgc ccagcaccaa cccaacggaa     420

accattatca acaacagtat cagcatgctg gcggcgggta acagcgtcta tttcagcccc     480

catcccggcg cgaaaaaggt ctcgttaacg cttatcgcca ggatcgaaga gatcgcctac     540

cgctgcagcg ggatccgtaa cctggtggtg accgttgccg agccgacctt tgaagccacc     600

cagcaaatga tggcccaccc gctgattgcc gttctggcta tcaccggcgg ccctggcatt     660

gtggcgatgg gcatgaaaag cggtaaaaaa gtgatcggcg ctggcgccgg caatccgccg     720

tgcatcgttg atgaaacggc cgatctcgtc aaagccgccg aagatatcat cagcggcgcc     780

gccttcgatt acaacctgcc ctgtatcgcc gaaaaaagcc tgatcgtcgt cgcctccgtc     840

gctgaccgcc tgatccagca gatgcaggat tttgacgcgc tgctgttgag cccgcaggag     900

accgataccc tgcgcgccgt ctgcctgccc gacggcgcgg cgaataaaaa actggttggt     960

aagagcccgg ctgagctgct ggcggccgcc ggtctcgccg tcccttcccg cccccctcgc    1020

ctgctgatag ccgaggtgca ggcgaacgac ccctgggtga cctgcgagca actgatgccg    1080

gtgctgccga tcgtccgggt cgccgatttt gatagcgccc tggcgctggc cctgcgcgtt    1140

gaggagggtc tgcaccacac cgccattatg cactcgcaga atgtctcgcg gctcaatctg    1200

gcggcacgca cgctgcagac ctccattttt gtcaaaaatg gtccgtctta cgcgggtatc    1260

ggcgtcggcg gcgaagggtt taccaccttc accatcgcca cgccaaccgg agaaggcacc    1320

acctccgcgc ggacgttcgc ccgcctgcgg cgctgcgtgt tgaccaacgg tttttccatt    1380

cgctaa                                                               1386

<210>  62
<211>  461
<212>  PRT
<213>  Klebsiella pneumoniae

<400>  62

Met Asn Thr Ala Glu Leu Glu Thr Leu Ile Arg Thr Ile Leu Ser Glu
1               5                   10                  15

Lys Leu Ala Pro Ala Pro Val Ser Gln Glu Gln Gln Gly Ile Tyr Arg
            20                  25                  30

Asp Val Gly Ser Ala Ile Asp Ala Ala His Gln Ala Phe Leu Arg Tyr
        35                  40                  45

Gln Gln Cys Pro Leu Lys Thr Arg Ser Ala Ile Ile Ser Ala Leu Arg
    50                  55                  60

Glu Thr Leu Ala Pro Glu Leu Ala Thr Leu Ala Glu Glu Ser Ala Thr
65                  70                  75                  80

Glu Thr Gly Met Gly Asn Lys Glu Asp Lys Tyr Leu Lys Asn Lys Ala
                85                  90                  95

Ala Leu Glu Asn Thr Pro Gly Ile Glu Asp Leu Thr Thr Ser Ala Leu
            100                 105                 110

Thr Gly Asp Gly Gly Met Val Leu Phe Glu Tyr Ser Pro Phe Gly Val
        115                 120                 125

Ile Gly Ala Val Ala Pro Ser Thr Asn Pro Thr Glu Thr Ile Ile Asn
    130                 135                 140

Asn Ser Ile Ser Met Leu Ala Ala Gly Asn Ser Val Tyr Phe Ser Pro
145                 150                 155                 160

His Pro Gly Ala Lys Lys Val Ser Leu Thr Leu Ile Ala Arg Ile Glu
                165                 170                 175

Glu Ile Ala Tyr Arg Cys Ser Gly Ile Arg Asn Leu Val Val Thr Val
            180                 185                 190

Ala Glu Pro Thr Phe Glu Ala Thr Gln Gln Met Met Ala His Pro Leu
        195                 200                 205

Ile Ala Val Leu Ala Ile Thr Gly Gly Pro Gly Ile Val Ala Met Gly
    210                 215                 220

Met Lys Ser Gly Lys Lys Val Ile Gly Ala Gly Ala Gly Asn Pro Pro
225                 230                 235                 240

Cys Ile Val Asp Glu Thr Ala Asp Leu Val Lys Ala Ala Glu Asp Ile
                245                 250                 255

Ile Ser Gly Ala Ala Phe Asp Tyr Asn Leu Pro Cys Ile Ala Glu Lys
            260                 265                 270

Ser Leu Ile Val Val Ala Ser Val Ala Asp Arg Leu Ile Gln Gln Met
        275                 280                 285

Gln Asp Phe Asp Ala Leu Leu Leu Ser Pro Gln Glu Thr Asp Thr Leu
    290                 295                 300

Arg Ala Val Cys Leu Pro Asp Gly Ala Ala Asn Lys Lys Leu Val Gly
305                 310                 315                 320

Lys Ser Pro Ala Glu Leu Leu Ala Ala Ala Gly Leu Ala Val Pro Ser
                325                 330                 335

Arg Pro Pro Arg Leu Leu Ile Ala Glu Val Gln Ala Asn Asp Pro Trp
            340                 345                 350

Val Thr Cys Glu Gln Leu Met Pro Val Leu Pro Ile Val Arg Val Ala
        355                 360                 365

Asp Phe Asp Ser Ala Leu Ala Leu Ala Leu Arg Val Glu Glu Gly Leu
    370                 375                 380

His His Thr Ala Ile Met His Ser Gln Asn Val Ser Arg Leu Asn Leu
385                 390                 395                 400

Ala Ala Arg Thr Leu Gln Thr Ser Ile Phe Val Lys Asn Gly Pro Ser
                405                 410                 415

Tyr Ala Gly Ile Gly Val Gly Gly Glu Gly Phe Thr Thr Phe Thr Ile
            420                 425                 430

Ala Thr Pro Thr Gly Glu Gly Thr Thr Ser Ala Arg Thr Phe Ala Arg
        435                 440                 445

Leu Arg Arg Cys Val Leu Thr Asn Gly Phe Ser Ile Arg
    450                 455                 460

<210>  63
<211>  1164
<212>  DNA
<213>  Klebsiella pneumoniae

<400>  63
atgagctatc gtatgtttga ttatctggtg ccaaacgtta acttttttgg ccccaacgcc        60

atttccgtag tcggcgaacg ctgccagctg ctggggggga aaaaagccct gctggtcacc 120

gacaaaggcc tgcgggcaat taaagatggc gcggtggaca aaaccctgca ttatctgcgg 180

gaggccggga tcgaggtggc gatctttgac ggcgtcgagc cgaacccgaa agacaccaac 240

gtgcgtgacg gcctcgccgt gtttcgccgc gaacagtgcg acatcatcgt caccgtgggc 300

ggcggcagcc cgcacgactg cggtaaaggc atcggcatcg ccgccaccca tgagggcgat 360

ctgtaccagt atgcgggaat cgagaccctg accaacccgc tgccgcctat cgtcgcggtc 420

aacaccactg ccggcaccgc cagcgaggtc acccgccact gcgtcctgac caacacccaa 480

accaaagtga agtttgtgat cgtcagttgg cgcaacctgc catcggtctc catcaacgat 540

ccgctgctga tgatcggtaa accggccgcc ctgaccgcgg cgaccgggat ggatgccctg 600

acccacgccg tagaggccta tatctccaaa gacgctaacc cggtgacgga cgccgccgcc 660

atgcaggcga tccgcctcat cgcccgcaac ctgcgccagg ccgtggccct cggcagcaat 720

ctgcaggcgc gggaaaacat ggcctatgcc tctctgctgg ccgggatggc tttcaataac 780

gccaacctcg gctacgtgca cgccatggcg caccagctgg gcggcctgta cgacatgccg 840

cacggcgtgg ccaacgctgt cctgctgccg catgtggccc gctacaacct gatcgccaat 900

ccggagaaat tcgccgatat cgctgaactg atgggcgaaa atatcaccgg actgtccacc 960

ctcgacgcgg cggaaaaagc catcgccgct atcacgcgtt tgtcgatgga tatcggtatt 1020

ccgcagcatc tgcgcgatct gggagtaaaa gaggccgact tcccctacat ggcggagatg 1080

gctctgaaag acggcaatgc gttctcgaac ccgcgtaaag gcaacgagca ggagattgcc 1140

gcgattttcc gccaggcatt ctga 1164

<210> 64
<211> 387
<212> PRT
<213> Klebsiella pneumoniae

<400> 64

Met Ser Tyr Arg Met Phe Asp Tyr Leu Val Pro Asn Val Asn Phe Phe
1               5                   10                  15

Gly Pro Asn Ala Ile Ser Val Val Gly Glu Arg Cys Gln Leu Leu Gly
            20                  25                  30

Gly Lys Lys Ala Leu Leu Val Thr Asp Lys Gly Leu Arg Ala Ile Lys
        35                  40                  45

Asp Gly Ala Val Asp Lys Thr Leu His Tyr Leu Arg Glu Ala Gly Ile
    50                  55                  60

Glu Val Ala Ile Phe Asp Gly Val Glu Pro Asn Pro Lys Asp Thr Asn
65                  70                  75                  80

Val Arg Asp Gly Leu Ala Val Phe Arg Arg Glu Gln Cys Asp Ile Ile
                85                  90                  95

Val Thr Val Gly Gly Gly Ser Pro His Asp Cys Gly Lys Gly Ile Gly
            100                 105                 110

Ile Ala Ala Thr His Glu Gly Asp Leu Tyr Gln Tyr Ala Gly Ile Glu
        115                 120                 125

Thr Leu Thr Asn Pro Leu Pro Pro Ile Val Ala Val Asn Thr Thr Ala
    130                 135                 140

Gly Thr Ala Ser Glu Val Thr Arg His Cys Val Leu Thr Asn Thr Gln
145                 150                 155                 160

Thr Lys Val Lys Phe Val Ile Val Ser Trp Arg Asn Leu Pro Ser Val
                165                 170                 175

Ser Ile Asn Asp Pro Leu Leu Met Ile Gly Lys Pro Ala Ala Leu Thr
            180                 185                 190

Ala Ala Thr Gly Met Asp Ala Leu Thr His Ala Val Glu Ala Tyr Ile
        195                 200                 205

Ser Lys Asp Ala Asn Pro Val Thr Asp Ala Ala Ala Met Gln Ala Ile
    210                 215                 220

Arg Leu Ile Ala Arg Asn Leu Arg Gln Ala Val Ala Leu Gly Ser Asn
225                 230                 235                 240

Leu Gln Ala Arg Glu Asn Met Ala Tyr Ala Ser Leu Leu Ala Gly Met
                245                 250                 255

Ala Phe Asn Asn Ala Asn Leu Gly Tyr Val His Ala Met Ala His Gln
            260                 265                 270

Leu Gly Gly Leu Tyr Asp Met Pro His Gly Val Ala Asn Ala Val Leu
        275                 280                 285

Leu Pro His Val Ala Arg Tyr Asn Leu Ile Ala Asn Pro Glu Lys Phe
    290                 295                 300

Ala Asp Ile Ala Glu Leu Met Gly Glu Asn Ile Thr Gly Leu Ser Thr
305                 310                 315                 320

Leu Asp Ala Ala Glu Lys Ala Ile Ala Ala Ile Thr Arg Leu Ser Met
                325                 330                 335

Asp Ile Gly Ile Pro Gln His Leu Arg Asp Leu Gly Val Lys Glu Ala
            340                 345                 350

Asp Phe Pro Tyr Met Ala Glu Met Ala Leu Lys Asp Gly Asn Ala Phe
        355                 360                 365

Ser Asn Pro Arg Lys Gly Asn Glu Gln Glu Ile Ala Ala Ile Phe Arg
    370                 375                 380

Gln Ala Phe
385

<210>  65
<211>  1158
<212>  DNA
<213>  Clostridium butyricum

<400>  65
atgagaatgt atgattattt agtaccaagt gtaaatttta tgggagcaaa ttcagtatct      60

gtagtaggtg aaagatgcaa aatattaggt ggaaagaaag cattgatagt tacagataag     120

tttctaaaag atatggaagg tggagctgtt gaattaacag ttaaatattt aaaagaagct     180

ggattagatg ctgtatatta tgacggagtt gaaccaaatc caaaagatgt taatgttata     240

gaaggattaa aaatatttaa agaagaaaat tgtgacatga tagtaactgt aggtggagga     300

agttctcatg attgcggtaa gggaatagga attgctgcaa cacatgaagg agatctttat     360

gattatgcag gaatagaaac acttgtcaat ccattgccac caatagtagc tgtaaatact     420

actgcaggaa ctgctagtga attaactcgt cattgtgtat tgactaatac aaaaaagaaa     480

ataaaatttg ttatagttag ctggagaaat ttgcctttag tatctataaa tgatccaatg     540

cttatggtta aaaaacctgc aggattaaca gcagctacag gaatggatgc tttaacacat     600

gcaatagaag catatgtatc aaaagatgca aatccagtaa cagatgcttc agcaatacaa     660

gctattaaat taatttcaca aaatttaaga caagctgtag ctttaggaga aaatcttgaa     720

gcaagagaaa atatggctta tgcatcatta ttagcaggaa tggcatttaa taatgctaat     780

ttaggatatg tacatgcaat ggctcatcaa ttagggggac tgtatgatat ggcacatggt     840

gttgctaatg caatgctatt accacatgtt gaacgttata atatgatatc aaatcctaag     900

aagtttgcag atatagcaga atttatggga gaaaatatat ctggactttc tgtaatggaa     960

gcagcagaga aagccataaa tgcaatgttt agactttcag aggatgttgg aattccgaaa    1020

agtttaaagg agatgggagt taaacaagaa gattttgagc atatggcaga actagctctt    1080

ttagatggaa atgcatttag caatccaaga aaaggaaatg caaaagatat tataaatatt 1140

tttaaggctg cttattaa 1158

<210> 66
<211> 385
<212> PRT
<213> Clostridium butyricum

<400> 66

Met Arg Met Tyr Asp Tyr Leu Val Pro Ser Val Asn Phe Met Gly Ala
1               5                   10                  15

Asn Ser Val Ser Val Val Gly Glu Arg Cys Lys Ile Leu Gly Gly Lys
            20                  25                  30

Lys Ala Leu Ile Val Thr Asp Lys Phe Leu Lys Asp Met Glu Gly Gly
        35                  40                  45

Ala Val Glu Leu Thr Val Lys Tyr Leu Lys Glu Ala Gly Leu Asp Ala
    50                  55                  60

Val Tyr Tyr Asp Gly Val Glu Pro Asn Pro Lys Asp Val Asn Val Ile
65                  70                  75                  80

Glu Gly Leu Lys Ile Phe Lys Glu Glu Asn Cys Asp Met Ile Val Thr
                85                  90                  95

Val Gly Gly Gly Ser Ser His Asp Cys Gly Lys Gly Ile Gly Ile Ala
            100                 105                 110

Ala Thr His Glu Gly Asp Leu Tyr Asp Tyr Ala Gly Ile Glu Thr Leu
        115                 120                 125

Val Asn Pro Leu Pro Pro Ile Val Ala Val Asn Thr Thr Ala Gly Thr
    130                 135                 140

Ala Ser Glu Leu Thr Arg His Cys Val Leu Thr Asn Thr Lys Lys Lys
145                 150                 155                 160

Ile Lys Phe Val Ile Val Ser Trp Arg Asn Leu Pro Leu Val Ser Ile
                165                 170                 175

Asn Asp Pro Met Leu Met Val Lys Lys Pro Ala Gly Leu Thr Ala Ala
            180                 185                 190

Thr Gly Met Asp Ala Leu Thr His Ala Ile Glu Ala Tyr Val Ser Lys
        195                 200                 205

Asp Ala Asn Pro Val Thr Asp Ala Ser Ala Ile Gln Ala Ile Lys Leu
    210                 215                 220

Ile Ser Gln Asn Leu Arg Gln Ala Val Ala Leu Gly Glu Asn Leu Glu
225                 230                 235                 240

Ala Arg Glu Asn Met Ala Tyr Ala Ser Leu Leu Ala Gly Met Ala Phe
                245                 250                 255

Asn Asn Ala Asn Leu Gly Tyr Val His Ala Met Ala His Gln Leu Gly
            260                 265                 270

Gly Leu Tyr Asp Met Ala His Gly Val Ala Asn Ala Met Leu Leu Pro
        275                 280                 285

His Val Glu Arg Tyr Asn Met Ile Ser Asn Pro Lys Lys Phe Ala Asp
    290                 295                 300

Ile Ala Glu Phe Met Gly Glu Asn Ile Ser Gly Leu Ser Val Met Glu
305                 310                 315                 320

Ala Ala Glu Lys Ala Ile Asn Ala Met Phe Arg Leu Ser Glu Asp Val
                325                 330                 335

Gly Ile Pro Lys Ser Leu Lys Glu Met Gly Val Lys Gln Glu Asp Phe
            340                 345                 350

Glu His Met Ala Glu Leu Ala Leu Leu Asp Gly Asn Ala Phe Ser Asn
        355                 360                 365

Pro Arg Lys Gly Asn Ala Lys Asp Ile Ile Asn Ile Phe Lys Ala Ala
    370                 375                 380

Tyr
385

<210>  67
<211>  1164
<212>  DNA
<213>  Citrobacter freundii

<400>  67
atgagctatc gtatgtttga ttacctggtg ccaaatgtga acttctttgg ccccaatgct      60

atttccgtgg tcggcgaacg ctgcaaactg ttgggcggta aaaaagcgct gctggtcact     120

gataaaggtc tgcgggcgat taaagacggc gcggtagata aaaccctcac acatctgcgt     180

gaagccggta ttgacgtcgt ggtttttgac ggcgttgagc caaaccccaa agacaccaac     240

gtgcgcgacg gcctggaggt ctttcggaaa gagcattgcg acatcatcgt taccgttggc 300

ggcggtagcc cgcatgactg cggtaaaggc atcggtatcg ccgcgactca cgaaggggat 360

ctctacagct atgccgggat tgaaaccctg accaacccgc tgccgccgat cgttgcggtg 420

aataccaccg ccggtaccgc cagcgaagtc acccgccact gcgtgctgac caataccaaa 480

accaaagtga agtttgtgat tgtcagctgg cgcaacctgc cgtcggtctc cattaacgat 540

ccgctgctaa tgctcggcaa gccagcccca ctgactgcgg ctaccgggat ggacgccctg 600

acccacgccg tggaagccta catttccaaa gatgccaacc cggtcaccga cgctgccgct 660

atccaggcga tccgcctgat cgcccgtaac ttgcgccagg ccgtggcgct gggcagcaac 720

ctgaaagctc gcgagaacat ggcctacgcc tccctgctgg cgggtatggc cttcaacaac 780

gccaacctcg gctacgttca cgcgatggcg catcagcttg gcggtcttta cgacatgccg 840

cacggcgtgg cgaatgccgt actgctgccg cacgtagcgc gctataacct gatcgctaac 900

ccggaaaaat ttgccgacat cgcagagttt atgggcgaga acacggacgg actctccacc 960

atggatgccg ccgagctggc cattcatgct attgcccgcc tctccgccga catcggtatt 1020

ccgcagcatc tgcgcgatct gggcgtcaaa gaagccgatt tcccgtatat ggctgaaatg 1080

gcactgaagg acggcaacgc cttctccaac ccacgcaaag ggaacgagaa agaaattgcc 1140

gagatcttcc gtcaggcatt ctga 1164

<210> 68
<211> 387
<212> PRT
<213> Citrobacter freundii

<400> 68

Met Ser Tyr Arg Met Phe Asp Tyr Leu Val Pro Asn Val Asn Phe Phe
1               5                   10                  15

Gly Pro Asn Ala Ile Ser Val Val Gly Glu Arg Cys Lys Leu Leu Gly
            20                  25                  30

Gly Lys Lys Ala Leu Leu Val Thr Asp Lys Gly Leu Arg Ala Ile Lys
        35                  40                  45

Asp Gly Ala Val Asp Lys Thr Leu Thr His Leu Arg Glu Ala Gly Ile
    50                  55                  60

Asp Val Val Val Phe Asp Gly Val Glu Pro Asn Pro Lys Asp Thr Asn
65                  70                  75                  80

Val Arg Asp Gly Leu Glu Val Phe Arg Lys Glu His Cys Asp Ile Ile
                85                  90                  95

Val Thr Val Gly Gly Gly Ser Pro His Asp Cys Gly Lys Gly Ile Gly
            100                 105                 110

Ile Ala Ala Thr His Glu Gly Asp Leu Tyr Ser Tyr Ala Gly Ile Glu
        115                 120                 125

Thr Leu Thr Asn Pro Leu Pro Pro Ile Val Ala Val Asn Thr Thr Ala
    130                 135                 140

Gly Thr Ala Ser Glu Val Thr Arg His Cys Val Leu Thr Asn Thr Lys
145                 150                 155                 160

Thr Lys Val Lys Phe Val Ile Val Ser Trp Arg Asn Leu Pro Ser Val
                165                 170                 175

Ser Ile Asn Asp Pro Leu Leu Met Leu Gly Lys Pro Ala Pro Leu Thr
            180                 185                 190

Ala Ala Thr Gly Met Asp Ala Leu Thr His Ala Val Glu Ala Tyr Ile
        195                 200                 205

Ser Lys Asp Ala Asn Pro Val Thr Asp Ala Ala Ala Ile Gln Ala Ile
    210                 215                 220

Arg Leu Ile Ala Arg Asn Leu Arg Gln Ala Val Ala Leu Gly Ser Asn
225                 230                 235                 240

Leu Lys Ala Arg Glu Asn Met Ala Tyr Ala Ser Leu Leu Ala Gly Met
                245                 250                 255

Ala Phe Asn Asn Ala Asn Leu Gly Tyr Val His Ala Met Ala His Gln
            260                 265                 270

Leu Gly Gly Leu Tyr Asp Met Pro His Gly Val Ala Asn Ala Val Leu
        275                 280                 285

Leu Pro His Val Ala Arg Tyr Asn Leu Ile Ala Asn Pro Glu Lys Phe
    290                 295                 300

Ala Asp Ile Ala Glu Phe Met Gly Glu Asn Thr Asp Gly Leu Ser Thr
305                 310                 315                 320

Met Asp Ala Ala Glu Leu Ala Ile His Ala Ile Ala Arg Leu Ser Ala
                325                 330                 335

Asp Ile Gly Ile Pro Gln His Leu Arg Asp Leu Gly Val Lys Glu Ala
            340                 345                 350

Asp Phe Pro Tyr Met Ala Glu Met Ala Leu Lys Asp Gly Asn Ala Phe
        355                 360                 365

Ser Asn Pro Arg Lys Gly Asn Glu Lys Glu Ile Ala Glu Ile Phe Arg
    370                 375                 380

Gln Ala Phe
385

<210>  69
<211>  1164
<212>  DNA
<213>  Escherichia coli

<400>  69
atgaacaact ttaatctgca caccccaacc cgcattctgt ttggtaaagg cgcaatcgct      60

ggtttacgcg aacaaattcc tcacgatgct cgcgtattga ttacctacgg tggcggcagc     120

gtgaaaaaaa ccggcgttct cgatcaagtt ctggatgccc tgaaaggcat ggacgtgctg     180

gaatttggcg gtattgagcc aaacccggct tatgaaacgc tgatgaacgc cgtgaaactg     240

gttcgcgaac agaaagtgac tttcctgctg gcggttggcg gcggttctgt actggacggc     300

accaaattta tcgccgcagc ggctaactat ccggaaaata tcgatccgtg gcacattctg     360

caaacgggcg gtaaagagat taaaagcgcc atcccgatgg gctgtgtgct gacgctgcca     420

gcaaccggtt cagaatccaa cgcaggcgcg gtgatctccc gtaaaaccac aggcgacaag     480

caggcgttcc attctgccca tgttcagccg gtatttgccg tgctcgatcc ggtttatacc     540

tacaccctgc cgccgcgtca ggtggctaac ggcgtagtgg acgcctttgt acacaccgtg     600

gaacagtatg ttaccaaacc ggttgatgcc aaaattcagg accgtttcgc agaaggcatt     660

ttgctgacgc tgatcgaaga tggtccgaaa gccctgaaag agccagaaaa ctacgatgtg     720

cgcgccaacg tcatgtgggg ggcgacgcag gcgctgaacg gtttgattgg cgctggcgta     780

ccgcaggact gggcaacgca tatgctgggc cacgaactga ctgcgatgca cggtctggat     840

cacgcgcaaa cactggctat cgtcctgcct gcactgtgga atgaaaaacg cgagaccaag     900

cgcgctaagc tgctgcaata tgctgaacgc gtctggaaca tcactgaagg ttccgatgat     960

gagcgtattg acgccgcgat tgccgcaacc cgcaatttct ttgagcaatt aggcgtgccg    1020

acccacctct ccgactacgg tctggacggc agctccatcc cggctttgct gaaaaaactg    1080

gaagagcacg gcatgaccca actgggcgaa aatcatgaca ttacgttgga tgtcagccgc    1140

cgtatatacg aagccgcccg ctaa                                           1164

<210>  70
<211>  387

<212>  PRT
<213>  Escherichia coli

<400>  70

Met Asn Asn Phe Asn Leu His Thr Pro Thr Arg Ile Leu Phe Gly Lys
1               5                   10                  15

Gly Ala Ile Ala Gly Leu Arg Glu Gln Ile Pro His Asp Ala Arg Val
            20                  25                  30

Leu Ile Thr Tyr Gly Gly Gly Ser Val Lys Lys Thr Gly Val Leu Asp
        35                  40                  45

Gln Val Leu Asp Ala Leu Lys Gly Met Asp Val Leu Glu Phe Gly Gly
    50                  55                  60

Ile Glu Pro Asn Pro Ala Tyr Glu Thr Leu Met Asn Ala Val Lys Leu
65                  70                  75                  80

Val Arg Glu Gln Lys Val Thr Phe Leu Leu Ala Val Gly Gly Gly Ser
                85                  90                  95

Val Leu Asp Gly Thr Lys Phe Ile Ala Ala Ala Ala Asn Tyr Pro Glu
            100                 105                 110

Asn Ile Asp Pro Trp His Ile Leu Gln Thr Gly Gly Lys Glu Ile Lys
        115                 120                 125

Ser Ala Ile Pro Met Gly Cys Val Leu Thr Leu Pro Ala Thr Gly Ser
    130                 135                 140

Glu Ser Asn Ala Gly Ala Val Ile Ser Arg Lys Thr Thr Gly Asp Lys
145                 150                 155                 160

Gln Ala Phe His Ser Ala His Val Gln Pro Val Phe Ala Val Leu Asp
                165                 170                 175

Pro Val Tyr Thr Tyr Thr Leu Pro Pro Arg Gln Val Ala Asn Gly Val
            180                 185                 190

Val Asp Ala Phe Val His Thr Val Glu Gln Tyr Val Thr Lys Pro Val
        195                 200                 205

Asp Ala Lys Ile Gln Asp Arg Phe Ala Glu Gly Ile Leu Leu Thr Leu
    210                 215                 220

Ile Glu Asp Gly Pro Lys Ala Leu Lys Glu Pro Glu Asn Tyr Asp Val
225                 230                 235                 240

Arg Ala Asn Val Met Trp Gly Ala Thr Gln Ala Leu Asn Gly Leu Ile
                245                 250                 255

Gly Ala Gly Val Pro Gln Asp Trp Ala Thr His Met Leu Gly His Glu
            260                 265                 270

Leu Thr Ala Met His Gly Leu Asp His Ala Gln Thr Leu Ala Ile Val
        275                 280                 285

Leu Pro Ala Leu Trp Asn Glu Lys Arg Glu Thr Lys Arg Ala Lys Leu
    290                 295                 300

Leu Gln Tyr Ala Glu Arg Val Trp Asn Ile Thr Glu Gly Ser Asp Asp
305                 310                 315                 320

Glu Arg Ile Asp Ala Ala Ile Ala Ala Thr Arg Asn Phe Phe Glu Gln
                325                 330                 335

Leu Gly Val Pro Thr His Leu Ser Asp Tyr Gly Leu Asp Gly Ser Ser
            340                 345                 350

Ile Pro Ala Leu Leu Lys Lys Leu Glu Glu His Gly Met Thr Gln Leu
        355                 360                 365

Gly Glu Asn His Asp Ile Thr Leu Asp Val Ser Arg Arg Ile Tyr Glu
    370                 375                 380

Ala Ala Arg
385

<210> 71
<211> 387
<212> PRT
<213> Escherichia coli

<400> 71
   Met Asn Asn Phe Asn Leu His Thr Pro Thr Arg Ile Leu Phe Gly Lys
   1 5 10 15
   Gly Ala Ile Ala Gly Leu Arg Glu Gln Ile Pro His Asp Ala Arg Val
   20 25 30
   Leu Ile Thr Tyr Gly Gly Gly Ser Val Lys Lys Thr Gly Val Leu Asp
   35 40 45
   Gln Val Leu Asp Ala Leu Lys Gly Met Asp Val Leu Glu Phe Gly Gly
   50 55 60
   Ile Glu Pro Asn Pro Ala Tyr Glu Thr Leu Met Asn Ala Val Lys Leu
   65                  70                  75                  80
   Val Arg Glu Gln Lys Val Thr Phe Leu Leu Ala Val Gly Gly Gly Ser
                   85                  90                  95
   Val Leu Gln Gly Thr Lys Phe Ile Ala Ala Ala Ala Asn Tyr Pro Glu
               100                 105                 110
   Asn Ile Asp Pro Trp His Ile Leu Gln Thr Gly Gly Lys Glu Ile Lys
           115                 120                 125
   Ser Ala Ile Pro Met Gly Cys Val Leu Thr Leu Pro Ala Thr Gly Ser
       130                 135                 140
   Glu Ser His Ala Gly Ala Val Ile Ser Arg Lys Thr Thr Gly Asp Lys
   145                 150                 155                 160
   Gln Ala Phe His Ser Ala His Val Gln Pro Val Phe Ala Val Leu Asp
                   165                 170                 175
   Pro Val Tyr Thr Tyr Thr Leu Pro Pro Arg Gln Val Ala Asn Gly Val
               180                 185                 190
   Val Asp Ala Phe Val His Thr Val Glu Gln Tyr Val Thr Lys Pro Val
           195                 200                 205
   Asp Ala Lys Ile Gln Asp Arg Phe Ala Glu Gly Ile Leu Leu Thr Leu
       210                 215                 220
   Ile Glu Asp Gly Pro Lys Ala Leu Lys Glu Pro Glu Asn Tyr Asp Val
   225                 230                 235                 240
   Arg Ala Asn Val Met Trp Gly Ala Thr Gln Ala Leu Asn Gly Leu Ile
                   245                 250                 255
   Gly Ala Gly Val Pro Gln Asp Trp Ala Thr His Met Leu Gly His Glu
               260                 265                 270
   Leu Thr Ala Met His Gly Leu Asp His Ala Gln Thr Leu Ala Ile Val
           275                 280                 285
   Leu Pro Ala Leu Trp Asn Glu Lys Arg Glu Thr Lys Arg Ala Lys Leu
       290                 295                 300
   Leu Gln Tyr Ala Glu Arg Val Trp Asn Ile Thr Glu Gly Ser Asp Asp
   305                 310                 315                 320
   Glu Arg Ile Asp Ala Ala Ile Ala Ala Thr Arg Asn Phe Phe Glu Gln
                   325                 330                 335
   Leu Gly Val Pro Thr His Leu Ser Asp Tyr Gly Leu Asp Gly Ser Ser
               340                 345                 350
   Ile Pro Ala Leu Leu Lys Lys Leu Glu Glu His Gly Met Thr Gln Leu
           355                 360                 365
   Gly Glu Asn His Asp Ile Thr Leu Asp Val Ser Arg Arg Ile Tyr Glu
       370                 375                 380
   Ala Ala Arg
   385

<210>  72
<211>  387
<212>  PRT
<213>  Escherichia coli

<400>  72

Met Asn Asn Phe Asn Leu His Thr Pro Thr Arg Ile Leu Phe Gly Lys
1               5                   10                  15

Gly Ala Ile Ala Gly Leu Arg Glu Gln Ile Pro His Asp Ala Arg Val
            20                  25                  30

Leu Ile Thr Tyr Gly Gly Gly Ser Val Lys Lys Thr Gly Val Leu Asp
        35                  40                  45

Gln Val Leu Asp Ala Leu Lys Gly Met Asp Val Leu Glu Phe Gly Gly
    50                  55                  60

Ile Glu Pro Asn Pro Ala Tyr Glu Thr Leu Met Asn Ala Val Lys Leu
65                  70                  75                  80

Val Arg Glu Gln Lys Val Thr Phe Leu Leu Ala Val Gly Gly Gly Ser
                85                  90                  95

Val Leu Asp Gly Thr Lys Phe Ile Ala Ala Ala Ala Asn Tyr Pro Glu
            100                 105                 110

Asn Ile Asp Pro Trp His Ile Leu Gln Thr Gly Gly Lys Glu Ile Lys
        115                 120                 125

Ser Ala Ile Pro Met Gly Cys Val Leu Thr Leu Pro Ala Thr Gly Ser

130                 135                 140

Glu Ser Asn Ala Gly Ala Val Ile Ser Arg Lys Thr Thr Gly Asp Lys
145                 150                 155                 160

Gln Ala Phe His Ser Ala His Val Gln Pro Val Phe Ala Val Leu Asp
                165                 170                 175

Pro Val Tyr Thr Tyr Thr Leu Pro Pro Arg Gln Val Ala Asn Gly Val
            180                 185                 190

Val Asp Ala Phe Val His Thr Val Glu Ala Tyr Val Thr Lys Pro Val
        195                 200                 205

Asp Ala Lys Ile Gln Asp Arg Phe Ala Glu Gly Ile Leu Leu Thr Leu
    210                 215                 220

Ile Glu Asp Gly Pro Lys Ala Leu Lys Glu Pro Glu Asn Tyr Asp Val
225                 230                 235                 240

Arg Ala Asn Val Met Trp Gly Ala Thr Gln Ala Leu Asn Gly Leu Ile
                245                 250                 255

Gly Ala Gly Val Pro Gln Asp Trp Ala Thr His Met Leu Gly His Glu
            260                 265                 270

Leu Thr Ala Met His Gly Leu Asp His Ala Gln Thr Leu Ala Ile Val
        275                 280                 285

Leu Pro Ala Leu Trp Asn Glu Lys Arg Glu Thr Lys Arg Ala Lys Leu
    290                 295                 300

Leu Gln Tyr Ala Glu Arg Val Trp Asn Ile Thr Glu Gly Ser Asp Asp
305                 310                 315                 320

Glu Arg Ile Asp Ala Ala Ile Ala Ala Thr Arg Asn Phe Phe Glu Gln
                325                 330                 335

Leu Gly Val Pro Thr His Leu Ser Asp Tyr Gly Leu Asp Gly Ser Ser
            340                 345                 350

Ile Pro Ala Leu Leu Lys Lys Leu Glu Glu His Gly Met Thr Gln Leu
        355                 360                 365

Gly Glu Asn His Asp Ile Thr Leu Asp Val Ser Arg Arg Ile Tyr Glu
    370                 375                 380

Ala Ala Arg
385

<210> 73
<211> 2433
<212> DNA
<213> Escherichia coli

<400> 73
atgagtgtga ttgcgcaggc aggggcgaaa ggtcgtcagc tgcataaatt tggtggcagt 60

agtctggctg atgtgaagtg ttatttgcgt gtcgcgggca ttatggcgga gtactctcag 120

cctgacgata tgatggtggt ttccgccgcc ggtagcacca ctaaccagtt gattaactgg 180

ttgaaactaa gccagaccga tcgtctctct gcgcatcagg ttcaacaaac gctgcgtcgc 240

tatcagtgcg atctgattag cggtctgcta cccgctgaag aagccgatag cctcattagc 300

gcttttgtca gcgaccttga gcgcctggcg gcgctgctcg acagcggtat taacgacgca 360

gtgtatgcgg aagtggtggg ccacggggaa gtatggtcgg cacgtctgat gtctgcggta 420

cttaatcaac aagggctgcc agcggcctgg cttgatgccc gcgagttttt acgcgctgaa 480

cgcgccgcac aaccgcaggt tgatgaaggg ctttcttacc cgttgctgca acagctgctg 540

gtgcaacatc cgggcaaacg tctggtggtg accggattta tcagccgcaa caacgccggt 600

gaaacggtgc tgctggggcg taacggttcc gactattccg cgacacaaat cggtgcgctg 660

gcgggtgttt ctcgcgtaac catctggagc gacgtcgccg gggtatacag tgccgacccg 720

cgtaaagtga aagatgcctg cctgctgccg ttgctgcgtc tggatgaggc cagcgaactg 780

gcgcgcctgg cggctcccgt tcttcacgcc cgtactttac agccggtttc tggcagcgaa 840

atcgacctgc aactgcgctg tagctacacg ccggatcaag gttccacgcg cattgaacgc 900

gtgctggcct ccggtactgg tgcgcgtatt gtcaccagcc acgatgatgt ctgtttgatt 960

gagtttcagg tgcccgccag tcaggatttc aaactggcgc ataaagagat cgaccaaatc 1020

ctgaaacgcg cgcaggtacg cccgctggcg gttggcgtac ataacgatcg ccagttgctg 1080

caattttgct acacctcaga agtggccgac agtgcgctga aaatcctcga cgaagcggga 1140

ttacctggcg aactgcgcct gcgtcagggg ctggcgctgg tggcgatggt cggtgcaggc 1200

gtcacccgta acccgctgca ttgccaccgc ttctggcagc aactgaaagg ccagccggtc 1260

gaatttacct ggcagtccga tgacggcatc agcctggtgg cagtactgcg caccggcccg 1320

accgaaagcc tgattcaggg gctgcatcag tccgtcttcc gcgcagaaaa acgcatcggc 1380

ctggtattgt tcggtaaggg caatatcggt tcccgttggc tggaactgtt cgcccgtgag 1440

cagagcacgc tttcggcacg taccggcttt gagtttgtgc tggcaggtgt ggtggacagc 1500

cgccgcagcc tgttgagcta tgacggcctg gacgccagcc gcgcgttagc cttcttcaac 1560

gatgaagcgg ttgagcagga tgaagagtcg ttgttcctgt ggatgcgcgc ccatccgtat 1620

gatgatttag tggtgctgga cgttaccgcc agccagcagc ttgctgatca gtatcttgat 1680

ttcgccagcc acggtttcca cgttatcagc gccaacaaac tggcgggagc cagcgacagc 1740

aataaatatc gccagatcca cgacgccttc gaaaaaaccg ggcgtcactg gctgtacaat 1800

gccaccgtcg gtgcgggctt gccgatcaac cacaccgtgc gcgatctgat cgacagcggc 1860

gatactattt tgtcgatcag cgggatcttc tccggcacgc tctcctggct gttcctgcaa 1920

ttcgacggta gcgtgccgtt taccgagctg gtggatcagg cgtggcagca gggcttaacc 1980

gaacctgacc cgcgtgacga tctctctggc aaagacgtga tgcgcaagct ggtgattctg 2040

gcgcgtgaag caggttacaa catcgaaccg gatcaggtac gtgtggaatc gctggtgcct 2100

gctcattgcg aaggcggcag catcgaccat ttctttgaaa atggcgatga actgaacgag 2160

cagatggtgc aacggctgga agcggcccgc gaaatggggc tggtgctgcg ctacgtggcg 2220

cgtttcgatg ccaacggtaa agcgcgtgta ggcgtggaag cggtgcgtga agatcatccg 2280

ttggcatcac tgctgccgtg cgataacgtc tttgccatcg aaagccgctg gtatcgcgat 2340

aaccctctgg tgatccgcgg acctggcgct gggcgcgacg tcaccgccgg ggcgattcag 2400

tcggatatca accggctggc acagttgttg taa 2433

<210> 74
<211> 810
<212> PRT
<213> Escherichia coli

<400> 74

Met Ser Val Ile Ala Gln Ala Gly Ala Lys Gly Arg Gln Leu His Lys
1               5                   10                  15

Phe Gly Gly Ser Ser Leu Ala Asp Val Lys Cys Tyr Leu Arg Val Ala
            20                  25                  30

Gly Ile Met Ala Glu Tyr Ser Gln Pro Asp Asp Met Met Val Val Ser
        35                  40                  45

Ala Ala Gly Ser Thr Thr Asn Gln Leu Ile Asn Trp Leu Lys Leu Ser
    50                  55                  60

Gln Thr Asp Arg Leu Ser Ala His Gln Val Gln Gln Thr Leu Arg Arg
65                  70                  75                  80

Tyr Gln Cys Asp Leu Ile Ser Gly Leu Leu Pro Ala Glu Glu Ala Asp
                85                  90                  95

Ser Leu Ile Ser Ala Phe Val Ser Asp Leu Glu Arg Leu Ala Ala Leu
            100                 105                 110

Leu Asp Ser Gly Ile Asn Asp Ala Val Tyr Ala Glu Val Val Gly His
        115                 120                 125

Gly Glu Val Trp Ser Ala Arg Leu Met Ser Ala Val Leu Asn Gln Gln
    130                 135                 140

Gly Leu Pro Ala Ala Trp Leu Asp Ala Arg Glu Phe Leu Arg Ala Glu
145                 150                 155                 160

Arg Ala Ala Gln Pro Gln Val Asp Glu Gly Leu Ser Tyr Pro Leu Leu
                165                 170                 175

Gln Gln Leu Leu Val Gln His Pro Gly Lys Arg Leu Val Val Thr Gly
            180                 185                 190

Phe Ile Ser Arg Asn Asn Ala Gly Glu Thr Val Leu Leu Gly Arg Asn
        195                 200                 205

Gly Ser Asp Tyr Ser Ala Thr Gln Ile Gly Ala Leu Ala Gly Val Ser
    210                 215                 220

Arg Val Thr Ile Trp Ser Asp Val Ala Gly Val Tyr Ser Ala Asp Pro
225                 230                 235                 240

Arg Lys Val Lys Asp Ala Cys Leu Leu Pro Leu Leu Arg Leu Asp Glu
                245                 250                 255

Ala Ser Glu Leu Ala Arg Leu Ala Ala Pro Val Leu His Ala Arg Thr
            260                 265                 270

Leu Gln Pro Val Ser Gly Ser Glu Ile Asp Leu Gln Leu Arg Cys Ser
        275                 280                 285

Tyr Thr Pro Asp Gln Gly Ser Thr Arg Ile Glu Arg Val Leu Ala Ser
    290                 295                 300

Gly Thr Gly Ala Arg Ile Val Thr Ser His Asp Asp Val Cys Leu Ile
305                 310                 315                 320

Glu Phe Gln Val Pro Ala Ser Gln Asp Phe Lys Leu Ala His Lys Glu
                325                 330                 335

Ile Asp Gln Ile Leu Lys Arg Ala Gln Val Arg Pro Leu Ala Val Gly
            340                 345                 350

Val His Asn Asp Arg Gln Leu Leu Gln Phe Cys Tyr Thr Ser Glu Val

355                 360                 365

Ala Asp Ser Ala Leu Lys Ile Leu Asp Glu Ala Gly Leu Pro Gly Glu
    370                 375                 380

Leu Arg Leu Arg Gln Gly Leu Ala Leu Val Ala Met Val Gly Ala Gly
385                 390                 395                 400

Val Thr Arg Asn Pro Leu His Cys His Arg Phe Trp Gln Gln Leu Lys
                405                 410                 415

Gly Gln Pro Val Glu Phe Thr Trp Gln Ser Asp Asp Gly Ile Ser Leu
            420                 425                 430

Val Ala Val Leu Arg Thr Gly Pro Thr Glu Ser Leu Ile Gln Gly Leu
        435                 440                 445

His Gln Ser Val Phe Arg Ala Glu Lys Arg Ile Gly Leu Val Leu Phe
    450                 455                 460

Gly Lys Gly Asn Ile Gly Ser Arg Trp Leu Glu Leu Phe Ala Arg Glu
465                 470                 475                 480

Gln Ser Thr Leu Ser Ala Arg Thr Gly Phe Glu Phe Val Leu Ala Gly
                485                 490                 495

Val Val Asp Ser Arg Arg Ser Leu Leu Ser Tyr Asp Gly Leu Asp Ala
            500                 505                 510

Ser Arg Ala Leu Ala Phe Phe Asn Asp Glu Ala Val Glu Gln Asp Glu
        515                 520                 525

Glu Ser Leu Phe Leu Trp Met Arg Ala His Pro Tyr Asp Asp Leu Val
    530                 535                 540

Val Leu Asp Val Thr Ala Ser Gln Gln Leu Ala Asp Gln Tyr Leu Asp
545                 550                 555                 560

Phe Ala Ser His Gly Phe His Val Ile Ser Ala Asn Lys Leu Ala Gly
                565                 570                 575

Ala Ser Asp Ser Asn Lys Tyr Arg Gln Ile His Asp Ala Phe Glu Lys
            580                 585                 590

Thr Gly Arg His Trp Leu Tyr Asn Ala Thr Val Gly Ala Gly Leu Pro
        595                 600                 605

Ile Asn His Thr Val Arg Asp Leu Ile Asp Ser Gly Asp Thr Ile Leu
    610                 615                 620

Ser Ile Ser Gly Ile Phe Ser Gly Thr Leu Ser Trp Leu Phe Leu Gln
625                 630                 635                 640

Phe Asp Gly Ser Val Pro Phe Thr Glu Leu Val Asp Gln Ala Trp Gln
                645                 650                 655

Gln Gly Leu Thr Glu Pro Asp Pro Arg Asp Asp Leu Ser Gly Lys Asp
            660                 665                 670

Val Met Arg Lys Leu Val Ile Leu Ala Arg Glu Ala Gly Tyr Asn Ile
        675                 680                 685

Glu Pro Asp Gln Val Arg Val Glu Ser Leu Val Pro Ala His Cys Glu
    690                 695                 700

Gly Gly Ser Ile Asp His Phe Phe Glu Asn Gly Asp Glu Leu Asn Glu
705                 710                 715                 720

Gln Met Val Gln Arg Leu Glu Ala Ala Arg Glu Met Gly Leu Val Leu
                725                 730                 735

Arg Tyr Val Ala Arg Phe Asp Ala Asn Gly Lys Ala Arg Val Gly Val
            740                 745                 750

Glu Ala Val Arg Glu Asp His Pro Leu Ala Ser Leu Leu Pro Cys Asp
        755                 760                 765

Asn Val Phe Ala Ile Glu Ser Arg Trp Tyr Arg Asp Asn Pro Leu Val
    770                 775                 780

Ile Arg Gly Pro Gly Ala Gly Arg Asp Val Thr Ala Gly Ala Ile Gln
785                 790                 795                 800

Ser Asp Ile Asn Arg Leu Ala Gln Leu Leu
                805                 810

<210>  75
<211>  2463
<212>  DNA
<213>  Escherichia coli

<400>  75
atgcgagtgt tgaagttcgg cggtacatca gtggcaaatg cagaacgttt tctgcgtgtt        60

gccgatattc tggaaagcaa tgccaggcag gggcaggtgg ccaccgtcct ctctgccccc       120

gccaaaatca ccaaccacct ggtggcgatg attgaaaaaa ccattagcgg ccaggatgct       180

ttacccaata tcagcgatgc cgaacgtatt tttgccgaac ttttgacggg actcgccgcc 240

gcccagccgg ggttcccgct ggcgcaattg aaaactttcg tcgatcagga atttgcccaa 300

ataaaacatg tcctgcatgg cattagtttg ttggggcagt gcccggatag catcaacgct 360

gcgctgattt gccgtggcga gaaaatgtcg atcgccatta tggccggcgt attagaagcg 420

cgcggtcaca acgttactgt tatcgatccg gtcgaaaaac tgctggcagt ggggcattac 480

ctcgaatcta ccgtcgatat tgctgagtcc acccgccgta ttgcggcaag ccgcattccg 540

gctgatcaca tggtgctgat ggcaggtttc accgccggta atgaaaaagg cgaactggtg 600

gtgcttggac gcaacggttc cgactactct gctgcggtgc tggctgcctg tttacgcgcc 660

gattgttgcg agatttggac ggacgttgac ggggtctata cctgcgaccc gcgtcaggtg 720

cccgatgcga ggttgttgaa gtcgatgtcc taccaggaag cgatggagct ttcctacttc 780

ggcgctaaag ttcttcaccc ccgcaccatt acccccatcg cccagttcca gatcccttgc 840

ctgattaaaa ataccggaaa tcctcaagca ccaggtacgc tcattggtgc cagccgtgat 900

gaagacgaat taccggtcaa gggcatttcc aatctgaata acatggcaat gttcagcgtt 960

tctggtccgg ggatgaaagg gatggtcggc atggcggcgc gcgtctttgc agcgatgtca 1020

cgcgcccgta ttttcgtggt gctgattacg caatcatctt ccgaatacag catcagtttc 1080

tgcgttccac aaagcgactg tgtgcgagct gaacgggcaa tgcaggaaga gttctacctg 1140

gaactgaaag aaggcttact ggagccgctg gcagtgacgg aacggctggc cattatctcg 1200

gtggtaggtg atggtatgcg caccttgcgt gggatctcgg cgaaattctt tgccgcactg 1260

gcccgcgcca atatcaacat tgtcgccatt gctcagggat cttctgaacg ctcaatctct 1320

gtcgtggtaa ataacgatga tgcgaccact ggcgtgcgcg ttactcatca gatgctgttc 1380

aataccgatc aggttatcga agtgtttgtg attggcgtcg gtggcgttgg cggtgcgctg 1440

ctggagcaac tgaagcgtca gcaaagctgg ctgaagaata aacatatcga cttacgtgtc 1500

tgcggtgttg ccaactcgaa ggctctgctc accaatgtac atggccttaa tctggaaaac 1560

tggcaggaag aactggcgca agccaaagag ccgtttaatc tcgggcgctt aattcgcctc 1620

gtgaaagaat atcatctgct gaacccggtc attgttgact gcacttccag ccaggcagtg 1680

gcggatcaat atgccgactt cctgcgcgaa ggtttccacg ttgtcacgcc gaacaaaaag 1740

gccaacacct cgtcgatgga ttactaccat cagttgcgtt atgcggcgga aaaatcgcgg 1800

cgtaaattcc tctatgacac caacgttggg gctggattac cggttattga gaacctgcaa 1860

aatctgctca atgcaggtga tgaattgatg aagttctccg gcattctttc tggttcgctt 1920

tcttatatct tcggcaagtt agacgaaggc atgagtttct ccgaggcgac cacgctggcg 1980

cgggaaatgg gttataccga accggacccg cgagatgatc tttctggtat ggatgtggcg 2040

cgtaaactat tgattctcgc tcgtgaaacg ggacgtgaac tggagctggc ggatattgaa 2100

attgaacctg tgctgcccgc agagtttaac gccgagggtg atgttgccgc ttttatggcg 2160

aatctgtcac aactcgacga tctctttgcc gcgcgcgtgg cgaaggcccg tgatgaagga 2220

aaagttttgc gctatgttgg caatattgat gaagatggcg tctgccgcgt gaagattgcc 2280

gaagtggatg gtaatgatcc gctgttcaaa gtgaaaaatg gcgaaaacgc cctggccttc 2340

tatagccact attatcagcc gctgccgttg gtactgcgcg gatatggtgc gggcaatgac 2400

gttacagctg ccggtgtctt tgctgatctg ctacgtaccc tctcatggaa gttaggagtc 2460

tga 2463

<210> 76
<211> 820
<212> PRT
<213> Escherichia coli

<400> 76

Met Arg Val Leu Lys Phe Gly Gly Thr Ser Val Ala Asn Ala Glu Arg
1               5                   10                  15

Phe Leu Arg Val Ala Asp Ile Leu Glu Ser Asn Ala Arg Gln Gly Gln
            20                  25                  30

Val Ala Thr Val Leu Ser Ala Pro Ala Lys Ile Thr Asn His Leu Val
        35                  40                  45

Ala Met Ile Glu Lys Thr Ile Ser Gly Gln Asp Ala Leu Pro Asn Ile
    50                  55                  60

Ser Asp Ala Glu Arg Ile Phe Ala Glu Leu Leu Thr Gly Leu Ala Ala
65                  70                  75                  80

Ala Gln Pro Gly Phe Pro Leu Ala Gln Leu Lys Thr Phe Val Asp Gln
                85                  90                  95

Glu Phe Ala Gln Ile Lys His Val Leu His Gly Ile Ser Leu Leu Gly
            100                 105                 110

Gln Cys Pro Asp Ser Ile Asn Ala Ala Leu Ile Cys Arg Gly Glu Lys
        115                 120                 125

Met Ser Ile Ala Ile Met Ala Gly Val Leu Glu Ala Arg Gly His Asn
    130                 135                 140

Val Thr Val Ile Asp Pro Val Glu Lys Leu Leu Ala Val Gly His Tyr
145                 150                 155                 160

Leu Glu Ser Thr Val Asp Ile Ala Glu Ser Thr Arg Arg Ile Ala Ala
                165                 170                 175

Ser Arg Ile Pro Ala Asp His Met Val Leu Met Ala Gly Phe Thr Ala
            180                 185                 190

Gly Asn Glu Lys Gly Glu Leu Val Val Leu Gly Arg Asn Gly Ser Asp
        195                 200                 205

Tyr Ser Ala Ala Val Leu Ala Ala Cys Leu Arg Ala Asp Cys Cys Glu
    210                 215                 220

Ile Trp Thr Asp Val Asp Gly Val Tyr Thr Cys Asp Pro Arg Gln Val
225                 230                 235                 240

Pro Asp Ala Arg Leu Leu Lys Ser Met Ser Tyr Gln Glu Ala Met Glu
                245                 250                 255

Leu Ser Tyr Phe Gly Ala Lys Val Leu His Pro Arg Thr Ile Thr Pro
            260                 265                 270

Ile Ala Gln Phe Gln Ile Pro Cys Leu Ile Lys Asn Thr Gly Asn Pro
        275                 280                 285

Gln Ala Pro Gly Thr Leu Ile Gly Ala Ser Arg Asp Glu Asp Glu Leu
    290                 295                 300

Pro Val Lys Gly Ile Ser Asn Leu Asn Asn Met Ala Met Phe Ser Val
305                 310                 315                 320

Ser Gly Pro Gly Met Lys Gly Met Val Gly Met Ala Ala Arg Val Phe
                325                 330                 335

Ala Ala Met Ser Arg Ala Arg Ile Phe Val Val Leu Ile Thr Gln Ser
            340                 345                 350

Ser Ser Glu Tyr Ser Ile Ser Phe Cys Val Pro Gln Ser Asp Cys Val
        355                 360                 365

Arg Ala Glu Arg Ala Met Gln Glu Glu Phe Tyr Leu Glu Leu Lys Glu
    370                 375                 380

Gly Leu Leu Glu Pro Leu Ala Val Thr Glu Arg Leu Ala Ile Ile Ser
385                 390                 395                 400

Val Val Gly Asp Gly Met Arg Thr Leu Arg Gly Ile Ser Ala Lys Phe
                405                 410                 415

Phe Ala Ala Leu Ala Arg Ala Asn Ile Asn Ile Val Ala Ile Ala Gln
            420                 425                 430

Gly Ser Ser Glu Arg Ser Ile Ser Val Val Val Asn Asn Asp Asp Ala
        435                 440                 445

Thr Thr Gly Val Arg Val Thr His Gln Met Leu Phe Asn Thr Asp Gln
    450                 455                 460

Val Ile Glu Val Phe Val Ile Gly Val Gly Gly Val Gly Gly Ala Leu
465                 470                 475                 480

Leu Glu Gln Leu Lys Arg Gln Gln Ser Trp Leu Lys Asn Lys His Ile
                485                 490                 495

Asp Leu Arg Val Cys Gly Val Ala Asn Ser Lys Ala Leu Leu Thr Asn
            500                 505                 510

Val His Gly Leu Asn Leu Glu Asn Trp Gln Glu Glu Leu Ala Gln Ala
        515                 520                 525

Lys Glu Pro Phe Asn Leu Gly Arg Leu Ile Arg Leu Val Lys Glu Tyr
    530                 535                 540

His Leu Leu Asn Pro Val Ile Val Asp Cys Thr Ser Ser Gln Ala Val
545                 550                 555                 560

Ala Asp Gln Tyr Ala Asp Phe Leu Arg Glu Gly Phe His Val Val Thr
                565                 570                 575

Pro Asn Lys Lys Ala Asn Thr Ser Ser Met Asp Tyr Tyr His Gln Leu
            580                 585                 590

Arg Tyr Ala Ala Glu Lys Ser Arg Arg Lys Phe Leu Tyr Asp Thr Asn
        595                 600                 605

Val Gly Ala Gly Leu Pro Val Ile Glu Asn Leu Gln Asn Leu Leu Asn
    610                 615                 620

Ala Gly Asp Glu Leu Met Lys Phe Ser Gly Ile Leu Ser Gly Ser Leu
625                 630                 635                 640

Ser Tyr Ile Phe Gly Lys Leu Asp Glu Gly Met Ser Phe Ser Glu Ala
                645                 650                 655

Thr Thr Leu Ala Arg Glu Met Gly Tyr Thr Glu Pro Asp Pro Arg Asp

660                 665                 670

Asp Leu Ser Gly Met Asp Val Ala Arg Lys Leu Leu Ile Leu Ala Arg
        675                 680                 685

Glu Thr Gly Arg Glu Leu Glu Leu Ala Asp Ile Glu Ile Glu Pro Val
    690                 695                 700

Leu Pro Ala Glu Phe Asn Ala Glu Gly Asp Val Ala Ala Phe Met Ala
705                 710                 715                 720

Asn Leu Ser Gln Leu Asp Asp Leu Phe Ala Ala Arg Val Ala Lys Ala
                725                 730                 735

Arg Asp Glu Gly Lys Val Leu Arg Tyr Val Gly Asn Ile Asp Glu Asp
            740                 745                 750

Gly Val Cys Arg Val Lys Ile Ala Glu Val Asp Gly Asn Asp Pro Leu
        755                 760                 765

Phe Lys Val Lys Asn Gly Glu Asn Ala Leu Ala Phe Tyr Ser His Tyr
    770                 775                 780

Tyr Gln Pro Leu Pro Leu Val Leu Arg Gly Tyr Gly Ala Gly Asn Asp
785                 790                 795                 800

Val Thr Ala Ala Gly Val Phe Ala Asp Leu Leu Arg Thr Leu Ser Trp
                805                 810                 815

Lys Leu Gly Val
            820

<210>  77
<211>  1350
<212>  DNA
<213>  Escherichia coli

<400>  77
atgtctgaaa ttgttgtctc caaatttggc ggtaccagcg tagctgattt tgacgccatg      60

aaccgcagcg ctgatattgt gctttctgat gccaacgtgc gtttagttgt cctctcggct     120

tctgctggta tcactaatct gctggtcgct ttagctgaag gactggaacc tggcgagcga     180

ttcgaaaaac tcgacgctat ccgcaacatc cagtttgcca ttctggaacg tctgcgttac     240

ccgaacgtta tccgtgaaga gattgaacgt ctgctggaga acattactgt tctggcagaa     300

gcggcggcgc tggcaacgtc tccggcgctg acagatgagc tggtcagcca cggcgagctg     360

atgtcgaccc tgctgtttgt tgagatcctg cgcgaacgcg atgttcaggc acagtggttt     420

gatgtacgta aagtgatgcg taccaacgac cgatttggtc gtgcagagcc agatatagcc 480

gcgctggcgg aactggccgc gctgcagctg ctcccacgtc tcaatgaagg cttagtgatc 540

acccagggat ttatcggtag cgaaaataaa ggtcgtacaa cgacgcttgg ccgtggaggc 600

agcgattata cggcagcctt gctggcggag gctttacacg catctcgtgt tgatatctgg 660

accgacgtcc cgggcatcta caccaccgat ccacgcgtag tttccgcagc aaaacgcatt 720

gatgaaatcg cgtttgccga agcggcagag atggcaactt ttggtgcaaa agtactgcat 780

ccggcaacgt tgctacccgc agtacgcagc gatatcccgg tctttgtcgg ctccagcaaa 840

gacccacgcg caggtggtac gctggtgtgc aataaaactg aaaatccgcc gctgttccgc 900

gctctggcgc ttcgtcgcaa tcagactctg ctcactttgc acagcctgaa tatgctgcat 960

tctcgcggtt tcctcgcgga agttttcggc atcctcgcgc ggcataatat ttcggtagac 1020

ttaatcacca cgtcagaagt gagcgtggca ttaatccttg ataccaccgg ttcaacctcc 1080

actggcgata cgttgctgac gcaatctctg ctgatggagc tttccgcact gtgtcgggtg 1140

gaggtggaag aaggtctggc gctggtcgcg ttgattggca atgacctgtc aaaagcctgc 1200

ggcgttggca aagaggtatt cggcgtactg gaaccgttca acattcgcat gatttgttat 1260

ggcgcatcca gccataacct gtgcttcctg gtgcccggcg aagatgccga gcaggtggtg 1320

caaaaactgc atagtaattt gtttgagtaa 1350

<210> 78
<211> 449
<212> PRT
<213> Escherichia coli

<400> 78

Met Ser Glu Ile Val Val Ser Lys Phe Gly Gly Thr Ser Val Ala Asp
1               5                   10                  15

Phe Asp Ala Met Asn Arg Ser Ala Asp Ile Val Leu Ser Asp Ala Asn
            20                  25                  30

Val Arg Leu Val Val Leu Ser Ala Ser Ala Gly Ile Thr Asn Leu Leu
        35                  40                  45

Val Ala Leu Ala Glu Gly Leu Glu Pro Gly Glu Arg Phe Glu Lys Leu
    50                  55                  60

Asp Ala Ile Arg Asn Ile Gln Phe Ala Ile Leu Glu Arg Leu Arg Tyr
65                  70                  75                  80

Pro Asn Val Ile Arg Glu Glu Ile Glu Arg Leu Leu Glu Asn Ile Thr
                85                  90                  95

Val Leu Ala Glu Ala Ala Ala Leu Ala Thr Ser Pro Ala Leu Thr Asp
            100                 105                 110

Glu Leu Val Ser His Gly Glu Leu Met Ser Thr Leu Leu Phe Val Glu
        115                 120                 125

Ile Leu Arg Glu Arg Asp Val Gln Ala Gln Trp Phe Asp Val Arg Lys
    130                 135                 140

Val Met Arg Thr Asn Asp Arg Phe Gly Arg Ala Glu Pro Asp Ile Ala
145                 150                 155                 160

Ala Leu Ala Glu Leu Ala Ala Leu Gln Leu Leu Pro Arg Leu Asn Glu
                165                 170                 175

Gly Leu Val Ile Thr Gln Gly Phe Ile Gly Ser Glu Asn Lys Gly Arg
            180                 185                 190

Thr Thr Thr Leu Gly Arg Gly Gly Ser Asp Tyr Thr Ala Ala Leu Leu
        195                 200                 205

Ala Glu Ala Leu His Ala Ser Arg Val Asp Ile Trp Thr Asp Val Pro
    210                 215                 220

Gly Ile Tyr Thr Thr Asp Pro Arg Val Val Ser Ala Ala Lys Arg Ile
225                 230                 235                 240

Asp Glu Ile Ala Phe Ala Glu Ala Ala Glu Met Ala Thr Phe Gly Ala
                245                 250                 255

Lys Val Leu His Pro Ala Thr Leu Leu Pro Ala Val Arg Ser Asp Ile
            260                 265                 270

Pro Val Phe Val Gly Ser Ser Lys Asp Pro Arg Ala Gly Gly Thr Leu
        275                 280                 285

Val Cys Asn Lys Thr Glu Asn Pro Pro Leu Phe Arg Ala Leu Ala Leu
    290                 295                 300

Arg Arg Asn Gln Thr Leu Leu Thr Leu His Ser Leu Asn Met Leu His
305                 310                 315                 320

Ser Arg Gly Phe Leu Ala Glu Val Phe Gly Ile Leu Ala Arg His Asn
                325                 330                 335

Ile Ser Val Asp Leu Ile Thr Thr Ser Glu Val Ser Val Ala Leu Ile
            340                 345                 350

Leu Asp Thr Thr Gly Ser Thr Ser Thr Gly Asp Thr Leu Leu Thr Gln
        355                 360                 365

Ser Leu Leu Met Glu Leu Ser Ala Leu Cys Arg Val Glu Val Glu Glu
    370                 375                 380

Gly Leu Ala Leu Val Ala Leu Ile Gly Asn Asp Leu Ser Lys Ala Cys
385                 390                 395                 400

Gly Val Gly Lys Glu Val Phe Gly Val Leu Glu Pro Phe Asn Ile Arg
                405                 410                 415

Met Ile Cys Tyr Gly Ala Ser Ser His Asn Leu Cys Phe Leu Val Pro
            420                 425                 430

Gly Glu Asp Ala Glu Gln Val Val Gln Lys Leu His Ser Asn Leu Phe
        435                 440                 445

Glu

<210>  79
<211>  930
<212>  DNA
<213>  Escherichia coli

<400>  79
atgccgattc gtgtgccgga cgagctaccc gccgtcaatt tcttgcgtga agaaaacgtc      60

tttgtgatga caacttctcg tgcgtctggt caggaaattc gtccacttaa ggttctgatc     120

cttaacctga tgccgaagaa gattgaaact gaaaatcagt ttctgcgcct gctttcaaac     180

tcacctttgc aggtcgatat tcagctgttg cgcatcgatt cccgtgaatc gcgcaacacg     240

cccgcagagc atctgaacaa cttctactgt aactttgaag atattcagga tcagaacttt     300

gacggtttga ttgtaactgg tgcgccgctg ggcctggtgg agtttaatga tgtcgcttac     360

tggccgcaga tcaaacaggt gctggagtgg tcgaaagatc acgtcacctc gacgctgttt     420

gtctgctggg cggtacaggc cgcgctcaat atcctctacg gcattcctaa gcaaactcgc     480

accgaaaaac tctctggcgt ttacgagcat catattctcc atcctcatgc gcttctgacg     540

cgtggctttg atgattcatt cctggcaccg cattcgcgct atgctgactt tccggcagcg     600

ttgattcgtg attacaccga tctggaaatt ctggcagaga cggaagaagg ggatgcatat     660

ctgtttgcca gtaaagataa gcgcattgcc tttgtgacgg gccatcccga atatgatgcg     720

caaacgctgg cgcaggaatt tttccgcgat gtggaagccg gactagaccc ggatgtaccg     780

tataactatt tcccgcacaa tgatccgcaa aatacaccgc gagcgagctg gcgtagtcac     840

ggtaatttac tgtttaccaa ctggctcaac tattacgtct accagatcac gccatacgat 900

ctacggcaca tgaatccaac gctggattaa 930

<210> 80
<211> 309
<212> PRT
<213> Escherichia coli

<400> 80

<210>  81
<211>  933
<212>  DNA
<213>  Escherichia coli

<400>  81
atggttaaag tttatgcccc ggcttccagt gccaatatga gcgtcgggtt tgatgtgctc      60

ggggcggcgg tgacacctgt tgatggtgca ttgctcggag atgtagtcac ggttgaggcg     120

gcagagacat tcagtctcaa caacctcgga cgctttgccg ataagctgcc gtcagaacca     180

cgggaaaata tcgtttatca gtgctgggag cgtttttgcc aggaactggg taagcaaatt     240

ccagtggcga tgaccctgga aaagaatatg ccgatcggtt cgggcttagg ctccagtgcc     300

tgttcggtgg tcgcggcgct gatggcgatg aatgaacact gcggcaagcc gcttaatgac     360

actcgtttgc tggctttgat gggcgagctg gaaggccgta tctccggcag cattcattac     420

gacaacgtgg caccgtgttt tctcggtggt atgcagttga tgatcgaaga aaacgacatc     480

atcagccagc aagtgccagg gtttgatgag tggctgtggg tgctggcgta tccggggatt     540

aaagtctcga cggcagaagc cagggctatt ttaccggcgc agtatcgccg ccaggattgc     600

attgcgcacg ggcgacatct ggcaggcttc attcacgcct gctattcccg tcagcctgag     660

cttgccgcga agctgatgaa agatgttatc gctgaaccct accgtgaacg gttactgcca     720

ggcttccggc aggcgcggca ggcggtcgcg gaaatcggcg cggtagcgag cggtatctcc     780

ggctccggcc cgaccttgtt cgctctgtgt gacaagccgg aaaccgccca gcgcgttgcc     840

gactggttgg gtaagaacta cctgcaaaat caggaaggtt ttgttcatat ttgccggctg 900

gatacggcgg gcgcacgagt actggaaaac taa 933

<210> 82
<211> 310
<212> PRT
<213> Escherichia coli

<400> 82

Met Val Lys Val Tyr Ala Pro Ala Ser Ser Ala Asn Met Ser Val Gly
1               5                   10                  15

Phe Asp Val Leu Gly Ala Ala Val Thr Pro Val Asp Gly Ala Leu Leu
            20                  25                  30

Gly Asp Val Val Thr Val Glu Ala Ala Glu Thr Phe Ser Leu Asn Asn
        35                  40                  45

Leu Gly Arg Phe Ala Asp Lys Leu Pro Ser Glu Pro Arg Glu Asn Ile
    50                  55                  60

Val Tyr Gln Cys Trp Glu Arg Phe Cys Gln Glu Leu Gly Lys Gln Ile
65                  70                  75                  80

Pro Val Ala Met Thr Leu Glu Lys Asn Met Pro Ile Gly Ser Gly Leu
                85                  90                  95

Gly Ser Ser Ala Cys Ser Val Val Ala Ala Leu Met Ala Met Asn Glu
            100                 105                 110

His Cys Gly Lys Pro Leu Asn Asp Thr Arg Leu Leu Ala Leu Met Gly
        115                 120                 125

Glu Leu Glu Gly Arg Ile Ser Gly Ser Ile His Tyr Asp Asn Val Ala
    130                 135                 140

Pro Cys Phe Leu Gly Gly Met Gln Leu Met Ile Glu Glu Asn Asp Ile
145                 150                 155                 160

Ile Ser Gln Gln Val Pro Gly Phe Asp Glu Trp Leu Trp Val Leu Ala
                165                 170                 175

Tyr Pro Gly Ile Lys Val Ser Thr Ala Glu Ala Arg Ala Ile Leu Pro
            180                 185                 190

Ala Gln Tyr Arg Arg Gln Asp Cys Ile Ala His Gly Arg His Leu Ala
        195                 200                 205

Gly Phe Ile His Ala Cys Tyr Ser Arg Gln Pro Glu Leu Ala Ala Lys
    210                 215                 220

Leu Met Lys Asp Val Ile Ala Glu Pro Tyr Arg Glu Arg Leu Leu Pro
225                 230                 235                 240

Gly Phe Arg Gln Ala Arg Gln Ala Val Ala Glu Ile Gly Ala Val Ala
                245                 250                 255

Ser Gly Ile Ser Gly Ser Gly Pro Thr Leu Phe Ala Leu Cys Asp Lys
            260                 265                 270

Pro Glu Thr Ala Gln Arg Val Ala Asp Trp Leu Gly Lys Asn Tyr Leu
        275                 280                 285

Gln Asn Gln Glu Gly Phe Val His Ile Cys Arg Leu Asp Thr Ala Gly
    290                 295                 300

Ala Arg Val Leu Glu Asn
305                 310

<210>  83
<211>  2652
<212>  DNA
<213>  Escherichia coli

<400>  83
atgaacgaac aatattccgc attgcgtagt aatgtcagta tgctcggcaa agtgctggga      60

gaaaccatca aggatgcgtt gggagaacac attcttgaac gcgtagaaac tatccgtaag     120

ttgtcgaaat cttcacgcgc tggcaatgat gctaaccgcc aggagttgct caccacctta     180

caaaatttgt cgaacgacga gctgctgccc gttgcgcgtg cgtttagtca gttcctgaac     240

ctggccaaca ccgccgagca ataccacagc atttcgccga aaggcgaagc tgccagcaac     300

ccggaagtga tcgcccgcac cctgcgtaaa ctgaaaaacc agccggaact gagcgaagac     360

accatcaaaa aagcagtgga atcgctgtcg ctggaactgg tcctcacggc tcacccaacc     420

gaaattaccc gtcgtacact gatccacaaa atggtggaag tgaacgcctg tttaaaacag     480

ctcgataaca aagatatcgc tgactacgaa cacaaccagc tgatgcgtcg cctgcgccag     540

ttgatcgccc agtcatggca taccgatgaa atccgtaagc tgcgtccaag cccggtagat     600

gaagccaaat ggggctttgc cgtagtggaa aacagcctgt ggcaaggcgt accaaattac     660

ctgcgcgaac tgaacgaaca actggaagag aacctcggct acaaactgcc cgtcgaattt     720

gttccggtcc gttttacttc gtggatgggc ggcgaccgcg acggcaaccc gaacgtcact     780

gccgatatca cccgccacgt cctgctactc agccgctgga aagccaccga tttgttcctg     840

aaagatattc aggtgctggt ttctgaactg tcgatggttg aagcgacccc tgaactgctg 900

gcgctggttg gcgaagaagg tgccgcagaa ccgtatcgct atctgatgaa aaacctgcgt 960

tctcgcctga tggcgacaca ggcatggctg gaagcgcgcc tgaaaggcga agaactgcca 1020

aaaccagaag gcctgctgac acaaaacgaa gaactgtggg aaccgctcta cgcttgctac 1080

cagtcacttc aggcgtgtgg catgggtatt atcgccaacg gcgatctgct cgacaccctg 1140

cgccgcgtga aatgtttcgg cgtaccgctg gtccgtattg atatccgtca ggagagcacg 1200

cgtcataccg aagcgctggg cgagctgacc cgctacctcg gtatcggcga ctacgaaagc 1260

tggtcagagg ccgacaaaca ggcgttcctg atccgcgaac tgaactccaa acgtccgctt 1320

ctgccgcgca actggcaacc aagcgccgaa acgcgcgaag tgctcgatac ctgccaggtg 1380

attgccgaag caccgcaagg ctccattgcc gcctacgtga tctcgatggc gaaaacgccg 1440

tccgacgtac tggctgtcca cctgctgctg aaagaagcgg gtatcgggtt tgcgatgccg 1500

gttgctccgc tgtttgaaac cctcgatgat ctgaacaacg ccaacgatgt catgacccag 1560

ctgctcaata ttgactggta tcgtggcctg attcagggca aacagatggt gatgattggc 1620

tattccgact cagcaaaaga tgcgggagtg atggcagctt cctgggcgca atatcaggca 1680

caggatgcat taatcaaaac ctgcgaaaaa gcgggtattg agctgacgtt gttccacggt 1740

cgcggcggtt ccattggtcg cggcggcgca cctgctcatg cggcgctgct gtcacaaccg 1800

ccaggaagcc tgaaaggcgg cctgcgcgta accgaacagg gcgagatgat ccgctttaaa 1860

tatggtctgc cagaaatcac cgtcagcagc ctgtcgcttt ataccggggc gattctggaa 1920

gccaacctgc tgccaccgcc ggagccgaaa gagagctggc gtcgcattat ggatgaactg 1980

tcagtcatct cctgcgatgt ctaccgcggc tacgtacgtg aaaacaaaga ttttgtgcct 2040

tacttccgct ccgctacgcc ggaacaagaa ctgggcaaac tgccgttggg ttcacgtccg 2100

gcgaaacgtc gcccaaccgg cggcgtcgag tcactacgcg ccattccgtg gatcttcgcc 2160

tggacgcaaa accgtctgat gctccccgcc tggctgggtg caggtacggc gctgcaaaaa 2220

gtggtcgaag acggcaaaca gagcgagctg gaggctatgt gccgcgattg gccattcttc 2280

tcgacgcgtc tcggcatgct ggagatggtc ttcgccaaag cagacctgtg gctggcggaa 2340

tactatgacc aacgcctggt agacaaagca ctgtggccgt taggtaaaga gttacgcaac 2400

ctgcaagaag aagacatcaa agtggtgctg gcgattgcca acgattccca tctgatggcc 2460

gatctgccgt ggattgcaga gtctattcag ctacggaata tttacaccga cccgctgaac 2520

gtattgcagg ccgagttgct gcaccgctcc cgccaggcag aaaaagaagg ccaggaaccg 2580

gatcctcgcg tcgaacaagc gttaatggtc actattgccg ggattgcggc aggtatgcgt 2640

aataccggct aa 2652

<210>  84
<211>  883
<212>  PRT
<213>  Escherichia coli

<400>  84

Met Asn Glu Gln Tyr Ser Ala Leu Arg Ser Asn Val Ser Met Leu Gly
1               5                   10                  15

Lys Val Leu Gly Glu Thr Ile Lys Asp Ala Leu Gly Glu His Ile Leu
            20                  25                  30

Glu Arg Val Glu Thr Ile Arg Lys Leu Ser Lys Ser Ser Arg Ala Gly
        35                  40                  45

Asn Asp Ala Asn Arg Gln Glu Leu Leu Thr Thr Leu Gln Asn Leu Ser
    50                  55                  60

Asn Asp Glu Leu Leu Pro Val Ala Arg Ala Phe Ser Gln Phe Leu Asn
65                  70                  75                  80

Leu Ala Asn Thr Ala Glu Gln Tyr His Ser Ile Ser Pro Lys Gly Glu
                85                  90                  95

Ala Ala Ser Asn Pro Glu Val Ile Ala Arg Thr Leu Arg Lys Leu Lys
            100                 105                 110

Asn Gln Pro Glu Leu Ser Glu Asp Thr Ile Lys Lys Ala Val Glu Ser
        115                 120                 125

Leu Ser Leu Glu Leu Val Leu Thr Ala His Pro Thr Glu Ile Thr Arg
    130                 135                 140

Arg Thr Leu Ile His Lys Met Val Glu Val Asn Ala Cys Leu Lys Gln
145                 150                 155                 160

Leu Asp Asn Lys Asp Ile Ala Asp Tyr Glu His Asn Gln Leu Met Arg
                165                 170                 175

Arg Leu Arg Gln Leu Ile Ala Gln Ser Trp His Thr Asp Glu Ile Arg
            180                 185                 190

Lys Leu Arg Pro Ser Pro Val Asp Glu Ala Lys Trp Gly Phe Ala Val
        195                 200                 205

Val Glu Asn Ser Leu Trp Gln Gly Val Pro Asn Tyr Leu Arg Glu Leu
    210                 215                 220

Asn Glu Gln Leu Glu Glu Asn Leu Gly Tyr Lys Leu Pro Val Glu Phe
225                 230                 235                 240

Val Pro Val Arg Phe Thr Ser Trp Met Gly Gly Asp Arg Asp Gly Asn
                245                 250                 255

Pro Asn Val Thr Ala Asp Ile Thr Arg His Val Leu Leu Leu Ser Arg
            260                 265                 270

Trp Lys Ala Thr Asp Leu Phe Leu Lys Asp Ile Gln Val Leu Val Ser
        275                 280                 285

Glu Leu Ser Met Val Glu Ala Thr Pro Glu Leu Leu Ala Leu Val Gly
    290                 295                 300

Glu Glu Gly Ala Ala Glu Pro Tyr Arg Tyr Leu Met Lys Asn Leu Arg
305                 310                 315                 320

Ser Arg Leu Met Ala Thr Gln Ala Trp Leu Glu Ala Arg Leu Lys Gly
                325                 330                 335

Glu Glu Leu Pro Lys Pro Glu Gly Leu Leu Thr Gln Asn Glu Glu Leu
            340                 345                 350

Trp Glu Pro Leu Tyr Ala Cys Tyr Gln Ser Leu Gln Ala Cys Gly Met
        355                 360                 365

Gly Ile Ile Ala Asn Gly Asp Leu Leu Asp Thr Leu Arg Arg Val Lys
    370                 375                 380

Cys Phe Gly Val Pro Leu Val Arg Ile Asp Ile Arg Gln Glu Ser Thr
385                 390                 395                 400

Arg His Thr Glu Ala Leu Gly Glu Leu Thr Arg Tyr Leu Gly Ile Gly
                405                 410                 415

Asp Tyr Glu Ser Trp Ser Glu Ala Asp Lys Gln Ala Phe Leu Ile Arg
            420                 425                 430

Glu Leu Asn Ser Lys Arg Pro Leu Leu Pro Arg Asn Trp Gln Pro Ser
        435                 440                 445

Ala Glu Thr Arg Glu Val Leu Asp Thr Cys Gln Val Ile Ala Glu Ala
    450                 455                 460

Pro Gln Gly Ser Ile Ala Ala Tyr Val Ile Ser Met Ala Lys Thr Pro
465                 470                 475                 480

Ser Asp Val Leu Ala Val His Leu Leu Leu Lys Glu Ala Gly Ile Gly
                485                 490                 495

Phe Ala Met Pro Val Ala Pro Leu Phe Glu Thr Leu Asp Asp Leu Asn
            500                 505                 510

Asn Ala Asn Asp Val Met Thr Gln Leu Leu Asn Ile Asp Trp Tyr Arg
        515                 520                 525

Gly Leu Ile Gln Gly Lys Gln Met Val Met Ile Gly Tyr Ser Asp Ser
    530                 535                 540

Ala Lys Asp Ala Gly Val Met Ala Ala Ser Trp Ala Gln Tyr Gln Ala
545                 550                 555                 560

Gln Asp Ala Leu Ile Lys Thr Cys Glu Lys Ala Gly Ile Glu Leu Thr
                565                 570                 575

Leu Phe His Gly Arg Gly Gly Ser Ile Gly Arg Gly Gly Ala Pro Ala
            580                 585                 590

His Ala Ala Leu Leu Ser Gln Pro Pro Gly Ser Leu Lys Gly Gly Leu
        595                 600                 605

Arg Val Thr Glu Gln Gly Glu Met Ile Arg Phe Lys Tyr Gly Leu Pro
    610                 615                 620

Glu Ile Thr Val Ser Ser Leu Ser Leu Tyr Thr Gly Ala Ile Leu Glu
625                 630                 635                 640

Ala Asn Leu Leu Pro Pro Pro Glu Pro Lys Glu Ser Trp Arg Arg Ile
                645                 650                 655

Met Asp Glu Leu Ser Val Ile Ser Cys Asp Val Tyr Arg Gly Tyr Val
            660                 665                 670

Arg Glu Asn Lys Asp Phe Val Pro Tyr Phe Arg Ser Ala Thr Pro Glu
        675                 680                 685

Gln Glu Leu Gly Lys Leu Pro Leu Gly Ser Arg Pro Ala Lys Arg Arg
    690                 695                 700

Pro Thr Gly Gly Val Glu Ser Leu Arg Ala Ile Pro Trp Ile Phe Ala
705                 710                 715                 720

Trp Thr Gln Asn Arg Leu Met Leu Pro Ala Trp Leu Gly Ala Gly Thr

                725                 730                 735

Ala Leu Gln Lys Val Val Glu Asp Gly Lys Gln Ser Glu Leu Glu Ala
            740                 745                 750

Met Cys Arg Asp Trp Pro Phe Phe Ser Thr Arg Leu Gly Met Leu Glu
        755                 760                 765

Met Val Phe Ala Lys Ala Asp Leu Trp Leu Ala Glu Tyr Tyr Asp Gln
    770                 775                 780

Arg Leu Val Asp Lys Ala Leu Trp Pro Leu Gly Lys Glu Leu Arg Asn
785                 790                 795                 800

Leu Gln Glu Glu Asp Ile Lys Val Val Leu Ala Ile Ala Asn Asp Ser
                805                 810                 815

His Leu Met Ala Asp Leu Pro Trp Ile Ala Glu Ser Ile Gln Leu Arg
            820                 825                 830

Asn Ile Tyr Thr Asp Pro Leu Asn Val Leu Gln Ala Glu Leu Leu His
        835                 840                 845

Arg Ser Arg Gln Ala Glu Lys Glu Gly Gln Glu Pro Asp Pro Arg Val
    850                 855                 860

Glu Gln Ala Leu Met Val Thr Ile Ala Gly Ile Ala Ala Gly Met Arg
865                 870                 875                 880

Asn Thr Gly

<210> 85
<211> 3465
<212> DNA
<213> Rhizobium etli

<400> 85
ttgcccatat ccaagatact cgttgccaat cgctctgaaa tagccatccg cgtgttccgc 60

gcggccaacg agcttggaat aaaaacggtg gcgatctggg cggaagagga caagctggcg 120

ctgcaccgct tcaaggcgga cgagagttat caggtcggcc gcggaccgca tcttgcccgc 180

gacctcgggc cgatcgaaag ctatctgtcg atcgacgagg tgatccgcgt cgccaagctt 240

tccggtgccg acgccatcca tccgggctac ggcctcttgt cggaaagccc cgaattcgtc 300

gatgcctgca acaaggccgg catcatcttc atcggcccga aggccgatac gatgcgccag 360

cttggcaaca aggtcgcagc gcgcaacctg gcgatctcgg tcggcgtacc ggtcgtgccg 420

gcgaccgagc cactgccgga cgatatggcc gaagtggcga agatggcggc ggcgatcggc 480

tatcccgtca tgctgaaggc atcctggggc ggcggcggtc gcggcatgcg cgtcattcgt 540

tccgaggccg acctcgccaa ggaagtgacg gaagccaagc gcgaggcgat ggcggccttc 600

ggcaaggacg aggtctatct cgaaaaactg gtcgagcgcg cccgccacgt cgaaagccag 660

atcctcggcg acacccacgg caatgtcgtg catctcttcg agcgcgactg ttccgttcag 720

cgccgcaatc agaaggtcgt cgagcgcgcg cccgcaccct atctttcgga agcgcagcgc 780

caggaactcg ccgcctattc gctgaagatc gcaggggcga ccaactatat cggcgccggc 840

accgtcgaat atctgatgga tgccgatacc ggcaaatttt acttcatcga agtcaatccg 900

cgcatccagg tcgagcacac ggtgaccgaa gtcgtcaccg gcatcgatat cgtcaaggcg 960

cagatccaca tcctggacgg cgccgcgatc ggcacgccgc aatccggcgt gccgaaccag 1020

gaagacatcc gtctcaacgg tcacgccctg cagtgccgcg tgacgacgga agatccggag 1080

cacaacttca ttccggatta cggccgcatc accgcctatc gctcggcttc cggcttcggc 1140

atccggcttg acggcggcac ctcttattcc ggcgccatca tcacccgcta ttacgatccg 1200

ctgctcgtca aggtcacggc ctgggcgccg aacccgctgg aagccatttc ccgcatggac 1260

cgggcgctgc gcgaattccg catccgtggc gtcgccacca acctgacctt cctcgaagcg 1320

atcatcggcc atccgaaatt ccgcgacaac agctacacca cccgcttcat cgacacgacg 1380

ccggagctct tccagcaggt caagcgccag gaccgcgcga cgaagcttct gacctatctc 1440

gccgacgtca ccgtcaatgg ccatcccgag gccaaggaca ggccgaagcc cctcgagaat 1500

gccgccaggc cggtggtgcc ctatgccaat ggcaacgggg tgaaggacgg caccaagcag 1560

ctgctcgata cgctcggccc gaaaaaattc ggcgaatgga tgcgcaatga gaagcgcgtg 1620

cttctgaccg acaccacgat gcgcgacggc caccagtcgc tgctcgcaac ccgcatgcgt 1680

acctatgaca tcgccaggat cgccggcacc tattcgcatg cgctgccgaa cctcttgtcg 1740

ctcgaatgct ggggcggcgc caccttcgac gtctcgatgc gcttcctcac cgaagatccg 1800

tgggagcggc tggcgctgat ccgagagggg gcgccgaacc tgctcctgca gatgctgctg 1860

cgcggcgcca atggcgtcgg ttacaccaac tatcccgaca atgtcgtcaa atacttcgtc 1920

cgccaggcgg ccaaaggcgg catcgatctc ttccgcgtct tcgactgcct gaactgggtc 1980

gagaatatgc gggtgtcgat ggatgcgatt gccgaggaga acaagctctg cgaggcggcg 2040

atctgctaca ccggcgatat cctcaattcc gcccgcccga aatacgactt gaaatattac 2100

accaaccttg ccgtcgagct tgagaaggcc ggcgcccata tcattgcggt caaggatatg 2160

gcgggccttc tgaagccggc tgctgccaag gttctgttca aggcgctgcg tgaagcaacc 2220

ggcctgccga tccatttcca cacgcatgac acctcgggca ttgcggcggc aacggttctt 2280

gccgccgtcg aagccggtgt cgatgccgtc gatgcggcga tggatgcgct ctccggcaac 2340

acctcgcaac cctgtctcgg ctcgatcgtc gaggcgctct ccggctccga gcgcgatccc 2400

ggcctcgatc cggcatggat ccgccgcatc tccttctatt gggaagcggt gcgcaaccag 2460

tatgccgcct tcgaaagcga cctcaaggga ccggcatcgg aagtctatct gcatgaaatg 2520

ccgggcggcc agttcaccaa cctcaaggag caggcccgct cgctggggct ggaaacccgc 2580

tggcaccagg tggcgcaggc ctatgccgac gccaaccaga tgttcggcga tatcgtcaag 2640

gtgacgccat cctccaaggt cgtcggcgac atggcgctga tgatggtctc ccaggacctg 2700

accgtcgccg atgtcgtcag ccccgaccgc gaagtctcct tcccggaatc ggtcgtctcg 2760

atgctgaagg gcgatctcgg ccagcctccg tctggatggc cggaagcgct gcagaagaaa 2820

gcattgaagg gcgaaaagcc ctatacggtg cgccccggct cgctgctcaa ggaagccgat 2880

ctcgatgcgg aacgcaaagt catcgagaag aagcttgagc gcgaggtcag cgacttcgaa 2940

ttcgcttcct atctgatgta tccgaaggtc ttcaccgact ttgcgcttgc ctccgatacc 3000

tacggtccgg tttcggtgct gccgacgccc gcctattttt acgggttggc ggacggcgag 3060

gagctgttcg ccgacatcga gaagggcaag acgctcgtca tcgtcaatca ggcggtgagc 3120

gccaccgaca gccagggcat ggtcactgtc ttcttcgagc tcaacggcca gccgcgccgt 3180

atcaaggtgc ccgatcgggc ccacggggcg acgggagccg ccgtgcgccg caaggccgaa 3240

cccggcaatg ccgcccatgt cggtgcgccg atgccgggcg tcatcagccg tgtctttgtc 3300

tcttcaggcc aggccgtcaa tgccggcgac gtgctcgtct ccatcgaggc catgaagatg 3360

gaaaccgcga tccatgcgga aaaggacggc accattgccg aagtgctggt caaggccggc 3420

gatcagatcg atgccaagga cctgctggcg gtttacggcg gatga 3465

<210> 86
<211> 1154
<212> PRT
<213> Rhizobium etli

<400> 86

Leu Pro Ile Ser Lys Ile Leu Val Ala Asn Arg Ser Glu Ile Ala Ile
1               5                   10                  15

Arg Val Phe Arg Ala Ala Asn Glu Leu Gly Ile Lys Thr Val Ala Ile
            20                  25                  30

Trp Ala Glu Glu Asp Lys Leu Ala Leu His Arg Phe Lys Ala Asp Glu
        35                  40                  45

Ser Tyr Gln Val Gly Arg Gly Pro His Leu Ala Arg Asp Leu Gly Pro
    50                  55                  60

Ile Glu Ser Tyr Leu Ser Ile Asp Glu Val Ile Arg Val Ala Lys Leu
65                  70                  75                  80

Ser Gly Ala Asp Ala Ile His Pro Gly Tyr Gly Leu Leu Ser Glu Ser
                85                  90                  95

Pro Glu Phe Val Asp Ala Cys Asn Lys Ala Gly Ile Ile Phe Ile Gly
            100                 105                 110

Pro Lys Ala Asp Thr Met Arg Gln Leu Gly Asn Lys Val Ala Ala Arg
        115                 120                 125

Asn Leu Ala Ile Ser Val Gly Val Pro Val Val Pro Ala Thr Glu Pro
    130                 135                 140

Leu Pro Asp Asp Met Ala Glu Val Ala Lys Met Ala Ala Ala Ile Gly
145                 150                 155                 160

Tyr Pro Val Met Leu Lys Ala Ser Trp Gly Gly Gly Gly Arg Gly Met
                165                 170                 175

Arg Val Ile Arg Ser Glu Ala Asp Leu Ala Lys Glu Val Thr Glu Ala
            180                 185                 190

Lys Arg Glu Ala Met Ala Ala Phe Gly Lys Asp Glu Val Tyr Leu Glu
        195                 200                 205

Lys Leu Val Glu Arg Ala Arg His Val Glu Ser Gln Ile Leu Gly Asp
    210                 215                 220

Thr His Gly Asn Val Val His Leu Phe Glu Arg Asp Cys Ser Val Gln
225                 230                 235                 240

Arg Arg Asn Gln Lys Val Val Glu Arg Ala Pro Ala Pro Tyr Leu Ser
                245                 250                 255

Glu Ala Gln Arg Gln Glu Leu Ala Ala Tyr Ser Leu Lys Ile Ala Gly
            260                 265                 270

Ala Thr Asn Tyr Ile Gly Ala Gly Thr Val Glu Tyr Leu Met Asp Ala
        275                 280                 285

Asp Thr Gly Lys Phe Tyr Phe Ile Glu Val Asn Pro Arg Ile Gln Val
    290                 295                 300

Glu His Thr Val Thr Glu Val Val Thr Gly Ile Asp Ile Val Lys Ala
305                 310                 315                 320

Gln Ile His Ile Leu Asp Gly Ala Ala Ile Gly Thr Pro Gln Ser Gly
                325                 330                 335

Val Pro Asn Gln Glu Asp Ile Arg Leu Asn Gly His Ala Leu Gln Cys
            340                 345                 350

Arg Val Thr Thr Glu Asp Pro Glu His Asn Phe Ile Pro Asp Tyr Gly
        355                 360                 365

Arg Ile Thr Ala Tyr Arg Ser Ala Ser Gly Phe Gly Ile Arg Leu Asp
    370                 375                 380

Gly Gly Thr Ser Tyr Ser Gly Ala Ile Ile Thr Arg Tyr Tyr Asp Pro
385                 390                 395                 400

Leu Leu Val Lys Val Thr Ala Trp Ala Pro Asn Pro Leu Glu Ala Ile
                405                 410                 415

Ser Arg Met Asp Arg Ala Leu Arg Glu Phe Arg Ile Arg Gly Val Ala
            420                 425                 430

Thr Asn Leu Thr Phe Leu Glu Ala Ile Ile Gly His Pro Lys Phe Arg
        435                 440                 445

Asp Asn Ser Tyr Thr Thr Arg Phe Ile Asp Thr Thr Pro Glu Leu Phe
    450                 455                 460

Gln Gln Val Lys Arg Gln Asp Arg Ala Thr Lys Leu Leu Thr Tyr Leu
465                 470                 475                 480

Ala Asp Val Thr Val Asn Gly His Pro Glu Ala Lys Asp Arg Pro Lys
                485                 490                 495

Pro Leu Glu Asn Ala Ala Arg Pro Val Val Pro Tyr Ala Asn Gly Asn
            500                 505                 510

Gly Val Lys Asp Gly Thr Lys Gln Leu Leu Asp Thr Leu Gly Pro Lys
        515                 520                 525

Lys Phe Gly Glu Trp Met Arg Asn Glu Lys Arg Val Leu Leu Thr Asp
    530                 535                 540

Thr Thr Met Arg Asp Gly His Gln Ser Leu Leu Ala Thr Arg Met Arg
545                 550                 555                 560

Thr Tyr Asp Ile Ala Arg Ile Ala Gly Thr Tyr Ser His Ala Leu Pro
                565                 570                 575

Asn Leu Leu Ser Leu Glu Cys Trp Gly Gly Ala Thr Phe Asp Val Ser
            580                 585                 590

Met Arg Phe Leu Thr Glu Asp Pro Trp Glu Arg Leu Ala Leu Ile Arg
        595                 600                 605

Glu Gly Ala Pro Asn Leu Leu Leu Gln Met Leu Leu Arg Gly Ala Asn
    610                 615                 620

Gly Val Gly Tyr Thr Asn Tyr Pro Asp Asn Val Val Lys Tyr Phe Val
625                 630                 635                 640

Arg Gln Ala Ala Lys Gly Gly Ile Asp Leu Phe Arg Val Phe Asp Cys
                645                 650                 655

Leu Asn Trp Val Glu Asn Met Arg Val Ser Met Asp Ala Ile Ala Glu
            660                 665                 670

Glu Asn Lys Leu Cys Glu Ala Ala Ile Cys Tyr Thr Gly Asp Ile Leu
        675                 680                 685

Asn Ser Ala Arg Pro Lys Tyr Asp Leu Lys Tyr Tyr Thr Asn Leu Ala
    690                 695                 700

Val Glu Leu Glu Lys Ala Gly Ala His Ile Ile Ala Val Lys Asp Met
705                 710                 715                 720

Ala Gly Leu Leu Lys Pro Ala Ala Ala Lys Val Leu Phe Lys Ala Leu
                725                 730                 735

Arg Glu Ala Thr Gly Leu Pro Ile His Phe His Thr His Asp Thr Ser
            740                 745                 750

Gly Ile Ala Ala Ala Thr Val Leu Ala Ala Val Glu Ala Gly Val Asp
        755                 760                 765

Ala Val Asp Ala Ala Met Asp Ala Leu Ser Gly Asn Thr Ser Gln Pro
    770                 775                 780

Cys Leu Gly Ser Ile Val Glu Ala Leu Ser Gly Ser Glu Arg Asp Pro
785                 790                 795                 800

Gly Leu Asp Pro Ala Trp Ile Arg Arg Ile Ser Phe Tyr Trp Glu Ala
                805                 810                 815

Val Arg Asn Gln Tyr Ala Ala Phe Glu Ser Asp Leu Lys Gly Pro Ala

            820                 825                 830

Ser Glu Val Tyr Leu His Glu Met Pro Gly Gly Gln Phe Thr Asn Leu
        835                 840                 845

Lys Glu Gln Ala Arg Ser Leu Gly Leu Glu Thr Arg Trp His Gln Val
    850                 855                 860

Ala Gln Ala Tyr Ala Asp Ala Asn Gln Met Phe Gly Asp Ile Val Lys
865                 870                 875                 880

Val Thr Pro Ser Ser Lys Val Val Gly Asp Met Ala Leu Met Met Val
                885                 890                 895

Ser Gln Asp Leu Thr Val Ala Asp Val Val Ser Pro Asp Arg Glu Val
            900                 905                 910

Ser Phe Pro Glu Ser Val Val Ser Met Leu Lys Gly Asp Leu Gly Gln
        915                 920                 925

Pro Pro Ser Gly Trp Pro Glu Ala Leu Gln Lys Lys Ala Leu Lys Gly
    930                 935                 940

Glu Lys Pro Tyr Thr Val Arg Pro Gly Ser Leu Leu Lys Glu Ala Asp
945                 950                 955                 960

Leu Asp Ala Glu Arg Lys Val Ile Glu Lys Lys Leu Glu Arg Glu Val
                965                 970                 975

Ser Asp Phe Glu Phe Ala Ser Tyr Leu Met Tyr Pro Lys Val Phe Thr
            980                 985                 990

Asp Phe Ala Leu Ala Ser Asp Thr Tyr Gly Pro Val Ser Val Leu Pro
        995                 1000                1005

Thr Pro Ala Tyr Phe Tyr Gly Leu Ala Asp Gly Glu Glu Leu Phe
    1010                1015                1020

Ala Asp Ile Glu Lys Gly Lys Thr Leu Val Ile Val Asn Gln Ala
    1025                1030                1035

Val Ser Ala Thr Asp Ser Gln Gly Met Val Thr Val Phe Phe Glu
    1040                1045                1050

Leu Asn Gly Gln Pro Arg Arg Ile Lys Val Pro Asp Arg Ala His
    1055                1060                1065

Gly Ala Thr Gly Ala Ala Val Arg Arg Lys Ala Glu Pro Gly Asn
    1070                1075                1080

Ala Ala His Val Gly Ala Pro Met Pro Gly Val Ile Ser Arg Val
    1085                1090                1095

Phe Val Ser Ser Gly Gln Ala Val Asn Ala Gly Asp Val Leu Val
    1100                1105                1110

Ser Ile Glu Ala Met Lys Met Glu Thr Ala Ile His Ala Glu Lys
    1115                1120                1125

Asp Gly Thr Ile Ala Glu Val Leu Val Lys Ala Gly Asp Gln Ile
    1130                1135                1140

Asp Ala Lys Asp Leu Leu Ala Val Tyr Gly Gly
    1145                1150

<210> 87
<211> 3501
<212> DNA
<213> Ralstonia eutropha

<400> 87
atggactacg cccctatccg ctccctgctg attgccaacc gttccgaggc gatccgcgtg 60

atgcgcgcgg ccgccgagat gaacgtgcgc acggtggcaa tctattcgaa ggaagaccgg 120

ctcgcgctcc atcgcttcaa ggccgatgag agctacctgg tcggcgaggg caagaagcca 180

ctggcggctt acctcgacat cgacgatatc ctgcgcattg ccaggcaggc gaaggtcgac 240

gccattcatc cgggctatgg cttcctttca gagaacccgg acttcgcgca ggccgtgatc 300

gacgcgggta tccgctggat cggcccgtcg cccgaggtca tgcgcaagct tggcaacaag 360

gtggcggcgc gcaacgcggc gatcgacgcg ggcgtgccgg tgatgccggc aaccgatccg 420

ctgccgcatg acctggacac gtgcaagcgc ctcgccgccg gcatcggcta tccgctgatg 480

ctcaaggcaa gctggggcgg cggcggacgc ggcatgcggg tcctggaacg cgagcaggac 540

cttgaggggg cgctcgccgc ggcgcggcgc gaggcgctgg ctgcgttcgg caacgacgag 600

gtgtatgtcg agaagctggt gcgcaacgcg cgccatgtcg aagtgcaggt gctcggcgac 660

acgcacggca acctcgtgca tctctatgag cgcgactgta ccgtgcagcg gcgcaaccag 720

aaggtggtgg agcgggcgcc cgcgccatac ctcgacgatg ccggccgggc cgcgctgtgc 780

gaatcggccc tgcggctgat gcgcgcggtc ggctacacgc atgccggtac ggtcgagttc 840

ctgatggatg ccgactccgg ccagttctac ttcatcgagg tcaatccgcg catccaggtc 900

gagcacacgg tcacggagat ggtcaccggg atcgatatcg tcaaggcgca gatccgcgtg 960

accgaaggcg gccatctcgg catgaccgag aacacgcgca atgagaacgg cgagatcgtc 1020

gtgcgcgccg cgggcgtgcc ggtgcaggaa gcgatttcgc tcaacggtca cgcgctgcaa 1080

tgccggatca ccaccgagga cccggagaac gggttcctgc cggactacgg ccgcctcact 1140

gcctaccgca gcgcggccgg cttcggcgtg cgcctggacg ccggcaccgc ctacggcggc 1200

gcggtgatca cgccgtacta cgattcgctg ctggtcaagg ttaccacctg ggcgccgacc 1260

gcgcccgaat cgatccggcg catggaccgc gcgctgcgcg agttccgcat ccgcggcgtc 1320

gcgtccaacc tgcagttcct cgagaacgtc atcaaccatc cctcgttccg gtccggcgac 1380

gtcaccacgc gctttatcga cctgacgccg gaactgctgg cgttcaccaa gcgcctggac 1440

cgcgccacca agctgctgcg ctacctgggc gaggtcagcg tcaacggcca cccggagatg 1500

agcggccgca cgctgccatc gctgccgctg cccgcaccgg tgctgcccgc cttcgacacc 1560

ggcggcgcgc tgccctacgg tacgcgcgac cggctgcgcg agctgggcgc ggagaagttc 1620

tcgcgctgga tgctggagca gaagcaggtg ctgctgaccg ataccaccat gcgcgacgcg 1680

caccagtcgc tgttcgccac gcgcatgcgc accgccgaca tgctgccgat cgcgccgttc 1740

tatgcgcgcg aactgtcgca gctgttctcg ctggagtgct ggggcggcgc caccttcgac 1800

gtggcgctgc gcttcctcaa ggaagacccg tggcagcgcc ttgagcaact gcgcgagcgc 1860

gttcccaacg tgctgttcca gatgctgctg cgcggctcca acgcggttgg ctacaccaat 1920

tatgcggaca acgtggtgcg cttcttcgtg cgccaggcgg ccagcgccgg cgtggatgtg 1980

ttccgcgtgt tcgattcact gaactgggtg cgcaacatgc gcgtggcgat cgatgctgtc 2040

ggcgagagcg gcgcgctgtg cgaaggcgcg atctgctata ccggcgacct gttcgacaag 2100

tcgcgcgcca aatacgacct gaagtactac gtaggcatcg cgcgcgagct gaagcaggcc 2160

ggcgtgcacg tgctgggcat caaggacatg gccggcatct gccgtccgca ggccgcggcg 2220

gcactggtca gggcgctcaa ggaagagacc gggctgccgg tgcatttcca tacccacgat 2280

accagcggca tctcggccgc ttcggcgctg gccgcgatcg aggccggctg cgatgcggtc 2340

gacggcgcgc tcgacgccat gagcgggctg acctcgcaac ccaacctgtc gagcatcgcc 2400

gcggccctgg ccggcagcga gcgcgatccc ggcctcagcc tggagcgcct gcacgaggcg 2460

tcgatgtact gggaaggggt gcgccgctac tacgcgccgt tcgaatccga aatccgcgcc 2520

ggcaccgccg acgtgtaccg ccacgagatg cccggcggcc agtacaccaa cctgcgcgag 2580

caggcgcgct cgctcggcat cgagcatcgc tggaccgagg tgtcgcgggc ctatgccgag 2640

gtcaaccaga tgtttggcga catcgtcaag gtgacgccga cgtccaaggt ggtcggcgac 2700

ctggccttga tgatggtggc caacgacctg agcgccgccg atgtgtgcga tcccgccagg 2760

gagactgcct tccctgaatc ggtggtgtcg ctgttcaagg gcgagctggg ctttccgccg 2820

gacggcttcc ccgcggaact gtcgcgcaag gtgctgcgcg gcgagccgcc cgtgccgtac 2880

cggcccggcg accagatccc gccggtcgac ctcgacgcgg cgcgcgccgc ggccgaagcg 2940

gcgtgcgagc agccgctcga cgaccgccag ctggcttcgt acctgatgta cccgaagcag 3000

gccggcgagt accacgcgca tgtgcgcaac tacagcgaca cctcggtggt acccacgccg 3060

gcatacctgt acggcctgca gccgcaggaa gaagtggcga tcgacatcgc tgccggcaag 3120

accctgctgg tctcgctgca aggcacgcac cccgatgccg aagagggtgt catcaaggtc 3180

cagttcgagc tgaacgggca gtcgcgcacc acgctggtcg agcagcgcag caccacgcaa 3240

gcggcggcag cgcgccatgg ccgtccggtt gccgaacccg acaatccgct gcatgtcgcc 3300

gcgcccatgc cgggctcgat cgtgacggtg gcggtgcagc cggggcagcg cgtggccgcg 3360

ggcacgacgc tgctggcgct ggaggcgatg aagatggaaa cccatatcgc ggcggagcgg 3420

gactgcgaga tcgccgcagt ccatgttcag cagggggatc gcgtggcggc gaaggatctg 3480

ctgatcgaac tgaagggctg a 3501

<210> 88
<211> 1167
<212> PRT
<213> Ralstonia eutropha

<400> 88

Met Asp Tyr Ala Pro Ile Arg Ser Leu Leu Ile Ala Asn Arg Ser Glu
1               5                   10                  15

Ile Ala Ile Arg Val Met Arg Ala Ala Ala Glu Met Asn Val Arg Thr
            20                  25                  30

Val Ala Ile Tyr Ser Lys Glu Asp Arg Leu Ala Leu His Arg Phe Lys
        35                  40                  45

Ala Asp Glu Ser Tyr Leu Val Gly Glu Gly Lys Lys Pro Leu Ala Ala
    50                  55                  60

Tyr Leu Asp Ile Asp Asp Ile Leu Arg Ile Ala Arg Gln Ala Lys Val
65                  70                  75                  80

Asp Ala Ile His Pro Gly Tyr Gly Phe Leu Ser Glu Asn Pro Asp Phe
                85                  90                  95

Ala Gln Ala Val Ile Asp Ala Gly Ile Arg Trp Ile Gly Pro Ser Pro
            100                 105                 110

Glu Val Met Arg Lys Leu Gly Asn Lys Val Ala Ala Arg Asn Ala Ala
        115                 120                 125

Ile Asp Ala Gly Val Pro Val Met Pro Ala Thr Asp Pro Leu Pro His

130                 135                 140

Asp Leu Asp Thr Cys Lys Arg Leu Ala Ala Gly Ile Gly Tyr Pro Leu
145                 150                 155                 160

Met Leu Lys Ala Ser Trp Gly Gly Gly Gly Arg Gly Met Arg Val Leu
                165                 170                 175

Glu Arg Glu Gln Asp Leu Glu Gly Ala Leu Ala Ala Ala Arg Arg Glu
            180                 185                 190

Ala Leu Ala Ala Phe Gly Asn Asp Glu Val Tyr Val Glu Lys Leu Val
        195                 200                 205

Arg Asn Ala Arg His Val Glu Val Gln Val Leu Gly Asp Thr His Gly
    210                 215                 220

Asn Leu Val His Leu Tyr Glu Arg Asp Cys Thr Val Gln Arg Arg Asn
225                 230                 235                 240

Gln Lys Val Val Glu Arg Ala Pro Ala Pro Tyr Leu Asp Asp Ala Gly
                245                 250                 255

Arg Ala Ala Leu Cys Glu Ser Ala Leu Arg Leu Met Arg Ala Val Gly
            260                 265                 270

Tyr Thr His Ala Gly Thr Val Glu Phe Leu Met Asp Ala Asp Ser Gly
        275                 280                 285

Gln Phe Tyr Phe Ile Glu Val Asn Pro Arg Ile Gln Val Glu His Thr
    290                 295                 300

Val Thr Glu Met Val Thr Gly Ile Asp Ile Val Lys Ala Gln Ile Arg
305                 310                 315                 320

Val Thr Glu Gly Gly His Leu Gly Met Thr Glu Asn Thr Arg Asn Glu
                325                 330                 335

Asn Gly Glu Ile Val Val Arg Ala Ala Gly Val Pro Val Gln Glu Ala
            340                 345                 350

Ile Ser Leu Asn Gly His Ala Leu Gln Cys Arg Ile Thr Thr Glu Asp
        355                 360                 365

Pro Glu Asn Gly Phe Leu Pro Asp Tyr Gly Arg Leu Thr Ala Tyr Arg
    370                 375                 380

Ser Ala Ala Gly Phe Gly Val Arg Leu Asp Ala Gly Thr Ala Tyr Gly
385                 390                 395                 400

Gly Ala Val Ile Thr Pro Tyr Tyr Asp Ser Leu Leu Val Lys Val Thr
                405                 410                 415

Thr Trp Ala Pro Thr Ala Pro Glu Ser Ile Arg Arg Met Asp Arg Ala
            420                 425                 430

Leu Arg Glu Phe Arg Ile Arg Gly Val Ala Ser Asn Leu Gln Phe Leu
        435                 440                 445

Glu Asn Val Ile Asn His Pro Ser Phe Arg Ser Gly Asp Val Thr Thr
    450                 455                 460

Arg Phe Ile Asp Leu Thr Pro Glu Leu Leu Ala Phe Thr Lys Arg Leu
465                 470                 475                 480

Asp Arg Ala Thr Lys Leu Leu Arg Tyr Leu Gly Glu Val Ser Val Asn
                485                 490                 495

Gly His Pro Glu Met Ser Gly Arg Thr Leu Pro Ser Leu Pro Leu Pro
            500                 505                 510

Ala Pro Val Leu Pro Ala Phe Asp Thr Gly Gly Ala Leu Pro Tyr Gly
        515                 520                 525

Thr Arg Asp Arg Leu Arg Glu Leu Gly Ala Glu Lys Phe Ser Arg Trp
    530                 535                 540

Met Leu Glu Gln Lys Gln Val Leu Leu Thr Asp Thr Thr Met Arg Asp
545                 550                 555                 560

Ala His Gln Ser Leu Phe Ala Thr Arg Met Arg Thr Ala Asp Met Leu
                565                 570                 575

Pro Ile Ala Pro Phe Tyr Ala Arg Glu Leu Ser Gln Leu Phe Ser Leu
            580                 585                 590

Glu Cys Trp Gly Gly Ala Thr Phe Asp Val Ala Leu Arg Phe Leu Lys
        595                 600                 605

Glu Asp Pro Trp Gln Arg Leu Glu Gln Leu Arg Glu Arg Val Pro Asn
    610                 615                 620

Val Leu Phe Gln Met Leu Leu Arg Gly Ser Asn Ala Val Gly Tyr Thr
625                 630                 635                 640

Asn Tyr Ala Asp Asn Val Val Arg Phe Phe Val Arg Gln Ala Ala Ser
                645                 650                 655

Ala Gly Val Asp Val Phe Arg Val Phe Asp Ser Leu Asn Trp Val Arg
            660                 665                 670

Asn Met Arg Val Ala Ile Asp Ala Val Gly Glu Ser Gly Ala Leu Cys
        675                 680                 685

Glu Gly Ala Ile Cys Tyr Thr Gly Asp Leu Phe Asp Lys Ser Arg Ala
    690                 695                 700

Lys Tyr Asp Leu Lys Tyr Tyr Val Gly Ile Ala Arg Glu Leu Lys Gln
705                 710                 715                 720

Ala Gly Val His Val Leu Gly Ile Lys Asp Met Ala Gly Ile Cys Arg
                725                 730                 735

Pro Gln Ala Ala Ala Ala Leu Val Arg Ala Leu Lys Glu Glu Thr Gly
            740                 745                 750

Leu Pro Val His Phe His Thr His Asp Thr Ser Gly Ile Ser Ala Ala
        755                 760                 765

Ser Ala Leu Ala Ala Ile Glu Ala Gly Cys Asp Ala Val Asp Gly Ala
    770                 775                 780

Leu Asp Ala Met Ser Gly Leu Thr Ser Gln Pro Asn Leu Ser Ser Ile
785                 790                 795                 800

Ala Ala Ala Leu Ala Gly Ser Glu Arg Asp Pro Gly Leu Ser Leu Glu
                805                 810                 815

Arg Leu His Glu Ala Ser Met Tyr Trp Glu Gly Val Arg Arg Tyr Tyr
            820                 825                 830

Ala Pro Phe Glu Ser Glu Ile Arg Ala Gly Thr Ala Asp Val Tyr Arg
        835                 840                 845

His Glu Met Pro Gly Gly Gln Tyr Thr Asn Leu Arg Glu Gln Ala Arg
    850                 855                 860

Ser Leu Gly Ile Glu His Arg Trp Thr Glu Val Ser Arg Ala Tyr Ala
865                 870                 875                 880

Glu Val Asn Gln Met Phe Gly Asp Ile Val Lys Val Thr Pro Thr Ser
                885                 890                 895

Lys Val Val Gly Asp Leu Ala Leu Met Met Val Ala Asn Asp Leu Ser
            900                 905                 910

Ala Ala Asp Val Cys Asp Pro Ala Arg Glu Thr Ala Phe Pro Glu Ser
        915                 920                 925

Val Val Ser Leu Phe Lys Gly Glu Leu Gly Phe Pro Pro Asp Gly Phe
    930                 935                 940

Pro Ala Glu Leu Ser Arg Lys Val Leu Arg Gly Glu Pro Pro Val Pro
945                 950                 955                 960

Tyr Arg Pro Gly Asp Gln Ile Pro Pro Val Asp Leu Asp Ala Ala Arg
                965                 970                 975

Ala Ala Ala Glu Ala Ala Cys Glu Gln Pro Leu Asp Asp Arg Gln Leu
            980                 985                 990

Ala Ser Tyr Leu Met Tyr Pro Lys Gln Ala Gly Glu Tyr His Ala His
        995                 1000                1005

Val Arg Asn Tyr Ser Asp Thr Ser Val Val Pro Thr Pro Ala Tyr
    1010                1015                1020

Leu Tyr Gly Leu Gln Pro Gln Glu Glu Val Ala Ile Asp Ile Ala
    1025                1030                1035

Ala Gly Lys Thr Leu Leu Val Ser Leu Gln Gly Thr His Pro Asp
    1040                1045                1050

Ala Glu Glu Gly Val Ile Lys Val Gln Phe Glu Leu Asn Gly Gln
    1055                1060                1065

Ser Arg Thr Thr Leu Val Glu Gln Arg Ser Thr Thr Gln Ala Ala
    1070                1075                1080

Ala Ala Arg His Gly Arg Pro Val Ala Glu Pro Asp Asn Pro Leu
    1085                1090                1095

His Val Ala Ala Pro Met Pro Gly Ser Ile Val Thr Val Ala Val
    1100                1105                1110

Gln Pro Gly Gln Arg Val Ala Ala Gly Thr Thr Leu Leu Ala Leu
    1115                1120                1125

Glu Ala Met Lys Met Glu Thr His Ile Ala Ala Glu Arg Asp Cys

1130                1135                1140

Glu Ile Ala Ala Val His Val Gln Gln Gly Asp Arg Val Ala Ala
    1145                1150                1155

Lys Asp Leu Leu Ile Glu Leu Lys Gly
    1160                1165

<210>  89
<211>  888
<212>  DNA
<213>  Escherichia coli

<400>  89
ggatccatgt ctagaatgag ccaagccctg aaaaacctgc tgacgctgct gaatctggaa      60

aaaatcgaag aaggcctgtt ccgtggtcaa tctgaagacc tgggcctgcg tcaggtgttt     120

ggcggtcagg tggttggtca agcgctgtat gcggccaaag aaaccgttcc ggaagaacgt     180

ctggtccata gctttcactc ttatttcctg cgcccgggcg atagcaaaaa accgattatc     240

tacgatgtgg aaaccctgcg cgacggcaac agtttttccg cccgtcgcgt tgcagctatt     300

cagaatggta aaccgatctt ttacatgacg gcatcattcc aggcaccgga agctggcttt     360

gaacatcaaa aaaccatgcc gagcgccccg gcaccggatg gtctgccgag tgaaacgcag     420

attgcacaat ccctggctca tctgctgccg ccggtcctga aagataaatt tatctgtgac     480

cgtccgctgg aagtccgtcc ggtggaattt cacaacccgc tgaaaggcca tgtcgcagaa     540

ccgcaccgtc aagtgtggat tcgcgctaat ggcagcgtgc cggatgacct gcgtgttcat     600

caatatctgc tgggttacgc gtctgatctg aactttctgc cggttgccct gcaaccgcac     660

ggcattggtt tcctggaacc gggtattcaa atcgccacga tcgaccattc aatgtggttt     720

caccgcccgt tcaacctgaa tgaatggctg ctgtattccg ttgaatcaac cagcgcgagc     780

agcgcccgtg gctttgtccg tggtgaattt tacacgcaag atggtgtcct ggtggcgtct     840

accgttcaag aaggcgttat gcgtaatcac aactaagagc tcaagctt                  888

<210>  90
<211>  286
<212>  PRT
<213>  Escherichia coli

<400>  90

Met Ser Gln Ala Leu Lys Asn Leu Leu Thr Leu Leu Asn Leu Glu Lys
1               5                   10                  15

Ile Glu Glu Gly Leu Phe Arg Gly Gln Ser Glu Asp Leu Gly Leu Arg
            20                  25                  30

Gln Val Phe Gly Gly Gln Val Val Gly Gln Ala Leu Tyr Ala Ala Lys
        35                  40                  45

Glu Thr Val Pro Glu Glu Arg Leu Val His Ser Phe His Ser Tyr Phe
    50                  55                  60

Leu Arg Pro Gly Asp Ser Lys Lys Pro Ile Ile Tyr Asp Val Glu Thr
65                  70                  75                  80

Leu Arg Asp Gly Asn Ser Phe Ser Ala Arg Arg Val Ala Ala Ile Gln
                85                  90                  95

Asn Gly Lys Pro Ile Phe Tyr Met Thr Ala Ser Phe Gln Ala Pro Glu
            100                 105                 110

Ala Gly Phe Glu His Gln Lys Thr Met Pro Ser Ala Pro Ala Pro Asp
        115                 120                 125

Gly Leu Pro Ser Glu Thr Gln Ile Ala Gln Ser Leu Ala His Leu Leu
    130                 135                 140

Pro Pro Val Leu Lys Asp Lys Phe Ile Cys Asp Arg Pro Leu Glu Val
145                 150                 155                 160

Arg Pro Val Glu Phe His Asn Pro Leu Lys Gly His Val Ala Glu Pro
                165                 170                 175

His Arg Gln Val Trp Ile Arg Ala Asn Gly Ser Val Pro Asp Asp Leu
            180                 185                 190

Arg Val His Gln Tyr Leu Leu Gly Tyr Ala Ser Asp Leu Asn Phe Leu
        195                 200                 205

Pro Val Ala Leu Gln Pro His Gly Ile Gly Phe Leu Glu Pro Gly Ile
    210                 215                 220

Gln Ile Ala Thr Ile Asp His Ser Met Trp Phe His Arg Pro Phe Asn
225                 230                 235                 240

Leu Asn Glu Trp Leu Leu Tyr Ser Val Glu Ser Thr Ser Ala Ser Ser
                245                 250                 255

Ala Arg Gly Phe Val Arg Gly Glu Phe Tyr Thr Gln Asp Gly Val Leu
            260                 265                 270

Val Ala Ser Thr Val Gln Glu Gly Val Met Arg Asn His Asn
        275                 280                 285

<210> 91
<211> 1602
<212> DNA
<213> Clostridium propionicum

<400> 91
ggatccatgt ctagaatgcg caaagtcccg attattacgg cagatgaagc ggctaaactg 60

attaaagacg gcgatacggt caccaccagc ggtttcgttg gcaacgcaat tccggaagct 120

ctggatcgtg cggttgaaaa acgctttctg gaaaccggcg aaccgaaaaa catcacgtat 180

gtctactgcg gcagtcaggg taatcgtgat ggccgcggtg ccgaacattt cgcacacgaa 240

ggcctgctga aacgttatat tgctggtcat tgggccaccg tcccggcact gggtaaaatg 300

gcaatggaaa acaaaatgga agcgtataat gtgtcacagg gcgcgctgtg tcacctgttt 360

cgtgatattg cctcgcacaa accgggtgtc tttaccaaag tgggcattgg tacgtttatc 420

gacccgcgca acggcggtgg caaagtgaat gatattacca aagaagacat cgtcgaactg 480

gtggaaatta aaggccagga atacctgttt tatccggcgt tcccgattca tgttgccctg 540

atccgcggca cctatgccga tgaatctggt aacattacgt ttgaaaaaga agtggcaccg 600

ctggaaggca ccagcgtgtg ccaggcagtc aaaaattctg gtggcatcgt ggttgtccaa 660

gttgaacgtg tggttaaagc gggcaccctg gacccgcgcc acgttaaagt cccgggtatt 720

tatgtggact acgtcgtggt tgctgatccg gaagaccatc agcaaagtct ggattgtgaa 780

tatgatccgg cactgtccgg tgaacaccgt cgcccggaag ttgtgggtga accgctgccg 840

ctgagtgcta aaaaagttat tggccgtcgc ggtgcgatcg aactggaaaa agatgtggcc 900

gttaacctgg gcgtgggtgc accggaatac gttgcgtccg tcgccgatga agaaggcatt 960

gttgacttta tgaccctgac ggcagatagc ggtgctattg gcggcgtgcc ggcgggcggc 1020

gttcgttttg gcgcgtctta taatgcggat gccctgatcg accagggtta ccaattcgat 1080

tattacgacg gtggcggtct ggatctgtgc tatctgggcc tggcggaatg tgacgaaaag 1140

ggtaacatta atgtgtcacg ttttggtccg cgtattgcgg gttgtggtgg tttcattaac 1200

atcacccaga atacgccgaa agtctttttc tgtggcacct ttacggcagg cggtctgaaa 1260

gtgaaaattg aagatggcaa agtgattatc gttcaggaag gtaaacagaa aaaattcctg 1320

aaagcggttg aacaaatcac cttcaacggt gatgtcgcac tggctaataa acagcaagtg 1380

acctatatca cggaacgttg cgtttttctg ctgaaagaag atggcctgca cctgtcggaa 1440

attgcgccgg gtattgatct gcaaacccaa attctggatg tgatggactt cgccccgatt 1500

atcgatcgcg acgcaaatgg ccagatcaaa ctgatggatg cggcactgtt tgcggaaggt 1560

ctgatgggtc tgaaagaaat gaaatcgtaa gagctcaagc tt 1602

<210> 92

<211>  524
<212>  PRT
<213>  Clostridium propionicum

<400>  92

Met Arg Lys Val Pro Ile Ile Thr Ala Asp Glu Ala Ala Lys Leu Ile
1               5                   10                  15

Lys Asp Gly Asp Thr Val Thr Thr Ser Gly Phe Val Gly Asn Ala Ile
            20                  25                  30

Pro Glu Ala Leu Asp Arg Ala Val Glu Lys Arg Phe Leu Glu Thr Gly
        35                  40                  45

Glu Pro Lys Asn Ile Thr Tyr Val Tyr Cys Gly Ser Gln Gly Asn Arg
    50                  55                  60

Asp Gly Arg Gly Ala Glu His Phe Ala His Glu Gly Leu Leu Lys Arg
65                  70                  75                  80

Tyr Ile Ala Gly His Trp Ala Thr Val Pro Ala Leu Gly Lys Met Ala
                85                  90                  95

Met Glu Asn Lys Met Glu Ala Tyr Asn Val Ser Gln Gly Ala Leu Cys
            100                 105                 110

His Leu Phe Arg Asp Ile Ala Ser His Lys Pro Gly Val Phe Thr Lys
        115                 120                 125

Val Gly Ile Gly Thr Phe Ile Asp Pro Arg Asn Gly Gly Gly Lys Val
    130                 135                 140

Asn Asp Ile Thr Lys Glu Asp Ile Val Glu Leu Val Glu Ile Lys Gly
145                 150                 155                 160

Gln Glu Tyr Leu Phe Tyr Pro Ala Phe Pro Ile His Val Ala Leu Ile
                165                 170                 175

Arg Gly Thr Tyr Ala Asp Glu Ser Gly Asn Ile Thr Phe Glu Lys Glu
            180                 185                 190

Val Ala Pro Leu Glu Gly Thr Ser Val Cys Gln Ala Val Lys Asn Ser
        195                 200                 205

Gly Gly Ile Val Val Val Gln Val Glu Arg Val Val Lys Ala Gly Thr
    210                 215                 220

Leu Asp Pro Arg His Val Lys Val Pro Gly Ile Tyr Val Asp Tyr Val

225                 230                 235                 240

Val Val Ala Asp Pro Glu Asp His Gln Gln Ser Leu Asp Cys Glu Tyr
                245                 250                 255

Asp Pro Ala Leu Ser Gly Glu His Arg Arg Pro Glu Val Val Gly Glu
            260                 265                 270

Pro Leu Pro Leu Ser Ala Lys Lys Val Ile Gly Arg Arg Gly Ala Ile
        275                 280                 285

Glu Leu Glu Lys Asp Val Ala Val Asn Leu Gly Val Gly Ala Pro Glu
    290                 295                 300

Tyr Val Ala Ser Val Ala Asp Glu Glu Gly Ile Val Asp Phe Met Thr
305                 310                 315                 320

Leu Thr Ala Asp Ser Gly Ala Ile Gly Gly Val Pro Ala Gly Gly Val
                325                 330                 335

Arg Phe Gly Ala Ser Tyr Asn Ala Asp Ala Leu Ile Asp Gln Gly Tyr
            340                 345                 350

Gln Phe Asp Tyr Tyr Asp Gly Gly Gly Leu Asp Leu Cys Tyr Leu Gly
        355                 360                 365

Leu Ala Glu Cys Asp Glu Lys Gly Asn Ile Asn Val Ser Arg Phe Gly
    370                 375                 380

Pro Arg Ile Ala Gly Cys Gly Gly Phe Ile Asn Ile Thr Gln Asn Thr
385                 390                 395                 400

Pro Lys Val Phe Phe Cys Gly Thr Phe Thr Ala Gly Gly Leu Lys Val
                405                 410                 415

Lys Ile Glu Asp Gly Lys Val Ile Ile Val Gln Glu Gly Lys Gln Lys
            420                 425                 430

Lys Phe Leu Lys Ala Val Glu Gln Ile Thr Phe Asn Gly Asp Val Ala
        435                 440                 445

Leu Ala Asn Lys Gln Gln Val Thr Tyr Ile Thr Glu Arg Cys Val Phe
    450                 455                 460

Leu Leu Lys Glu Asp Gly Leu His Leu Ser Glu Ile Ala Pro Gly Ile
465                 470                 475                 480

Asp Leu Gln Thr Gln Ile Leu Asp Val Met Asp Phe Ala Pro Ile Ile
                485                 490                 495

Asp Arg Asp Ala Asn Gly Gln Ile Lys Leu Met Asp Ala Ala Leu Phe
            500                 505                 510

Ala Glu Gly Leu Met Gly Leu Lys Glu Met Lys Ser
        515                 520

<210>  93
<211>  1581
<212>  DNA
<213>  Megasphaera elsdenii

<400>  93
ggatccatgt ctagaatgcg taaagttgaa attattaccg cagaacaggc agcacagctg      60

gttaaagata atgataccat taccagcatt ggctttgtta gcagcgcaca tccggaagca     120

ctgaccaaag cactggaaaa acgttttctg gataccaata caccgcagaa tctgacctat     180

atttatgcag gtagccaggg taaacgtgat ggtcgtgcag cagaacatct ggcacataca     240

ggtctgctga aacgtgcaat tattggtcat tggcagaccg ttccggcaat tggtaaactg     300

gcagtggaaa ataaaattga agcctataat tttagccagg gcaccctggt tcattggttt     360

cgtgcactgg caggtcataa actgggtgtt tttaccgata ttggcctgga aacctttctg     420

gacccgcgtc agctgggtgg taaactgaat gatgttacca aagaggatct ggttaaactg     480

attgaagtgg atggtcatga acagctgttt tatccgacct ttccggttaa tgttgcattt     540

ctgcgtggca cctatgcaga tgaaagcggt aatattacaa tggatgaaga aattggtccg     600

tttgaaagca ccagcgttgc acaggcagtt cataattgtg gtggtaaagt tgtggttcag     660

gttaaagatg ttgttgcaca tggtagcctg gacccgcgta tggttaaaat tccgggtatt     720

tatgtggatt atgttgttgt tgcagcaccg gaagatcatc agcagaccta tgattgtgaa     780

tatgatccga gcctgagcgg tgaacatcgt gcaccggaag gtgcagcaga tgcagcactg     840

ccgatgagcg caaaaaaaat tattggtcgt cgtggtgcac tggaactgac cgaaaatgca     900

gttgttaatc tgggtgttgg tgcaccggaa tatgttgcaa gcgttgcggg tgaagaaggt     960

attgcagata ccattacact gaccgttgat ggtggtgcaa ttggtggtgt tccgcagggt    1020

ggtgcacgtt ttggtagcag ccgtaatgca gatgccatta ttgatcatac ctatcagttt    1080

gatttttatg atggtggtgg tctggatatt gcatatctgg gtctggcaca gtgtgatggt    1140

agtggtaata ttaatgtgag caaatttggc accaatgttg caggttgtgg tggttttccg    1200

aatattagcc agcagacccc gaatgtttat ttttgtggca cctttaccgc aggcggtctg    1260

aaaattgcag ttgaagatgg caaagtgaaa attctgcaag aaggcaaagc caaaaaattt    1320

attaaagccg tggatcagat tacctttaat ggtagctatg cagcccgtaa tggtaaacat    1380

gttctgtata ttaccgaacg ctgcgttttt gaactgacaa aagaaggtct gaaactgatc 1440

gaagttgcac cgggtattga tattgaaaaa gatattctgg cccacatgga ttttaaaccg 1500

attattgata atccgaaact gatggatgcc cgtctgtttc aggatggtcc gatgggtctg 1560

aaacgttaag agctcaagct t 1581

<210> 94
<211> 516
<212> PRT
<213> Megasphaera elsdenii

<400> 94

Met Arg Lys Val Glu Ile Ile Thr Ala Glu Gln Ala Ala Gln Leu Val
1               5                   10                  15

Lys Asp Asn Asp Thr Ile Thr Ser Ile Gly Phe Val Ser Ser Ala His
            20                  25                  30

Pro Glu Ala Leu Thr Lys Ala Leu Glu Lys Arg Phe Leu Asp Thr Asn
        35                  40                  45

Thr Pro Gln Asn Leu Thr Tyr Ile Tyr Ala Gly Ser Gln Gly Lys Arg
    50                  55                  60

Asp Gly Arg Ala Ala Glu His Leu Ala His Thr Gly Leu Leu Lys Arg
65                  70                  75                  80

Ile Ile Gly His Trp Gln Thr Val Pro Ala Ile Gly Lys Leu Ala Val
                85                  90                  95

Glu Asn Lys Ile Glu Ala Tyr Asn Phe Ser Gln Gly Thr Leu Val His
            100                 105                 110

Trp Phe Arg Ala Leu Ala Gly His Lys Leu Gly Val Phe Thr Asp Ile
        115                 120                 125

Gly Leu Glu Thr Phe Leu Asp Pro Arg Gln Leu Gly Gly Lys Leu Asn
    130                 135                 140

Asp Val Thr Lys Glu Asp Leu Val Lys Leu Ile Glu Val Asp Gly His
145                 150                 155                 160

Glu Gln Leu Phe Tyr Pro Thr Phe Pro Val Asn Val Ala Phe Leu Arg
                165                 170                 175

Gly Thr Tyr Ala Asp Glu Ser Gly Asn Ile Thr Met Asp Glu Glu Ile
            180                 185                 190

Gly Pro Phe Glu Ser Thr Ser Val Ala Gln Ala Val His Asn Cys Gly
        195                 200                 205

Gly Lys Val Val Val Gln Val Lys Asp Val Val Ala His Gly Ser Leu
    210                 215                 220

Asp Pro Arg Met Val Lys Ile Pro Gly Ile Tyr Val Asp Tyr Val Val
225                 230                 235                 240

Val Ala Ala Pro Glu Asp His Gln Gln Thr Tyr Asp Cys Glu Tyr Asp
                245                 250                 255

Pro Ser Leu Ser Gly Glu His Arg Ala Pro Glu Gly Ala Ala Asp Ala
            260                 265                 270

Ala Leu Pro Met Ser Ala Lys Lys Ile Ile Gly Arg Arg Gly Ala Leu
        275                 280                 285

Glu Leu Thr Glu Asn Ala Val Val Asn Leu Gly Val Gly Ala Pro Glu
    290                 295                 300

Tyr Val Ala Ser Val Ala Gly Glu Glu Gly Ile Ala Asp Thr Ile Thr
305                 310                 315                 320

Leu Thr Val Asp Gly Gly Ala Ile Gly Gly Val Pro Gln Gly Gly Ala
                325                 330                 335

Arg Phe Gly Ser Ser Arg Asn Ala Asp Ala Ile Ile Asp His Thr Tyr
            340                 345                 350

Gln Phe Asp Phe Tyr Asp Gly Gly Gly Leu Asp Ile Ala Tyr Leu Gly
        355                 360                 365

Leu Ala Gln Cys Asp Gly Ser Gly Asn Ile Asn Val Ser Lys Phe Gly
    370                 375                 380

Thr Asn Val Ala Gly Cys Gly Gly Phe Pro Asn Ile Ser Gln Gln Thr
385                 390                 395                 400

Pro Asn Val Tyr Phe Cys Gly Thr Phe Thr Ala Gly Gly Leu Lys Ile
                405                 410                 415

Ala Val Glu Asp Gly Lys Val Lys Ile Leu Gln Glu Gly Lys Ala Lys
            420                 425                 430

Lys Phe Ile Lys Ala Val Asp Gln Ile Thr Phe Asn Gly Ser Tyr Ala

435                 440                 445

Ala Arg Asn Gly Lys His Val Leu Tyr Ile Thr Glu Arg Cys Val Phe
    450                 455                 460

Glu Leu Thr Lys Glu Gly Leu Lys Leu Ile Glu Val Ala Pro Gly Ile
465                 470                 475                 480

Asp Ile Glu Lys Asp Ile Leu Ala His Met Asp Phe Lys Pro Ile Ile
                485                 490                 495

Asp Asn Pro Lys Leu Met Asp Ala Arg Leu Phe Gln Asp Gly Pro Met
            500                 505                 510

Gly Leu Lys Arg
        515

<210>  95
<211>  1077
<212>  DNA
<213>  Saccharomyces cerevisiae

<400>  95
ggatccatgt ctagaatgtc tgcgagcaaa atggcgatga gcaacctgga aaaaattctg      60

gaactggtgc cgctgtctcc gacctccttt gtgacgaaat acctgccggc ggcaccggtt     120

ggctctaaag gcaccttcgg cggtacgctg gtcagccagt ccctgctggc cagtctgcat     180

accgtgccgc tgaacttttt cccgacgtct ctgcacagtt atttcattaa aggcggtgac     240

ccgcgtacca aaatcacgta ccatgttcag aacctgcgta atggccgcaa ctttattcat     300

aaacaggtct ccgcttatca acacgataaa ctgattttta cctcaatgat cctgttcgcg     360

gttcagcgta gcaaagaaca tgatagcctg caacactggg aaaccatccc gggcctgcaa     420

ggtaaacaac cggacccgca ccgctacgaa gaagcgacgt cgctgtttca gaaagaagtg     480

ctggacccgc aaaaactgtc acgttatgcg tcactgtcgg atcgcttcca ggacgccacc     540

agcatgtcta aatacgtcga tgcatttcag tatggcgtga tggaatacca atttccgaaa     600

gacatgttct atagcgcccg tcatacggat gaactggact acttcgtgaa agttcgcccg     660

ccgattacca cggtcgaaca tgcaggtgat gaaagctctc tgcacaaaca tcacccgtat     720

cgtattccga aaagcatcac cccggaaaat gatgctcgct ataactacgt ggcatttgct     780

tatctgagtg actcctacct gctgctgacc attccgtatt ttcataatct gccgctgtac     840

tgccactcat tcagcgtgag cctggatcat acgatctatt ttcatcagct gccgcacgtt     900

aacaattgga tttacctgaa aatctccaac ccgcgttcac attgggataa acacctggtt     960

cagggcaaat attttgacac ccaatctggt cgcatcatgg cgagtgtctc ccaggaaggc    1020

tatgtggtgt atggtagtga acgtgatatt cgtgccaaat tctaagagct caagctt        1077

<210>  96
<211>  349
<212>  PRT
<213>  Saccharomyces cerevisiae

<400>  96

Met Ser Ala Ser Lys Met Ala Met Ser Asn Leu Glu Lys Ile Leu Glu
1               5                   10                  15

Leu Val Pro Leu Ser Pro Thr Ser Phe Val Thr Lys Tyr Leu Pro Ala
            20                  25                  30

Ala Pro Val Gly Ser Lys Gly Thr Phe Gly Gly Thr Leu Val Ser Gln
        35                  40                  45

Ser Leu Leu Ala Ser Leu His Thr Val Pro Leu Asn Phe Phe Pro Thr
    50                  55                  60

Ser Leu His Ser Tyr Phe Ile Lys Gly Gly Asp Pro Arg Thr Lys Ile
65                  70                  75                  80

Thr Tyr His Val Gln Asn Leu Arg Asn Gly Arg Asn Phe Ile His Lys
                85                  90                  95

Gln Val Ser Ala Tyr Gln His Asp Lys Leu Ile Phe Thr Ser Met Ile
            100                 105                 110

Leu Phe Ala Val Gln Arg Ser Lys Glu His Asp Ser Leu Gln His Trp
        115                 120                 125

Glu Thr Ile Pro Gly Leu Gln Gly Lys Gln Pro Asp Pro His Arg Tyr
    130                 135                 140

Glu Glu Ala Thr Ser Leu Phe Gln Lys Glu Val Leu Asp Pro Gln Lys
145                 150                 155                 160

Leu Ser Arg Tyr Ala Ser Leu Ser Asp Arg Phe Gln Asp Ala Thr Ser
                165                 170                 175

Met Ser Lys Tyr Val Asp Ala Phe Gln Tyr Gly Val Met Glu Tyr Gln
            180                 185                 190

Phe Pro Lys Asp Met Phe Tyr Ser Ala Arg His Thr Asp Glu Leu Asp
        195                 200                 205

Tyr Phe Val Lys Val Arg Pro Pro Ile Thr Thr Val Glu His Ala Gly

210                 215                 220

Asp Glu Ser Ser Leu His Lys His His Pro Tyr Arg Ile Pro Lys Ser
225                 230                 235                 240

Ile Thr Pro Glu Asn Asp Ala Arg Tyr Asn Tyr Val Ala Phe Ala Tyr
                245                 250                 255

Leu Ser Asp Ser Tyr Leu Leu Leu Thr Ile Pro Tyr Phe His Asn Leu
            260                 265                 270

Pro Leu Tyr Cys His Ser Phe Ser Val Ser Leu Asp His Thr Ile Tyr
        275                 280                 285

Phe His Gln Leu Pro His Val Asn Asn Trp Ile Tyr Leu Lys Ile Ser
    290                 295                 300

Asn Pro Arg Ser His Trp Asp Lys His Leu Val Gln Gly Lys Tyr Phe
305                 310                 315                 320

Asp Thr Gln Ser Gly Arg Ile Met Ala Ser Val Ser Gln Glu Gly Tyr
                325                 330                 335

Val Val Tyr Gly Ser Glu Arg Asp Ile Arg Ala Lys Phe
            340                 345

<210>  97
<211>  990
<212>  DNA
<213>  Mus musculus

<400>  97
ggatccatgt ctagaatgtc tgccccggaa ggcctgggtg atgcacacgg tgatgctgat      60

cgcggtgacc tgagcggtga cctgcgttcg gttctggtta cgagcgtcct gaacctggaa     120

ccgctggatg aagacctgta tcgtggccgc cattactggg ttccgacctc tcagcgtctg     180

tttggcggtc agattatggg tcaagccctg gtcgcggccg caaaaagcgt gtctgaagat     240

gtccatgtgc actcactgca ttgctatttt gttcgtgccg gcgatccgaa agttccggtc     300

ctgtaccacg tcgaacgtat ccgcacgggt gcaagtttct ccgtgcgtgc tgttaaagcg     360

gtccagcatg gtaaagccat ttttatctgt caggcaagtt tccagcaaat gcaaccgtcc     420

ccgctgcaac accaattttc aatgccgtcg gtgccgccgc cggaagacct gctggatcat     480

gaagcgctga ttgatcagta tctgcgtgac ccgaacctgc acaaaaaata ccgtgtcggt     540

ctgaatcgcg tggctgcgca agaagttccg attgaaatca aagtggttaa cccgccgacc     600

ctgacgcagc tgcaagctct ggaaccgaaa cagatgttct gggtgcgtgc gcgcggctat     660

attggcgaag gtgatatcaa aatgcattgc tgtgttgccg catatatctc agactacgct 720

tttctgggca ccgccctgct gccgcaccag agcaaatata aagtgaattt catggccagc 780

ctggatcatt ctatgtggtt tcacgccccg ttccgcgcag accattggat gctgtacgaa 840

tgcgaatccc cgtgggctgg cggttttcgc ggtctggttc atggtcgcct gtggcgtcgc 900

gatggtgtgc tggcagttac ctgtgcccaa gaaggcgtca tccgtctgaa accgcaagtg 960

tctgaatcta aactgtgaga gctcaagctt 990

<210> 98
<211> 320
<212> PRT
<213> Mus musculus

<400> 98

Met Ser Ala Pro Glu Gly Leu Gly Asp Ala His Gly Asp Ala Asp Arg
1               5                   10                  15

Gly Asp Leu Ser Gly Asp Leu Arg Ser Val Leu Val Thr Ser Val Leu
            20                  25                  30

Asn Leu Glu Pro Leu Asp Glu Asp Leu Tyr Arg Gly Arg His Tyr Trp
        35                  40                  45

Val Pro Thr Ser Gln Arg Leu Phe Gly Gly Gln Ile Met Gly Gln Ala
    50                  55                  60

Leu Val Ala Ala Ala Lys Ser Val Ser Glu Asp Val His Val His Ser
65                  70                  75                  80

Leu His Cys Tyr Phe Val Arg Ala Gly Asp Pro Lys Val Pro Val Leu
                85                  90                  95

Tyr His Val Glu Arg Ile Arg Thr Gly Ala Ser Phe Ser Val Arg Ala
            100                 105                 110

Val Lys Ala Val Gln His Gly Lys Ala Ile Phe Ile Cys Gln Ala Ser
        115                 120                 125

Phe Gln Gln Met Gln Pro Ser Pro Leu Gln His Gln Phe Ser Met Pro
    130                 135                 140

Ser Val Pro Pro Pro Glu Asp Leu Leu Asp His Glu Ala Leu Ile Asp
145                 150                 155                 160

Gln Tyr Leu Arg Asp Pro Asn Leu His Lys Lys Tyr Arg Val Gly Leu
                165                 170                 175

Asn Arg Val Ala Ala Gln Glu Val Pro Ile Glu Ile Lys Val Val Asn
            180                 185                 190

Pro Pro Thr Leu Thr Gln Leu Gln Ala Leu Glu Pro Lys Gln Met Phe
        195                 200                 205

Trp Val Arg Ala Arg Gly Tyr Ile Gly Glu Gly Asp Ile Lys Met His
    210                 215                 220

Cys Cys Val Ala Ala Tyr Ile Ser Asp Tyr Ala Phe Leu Gly Thr Ala
225                 230                 235                 240

Leu Leu Pro His Gln Ser Lys Tyr Lys Val Asn Phe Met Ala Ser Leu
                245                 250                 255

Asp His Ser Met Trp Phe His Ala Pro Phe Arg Ala Asp His Trp Met
            260                 265                 270

Leu Tyr Glu Cys Glu Ser Pro Trp Ala Gly Gly Phe Arg Gly Leu Val
        275                 280                 285

His Gly Arg Leu Trp Arg Arg Asp Gly Val Leu Ala Val Thr Cys Ala
    290                 295                 300

Gln Glu Gly Val Ile Arg Leu Lys Pro Gln Val Ser Glu Ser Lys Leu
305                 310                 315                 320

<210>  99
<211>  1698
<212>  DNA
<213>  Rattus norvegicus

<400>  99
ggatccatgt ctagaatgga accgaccgtg gccccgggtg aagtgctgat gtcgcaagct      60

atccaaccgg ctcatgcaga ttcgcgtggt gaactgtccg ctggccagct gctgaaatgg     120

atggatacca ccgcatgcct ggcggccgaa aaacatgcag gtatctcttg tgttaccgct     180

agtatggatg acattctgtt tgaagacacg gcacgcattg gccagatcgt caccattcgc     240

gcgaaagtga cgcgtgcctt ttccacctca atggaaattt cgatcaaagt ccgtgtgcag     300

gataaattca cgggcattca aaaactgctg tgcgtggcgt tttctacctt cgtggttaaa     360

ccgctgggta aagaaaaagt gcatctgaaa ccggttctgc tgcaaacgga acaggaacaa     420

gtcgaacatc gcctggcctc cgaacgtcgc aaagtgcgtc tgcaacacga aaacaccttt     480

tccaatatca tgaaagaatc aaactggctg cgcgatccgg tgtgtaatga agaagaaggc     540

accgcgacca cgatggccac ctccgttcag tcaattgaac tggtcctgcc gccgcacgca     600

aaccatcacg gtaatacctt tggcggtcaa atcatggctt ggatggaaac ggttgcaacc 660

atttcggcta gccgtctgtg ccatggccac ccgttcctga aatccgtgga tatgtttaaa 720

tttcgtggtc cgtcaacggt tggtgaccgt ctggtgttta acgcgattgt taacaatacc 780

ttccagaaca gcgttgaagt cggtgtgcgc gttgaagcgt ttgattgtcg tgaatgggcc 840

gaaggccagg gtcgccatat caacagtgcg ttcctgattt ataatgccgt tgatgaccag 900

gaagaactga tcacctttcc gcgtatccaa ccgattagca aagatgactt ccgtcgctat 960

cagggtgcca ttgcacgtcg ccgtattcgc ctgggtcgta aatacgtcat ttcgcacaaa 1020

aaagaagtgc cgctgggcac gcagtgggat atcagcaaaa aaggttctat tagtaacacc 1080

aatgtggaag cactgaaaaa cctggcttcc aaatcaggct gggaaatcac cacgaccctg 1140

gaaaaaatca aaatctatac cctggaagaa caggatgcga tttctgtcaa agtggaaaaa 1200

caagtgggta gtccggcacg cgttgcttat catctgctga gcgattttac caaacgtccg 1260

ctgtgggacc cgcactacat ctcgtgcgaa gttattgatc aagtcagcga agatgaccaa 1320

atctattaca ttacgtgttc tgtcgtgaac ggtgataaac cgaaagactt cgttgtcctg 1380

gttagccagc gcaaaccgct gaaagatgac aatacctata ttgtggcact gatgtccgtg 1440

gttctgccgt cggttccgcc gagcccgcag tacatccgtt cacaagtgat ttgcgctggc 1500

tttctgatcc agccggttga tagctctagt tgtaccgtcg cgtatctgaa ccaaatgtcg 1560

gacagcattc tgccgtactt cgccggtaat atcggcggtt ggtctaaaag tattgaagaa 1620

gcagctgcga gttgtatcaa attcatcgaa aacgctacgc acgacggtct gaaatcggtt 1680

ctgtaagagc tcaagctt 1698

<210> 100
<211> 556
<212> PRT
<213> Rattus norvegicus

<400> 100

Met Glu Pro Thr Val Ala Pro Gly Glu Val Leu Met Ser Gln Ala Ile
1               5                   10                  15

Gln Pro Ala His Ala Asp Ser Arg Gly Glu Leu Ser Ala Gly Gln Leu
            20                  25                  30

Leu Lys Trp Met Asp Thr Thr Ala Cys Leu Ala Ala Glu Lys His Ala
        35                  40                  45

Gly Ile Ser Cys Val Thr Ala Ser Met Asp Asp Ile Leu Phe Glu Asp
    50                  55                  60

Thr Ala Arg Ile Gly Gln Ile Val Thr Ile Arg Ala Lys Val Thr Arg

65                  70                  75                  80

Ala Phe Ser Thr Ser Met Glu Ile Ser Ile Lys Val Arg Val Gln Asp
                85                  90                  95

Lys Phe Thr Gly Ile Gln Lys Leu Leu Cys Val Ala Phe Ser Thr Phe
            100                 105                 110

Val Val Lys Pro Leu Gly Lys Glu Lys Val His Leu Lys Pro Val Leu
        115                 120                 125

Leu Gln Thr Glu Gln Glu Gln Val Glu His Arg Leu Ala Ser Glu Arg
    130                 135                 140

Arg Lys Val Arg Leu Gln His Glu Asn Thr Phe Ser Asn Ile Met Lys
145                 150                 155                 160

Glu Ser Asn Trp Leu Arg Asp Pro Val Cys Asn Glu Glu Glu Gly Thr
                165                 170                 175

Ala Thr Thr Met Ala Thr Ser Val Gln Ser Ile Glu Leu Val Leu Pro
            180                 185                 190

Pro His Ala Asn His His Gly Asn Thr Phe Gly Gly Gln Ile Met Ala
        195                 200                 205

Trp Met Glu Thr Val Ala Thr Ile Ser Ala Ser Arg Leu Cys His Gly
    210                 215                 220

His Pro Phe Leu Lys Ser Val Asp Met Phe Lys Phe Arg Gly Pro Ser
225                 230                 235                 240

Thr Val Gly Asp Arg Leu Val Phe Asn Ala Ile Val Asn Asn Thr Phe
                245                 250                 255

Gln Asn Ser Val Glu Val Gly Val Arg Val Glu Ala Phe Asp Cys Arg
            260                 265                 270

Glu Trp Ala Glu Gly Gln Gly Arg His Ile Asn Ser Ala Phe Leu Ile
        275                 280                 285

Tyr Asn Ala Val Asp Asp Gln Glu Glu Leu Ile Thr Phe Pro Arg Ile
    290                 295                 300

Gln Pro Ile Ser Lys Asp Asp Phe Arg Arg Tyr Gln Gly Ala Ile Ala
305                 310                 315                 320

Arg Arg Arg Ile Arg Leu Gly Arg Lys Tyr Val Ile Ser His Lys Lys
                325                 330                 335

Glu Val Pro Leu Gly Thr Gln Trp Asp Ile Ser Lys Lys Gly Ser Ile
            340                 345                 350

Ser Asn Thr Asn Val Glu Ala Leu Lys Asn Leu Ala Ser Lys Ser Gly
        355                 360                 365

Trp Glu Ile Thr Thr Thr Leu Glu Lys Ile Lys Ile Tyr Thr Leu Glu
    370                 375                 380

Glu Gln Asp Ala Ile Ser Val Lys Val Glu Lys Gln Val Gly Ser Pro
385                 390                 395                 400

Ala Arg Val Ala Tyr His Leu Leu Ser Asp Phe Thr Lys Arg Pro Leu
                405                 410                 415

Trp Asp Pro His Tyr Ile Ser Cys Glu Val Ile Asp Gln Val Ser Glu
            420                 425                 430

Asp Asp Gln Ile Tyr Tyr Ile Thr Cys Ser Val Val Asn Gly Asp Lys
        435                 440                 445

Pro Lys Asp Phe Val Val Leu Val Ser Gln Arg Lys Pro Leu Lys Asp
    450                 455                 460

Asp Asn Thr Tyr Ile Val Ala Leu Met Ser Val Val Leu Pro Ser Val
465                 470                 475                 480

Pro Pro Ser Pro Gln Tyr Ile Arg Ser Gln Val Ile Cys Ala Gly Phe
                485                 490                 495

Leu Ile Gln Pro Val Asp Ser Ser Ser Cys Thr Val Ala Tyr Leu Asn
            500                 505                 510

Gln Met Ser Asp Ser Ile Leu Pro Tyr Phe Ala Gly Asn Ile Gly Gly
        515                 520                 525

Trp Ser Lys Ser Ile Glu Glu Ala Ala Ala Ser Cys Ile Lys Phe Ile
    530                 535                 540

Glu Asn Ala Thr His Asp Gly Leu Lys Ser Val Leu
545                 550                 555

<210>  101
<211>  756
<212>  DNA

<213>  Bacillus subtilis

<400>  101
ggatccatgt ctagaatgtc acaactgttc aaatccttcg atgcttcaga aaaaacccaa      60

ctgatttgct tcccgttcgc aggtggctat tccgcctcct tccgtccgct gcatgcgttt     120

ctgcaaggcg aatgtgaaat gctggcggcc gaaccgccgg gtcatggcac caaccagacg     180

agcgccattg aagacctgga agaactgacc gacctgtata aacaggaact gaatctgcgt     240

ccggatcgcc cgtttgtgct gttcggccat tctatgggcg gtatgattac gtttcgtctg     300

gcacagaaac tggaacgcga aggtatcttc ccgcaagcag ttattatcag tgctattcag     360

ccgccgcata tccaacgtaa aaaagtctcc cacctgccgg atgaccagtt tctggatcat     420

attatccaac tgggcggtat gccggcggaa ctggtcgaaa acaaagaagt gatgtcattt     480

ttcctgccga gctttcgttc tgattatcgc gcactggaac agtttgaact gtacgacctg     540

gctcagatcc aatcgccggt gcacgttttt aatggcctgg atgacaaaaa atgtattcgc     600

gatgcggaag gttggaaaaa atgggccaaa gatatcacct ttcatcagtt cgacggcggt     660

cacatgttcc tgctgtccca aacggaagaa gtggcagaac gcattttcgc tatcctgaac     720

cagcatccga ttatccagcc gtaagagctc aagctt                               756

<210>  102
<211>  242
<212>  PRT
<213>  Bacillus subtilis

<400>  102

Met Ser Gln Leu Phe Lys Ser Phe Asp Ala Ser Glu Lys Thr Gln Leu
1               5                   10                  15

Ile Cys Phe Pro Phe Ala Gly Gly Tyr Ser Ala Ser Phe Arg Pro Leu
            20                  25                  30

His Ala Phe Leu Gln Gly Glu Cys Glu Met Leu Ala Ala Glu Pro Pro
        35                  40                  45

Gly His Gly Thr Asn Gln Thr Ser Ala Ile Glu Asp Leu Glu Glu Leu
    50                  55                  60

Thr Asp Leu Tyr Lys Gln Glu Leu Asn Leu Arg Pro Asp Arg Pro Phe
65                  70                  75                  80

Val Leu Phe Gly His Ser Met Gly Gly Met Ile Thr Phe Arg Leu Ala
                85                  90                  95

Gln Lys Leu Glu Arg Glu Gly Ile Phe Pro Gln Ala Val Ile Ile Ser
            100                 105                 110

Ala Ile Gln Pro Pro His Ile Gln Arg Lys Lys Val Ser His Leu Pro
        115                 120                 125

Asp Asp Gln Phe Leu Asp His Ile Ile Gln Leu Gly Gly Met Pro Ala
    130                 135                 140

Glu Leu Val Glu Asn Lys Glu Val Met Ser Phe Phe Leu Pro Ser Phe
145                 150                 155                 160

Arg Ser Asp Tyr Arg Ala Leu Glu Gln Phe Glu Leu Tyr Asp Leu Ala
                165                 170                 175

Gln Ile Gln Ser Pro Val His Val Phe Asn Gly Leu Asp Asp Lys Lys
            180                 185                 190

Cys Ile Arg Asp Ala Glu Gly Trp Lys Lys Trp Ala Lys Asp Ile Thr
        195                 200                 205

Phe His Gln Phe Asp Gly Gly His Met Phe Leu Leu Ser Gln Thr Glu
    210                 215                 220

Glu Val Ala Glu Arg Ile Phe Ala Ile Leu Asn Gln His Pro Ile Ile
225                 230                 235                 240

Gln Pro

<210>  103
<211>  1602
<212>  DNA
<213>  Clostridium propionicum

<400>  103
ggatccatgt ctagaatgcg caaagtcccg attattacgg cagatgaagc ggctaaactg      60

attaaagacg gcgatacggt caccaccagc ggtttcgttg gcaacgcaat tccggaagct     120

ctggatcgtg cggttgaaaa acgctttctg gaaaccggcg aaccgaaaaa catcacgtat     180

gtctactgcg gcagtcaggg taatcgtgat ggccgcggtg ccgaacattt cgcacacgaa     240

ggcctgctga aacgttatat tgctggtcat tgggccaccg tcccggcact gggtaaaatg     300

gcaatggaaa acaaaatgga agcgtataat gtgtcacagg gcgcgctgtg tcacctgttt     360

cgtgatattg cctcgcacaa accgggtgtc tttaccaaag tgggcattgg tacgtttatc     420

gacccgcgca acggcggtgg caaagtgaat gatattacca aagaagacat cgtcgaactg     480

gtggaaatta aaggccagga atacctgttt tatccggcgt tcccgattca tgttgccctg     540

atccgcggca cctatgccga tgaatctggt aacattacgt ttgaaaaaga agtggcaccg     600

ctggaaggca ccagcgtgtg ccaggcagtc aaaaattctg gtggcatcgt ggttgtccaa 660

gttgaacgtg tggttaaagc gggcaccctg gacccgcgcc acgttaaagt cccgggtatt 720

tatgtggact acgtcgtggt tgctgatccg gaagaccatc agcaaagtct ggattgtgaa 780

tatgatccgg cactgtccgg tgaacaccgt cgcccggaag ttgtgggtga accgctgccg 840

ctgagtgcta aaaaagttat tggccgtcgc ggtgcgatcg aactggaaaa agatgtggcc 900

gttaacctgg gcgtgggtgc accggaatac gttgcgtccg tcgccgatga agaaggcatt 960

gttgacttta tgaccctgac ggcagaaagc ggtgctattg gcggcgtgcc ggcgggcggc 1020

gttcgttttg gcgcgtctta taatgcggat gccctgatcg accagggtta ccaattcgat 1080

tattacgacg gtggcggtct ggatctgtgc tatctgggcc tggcggaatg tgacgaaaag 1140

ggtaacatta atgtgtcacg ttttggtccg cgtattgcgg gttgtggtgg tttcattaac 1200

atcacccaga atacgccgaa agtctttttc tgtggcacct ttacggcagg cggtctgaaa 1260

gtgaaaattg aagatggcaa agtgattatc gttcaggaag gtaaacagaa aaaattcctg 1320

aaagcggttg aacaaatcac cttcaacggt gatgtcgcac tggctaataa acagcaagtg 1380

acctatatca cggaacgttg cgtttttctg ctgaaagaag atggcctgca cctgtcggaa 1440

attgcgccgg gtattgatct gcaaacccaa attctggatg tgatggactt cgccccgatt 1500

atcgatcgcg acgcaaatgg ccagatcaaa ctgatggatg cggcactgtt tgcggaaggt 1560

ctgatgggtc tgaaagaaat gaaatcgtaa gagctcaagc tt 1602

<210> 104
<211> 524
<212> PRT
<213> Clostridium propionicum

<400> 104

Met Arg Lys Val Pro Ile Ile Thr Ala Asp Glu Ala Ala Lys Leu Ile
1               5                   10                  15

Lys Asp Gly Asp Thr Val Thr Thr Ser Gly Phe Val Gly Asn Ala Ile
            20                  25                  30

Pro Glu Ala Leu Asp Arg Ala Val Glu Lys Arg Phe Leu Glu Thr Gly
        35                  40                  45

Glu Pro Lys Asn Ile Thr Tyr Val Tyr Cys Gly Ser Gln Gly Asn Arg
    50                  55                  60

Asp Gly Arg Gly Ala Glu His Phe Ala His Glu Gly Leu Leu Lys Arg
65                  70                  75                  80

Tyr Ile Ala Gly His Trp Ala Thr Val Pro Ala Leu Gly Lys Met Ala
                85                  90                  95

Met Glu Asn Lys Met Glu Ala Tyr Asn Val Ser Gln Gly Ala Leu Cys
            100                 105                 110

His Leu Phe Arg Asp Ile Ala Ser His Lys Pro Gly Val Phe Thr Lys
        115                 120                 125

Val Gly Ile Gly Thr Phe Ile Asp Pro Arg Asn Gly Gly Gly Lys Val
    130                 135                 140

Asn Asp Ile Thr Lys Glu Asp Ile Val Glu Leu Val Glu Ile Lys Gly
145                 150                 155                 160

Gln Glu Tyr Leu Phe Tyr Pro Ala Phe Pro Ile His Val Ala Leu Ile
                165                 170                 175

Arg Gly Thr Tyr Ala Asp Glu Ser Gly Asn Ile Thr Phe Glu Lys Glu
            180                 185                 190

Val Ala Pro Leu Glu Gly Thr Ser Val Cys Gln Ala Val Lys Asn Ser
        195                 200                 205

Gly Gly Ile Val Val Val Gln Val Glu Arg Val Val Lys Ala Gly Thr
    210                 215                 220

Leu Asp Pro Arg His Val Lys Val Pro Gly Ile Tyr Val Asp Tyr Val
225                 230                 235                 240

Val Val Ala Asp Pro Glu Asp His Gln Gln Ser Leu Asp Cys Glu Tyr
                245                 250                 255

Asp Pro Ala Leu Ser Gly Glu His Arg Arg Pro Glu Val Val Gly Glu
            260                 265                 270

Pro Leu Pro Leu Ser Ala Lys Lys Val Ile Gly Arg Arg Gly Ala Ile
        275                 280                 285

Glu Leu Glu Lys Asp Val Ala Val Asn Leu Gly Val Gly Ala Pro Glu
    290                 295                 300

Tyr Val Ala Ser Val Ala Asp Glu Glu Gly Ile Val Asp Phe Met Thr
305                 310                 315                 320

Leu Thr Ala Glu Ser Gly Ala Ile Gly Gly Val Pro Ala Gly Gly Val
                325                 330                 335

Arg Phe Gly Ala Ser Tyr Asn Ala Asp Ala Leu Ile Asp Gln Gly Tyr
            340                 345                 350

Gln Phe Asp Tyr Tyr Asp Gly Gly Gly Leu Asp Leu Cys Tyr Leu Gly
        355                 360                 365

Leu Ala Glu Cys Asp Glu Lys Gly Asn Ile Asn Val Ser Arg Phe Gly
    370                 375                 380

Pro Arg Ile Ala Gly Cys Gly Gly Phe Ile Asn Ile Thr Gln Asn Thr
385                 390                 395                 400

Pro Lys Val Phe Phe Cys Gly Thr Phe Thr Ala Gly Gly Leu Lys Val
                405                 410                 415

Lys Ile Glu Asp Gly Lys Val Ile Ile Val Gln Glu Gly Lys Gln Lys
            420                 425                 430

Lys Phe Leu Lys Ala Val Glu Gln Ile Thr Phe Asn Gly Asp Val Ala
        435                 440                 445

Leu Ala Asn Lys Gln Gln Val Thr Tyr Ile Thr Glu Arg Cys Val Phe
    450                 455                 460

Leu Leu Lys Glu Asp Gly Leu His Leu Ser Glu Ile Ala Pro Gly Ile
465                 470                 475                 480

Asp Leu Gln Thr Gln Ile Leu Asp Val Met Asp Phe Ala Pro Ile Ile
                485                 490                 495

Asp Arg Asp Ala Asn Gly Gln Ile Lys Leu Met Asp Ala Ala Leu Phe
            500                 505                 510

Ala Glu Gly Leu Met Gly Leu Lys Glu Met Lys Ser
        515                 520

<210>  105
<211>  1581
<212>  DNA
<213>  Megasphaera elsdenii

<400>  105
ggatccatgt ctagaatgcg taaagttgaa attattaccg cagaacaggc agcacagctg      60

gttaaagata atgataccat taccagcatt ggctttgtta gcagcgcaca tccggaagca     120

ctgaccaaag cactggaaaa acgttttctg gataccaata caccgcagaa tctgacctat     180

atttatgcag gtagccaggg taaacgtgat ggtcgtgcag cagaacatct ggcacataca     240

ggtctgctga aacgtgcaat tattggtcat tggcagaccg ttccggcaat tggtaaactg 300

gcagtggaaa ataaaattga agcctataat tttagccagg gcaccctggt tcattggttt 360

cgtgcactgg caggtcataa actgggtgtt tttaccgata ttggcctgga aacctttctg 420

gacccgcgtc agctgggtgg taaactgaat gatgttacca aagaggatct ggttaaactg 480

attgaagtgg atggtcatga acagctgttt tatccgacct ttccggttaa tgttgcattt 540

ctgcgtggca cctatgcaga tgaaagcggt aatattacaa tggatgaaga aattggtccg 600

tttgaaagca ccagcgttgc acaggcagtt cataattgtg gtggtaaagt tgtggttcag 660

gttaaagatg ttgttgcaca tggtagcctg gacccgcgta tggttaaaat tccgggtatt 720

tatgtggatt atgttgttgt tgcagcaccg gaagatcatc agcagaccta tgattgtgaa 780

tatgatccga gcctgagcgg tgaacatcgt gcaccggaag gtgcagcaga tgcagcactg 840

ccgatgagcg caaaaaaaat tattggtcgt cgtggtgcac tggaactgac cgaaaatgca 900

gttgttaatc tgggtgttgg tgcaccggaa tatgttgcaa gcgttgcggg tgaagaaggt 960

attgcagata ccattacact gaccgttgaa ggtggtgcaa ttggtggtgt tccgcagggt 1020

ggtgcacgtt ttggtagcag ccgtaatgca gatgccatta ttgatcatac ctatcagttt 1080

gatttttatg atggtggtgg tctggatatt gcatatctgg gtctggcaca gtgtgatggt 1140

agtggtaata ttaatgtgag caaatttggc accaatgttg caggttgtgg tggttttccg 1200

aatattagcc agcagacccc gaatgtttat ttttgtggca cctttaccgc aggcggtctg 1260

aaaattgcag ttgaagatgg caaagtgaaa attctgcaag aaggcaaagc caaaaaattt 1320

attaaagccg tggatcagat tacctttaat ggtagctatg cagcccgtaa tggtaaacat 1380

gttctgtata ttaccgaacg ctgcgttttt gaactgacaa aagaaggtct gaaactgatc 1440

gaagttgcac cgggtattga tattgaaaaa gatattctgg cccacatgga ttttaaaccg 1500

attattgata atccgaaact gatggatgcc cgtctgtttc aggatggtcc gatgggtctg 1560

aaacgttaag agctcaagct t 1581

<210> 106
<211> 517
<212> PRT
<213> Megasphaera elsdenii

<400> 106

Met Arg Lys Val Glu Ile Ile Thr Ala Glu Gln Ala Ala Gln Leu Val
1               5                   10                  15

Lys Asp Asn Asp Thr Ile Thr Ser Ile Gly Phe Val Ser Ser Ala His
            20                  25                  30

Pro Glu Ala Leu Thr Lys Ala Leu Glu Lys Arg Phe Leu Asp Thr Asn

35                  40                  45

Thr Pro Gln Asn Leu Thr Tyr Ile Tyr Ala Gly Ser Gln Gly Lys Arg
    50                  55                  60

Asp Gly Arg Ala Ala Glu His Leu Ala His Thr Gly Leu Leu Lys Arg
65                  70                  75                  80

Ala Ile Ile Gly His Trp Gln Thr Val Pro Ala Ile Gly Lys Leu Ala
                85                  90                  95

Val Glu Asn Lys Ile Glu Ala Tyr Asn Phe Ser Gln Gly Thr Leu Val
            100                 105                 110

His Trp Phe Arg Ala Leu Ala Gly His Lys Leu Gly Val Phe Thr Asp
        115                 120                 125

Ile Gly Leu Glu Thr Phe Leu Asp Pro Arg Gln Leu Gly Gly Lys Leu
    130                 135                 140

Asn Asp Val Thr Lys Glu Asp Leu Val Lys Leu Ile Glu Val Asp Gly
145                 150                 155                 160

His Glu Gln Leu Phe Tyr Pro Thr Phe Pro Val Asn Val Ala Phe Leu
                165                 170                 175

Arg Gly Thr Tyr Ala Asp Glu Ser Gly Asn Ile Thr Met Asp Glu Glu
            180                 185                 190

Ile Gly Pro Phe Glu Ser Thr Ser Val Ala Gln Ala Val His Asn Cys
        195                 200                 205

Gly Gly Lys Val Val Val Gln Val Lys Asp Val Val Ala His Gly Ser
    210                 215                 220

Leu Asp Pro Arg Met Val Lys Ile Pro Gly Ile Tyr Val Asp Tyr Val
225                 230                 235                 240

Val Val Ala Ala Pro Glu Asp His Gln Gln Thr Tyr Asp Cys Glu Tyr
                245                 250                 255

Asp Pro Ser Leu Ser Gly Glu His Arg Ala Pro Glu Gly Ala Ala Asp
            260                 265                 270

Ala Ala Leu Pro Met Ser Ala Lys Lys Ile Ile Gly Arg Arg Gly Ala
        275                 280                 285

Leu Glu Leu Thr Glu Asn Ala Val Val Asn Leu Gly Val Gly Ala Pro
    290                 295                 300

Glu Tyr Val Ala Ser Val Ala Gly Glu Glu Gly Ile Ala Asp Thr Ile
305                 310                 315                 320

Thr Leu Thr Val Glu Gly Gly Ala Ile Gly Gly Val Pro Gln Gly Gly
                325                 330                 335

Ala Arg Phe Gly Ser Ser Arg Asn Ala Asp Ala Ile Ile Asp His Thr
            340                 345                 350

Tyr Gln Phe Asp Phe Tyr Asp Gly Gly Gly Leu Asp Ile Ala Tyr Leu
        355                 360                 365

Gly Leu Ala Gln Cys Asp Gly Ser Gly Asn Ile Asn Val Ser Lys Phe
    370                 375                 380

Gly Thr Asn Val Ala Gly Cys Gly Gly Phe Pro Asn Ile Ser Gln Gln
385                 390                 395                 400

Thr Pro Asn Val Tyr Phe Cys Gly Thr Phe Thr Ala Gly Gly Leu Lys
                405                 410                 415

Ile Ala Val Glu Asp Gly Lys Val Lys Ile Leu Gln Glu Gly Lys Ala
            420                 425                 430

Lys Lys Phe Ile Lys Ala Val Asp Gln Ile Thr Phe Asn Gly Ser Tyr
        435                 440                 445

Ala Ala Arg Asn Gly Lys His Val Leu Tyr Ile Thr Glu Arg Cys Val
    450                 455                 460

Phe Glu Leu Thr Lys Glu Gly Leu Lys Leu Ile Glu Val Ala Pro Gly
465                 470                 475                 480

Ile Asp Ile Glu Lys Asp Ile Leu Ala His Met Asp Phe Lys Pro Ile
                485                 490                 495

Ile Asp Asn Pro Lys Leu Met Asp Ala Arg Leu Phe Gln Asp Gly Pro
            500                 505                 510

Met Gly Leu Lys Arg
        515

<210>  107
<211>  438
<212>  DNA

<213>  Haemophilus influenzae

<400>  107
ggatccatgt ctagaatgct ggataattgc tttagctttc cggtgcgtgt gtattatgaa      60

gataccgatg ccggtggtgt tgtttatcat gcacgttatc tgcatttttt tgaacgtgca     120

cgtaccgaat atctgcgtac cctgaatttt acccagcaga ccctgctgga agaacagcag     180

ctggcatttg ttgttaaaac cctggcaatt gattattgcg ttgcagcaaa actggatgat     240

ctgctgatgg ttgaaaccga agttagcgaa gttaaaggtg caaccattct gtttgaacag     300

cgtctgatgc gtaataccct gatgctgagc aaagcaaccg ttaaagttgc atgtgttgat     360

ctgggtaaaa tgaaaccggt tgcctttccg aaagaagtta aagcagcatt tcatcatctg     420

aaataagagc tcaagctt                                                   438


<210>  108
<211>  136
<212>  PRT
<213>  Haemophilus influenzae

<400>  108

Met Leu Asp Asn Cys Phe Ser Phe Pro Val Arg Val Tyr Tyr Glu Asp
1               5                   10                  15

Thr Asp Ala Gly Gly Val Val Tyr His Ala Arg Tyr Leu His Phe Phe
            20                  25                  30

Glu Arg Ala Arg Thr Glu Tyr Leu Arg Thr Leu Asn Phe Thr Gln Gln
        35                  40                  45

Thr Leu Leu Glu Glu Gln Gln Leu Ala Phe Val Val Lys Thr Leu Ala
    50                  55                  60

Ile Asp Tyr Cys Val Ala Ala Lys Leu Asp Asp Leu Leu Met Val Glu
65                  70                  75                  80

Thr Glu Val Ser Glu Val Lys Gly Ala Thr Ile Leu Phe Glu Gln Arg
                85                  90                  95

Leu Met Arg Asn Thr Leu Met Leu Ser Lys Ala Thr Val Lys Val Ala
            100                 105                 110

Cys Val Asp Leu Gly Lys Met Lys Pro Val Ala Phe Pro Lys Glu Val
        115                 120                 125

Lys Ala Ala Phe His His Leu Lys
    130                 135

<210>  109
<211>  523
<212>  PRT
<213>  Haemophilus influenzae

<400>  109

Met Gly Lys Val Lys Val Leu Thr Ala Arg Gln Ala Ala Asp Leu Ile
1               5                   10                  15

Lys Asp Gly Asp Thr Val Thr Leu Ser Gly Phe Val Ala Asn Gly Ile
            20                  25                  30

Ala Glu Ala Leu Asn Ala Ala Ala Glu Glu Arg Phe Leu Glu Thr Gly
        35                  40                  45

His Pro Lys Asp Leu Thr Leu Phe Trp Val Ala Gly Thr Gly Asn Lys
    50                  55                  60

Asp Gly Ser His Ala Asp His Tyr Ala His Glu Gly Met Val Lys Lys
65                  70                  75                  80

Val Ile Gly Gly His Phe Asn Phe Val Pro Lys Ile Cys Glu Met Leu
                85                  90                  95

Ser Glu Asn Lys Ile Glu Gly Tyr Asn Val Pro Gln Gly Ala Ile Ala
            100                 105                 110

Gln Met Leu Arg Asp Asn Ala Ala Arg Lys Val Gly Thr Ile Ser His
        115                 120                 125

Val Gly Ile Gly Thr Phe Ala Asp Pro Arg Asn Gly Gly Gly Arg Leu
    130                 135                 140

Ser Glu Lys Thr Lys Glu Asp Ile Val Lys Ile Ile Glu Leu Glu Gly
145                 150                 155                 160

Gln Glu Gln Leu Phe Tyr Pro Arg Ile Pro Leu Asp Val Ala Phe Ile
                165                 170                 175

Arg Gly Thr Tyr Ala Asp Glu Leu Gly Asn Ile Thr Leu Glu Lys Glu
            180                 185                 190

Met Ala Pro Leu Asp Ala Thr Ser Gln Ala Met Ala Val His Asn Asn
        195                 200                 205

Gly Gly Leu Val Val Val Gln Val Glu Arg Val Val Lys Ala Gly His
    210                 215                 220

Leu Asp Pro Lys Leu Val Lys Ile Pro Gly Ile Tyr Val Asp Ala Val
225                 230                 235                 240

Val Glu Cys Pro Ala Asp Asp Pro Lys Gln Ser Gln Ser Ile Asn Cys
                245                 250                 255

Thr Tyr Asp Pro Ala Tyr Ala Gly Asn Thr Gln Val Pro Val Ser Ser
            260                 265                 270

Leu Glu Pro Lys Lys Leu Asp Ala Lys Lys Ile Ile Gly Arg Arg Ala
        275                 280                 285

Ala Met Glu Leu Lys Lys Asn Val Val Val Asn Leu Gly Val Gly Val
    290                 295                 300

Pro Glu Trp Val Ser Ser Val Ala Ala Glu Glu Gly Val Ala Asp Glu
305                 310                 315                 320

Met Thr Leu Thr Val Asp Cys Gly Pro Val Gly Gly Val Pro Gly Gly
                325                 330                 335

Gly Leu Arg Phe Gly Gly Ser Val Asn Ala Gln Ala Tyr Met Asp Glu
            340                 345                 350

Gly Tyr Gln Phe Asp Phe Tyr Asp Gly Gly Gly Leu Asp Leu Cys Phe
        355                 360                 365

Leu Gly Leu Ala Glu Val Asp Asn Asn Gly Asp Val Asn Val Ser Arg
    370                 375                 380

Leu Gly Thr Arg Ile Thr Gly Ser Gly Gly Phe Thr Asn Ile Ser Ser
385                 390                 395                 400

Asn Ser Lys Lys Ala Val Phe Cys Gly Thr Phe Thr Asn Gly Val Lys
                405                 410                 415

Ile Gln Thr Gly Asp Gly Lys Leu Thr Ile Leu Glu Glu Gly Lys Lys
            420                 425                 430

His Lys Phe Val Asn Lys Val Thr Glu Ile Thr Phe Ser Gly Val Val
        435                 440                 445

Ala Gly Lys Ala Gly Lys Asp Val Leu Tyr Val Thr Glu Arg Ala Val
    450                 455                 460

Phe Ala Leu Lys Ala Asp Gly Ile His Leu Ile Glu Val Ala Pro Gly
465                 470                 475                 480

Ile Asp Val Gln Thr Gln Val Leu Asp Glu Met Asp Phe Ala Pro Ile
                485                 490                 495

Val Asp Arg Asp Ala Asp Gly Asn Val Lys Leu Met Asp Ala Arg Ile
            500                 505                 510

Phe Lys Asp Glu Val Met Gly Met Thr Ile Asp
        515                 520

<210>  110
<211>  527
<212>  PRT
<213>  Listeria monocytogenes

<400>  110

Met Ser Lys Val Ile Lys Ala Ser Glu Ala Ala Lys Leu Leu Lys Asp
1               5                   10                  15

Gly Asp Thr Val Ala Phe Ser Gly Phe Gly Leu Ala Cys Val Asn Glu
            20                  25                  30

Glu Met Ala Ile Ala Val Glu Lys Arg Phe Leu Glu Glu Gly Ala Pro
        35                  40                  45

Arg Asn Leu Thr Val Met His Ala Ser Ala Leu Gly Asp Arg Arg Glu
    50                  55                  60

Lys Gly Met Ser His Trp Gly His Glu Gly Leu Ile Lys Arg Trp Ile
65                  70                  75                  80

Gly Gly Ile Ala Ile Ala Ser Pro Lys Met Ala Lys Leu Ile Glu Glu
                85                  90                  95

Asp Lys Cys Glu Ala Tyr Asn Leu Pro Gln Gly Val Ile Thr Gln Leu
            100                 105                 110

Tyr Arg Glu Ile Ala Ala Lys Arg Pro Gly Val Ile Thr Lys Ile Gly
        115                 120                 125

Met Gly Thr Phe Val Asp Pro Arg Ile Glu Gly Ala Lys Met Ser Ala
    130                 135                 140

Ser Ser Lys Asp Asn Leu Val Glu Leu Leu Thr Ile His Asn Glu Glu
145                 150                 155                 160

Trp Leu Phe Tyr Pro Ser Phe Pro Ile Gln Val Ala Leu Ile Arg Gly
                165                 170                 175

Thr Val Ala Asp Glu Phe Gly Asn Leu Thr Leu Glu Lys Glu Gly Leu
            180                 185                 190

His Met Glu Val Leu Pro Ile Ala Gln Ala Val Arg Asn Ser Gly Gly
        195                 200                 205

Ile Val Ile Ala Gln Val Glu Ser Val Ala Lys Lys Gly Ser Leu Asn
    210                 215                 220

Pro Lys Asp Val Arg Val Pro Gly Ile Leu Val Asp His Ile Ile Ile
225                 230                 235                 240

Ser Glu Pro Glu Asn His Phe Gln Thr Glu Asn Thr Gln Tyr Asn Pro
                245                 250                 255

Ala Phe Ser Gly His Ile Gln Val Pro Leu Gly Asp Ile Glu Pro Leu
            260                 265                 270

Pro Leu Asp Asp Arg Lys Val Ile Ala Arg Arg Ser Ala Ala Glu Leu
        275                 280                 285

Glu Pro Gln Thr Ile Leu Asn Leu Gly Val Gly Ile Pro Val Asn Val
    290                 295                 300

Ser Thr Val Ala Ala Glu Glu Gly Val Ser Asp Gln Leu Ile Leu Thr
305                 310                 315                 320

Thr Asp Ala Gly Ser Val Gly Gly Val Pro Ala Gly Leu Ala Asp Phe
                325                 330                 335

Gly His Ala Tyr Asn Ser Glu Ala Ile Val Asp His His Ser Gln Phe
            340                 345                 350

Asp Phe Tyr Asp Gly Gly Gly Leu Asp Leu Ser Val Leu Gly Leu Ala
        355                 360                 365

Gln Thr Asp Glu Ser Gly Asn Val Asn Val Ser Lys Phe Gly Ser Arg
    370                 375                 380

Val Ala Gly Cys Gly Gly Phe Ile Asn Ile Ser Gln Ser Ala Lys Lys
385                 390                 395                 400

Leu Ile Phe Ala Gly Thr Phe Thr Ala Gly Gly Leu Lys Thr Arg Val
                405                 410                 415

Ala Asp Gly Lys Leu Glu Ile Leu Gln Glu Gly Lys Ala Lys Lys Phe
            420                 425                 430

Ile Lys Gln Val Gln Gln Ile Thr Phe Ser Gly Glu Tyr Ala Ser Thr
        435                 440                 445

Thr Asn Gln Thr Ile Leu Tyr Val Thr Glu Arg Ala Val Phe Arg Leu
    450                 455                 460

Glu Asn Gly Lys Met Val Leu Thr Glu Ile Ala Pro Gly Ile Asp Leu
465                 470                 475                 480

Glu Lys Asp Ile Leu Gly Gln Met Glu Phe Glu Pro Ile Ile Ala Ser
                485                 490                 495

Asp Leu Lys Val Met Asp Gly Gly Met Phe Ser Glu Glu Trp Gly Gly
            500                 505                 510

Leu Lys Ala Ile Ile Glu Lys Gln Thr Arg Glu Gly Val Ser Ile
        515                 520                 525

<210>  111
<211>  472
<212>  PRT
<213>  Pseudomonas mendocina

<400>  111

Pro Arg Asp Leu Thr Leu Val Tyr Ala Ala Gly Gln Gly Asp Gly Lys
1               5                   10                  15

Gly Arg Gly Leu Asn His Leu Ala His Glu Gly Leu Val Arg Arg Val
            20                  25                  30

Ile Gly Gly His Trp Gly Leu Val Pro Gly Leu Gln Lys Leu Ala Val
        35                  40                  45

Asp Asn Arg Ile Glu Ala Tyr Asn Leu Pro Gln Gly Val Ile Ser Gln
    50                  55                  60

Leu Phe Arg Asp Ile Ala Ala Gly Lys Pro Gly Gln Leu Ser Arg Val
65                  70                  75                  80

Gly Leu Gly Thr Tyr Val Asp Pro Arg His Gly Gly Gly Lys Leu Asn
                85                  90                  95

Ala Arg Thr Thr Ala Asp Leu Val Arg Leu Met Pro Ile Asp Gly Glu
            100                 105                 110

Asp Tyr Leu Phe Tyr Pro Thr Phe Pro Ile Asp Val Ala Val Val Arg
        115                 120                 125

Ala Thr Ser Ser Asp Pro Asp Gly Asn Leu Ser Phe Glu Arg Glu Ala
    130                 135                 140

Leu Thr Ile Glu Ser Leu Ala Ile Ala Met Ala Ala Arg Asn Cys Gly
145                 150                 155                 160

Gly Leu Val Ile Ala Gln Val Glu Arg Ile Val Glu Arg Gly Ser Leu
                165                 170                 175

Asn Ala Arg Glu Val Lys Ile Pro Gly Ile Leu Val Asp Cys Val Val
            180                 185                 190

Gln Ala Glu Pro Ala Asn His Gln Gln Thr Phe Ala Thr Ala Tyr Asn
        195                 200                 205

Pro Ala Phe Ala Ala Glu Thr Arg Val Pro Val Asp Ser Leu Ala Pro
    210                 215                 220

Met Pro Leu Asp Val Arg Lys Leu Ile Ala Arg Arg Ala Ala Leu Glu
225                 230                 235                 240

Leu Lys Gly Gly Ala Val Val Asn Leu Gly Ile Gly Met Pro Asp Gly
                245                 250                 255

Val Ala Ala Val Ala Ala Glu Glu Gly Val Ile Glu Arg Leu Thr Leu
            260                 265                 270

Thr Ala Glu Pro Gly Val Ile Gly Gly Val Pro Ala Ser Gly Leu Asp
        275                 280                 285

Phe Gly Ala Ala Ser Asn His Ser Ala Leu Leu Asp Gln Pro Tyr Gln
    290                 295                 300

Phe Asp Phe Tyr Asp Gly Gly Gly Leu Asp Ile Ala Phe Leu Gly Leu
305                 310                 315                 320

Ala Gln Ala Asp Ala Ala Gly Asn Leu Asn Val Ser Lys Phe Gly Ser
                325                 330                 335

Arg Leu Ala Gly Ala Gly Gly Phe Ile Asn Ile Ser Gln Asn Ala Lys
            340                 345                 350

Gln Val Val Phe Val Gly Thr Phe Ser Ala Gly Ala Gln Asp Ile Arg
        355                 360                 365

Ile Glu Gly Gly Gln Leu Arg Ile Val Gln Asp Gly Glu Leu Arg Lys

370                 375                 380

Phe Val Ala Glu Val Glu His Arg Thr Phe Ala Gly Arg Leu Ala Ala
385                 390                 395                 400

Glu Arg Gly Gln Pro Val Leu Tyr Val Thr Glu Arg Cys Val Leu Arg
                405                 410                 415

Leu Ser Arg Glu Gly Leu Glu Leu Ile Glu Val Ala Pro Gly Val Asp
            420                 425                 430

Ile Gln Arg Asp Ile Leu Ser Arg Met Asp Phe Ala Pro Ile Val Arg
        435                 440                 445

Glu Pro Lys Leu Met Asp Ala Arg Leu Phe Arg Pro Glu Pro Ile Gly
    450                 455                 460

Leu Ala Gln Cys Leu Glu Asn Leu
465                 470

<210>  112
<211>  515
<212>  PRT
<213>  Erysipelotrichaceae bacterium

<400>  112

Met Ser Lys Val Ile Ser Ile Glu Gln Ala Val Ser Met Ile Pro Asp
1               5                   10                  15

Gly Ala Ala Ile Gly Ile Gly Gly Phe Ile Gly Ser Gly His Pro Gln
            20                  25                  30

Glu Phe Ser Val Gly Ile Glu Glu Ser Phe Leu Lys Ser Gly His Pro
        35                  40                  45

Lys Asp Leu Thr Ile Met Phe Ser Ala Gly Ile Gly Asp Gly Thr Asp
    50                  55                  60

Arg Leu Gly Leu Asn Lys Leu Gly His Glu Gly Leu Leu Lys Arg Ile
65                  70                  75                  80

Ile Gly Gly His Trp Gly Leu Ile Pro Lys Leu Gln Lys Leu Val Phe
                85                  90                  95

Glu Asn Lys Val Glu Gly Tyr Asn Leu Pro Leu Gly Thr Ile Ser Leu
            100                 105                 110

Met Phe Arg Asp Ile Ala Gly His Arg Pro Gly Thr Ile Thr Lys Val

115                 120                 125

Gly Leu Lys Thr Phe Val Asp Pro Arg Ile Glu Gly Ala Lys Met Asn
    130                 135                 140

Glu Arg Ser Lys Glu Asp Leu Val Glu Leu Met His Ile Asp Gly Glu
145                 150                 155                 160

Glu Trp Leu Arg Tyr Lys Ser Phe Pro Leu Asn Val Ala Leu Ile Arg
                165                 170                 175

Ala Thr Tyr Cys Asp Glu Asp Gly Asn Ala Thr Met Glu Lys Glu Ala
            180                 185                 190

Ala Thr Leu Asp Ser Leu Ser Ile Ala Gln Ala Ala Lys Asn Ser Gly
        195                 200                 205

Gly Ile Val Leu Leu Gln Val Glu Lys Val Val Gln Asn Gly Thr Leu
    210                 215                 220

Asp Pro Arg Lys Val Lys Ile Pro Gly Ile Tyr Val Asp Gly Ile Val
225                 230                 235                 240

Val Ala Arg Pro Glu Asn His Trp Gln Thr Tyr Ala Asn Pro Tyr Asp
                245                 250                 255

Pro Ala Leu Ser Gly Glu Val Lys Val Pro Val Asn Ser Ile Ala Pro
            260                 265                 270

Met Lys Leu Asn Glu Arg Lys Val Ile Cys Arg Arg Ala Ala Met Glu
        275                 280                 285

Leu Asp Pro Ala Ala Ile Ile Asn Leu Gly Ile Gly Met Pro Asp Gly
    290                 295                 300

Ile Ala Asn Val Ala Asn Glu Glu Gly Leu Pro Gly Leu Lys Leu Thr
305                 310                 315                 320

Val Glu Ala Gly Gly Ile Gly Gly Val Pro Asn Ala Gly Thr Ala Phe
                325                 330                 335

Gly Thr Cys Thr Asn Pro Asp Ala Ile Ile Asp Gln Pro Tyr Gln Phe
            340                 345                 350

Asp Phe Tyr Asp Gly Gly Gly Leu Asp Gln Ala Phe Leu Gly Leu Ala
        355                 360                 365

Glu Cys Asp Cys Ser Gly Asn Ile Asn Val Ser Arg Phe Gly Pro Lys
    370                 375                 380

Ile Ala Gly Cys Gly Gly Phe Ile Asn Ile Thr Gln Thr Ser Pro Val
385                 390                 395                 400

Val Val Tyr Cys Gly Thr Phe Thr Ala Gly Gly Leu Lys Val Glu Val
                405                 410                 415

Arg Asp Gly Lys Leu His Ile Leu Gln Glu Gly Arg Ile Lys Lys Phe
            420                 425                 430

Lys Lys Glu Val Glu Gln Ile Thr Phe Ser Ala Glu Phe Ala Thr Glu
        435                 440                 445

Thr Gly Gln Lys Val Leu Tyr Val Thr Glu Arg Ala Val Phe Glu Leu
    450                 455                 460

Leu Asp Gly Lys Leu Thr Leu Thr Glu Ile Ala Pro Gly Val Asp Leu
465                 470                 475                 480

Glu Gln Asp Val Leu Gly Gln Met Glu Phe Lys Pro Ala Val Ala Glu
                485                 490                 495

His Leu Lys Thr Met Asp Glu Arg Leu Phe Arg Asp Glu Leu Met Gly
            500                 505                 510

Leu Lys Ala
        515

<210>  113
<211>  1263
<212>  DNA
<213>  Escherichia coli

<400>  113
atgccacatt cactgttcag caccgatacc gatctcaccg ccgaaaatct gctgcgtttg      60

cccgctgaat ttggctgccc ggtgtgggtc tacgatgcgc aaattattcg tcggcagatt     120

gcagcgctga aacagtttga tgtggtgcgc tttgcacaga aagcctgttc caatattcat     180

attttgcgct taatgcgtga gcagggcgtg aaagtggatt ccgtctcgtt aggcgaaata     240

gagcgtgcgt tggcggcggg ttacaatccg caaacgcacc ccgatgatat tgtttttacg     300

gcagatgtta tcgatcaggc gacgcttgaa cgcgtcagtg aattgcaaat tccggtgaat     360

gcgggttctg ttgatatgct cgaccaactg ggccaggttt cgccagggca tcgggtatgg     420

ctgcgcgtta atccggggtt tggtcacgga catagccaaa aaaccaatac cggtggcgaa     480

aacagcaagc acggtatctg gtacaccgat ctgcccgccg cactggacgt gatacaacgt     540

catcatctgc agctggtcgg cattcacatg cacattggtt ctggcgttga ttatgcccat 600

ctggaacagg tgtgtggtgc tatggtgcgt caggtcatcg aattcggtca ggatttacag 660

gctatttctg cgggcggtgg gctttctgtt ccttatcaac agggtgaaga ggcggttgat 720

accgaacatt attatggtct gtggaatgcc gcgcgtgagc aaatcgcccg ccatttgggc 780

caccctgtga aactggaaat tgaaccgggt cgcttcctgg tagcgcagtc tggcgtatta 840

attactcagg tgcggagcgt caaacaaatg gggagccgcc actttgtgct ggttgatgcc 900

gggttcaacg atctgatgcg cccggcaatg tacggtagtt accaccatat cagtgccctg 960

gcagctgatg gtcgttctct ggaacacgcg ccaacggtgg aaaccgtcgt cgccggaccg 1020

ttatgtgaat cgggcgatgt ctttacccag caggaagggg gaaatgttga aacccgcgcc 1080

ttgccggaag tgaaggcagg tgattatctg gtactgcatg atacaggggc atatggcgca 1140

tcaatgtcat ccaactacaa tagccgtccg ctgttaccag aagttctgtt tgataatggt 1200

caggcgcggt tgattcgccg tcgccagacc atcgaagaat tactggcgct ggaattgctt 1260

taa 1263

<210> 114
<211> 420
<212> PRT
<213> Escherichia coli

<400> 114

Met Pro His Ser Leu Phe Ser Thr Asp Thr Asp Leu Thr Ala Glu Asn
1               5                   10                  15

Leu Leu Arg Leu Pro Ala Glu Phe Gly Cys Pro Val Trp Val Tyr Asp
            20                  25                  30

Ala Gln Ile Ile Arg Arg Gln Ile Ala Ala Leu Lys Gln Phe Asp Val
        35                  40                  45

Val Arg Phe Ala Gln Lys Ala Cys Ser Asn Ile His Ile Leu Arg Leu
    50                  55                  60

Met Arg Glu Gln Gly Val Lys Val Asp Ser Val Ser Leu Gly Glu Ile
65                  70                  75                  80

Glu Arg Ala Leu Ala Ala Gly Tyr Asn Pro Gln Thr His Pro Asp Asp
                85                  90                  95

Ile Val Phe Thr Ala Asp Val Ile Asp Gln Ala Thr Leu Glu Arg Val
            100                 105                 110

Ser Glu Leu Gln Ile Pro Val Asn Ala Gly Ser Val Asp Met Leu Asp
        115                 120                 125

Gln Leu Gly Gln Val Ser Pro Gly His Arg Val Trp Leu Arg Val Asn
    130                 135                 140

Pro Gly Phe Gly His Gly His Ser Gln Lys Thr Asn Thr Gly Gly Glu
145                 150                 155                 160

Asn Ser Lys His Gly Ile Trp Tyr Thr Asp Leu Pro Ala Ala Leu Asp
                165                 170                 175

Val Ile Gln Arg His His Leu Gln Leu Val Gly Ile His Met His Ile
            180                 185                 190

Gly Ser Gly Val Asp Tyr Ala His Leu Glu Gln Val Cys Gly Ala Met
        195                 200                 205

Val Arg Gln Val Ile Glu Phe Gly Gln Asp Leu Gln Ala Ile Ser Ala
    210                 215                 220

Gly Gly Gly Leu Ser Val Pro Tyr Gln Gln Gly Glu Glu Ala Val Asp
225                 230                 235                 240

Thr Glu His Tyr Tyr Gly Leu Trp Asn Ala Ala Arg Glu Gln Ile Ala
                245                 250                 255

Arg His Leu Gly His Pro Val Lys Leu Glu Ile Glu Pro Gly Arg Phe
            260                 265                 270

Leu Val Ala Gln Ser Gly Val Leu Ile Thr Gln Val Arg Ser Val Lys
        275                 280                 285

Gln Met Gly Ser Arg His Phe Val Leu Val Asp Ala Gly Phe Asn Asp
    290                 295                 300

Leu Met Arg Pro Ala Met Tyr Gly Ser Tyr His His Ile Ser Ala Leu
305                 310                 315                 320

Ala Ala Asp Gly Arg Ser Leu Glu His Ala Pro Thr Val Glu Thr Val
                325                 330                 335

Val Ala Gly Pro Leu Cys Glu Ser Gly Asp Val Phe Thr Gln Gln Glu
            340                 345                 350

Gly Gly Asn Val Glu Thr Arg Ala Leu Pro Glu Val Lys Ala Gly Asp
        355                 360                 365

Tyr Leu Val Leu His Asp Thr Gly Ala Tyr Gly Ala Ser Met Ser Ser
    370                 375                 380

Asn Tyr Asn Ser Arg Pro Leu Leu Pro Glu Val Leu Phe Asp Asn Gly
385                 390                 395                 400

Gln Ala Arg Leu Ile Arg Arg Arg Gln Thr Ile Glu Glu Leu Leu Ala
                405                 410                 415

Leu Glu Leu Leu
            420

<210>  115
<211>  879
<212>  DNA
<213>  Escherichia coli

<400>  115
atgttcacgg gaagtattgt cgcgattgtt actccgatgg atgaaaaagg taatgtctgt      60

cgggctagct tgaaaaaact gattgattat catgtcgcca gcggtacttc ggcgatcgtt     120

tctgttggca ccactggcga gtccgctacc ttaaatcatg acgaacatgc tgatgtggtg     180

atgatgacgc tggatctggc tgatgggcgc attccggtaa ttgccgggac cggcgctaac     240

gctactgcgg aagccattag cctgacgcag cgcttcaatg acagtggtat cgtcggctgc     300

ctgacggtaa ccccttacta caatcgtccg tcgcaagaag gtttgtatca gcatttcaaa     360

gccatcgctg agcatactga cctgccgcaa attctgtata atgtgccgtc ccgtactggc     420

tgcgatctgc tcccggaaac ggtgggccgt ctggcgaaag taaaaaatat tatcggaatc     480

aaagaggcaa cagggaactt aacgcgtgta aaccagatca aagagctggt ttcagatgat     540

tttgttctgc tgagcggcga tgatgcgagc gcgctggact tcatgcaatt gggcggtcat     600

ggggttattt ccgttacggc taacgtcgca gcgcgtgata tggcccagat gtgcaaactg     660

gcagcagaag ggcattttgc cgaggcacgc gttattaatc agcgtctgat gccattacac     720

aacaaactat ttgtcgaacc caatccaatc ccggtgaaat gggcatgtaa ggaactgggt     780

cttgtggcga ccgatacgct gcgcctgcca atgacaccaa tcaccgacag tggtcgtgag     840

acggtcagag cggcgcttaa gcatgccggt ttgctgtaa                            879

<210>  116
<211>  292
<212>  PRT
<213>  Escherichia coli

<400>  116

Met Phe Thr Gly Ser Ile Val Ala Ile Val Thr Pro Met Asp Glu Lys
1               5                   10                  15

Gly Asn Val Cys Arg Ala Ser Leu Lys Lys Leu Ile Asp Tyr His Val
            20                  25                  30

Ala Ser Gly Thr Ser Ala Ile Val Ser Val Gly Thr Thr Gly Glu Ser
        35                  40                  45

Ala Thr Leu Asn His Asp Glu His Ala Asp Val Val Met Met Thr Leu
    50                  55                  60

Asp Leu Ala Asp Gly Arg Ile Pro Val Ile Ala Gly Thr Gly Ala Asn
65                  70                  75                  80

Ala Thr Ala Glu Ala Ile Ser Leu Thr Gln Arg Phe Asn Asp Ser Gly
                85                  90                  95

Ile Val Gly Cys Leu Thr Val Thr Pro Tyr Tyr Asn Arg Pro Ser Gln
            100                 105                 110

Glu Gly Leu Tyr Gln His Phe Lys Ala Ile Ala Glu His Thr Asp Leu
        115                 120                 125

Pro Gln Ile Leu Tyr Asn Val Pro Ser Arg Thr Gly Cys Asp Leu Leu
    130                 135                 140

Pro Glu Thr Val Gly Arg Leu Ala Lys Val Lys Asn Ile Ile Gly Ile
145                 150                 155                 160

Lys Glu Ala Thr Gly Asn Leu Thr Arg Val Asn Gln Ile Lys Glu Leu
                165                 170                 175

Val Ser Asp Asp Phe Val Leu Leu Ser Gly Asp Asp Ala Ser Ala Leu
            180                 185                 190

Asp Phe Met Gln Leu Gly Gly His Gly Val Ile Ser Val Thr Ala Asn
        195                 200                 205

Val Ala Ala Arg Asp Met Ala Gln Met Cys Lys Leu Ala Ala Glu Gly
    210                 215                 220

His Phe Ala Glu Ala Arg Val Ile Asn Gln Arg Leu Met Pro Leu His
225                 230                 235                 240

Asn Lys Leu Phe Val Glu Pro Asn Pro Ile Pro Val Lys Trp Ala Cys
                245                 250                 255

Lys Glu Leu Gly Leu Val Ala Thr Asp Thr Leu Arg Leu Pro Met Thr

260                 265                 270

Pro Ile Thr Asp Ser Gly Arg Glu Thr Val Arg Ala Ala Leu Lys His
        275                 280                 285

Ala Gly Leu Leu
    290

<210>  117
<211>  410
<212>  DNA
<213>  Artificial Sequence

<220>
<223>  modification to pET30a vector

<400>  117
gcatgcaagg agatggcgcc caacagtccc ccggccacgg ggcctgccac catacccacg      60

ccgaaacaag cgctcatgag cccgaagtgg cgagcccgat cttccccatc ggtgatgtcg     120

gcgatatagg cgccagcaac cgcacctgtg gcgccggtga tgccggccac gatgcgtccg     180

gcgtagagga tcgagatcga tctcgatccc gcgaaattaa tacgactcac tataggggaa     240

ttgtgagcgg ataacaattc ccctctagaa ataattttgt ttaactttaa gaaggagata     300

tacatatgca ccatcatcat catcattctt ctggtaccgg tggtggctcc ggtattgagg     360

gtcgcgccat ggcgatatcg aattcggatc cgagctccct gcagctcgag                410

<210>  118
<211>  33
<212>  DNA
<213>  Aritifical Sequence

<400>  118
ggtaccggtg gtggctccgg tattgagggt cgc                                   33

<210>  119
<211>  21
<212>  DNA
<213>  Artificial Sequence

<220>
<223>  3' prime suffix sequence to stop codon of aminotransferase

<400>  119
tactagtagc ggccgctgca g                                                21

<210>  120
<211>  1242
<212>  DNA
<213>  Sus scrofa

<400>  120
atggctccgc cgagtgtgtt tgctgaagtt ccgcaggccc aaccggtgct ggtgtttaag      60

ctgattgctg attttcgtga agacccggac ccgcgtaaag ttaatctggg cgtcggtgca 120

tatcgcaccg atgactgcca gccgtgggtg ctgccggtgg ttcgtaaggt tgaacaacgc 180

attgcgaacg atagctctct gaatcatgaa tacctgccga tcctgggcct ggccgaattt 240

cgtacctgtg caagccgcct ggctctgggt gatgactctc cggcgctgca agaaaaacgt 300

gtcggcggtg tgcagagcct gggcggcacc ggcgctctgc gtattggtgc ggaatttctg 360

gcccgctggt ataacggcac caacaataaa gacacgccgg tttacgtcag ttccccgacc 420

tgggaaaacc acaatggcgt gtttaccacc gcgggcttca aagatattcg tagctatcgc 480

tactgggata cggaaaagcg cggcctggat ctgcaaggtt ttctgagtga tctggaaaat 540

gcgccggaat tttccatttt cgtcctgcat gcatgcgcac acaacccgac cggcaccgat 600

ccgaccccgg aacagtggaa acaaatcgcc agtgttatga agcgtcgctt tctgttcccg 660

tttttcgact cagcgtatca gggctttgcc tcgggtaatc tggaaaaaga tgcatgggct 720

attcgttact tcgtttctga aggttttgaa ctgttctgtg cacagagctt tagcaaaaac 780

ttcggcctgt ataatgaacg cgttggtaac ctgaccgtcg tggctaaaga accggatagt 840

attctgcgtg tcctgtccca gatggaaaag atcgtgcgcg ttacctggtc aaatccgccg 900

gcacaaggcg cccgtattgt ggcacgtacg ctgtcggacc cggaactgtt tcatgaatgg 960

accggtaacg ttaaaacgat ggccgatcgt atcctgagca tgcgctctga actgcgtgca 1020

cgcctggaag ctctgaagac cccgggtacg tggaaccata ttaccgacca gatcggcatg 1080

tttagcttca cgggtctgaa tccgaaacaa gtggaatatc tgattaacga aaagcacatc 1140

tacctgctgc cgagcggtcg tattaatatg tgcggtctga ccacgaaaaa cctggattat 1200

gtcgcaacct ctattcacga agctgtgacg aagatccagt aa 1242

<210> 121
<211> 1491
<212> DNA
<213> Rattus norvegicus

<400> 121
atggcgtccc gtgtcaacga tcaatcacag gcgtctcgca atggtctgaa aggcaaggtg 60

ctgaccctgg atacgatgaa cccgtgtgtc cgtcgcgtgg aatatgcagt gcgtggtccg 120

atcgttcagc gcgccctgga actggaacag gaactgcgtc aaggtgtcaa aaagccgttt 180

accgaagtga ttcgcgcaaa catcggtgat gcacaggcga tgggccaacg tccgattacg 240

tttttccgtc aggtcctggc gctgtgcgtg tacccgaacc tgctgagcag cccggatttc 300

ccggaagacg caaaacgtcg cgctgaacgt attctgcaag cgtgcggtgg tcatagcctg 360

ggcgcttatt ctattagttc cggtattcag ccgatccgcg aagatgtggc acaatacatt 420

gaacgtcgcg atggtggtat cccggctgac ccgaacaata tttttctgag taccggcgcg 480

tccgacgcca tcgttacgat gctgaaactg ctggtcagcg gtgaaggtcg tgcgcgcacc 540

ggtgttctga ttccgatccc gcagtatccg ctgtactctg cggccctggc ggaactggat 600

gcagtgcagg ttgattatta cctggacgaa gaacgtgcat gggcgctgga tattgccgaa 660

ctgcgtcgcg cactgtgcca ggctcgtgac cgttgctgtc cgcgcgttct gtgtgtcatt 720

aacccgggca atccgaccgg tcaggtccaa acgcgtgaat gcattgaagc agtgatccgc 780

tttgctttca aggaaggcct gtttctgatg gcagatgaag tttatcagga caacgtctac 840

gctgaaggct cacaatttca ttcgttcaaa aaggtgctga tggaaatggg tccgccgtat 900

agtacccagc aagaactggc gtcctttcac tcagtgagca aaggttatat gggcgaatgc 960

ggtttccgtg gcggttacgt tgaagtggtt aatatggatg ccgaagtcca gaaacaaatg 1020

ggcaagctga tgagcgtgcg cctgtgtccg ccggttccgg gtcaggccct gatggacatg 1080

gtcgtgtctc cgccgacccc gagcgaaccg tcttttaaac agttccaagc agaacgtcag 1140

gaagtgctgg cggaactggc agctaaagcc aagctgacgg aacaggtgtt taacgaagcg 1200

ccgggcattc gttgcaatcc ggttcagggt gccatgtata gcttcccgca ggtgcaactg 1260

ccgctgaaag cggttcagcg cgcccaagaa ctgggtctgg cgccggatat gtttttctgc 1320

ctgtgtctgc tggaagaaac cggcatctgt gttgtcccgg gctccggttt tggccagcag 1380

gaaggcacct atcatttccg tatgacgatt ctgccgccga tggaaaaact gcgcctgctg 1440

ctggaaaagc tgtcacattt tcacgcgaaa ttcacgcacg aatactcgta a 1491

<210> 122
<211> 1020
<212> DNA
<213> Pseudomonas fluorescens

<400> 122
atgggtaatg aatcaatcaa ctgggacaaa ctgggcttcg actacatcaa gacggacaaa 60

cgctttctgc aagtgtggaa aaacggtgaa tggcaggcgg gcaccctgac ggatgacaac 120

gtgctgcata tcagtgaagg ttccaccgca ctgcactatg gccagcaatg ctttgaaggt 180

ctgaaagcgt atcgttgtaa ggatggctca attaacctgt tccgtccgga ccagaatgcg 240

gcccgtatgc aacgtagctg cgcgcgtctg ctgatgccgc atgtttctac cgaagatttt 300

atcgacgcgt gtaaacaggt ggttaaggcc aacgaacgct tcattccgcc gtatggttcc 360

ggcggtgcgc tgtacctgcg tccgtttgtg atcggcaccg gtgataatat tggcgttcgc 420

acggccccgg aatttatctt cagcgtgttt gcaattccgg ttggtgctta tttcaaaggc 480

ggtctggttc cgcacaactt tcagattagc accttcgacc gtgcagctcc gcaaggcacg 540

ggtgcagcaa aggtcggcgg taattatgca gctagtctga tgccgggcgc agaagctaaa 600

aagtccggtt ttgcggatgc catctacctg gacccgatga cccattcaaa aattgaagaa 660

gtgggctcgg caaacttttt cggtatcacc cacgataata aattcattac gccgaaaagc 720

ccgtctgttc tgccgggcat cacccgtctg agcctgattg aactggccaa aacgcgcctg 780

ggcctggaag tcgtggaagg tgaagtcttt attgataaac tggaccagtt caaggaagca 840

ggtgcttgcg gcaccgcggc ggtgattagc ccgatcggcg gtattcaata caacggcaaa 900

ctgcatgttt ttcactcgga aaccgaagtc ggtccgatca cgcagaaact gtacaaggaa 960

ctgaccggcg tccaaacggg tgatgtggaa gcgccggcgg gttggattgt taaagtctaa 1020

<210> 123
<211> 1218
<212> DNA
<213> Escherichia coli

<400> 123
atgagcccga ttgaaaagag cagcaaactg gaaaacgtct gttatgacat ccgtggtccg 60

gtcctgaaag aagcaaaacg cctggaagaa gaaggcaaca aagtgctgaa gctgaacatt 120

ggcaatccgg caccgtttgg tttcgatgct ccggacgaaa ttctggtgga cgttatccgt 180

aatctgccga ccgcgcaggg ctattgcgat agtaaaggtc tgtactccgc acgcaaggct 240

attatgcagc attatcaagc ccgtggtatg cgcgacgtca cggtggaaga tatttacatc 300

ggcaacggtg tctcagaact gatcgtgcag gcgatgcagg cgctgctgaa cagcggtgac 360

gaaatgctgg tgccggcacc ggattatccg ctgtggaccg cggcggtgag cctgagcagc 420

ggtaaagccg ttcattacct gtgtgatgaa agttccgact ggtttccgga tctggatgac 480

attcgtgcaa agatcacccc gcgtacgcgc ggcattgtta ttatcaaccc gaacaatccg 540

accggtgctg tttattccaa agaactgctg atggaaattg tcgaaatcgc acgccagcac 600

aatctgatca tcttcgctga cgaaatttat gataaaatcc tgtacgatga cgcggaacat 660

cacagcattg caccgctggc tccggatctg ctgaccatca cgtttaacgg cctgtctaaa 720

acctatcgtg tcgcaggctt ccgccaaggt tggatggtgc tgaatggtcc gaaaaagcat 780

gctaagggct acattgaagg tctggaaatg ctggcgtcta tgcgtctgtg cgcaaacgtg 840

ccggcacagc acgcaatcca aaccgcactg ggcggttatc agtcaatttc ggaatttatc 900

acgccgggcg gtcgcctgta cgaacaacgt aaccgcgcgt gggaactgat taatgatatt 960

ccgggcgtta gctgtgtcaa accgcgtggt gcgctgtata tgtttccgaa aattgatgcc 1020

aagcgcttca acatccacga tgaccagaaa atggttctgg attttctgct gcaagaaaag 1080

gtgctgctgg ttcaaggcac cgcctttaat tggccgtggc cggatcattt ccgtattgtt 1140

acgctgccgc gcgtcgatga catcgaactg agcctgtcta aatttgcacg tttcctgagt 1200

ggttaccacc aactgtaa 1218

<210>  124
<211>  1699
<212>  DNA
<213>  Lactococcus lactis

<400>  124
gaattcgcgg ccgcttctag aaggagatat acatatgtat accgtgggtg actacctgct      60

ggaccgtctg catgaactgg gcattgaaga aatctttggt gttccgggtg actacaacct     120

gcaatttctg gatcaaatta tctcacgtga agacatgaaa tggattggta acgcaaatga     180

actgaacgca tcgtatatgg ctgatggcta cgcgcgcacc aaaaaagcgg cggcgtttct     240

gaccacgttc ggcgttggtg aactgagcgc gattaacggc ctggccggtt cttatgcaga     300

aaatctgccg gtggttgaaa tcgttggctc accgacgtcg aaagtccaga atgatggtaa     360

atttgtgcat cacaccctgg cggatggcga ctttaaacat ttcatgaaaa tgcacgaacc     420

ggtgacggct gcgcgtaccc tgctgacggc ggaaaacgcc acctatgaaa ttgatcgtgt     480

gctgagtcaa ctgctgaaag aacgcaaacc ggtttacatc aatctgccgg ttgacgtcgc     540

cgcagctaaa gctgaaaaac cggcgctgtc cctggaaaaa gaaagctcta ccacgaacac     600

cacggaacag gttattctga gcaaaatcga agaatctctg aaaaatgccc aaaaaccggt     660

cgtgattgca ggccatgaag tgatcagttt tggtctggaa aaaaccgtca cgcagttcgt     720

gtccgaaacc aaactgccga ttaccacgct gaactttggt aaaagcgccg tggatgaaag     780

cctgccgtct ttcctgggca tttataacgg taaactgagt gaaatctccc tgaaaaactt     840

cgtcgaatct gctgatttca tcctgatgct gggcgtgaaa ctgaccgaca gttccacggg     900

tgcctttacc catcacctgg atgaaaacaa aatgattagc ctgaatatcg acgaaggcat     960

catcttcaac aaagttgtcg aagatttcga cttccgtgcg gtggtttcat cgctgtctga    1020

actgaaaggc attgaatatg aaggccagta catcgataaa caatacgaag aatttatccc    1080

gagcagcgca ccgctgagtc aggaccgtct gtggcaagca gttgaatcac tgacgcagtc    1140

gaacgaaacc attgtcgctg aacaaggcac cagctttttc ggtgcgtcca ccatctttct    1200

gaaaagtaat tcccgtttca ttggtcagcc gctgtggggc agcatcggtt atacctttcc    1260

ggcggccctg ggctcacaaa ttgccgataa agaatcgcgc catctgctgt tcatcggcga    1320

cggcagcctg caactgaccg ttcaagaact gggtctgtcg attcgtgaaa aactgaaccc    1380

gatctgcttt attatcaaca atgatggcta cacggtggaa cgcgaaattc acggtccgac    1440

ccagagttat aacgacatcc cgatgtggaa ttactccaaa ctgccggaaa cgtttggcgc    1500

aaccgaagat cgtgtcgtga gcaaaattgt gcgcaccgaa aacgaatttg tgtctgttat    1560

gaaagaagca caggctgatg ttaatcgcat gtattggatc gaactggtcc tggaaaaaga    1620

agatgctccg aaactgctga aaaaaatggg taaactgttc gctgaacaaa ataaataata    1680

ctagtagcgg ccgctgcag                                                 1699

<210>  125
<211>  1450
<212>  DNA
<213>  Salmonella enterica

<400>  125
gaattcgcgg ccgcttctag aaggagatat acatatgaac acctcggaac tggaaaccct      60

gattcgcacc atcctgtcgg aacaactgac caccccggct caaaccccgg tccaaccgca     120

gggcaaaggt atctttcaga gcgtttctga agcaattgat gcggcccatc aggcgtttct     180

gcgttatcag caatgcccgc tgaaaacgcg tagcgctatt atctctgcga tgcgtcagga     240

actgaccccg ctgctggctc cgctggcgga agaaagtgcg aacgaaaccg gcatgggtaa     300

caaagaagat aaattcctga agaacaaggc agctctggat aatacgccgg gtgtcgaaga     360

cctgaccacg accgcactga ccggtgatgg tggtatggtg ctgtttgaat atagcccgtt     420

cggtgtgatt ggcagtgttg caccgtccac caacccgacg gaaaccatta tcaacaatag     480

tatctccatg ctggcggcgg gcaacagcat ttacttttcg ccgcatccgg gcgcgaaaaa     540

ggtttcactg aaactgattt cgctgatcga agaaattgcc tttcgttgct gtggtatccg     600

caacctggtg gttacggtgg ccgaaccgac gtttgaagca acccagcaaa tgatggctca     660

cccgcgtatc gcagtcctgg caattaccgg cggtccgggc attgtggcga tgggtatgaa     720

aagcggcaaa aaggttatcg gtgcaggtgc aggtaatccg ccgtgcattg ttgatgaaac     780

cgccgacctg gtcaaagcgg cggaagatat tatcaacggt gcctcttttg actataatct     840

gccgtgtatc gcagaaaaga gcctgattgt cgtggaatct gtcgcggaac gtctggtgca     900

gcaaatgcag acgttcggcg cgctgctgct gtccccggcg gataccgaca aactgcgtgc     960

agtttgcctg ccggagggtc aggccaacaa aaagctggtc ggcaaatcac cgtcggcaat    1020

gctggaagcg gcgggtatcg ctgtgccggc aaaggctccg cgtctgctga ttgccctggt    1080

gaatgcagat gacccgtggg ttacctctga acaactgatg ccgatgctgc cggttgtcaa    1140

agtgagcgat tttgactctg cgctggccct ggcactgaag gttgaagaag gcctgcatca    1200

caccgcgatt atgcacagtc agaacgtttc ccgtctgaat ctggcagctc gcacgctgca    1260

aacctcaatc ttcgtcaaaa acggtccgtc gtacgcaggt attggcgtgg gcggtgaagg    1320

ctttacgacc ttcaccatcg caacgccgac cggtgaaggc acgaccagtg ctcgtacgtt    1380

tgcgcgctcc cgtcgctgtg tgctgaccaa tggtttcagc attcgctaat actagtagcg    1440

gccgctgcag                                                            1450

<210>  126
<211>  1825
<212>  DNA
<213>  Cupriavidus necator

<400> 126
gaattcgcgg ccgcttctag aaggagatat acatatggca accggcaagg gcgcagcagc 60
atccacgcag gaaggcaaat cccaaccgtt caaagttacc ccgggtccgt tcgatccggc 120
tacctggctg gaatggagtc gccagtggca gggtacggag ggtaacggtc atgcggcggc 180
gagcggtatt ccgggcctgg atgcgctggc cggtgtgaaa attgcaccgg ctcaactggg 240
cgatattcag caacgttaca tgaaggactt cagcgcgctg tggcaagcga tggccgaagg 300
caaggcagaa gctaccggtc cgctgcacga tcgtcgcttc gcaggtgacg catggcgtac 360
gaacctgccg tatcgttttg ctgcggcctt ctacctgctg aatgcgcgcg ccctgaccga 420
actggcagat gctgtcgaag cggacgccaa aacgcgtcag cgcattcgtt ttgcgatcag 480
tcaatgggtg gatgccatgt ccccggcaaa cttcctggct accaatccgg aagcacagcg 540
tctgctgatt gaaagtggcg gtgaatccct gcgcgcgggt gttcgtaaca tgatggaaga 600
cctgacccgc ggcaagatca gccagacgga cgaatctgcc tttgaagtgg gtcgtaacgt 660
ggccgttacc gaaggcgcag tggtttttga aaacgaatac ttccaactgc tgcaatacaa 720
accgctgacg gataaggttc atgcgcgtcc gctgctgatg gtcccgccgt gcatcaacaa 780
gtactacatt ctggatctgc aaccggaaag ctctctggtt cgccatgtcg tggaacaagg 840
tcacaccgtg tttctggtta gttggcgtaa tccggatgca tcaatggctg gctcgacgtg 900
ggatgactat attgaacatg cagctattcg cgcgatcgaa gtggcccgtg atatttctgg 960
tcaggacaaa atcaatgtcc tgggcttctg cgtgggcggc accatcgtta gcacggcact 1020
ggctgtcctg gcggcccgcg gtgaacatcc ggcagcttct gtgaccctgc tgaccacgct 1080
gctggatttt gccgacacgg gtattctgga tgtcttcgtg gacgaaggcc acgttcaact 1140
gcgtgaagca accctgggcg gtggcgcagg tgccccgtgt gccctgctgc gcggcctgga 1200
actggcaaac acctttagct tcctgcgtcc gaacgatctg gtgtggaatt atgttgtgga 1260
taactacctg aaaggcaata ccccggttcc gtttgatctg ctgttctgga acggtgacgc 1320
aacgaatctg ccgggtccgt ggtattgctg gtatctgcgc catacctatc tgcaaaatga 1380
actgaaagtc ccgggcaagc tgacggtgtg tggcgttccg gtcgatctgg cgtcaattga 1440
cgttccgacc tatatctacg gttcgcgtga agatcacatc gtcccgtgga ccgcggccta 1500
cgcgtcaacg gccctgctgg caaacaagct gcgctttgtg ctgggtgctt cgggccatat 1560
tgcgggcgtt atcaacccgc cggcgaaaaa taagcgtagc cactggacca atgatgccct 1620
gccggaatct ccgcagcaat ggctggcagg tgctattgaa catcacggct cctggtggcc 1680
ggattggacc gcttggctgg cgggtcaggc aggtgccaaa cgcgcagctc cggcgaacta 1740
tggtaatgca cgctaccgtg ctatcgaacc ggcaccgggc cgttatgtga aagcaaaggc 1800
ttaatactag tagcggccgc tgcag 1825

<210>  127
<211>  835
<212>  DNA
<213>  Metallosphaera sedula

<400>  127
gaattcgcgg ccgcttctag aaggagatat acatatggaa tttgaaacca ttgaaacgaa      60

aaaggaaggc aacctgttct ggatcaccct gaatcgcccg gacaagctga acgcactgaa     120

tgcaaaactg ctggaagaac tggatcgtgc cgtgagtcag gccgaatccg acccggaaat     180

ccgcgttatt atcattaccg gtaaaggcaa ggctttttgc gcaggtgctg atattaccca     240

gttcaaccaa ctgacgccgg cggaagcctg gaaattttcc aaaaagggtc gtgaaatcat     300

ggacaaaatt gaagcgctga gcaagccgac catcgcgatg attaacggct atgccctggg     360

cggtggcctg gaactggcac tggcttgtga tattcgtatt gcggcggaag aagcgcaact     420

gggtctgccg gaaatcaatc tgggtattta tccgggctac ggtggcaccc aacgtctgac     480

gcgcgtgatc ggtaaaggcc gtgccctgga aatgatgatg accggtgatc gcattccggg     540

caaagacgca gaaaagtacg gcctggttaa ccgtgtggtt ccgctggcta atctggaaca     600

agaaacgcgc aaactggcag aaaagattgc taaaaagagc ccgatctctc tggcgctgat     660

taaagaagtc gtgaatcgtg gtctggattc accgctgctg tcgggcctgg ccctggaaag     720

cgtcggttgg ggcgttgtct tctctaccga agacaaaaag gaaggtgtta gtgcctttct     780

ggaaaaacgc gaaccgacgt tcaaaggcaa gtaatactag tagcggccgc tgcag          835

## Description

### FIELD OF THE INVENTION

Micro-organism that is able to convert homoserine to 3-hydroxypropionaldehyde, wherein the micro-organism expresses a protein capable of converting homoserine to 2-keto-4-hydroxybutyrate wherein the protein is selected from the group consisting of: aminotransferases, an amino acid oxidases, and amino acid dehydrogenases.

### BACKGROUND OF THE INVENTION

One organic chemical used to make super absorbent polymers (used in diapers), plastics, coatings, paints, adhesives, and binders (used in leather, paper and textile products) is acrylic acid. Acrylic acid (IUPAC: prop-2-enoic acid) is the simplest unsaturated carboxylic acid.

Traditionally, acrylic acid is made from propene. Propene itself is a byproduct of oil refining from petroleum (i.e., crude oil) and of natural gas production. Disadvantages associated with traditional acrylic acid production are that petroleum is a nonrenewable starting material and that the oil refining process pollutes the environment. Synthesis methods for acrylic acid utilizing other starting materials have not been adopted for widespread use due to expense or environmental concerns. These starting materials included, for example, acetylene, ethenone and ethylene cyanohydrins.

To avoid petroleum-based production, researchers have proposed other methods for producing acrylic acid involving the fermentation of sugars by engineered microorganisms. Straathof et al., Appl Microbiol Biotechnol, 67: 727-734 (2005) discusses a conceptual fermentation process for acrylic acid production from sugars. The process proposed in the article proceeds via a β-alanine, methylcitrate, malonyl-CoA or methylmalonate-CoA intermediate in the microorganism. Another process described in Lynch, U.S. Patent Publication No. 2011/0125118 relates to using synthesis gas components as a carbon source in a microbial system to produce 3-hydroxypropionic acid, with subsequent conversion of the 3-hydroxyproprionic acid to acrylic acid.

Methods to manufacture other organic chemicals in genetically engineered microorganisms have been proposed. See, for example, U.S. Patent Publication No. 2011/0014669 published January 20, 2011 relating to microorganisms for converting L-glutamate to 1,4-butanediol.

Since at least four million metric tons of acrylic acid are produced annually, there remains a need in the art for cost-effective, environmentally-friendly methods for its production from renewable carbon sources.

### SUMMARY OF THE INVENTION

Claim 1 relates to a micro-organism that is able to convert homoserine to 3-hydroxypropionaldehyde, wherein the micro-organism expresses a protein capable of converting homoserine to 2-keto-4-hydroxybutyrate wherein the protein is selected from the group consisting of: aminotransferases, an amino acid oxidases, and amino acid dehydrogenases. The micro-organism of claim 1, wherein the protein is an aminotransferase and wherein the aminotransferase has the amino acid sequence as set out in SEQ ID NOs: 2, 4, 6, 8, 10 or 12, or a polypeptide having at least 60% amino acid homology thereto, preferably at least 80% amino acid homology thereto. The micro-organism of claim 1, wherein the protein is an aminotransferase and wherein the aminotransferase is the pig heart glutamate-oxaloacetate aminotransferase set out in SEQ ID NO: 2 or a polypeptide having at least 60% amino acid homology thereto, preferably at least 80% amino acid homology thereto. The micro-organism of claim 1, wherein the protein is selected from the group consisting of: glutamate-pyruvate aminotransferase; L-aspartate:2-oxoglutarate aminotransferase; L-alanine:2-oxoglutarate aminotransferase; L-amino acid oxidase; L-amino acid dehydrogenase. The micro-organism of any of the preceding claims, wherein the micro-organism expresses a protein capable of converting 2-keto-4-hydroxybutyrate to 3-hydroxypropionaldehyde. The micro-organism of claim 4, wherein the protein capable of converting 2-keto-4-hydroxybutyrate to 3-hydroxypropionaldehyde is 2-keto acid decarboxylase. The micro-organism of claims 4, wherein the protein capable of converting 2-keto-4-hydroxybutyrate to 3-hydroxypropionaldehyde is selected from the group consisting of 2-keto acid decarboxylase KdcA set out in SEQ ID NO: 54, or a polypeptide having at least 60% amino acid homology thereto, preferably at least 80% amino acid homology thereto. The micro-organism of any of the preceding claims, wherein the micro-organism is able to convert homoserine to 2-keto-4-hydroxybutyrate, 2-keto-4-hydroxybutyrate to 3-hydroxy-propionaldehyde, and 3-hydroxy-propionaldehyde to 3-hydroxypropionate. The micro-organism of claim 7, wherein the micro-organism expresses a protein capable of converting 3-hydroxy-propionaldehyde to 3-hydroxypropionate and wherein the protein capable of converting 3-hydroxy-propionaldehyde to 3-hydroxypropionate is a dehydrogenase, preferably an aldehyde dehydrogenase. The micro-organism of claim 8, wherein the aldehyde dehydrogenase is as set out in SEQ ID NOs: 56 or 58, or a polypeptide having at least 60% amino acid homology thereto, preferably at least 80% amino acid homology thereto. The micro-organism of any of the preceding claims, wherein the micro-organism is able to convert 3-hydroxy-propionaldehyde to 3-hydroxypropionyl-CoA and 3-hydroxypropionyl-CoA to 3-hydroxypropionate; and wherein the the micro-organism expresses a protein capable of converting 3-hydroxy-propionaldehyde to 3-hydroxypropionyl-CoA; and wherein the micro-organism expresses a protein capable of converting 3-hydroxypropionyl-CoA to 3-hydroxypropionate; and wherein the protein capable of converting 3-hydroxy-propionaldehyde to 3-hydroxypropionyl-CoA is a propionaldehyde dehydrogenase (pduP); and wherein the protein capable of converting 3-hydroxypropionyl-CoA to 3-hydroxypropionate is a 3-hydroxypropionyl-CoA transferase or thioesterase. The micro-organism of claim 10, wherein the protein capable of converting 3-hydroxypropionyl-CoA to 3-hydroxypropionate is as set out in SEQ ID NOs: 90, 92, or 94, or a polypeptide having at least 60% amino acid homology thereto, preferably at least 80% amino acid homology thereto. The micro-organism of any of claims 1 to 12, wherein the micro-organism expresses:
- the transferase of SEQ ID NO: 8, or a polypeptide having at least 60% amino acid homology thereto, preferably at least 80% amino acid homology thereto; and
- the decarboxylase of SEQ ID NO: 54, or a polypeptide having at least 60% amino acid homology thereto, preferably at least 80% amino acid homology thereto; and
- the dehydrogenase of SEQ ID NO: 60, or a polypeptide having at least 60% amino acid homology thereto, preferably at least 80% amino acid homology thereto.

A method for producing 3-hydroxypropionate or 3-hydroxypropionic acid, comprising culturing a micro-organism of any of claims 1 to 12 in media comprising L-homoserine.

A process for producing acrylate comprising:
(a) culturing a micro-organism of any of claims 1 to 12 in media comprising L-homoserine;
(b) isolating 3-hydroxypropionate or 3-hydroxypropionic acid from the micro-organism;
(c) converting 3-hydroxypropionate or 3-hydroxypropionic acid into acrylate.

Use of a micro-organism according to any of claims 1 to 12 for producing 3-hydroxypropionate or 3-hydroxypropionic acid.

### BRIEF DESCRIPTION OF THE DRAWINGS

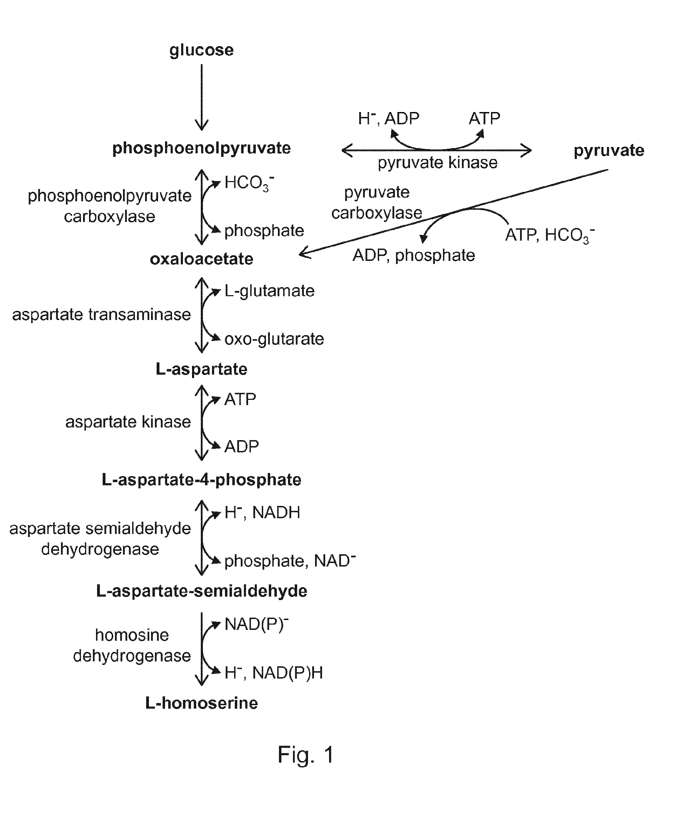

Figure 1 shows steps in the conversion of glucose to homoserine.

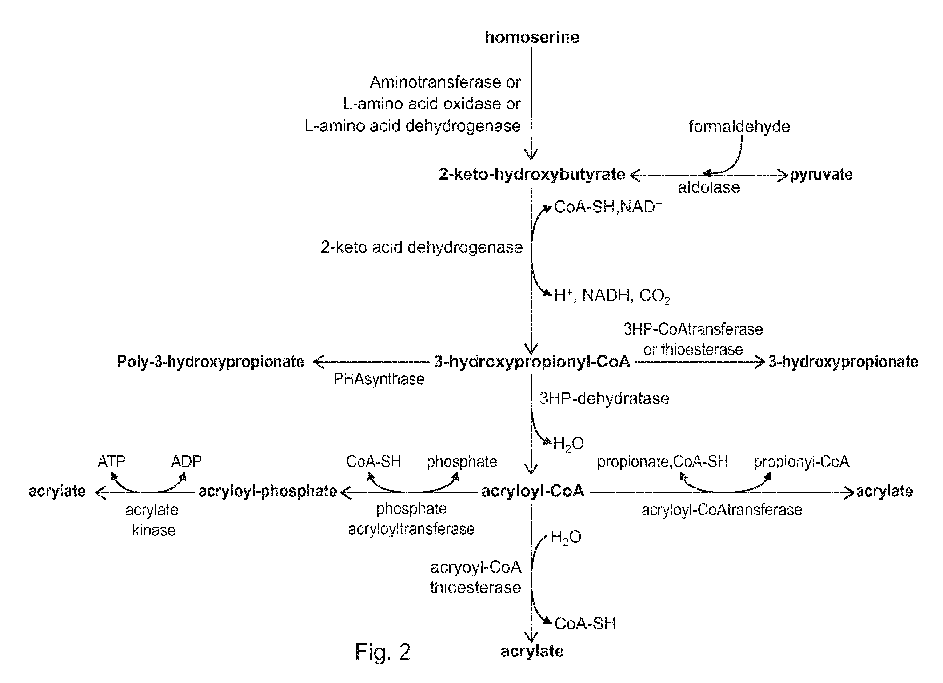

Figure 2 shows steps in methods of the invention for producing acrylate, 3-hydroxypropionyl-CoA, 3-hydroxypropionate and poly-3-hydroxypropionate from homoserine.

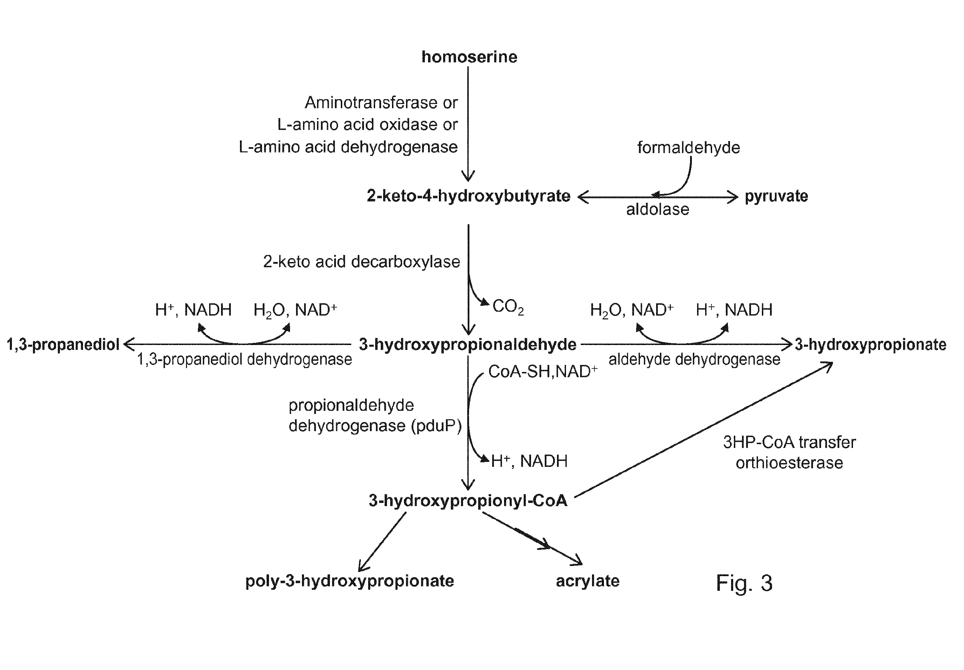

Figure 3 shows steps in methods of the invention for producing acrylate, 3-hydroxypropionate, 1,3-propanediol and 3-hydroxypropionyl-CoA from homoserine.
Figure 4 shows single ion monitoring (SIM) LC-MS chromatograms of 2-keto-4-hydroxybutyrate and glutamate, after incubation of 1-homoserine and α-ketoglutarate with (reaction) or without (control) Pf_AT aminotransferase.
Figure 5 show initial rates of deamination as a function of 1-homoserine concentration by Pf_AT aminotransferase.
Figure 6 shows the production of 3-hydroxypropionyl-CoA from 1-homoserine catalyzed by d-amino acid oxidase and 2-ketoglutarate dehydrogenase or d-amino acid oxidase, KdcA decarboxylase, and PduP dehydrogenase.
Figure 7 shows HPLC chromatograms of samples of acryloyl-CoA after incubation with (top) or without (bottom) a dehydratase, evidencing the formation of 3-hydroxypropionyl-CoA only when the enzyme was present.
Figure 8 shows the production 3-hydroxypropionyl-CoA from acryloyl-CoA catalyzed by a dehydratase.
Figure 9 shows the consumption of 3-hydroxypropionyl-CoA after incubation with PHA synthase suggesting the formation of the poly(3-hydroxypropionate).

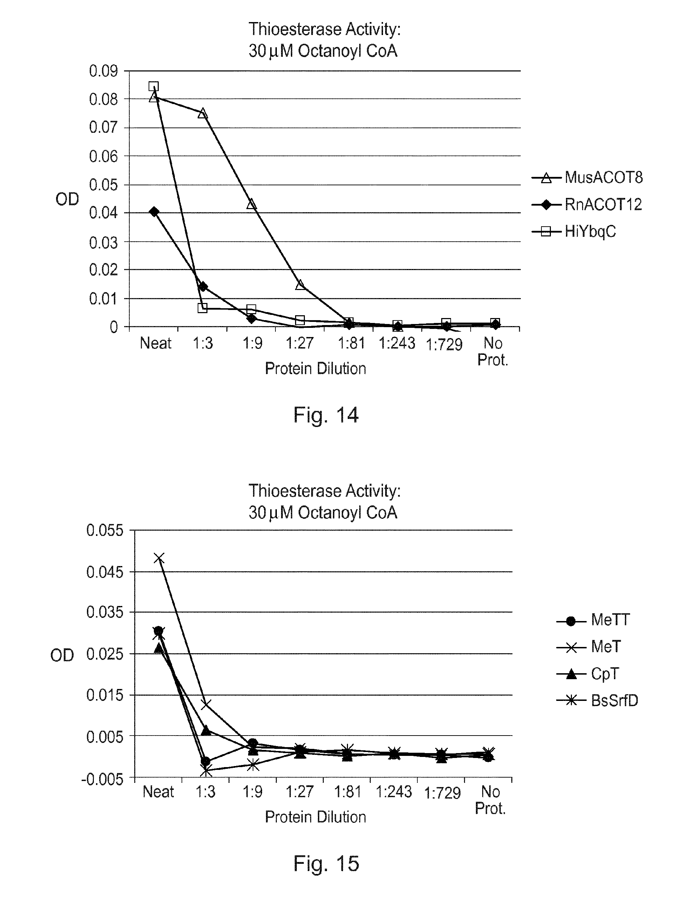

Figure 10-15 show thioesterase activity against an acryloyl-CoA substrate. Activity is monitored by optical density (OD) at 412 nm.

### DETAILED DESCRIPTION OF THE INVENTION

"Homology" as used herein, is for example, measured by sequence alignment and then using sequence comparison algorithms or by visual inspection. Such an algorithm includes CLUSTAL http://www.ebi.ac.uk/Tools/msa/clustalo/, for example, wherein the homology is a pairwise score calculated as the number of identities between the two sequences compared, divided by the length of the sequence, and represented as a percentage. For example, "having at least 60% amino acid homology to SED ID NO: X" means that the query amino acid sequence must have at least 60% amino acid homology to SED ID NO: X. The length of the sequence for calculating the homology is that of SED ID NO: X, thus where the query sequence is shorter than SED ID NO: X, then missing residues in the query amino acid sequence would reduce the percentage calculation. Similarly, where the query amino acid sequence is longer than the SED ID NO: X, a 100% amino acid homology is still possible because e.g. where the first half of the polypeptide sequence aligns with 100% amino acid homology with SED ID NO: X.

### Producing acrylate

At least one embodiment relates to a microorganism that converts homoserine to acrylate, wherein the microorganism expresses recombinant genes encoding a deaminase or transaminase; a dehydrogenase or decarboxylase; a dehydratase; and a thioesterase, a phosphate transferase/kinase combination, or an acyl-CoA transferase. The deaminase or transaminase catalyzes a reaction to convert homoserine to 2-keto-4-hydroxybutyrate. In at least one embodiment, the deaminase or transaminase is an aminotransferase, an L-amino acid oxidase or an L-amino acid dehydrogenase. Aminotransferases include, but are not limited to, a glutamate-oxaloacetate aminotransferase, a glutamate-pyruvate aminotransferase, an L-aspartate:2-oxoglutarate aminotransferase, and an L-alanine:2-oxoglutarate aminotransferase. Amino acid sequences of some aminotransferases known in the art are set out in SEQ ID NOs: 2, 4, 6, 8, 10 and 12. Exemplary DNA sequences encoding those aminotransferases are respectively set out in SEQ ID NOs: 1, 3, 5, 7, 9 and 11. Amino acid sequences of some L-amino acid oxidases known in the art are set out in SEQ ID NOs: 14 and 16. Exemplary DNA sequences encoding those L-amino acid oxidases are respectively set out in SEQ ID NOs: 13 and 15. Amino acid sequences of some L-amino acid dehydrogenases known in the art are set out in SEQ ID NOs: 18 and 20. Exemplary DNA sequences encoding those L-amino acid dehydrogenases are respectively set out in SEQ ID NOs: 17 and 19.

Dehydrogenase catalyzes a reaction to convert 2-keto-4-hydroxybutyrate to 3-hydroxypropionyl-CoA. In at least one embodiment, the dehydrogenase is a 2-keto acid dehydrogenase (or an alpha keto acid dehydrogenase). Dehydrogenases include, but are not limited to, a pyruvate dehydrogenase, a 2-keto-glutarate dehydrogenase or a branched chain keto acid dehydrogenase. A pyruvate dehydrogenase known in the art is the pyruvate dehydrogenase PDH, the amino acid sequences of the subunits of which are set out in SEQ ID NOs: 30, 32 and 34. Exemplary DNA sequences encoding those subunits are respectively set out in SEQ ID NOs: 29, 31 and 33. A 2-keto-glutarate dehydrogenase known in the art similarly comprises three subunits, the amino acid sequences of which are set out in SEQ ID NOs: 36, 38 and 40. Exemplary DNA sequences encoding those subunits are respectively set out in SEQ ID NOs: 35, 37 and 39. A branched chain keto acid dehydrogenase known in the art is the branched chain keto acid dehydrogenase BKD, the amino acid sequences of the subunits of which are set out in SEQ ID NOs: 22, 24, 26 and 28. Exemplary DNA sequences encoding those subunits are respectively set out in SEQ ID NOs: 21, 23, 25 and 27.

Dehydratase catalyzes a reaction to convert 3-hydroxypropionyl-CoA to acryloyl-CoA. In at least one embodiment, the dehydratase is a 3-hydroxypropionyl-CoA-dehydratase. The amino acid sequence of a 3-hydroxypropionyl-CoA-dehydratase known in the art is set out in SEQ ID NO: 48. An exemplary DNA sequence encoding the 3-hydroxypropionyl-CoA-dehydratase is set out in SEQ ID NO: 47.

Thioesterase, the phosphate transferase/kinase combination or the acyl-CoA transferase catalyzes a reaction to convert acryloyl-CoA to acrylate. In some embodiments, the thioesterase is acryloyl-CoA thioesterase. The amino acid sequence of a phosphate acryloyltransferase known in the art is set out in SEQ ID NO: 50. An exemplary DNA sequence encoding the phosphate acryloyltransferase is SEQ ID NO: 49. The amino acid sequence of an acrylate kinase known in the art is set out in SEQ ID NO: 52. An exemplary DNA sequence encoding the acrylate kinase is set out in SEQ ID NO: 51. The amino acid sequence of an acyl-CoA transferase known in the art is set out in SEQ ID NO: 46. An exemplary DNA sequence encoding the acyl-CoA transferase is set out in SEQ ID NO: 45.

At least one embodiment relates to a method for producing acrylate in which the microorganism is cultured to produce acrylate. The method for producing acrylate converts homoserine to 2-keto-4-hydroxybutyrate, 2-keto-4-hydroxybutyrate to 3-hydroxypropionyl-CoA, 3-hydroxypropionyl-CoA to acryloyl-CoA and then acryloyl-CoA to acrylate.

At least one embodiment relates to a microorganism that converts homoserine to acrylate, wherein the microorganism expresses recombinant genes encoding: a deaminase or transaminase, a decarboxylase, a dehydrogenase, a dehydratase and a thioesterase.

Deaminase or transaminase catalyzes a reaction to convert homoserine to 2-keto-4-hydroxybutyrate. In at least one embodiment, the deaminase or transaminase is an aminotransferase, an L-amino acid oxidase or an L-amino acid dehydrogenase. Aminotransferases include, but are not limited to, a glutamate-oxaloacetate aminotransferase, a glutamate-pyruvate aminotransferase, an L-aspartate:2-oxoglutarate aminotransferase, an L-alanine:2-oxoglutarate aminotransferase. Amino acid sequences of some aminotransferases known in the art are set out in SEQ ID NOs: 2, 4, 6, 8, 10 and 12. Exemplary DNA sequences encoding those aminotransferases are respectively set out in SEQ ID NOs: 1, 3, 5, 7, 9 and 11. Amino acid sequences of some L-amino acid oxidases known in the art are set out in SEQ ID NOs: 14 and 16. Exemplary DNA sequences encoding those L-amino acid oxidases are respectively set out in SEQ ID NOs: 13 and 15. Amino acid sequences of some L-amino acid dehydrogenases known in the art are set out in SEQ ID NOs: 18 and 20. Exemplary DNA sequences encoding those L-amino acid dehydrogenases are set out in SEQ ID NOs: 17 and 19.

Decarboxylase catalyzes a reaction to convert 2-keto-4-hydroxybutyrate to 3-hydroxy-propionaldehyde. In some embodiments, the decarboxylase is a 2-keto acid decarboxylase. The 2-keto acid decarboxylases include, but are not limited to, the 2-keto acid decarboxylase KdcA set out in SEQ ID NO: 54 and its derivatives. An exemplary DNA sequence encoding KdcA is set out in SEQ ID NO: 53.

Dehydrogenase catalyzes a reaction to convert 3-hydroxy-propionaldehyde to 3-hydroxypropionyl-CoA. In some embodiments, the dehydrogenase is a propionaldehyde dehydrogenase. Propionaldehyde dehydrogenases include, but are not limited to, a PduP. Amino acid sequences of some PduP propionaldehyde dehydrogenases known in the art are set out in SEQ ID NOs: 60 and 62. Exemplary DNA sequences encoding the PduP propionaldehyde dehydrogenases are respectively set out in SEQ ID NOs: 59 and 61.

Dehydratase catalyzes a reaction to convert 3-hydroxypropionyl-CoA to acryloyl-CoA. In some embodiments, the dehydratase is a 3-hydroxypropionyl-CoA dehydratase. The amino acid sequence of 3-hydroxypropionyl-CoA dehydratase known in the art is set out in SEQ ID NO: 48. An exemplary DNA sequence encoding the 3-hydroxypropionyl-CoA dehydratase is set out in SEQ ID NO: 47.

Thioesterase catalyzes a reaction to convert acryloyl-CoA to acrylate. In some embodiments, the thioesterase is an acryloyl-CoA thioesterase. Acryloyl-CoA thioesterases include, but are not limited to *E. coli* TesB set out in SEQ ID NO: 90, the *Clostridium propionicum*-derived thioesterase including an E324D substitution set out in SEQ ID NO: 92 and the *Megasphaera elsdenii*-derived thioesterase including an E325D substitution set out in SEQ ID NO: 94. Exemplary DNA sequences encoding these acryloyl-CoA thioesterases are respectively set out in SEQ ID NOs: 89, 91 (codon-optimized for *E. coli*) and 93 (codon-optimized for *E. coli*).

At least one embodiment relates to a method for producing acrylate in which the second type of microorganism is cultured to produce acrylate. The method for producing acrylate converts homoserine to 2-keto-4-hydroxybutyrate, 2-keto-4-hydroxybutyrate to 3-hydroxy-propionaldehyde, 3-hydroxy-propionaldehyde to 3-hydroxypropionyl-CoA, 3-hydroxy-propionyl-CoA to acryloyl-CoA and then acryloyl-CoA to acrylate.

### Producing 3-hydroxypropionate

At least one embodiment relates a microorganism that converts homoserine to 3-hydroxypropionate, wherein the microorganism expresses recombinant genes encoding: a deaminase or transaminase, a dehydrogenase or decarboxylase, and acyl-CoA transferase or athioesterase.

The deaminase or transaminase catalyzes a reaction to convert homoserine to 2-keto-4-hydroxybutyrate. In some embodiments, the deaminase or transaminase is an aminotransferase, an L-amino acid oxidase or an L-amino acid dehydrogenase. Aminotransferases include, but are not limited to, a glutamate-oxaloacetate aminotransferase, a glutamate-pyruvate aminotransferase, an L-aspartate:2-oxoglutarate aminotransferase, an L-alanine:2-oxoglutarate aminotransferase. Amino acid sequences of some aminotransferases known in the art are set out in SEQ ID NOs: 2, 4, 6, 8, 10 and 12. Exemplary DNA sequences encoding those aminotransferases are respectively set out in SEQ ID NOs: 1, 3, 5, 7, 9 and 11. Amino acid sequences of some L-amino acid oxidases known in the art are set out in SEQ ID NOs: 14 and 16. Exemplary DNA sequences encoding those L-amino acid oxidases are respectively set out in SEQ ID NOs: 13 and 15. Amino acid sequences of some L-amino acid dehydrogenases known in the art are set out in SEQ ID NOs: 18 and 20. Exemplary DNA sequences encoding those L-amino acid dehydrogenases are set out in SEQ ID NOs: 17 and 19.

The dehydrogenase or decarboxylase catalyzes a reaction to convert 2-keto-4-hydroxybutyrate to 3-hydroxypropionyl-CoA. In some embodiments, the dehydrogenase is a 2-keto acid dehydrogenase (or an alpha keto acid dehydrogenase). Dehydrogenases include, but are not limited to, a pyruvate dehydrogenase, a 2-keto-glutarate dehydrogenase or a branched chain keto acid dehydrogenase. A pyruvate dehydrogenase known in the art is the pyruvate dehydrogenase PDH, the amino acid sequences of the subunits of which are set out in SEQ ID NOs: 30, 32 and 34. Exemplary DNA sequences encoding those subunits are respectively set out in SEQ ID NOs: 29, 31 and 33. A 2-keto-glutarate dehydrogenase known in the art similarly comprises three subunits, the amino acid sequences of which are set out in SEQ ID NOs: 36, 38 and 40. Exemplary DNA sequences encoding those subunits are respectively set out in SEQ ID NOs: 35, 37 and 39. A branched chain keto acid dehydrogenase known in the art is the branched chain keto acid dehydrogenase BKD, the amino acid sequences of the subunits of which are set out in SEQ ID NOs: 22, 24, 26 and 28. Exemplary DNA sequences encoding those subunits are respectively set out in SEQ ID NOs: 21, 23, 25 and 27.

The acyl-CoA transferase or the acyl-CoA thioesterase catalyzes a reaction to convert 3-hydroxypropionyl-CoA to 3-hydroxypropionate. Contemplated thioesterases include, but are not limited to *E. coli* TesB set out in SEQ ID NO: 90, the *C. propionicum*-derived thioesterase including an E324D substitution set out in SEQ ID NO: 92 and the *M. elsdenii*-derived thioesterase including an E325D substitution set out in SEQ ID NO: 94. Exemplary (codon-optimized for *E. coli*) DNA sequences encoding these thioesterases are respectively set out in SEQ ID NOs: 89, 91 (codon-optimized for *E. coli*) and 93 (codon-optimized for *E. coli*).

At least one embodiment relates to a method for producing 3-hydroxypropionate in which the third type of microorganism is cultured to produce 3-hydroxypropionate. The method converts homoserine to 2-keto-4-hydroxybutyrate, 2-keto-4-hydroxybutyrate to 3-hydroxy-propionaldehyde,3-hydroxy-propionaldehyde to 3-hydroxypropionyl-CoA and then 3-hydroxypropionyl-CoA to 3-hydroxypropionate.

At least one embodiment relates to a microorganism that converts homoserine to 3-hydroxypropionate, wherein the microorganism expresses recombinant genes encoding: a deaminase or transaminase, a decarboxylase and a dehydrogenase.

The deaminase or transaminase catalyzes a reaction to convert homoserine to 2-keto-4-hydroxybutyrate. In some embodiments, the deaminase or transaminase is an aminotransferase, an L-amino acid oxidase or an L-amino acid dehydrogenase. Aminotransferases include, but are not limited to, a glutamate-oxaloacetate aminotransferase, a glutamate-pyruvate aminotransferase, an L-aspartate:2-oxoglutarate aminotransferase, an L-alanine:2-oxoglutarate aminotransferase. Amino acid sequences of some aminotransferases known in the art are set out in SEQ ID NOs: 2, 4, 6, 8, 10 and 12. Exemplary DNA sequences encoding those aminotransferases are respectively set out in SEQ ID NOs: 1, 3, 5, 7, 9 and 11. Amino acid sequences of some L-amino acid oxidases known in the art are set out in SEQ ID NOs: 14 and 16. Exemplary DNA sequences encoding those L-amino acid oxidases are respectively set out in SEQ ID NOs: 13 and 15. Amino acid sequences of some L-amino acid dehydrogenases known in the art are set out in SEQ ID NOs: 18 and 20. Exemplary DNA sequences encoding those L-amino acid dehydrogenases are set out in SEQ ID NOs: 17 and 19.

The decarboxylase catalyzes a reaction to convert 2-keto-4-hydroxybutyrate to 3-hydroxy-propionaldehyde. In some embodiments the decarboxylase is a 2-keto acid decarboxylase. The 2-keto acid decarboxylases include, but are not limited to, the 2-keto acid decarboxylase KdcA set out in SEQ ID NO: 54 and its derivatives. An exemplary DNA sequence encoding KdcA is set out in SEQ ID NO: 53.

The dehydrogenase catalyzes a reaction to convert 3-hydroxy-propionaldehyde to 3-hydroxypropionate. In some embodiments, the dehydrogenase is an aldehyde dehydrogenase. Amino acid sequences of aldehyde dehydrogenases known in the art are set out in SEQ ID NOs: 56 and 58. Exemplary DNA sequences encoding the aldehyde dehydrogenases are respectively set out in SEQ ID NOs: 55 and 57.

At least one embodiment relates to a method for producing 3-hydroxypropionate in which the fourth type of microorganism is cultured to produce 3-hydroxypropionate . The fourth type of method converts homoserine to 2-keto-4-hydroxybutyrate, 2-keto-4-hydroxybutyrate to 3-hydroxy-propionaldehyde, 3-hydroxy-propionaldehyde to 3-hydroxypropionate.

At least one embodiment relates to a microorganism that converts homoserine to 3-hydroxypropionate, wherein the microorganism expresses recombinant genes encoding: a deaminase or transaminase, a decarboxylase, a dehydrogenase, and a acyl-CoA transferase or a thioesterase.

The deaminase or transaminase catalyzes a reaction to convert homoserine to 2-keto-4-hydroxybutyrate. In some embodiments, the deaminase or transaminase is an aminotransferase, an L-amino acid oxidase or an L-amino acid dehydrogenase. Aminotransferases include, but are not limited to, a glutamate-oxaloacetate aminotransferase, a glutamate-pyruvate aminotransferase, an L-aspartate:2-oxoglutarate aminotransferase, an L-alanine:2-oxoglutarate aminotransferase. Amino acid sequences of some aminotransferases known in the art are set out in SEQ ID NOs: 2, 4, 6, 8, 10 and 12. Exemplary DNA sequences encoding those aminotransferases are respectively set out in SEQ ID NOs: 1, 3, 5, 7, 9 and 11. Amino acid sequences of some L-amino acid oxidases known in the art are set out in SEQ ID NOs: 14 and 16. Exemplary DNA sequences encoding those L-amino acid oxidases are respectively set out in SEQ ID NOs: 13 and 15. Amino acid sequences of some L-amino acid dehydrogenases known in the art are set out in SEQ ID NOs: 18 and 20. Exemplary DNA sequences encoding those L-amino acid dehydrogenases are set out in SEQ ID NOs: 17 and 19.

The decarboxylase catalyzes a reaction to convert 2-keto-4-hydroxybutyrate to 3-hydroxy-propionaldehyde. In some embodiments, the decarboxylase is a 2-keto acid decarboxylase. The 2-keto acid decarboxylases include, but are not limited to, the 2-keto acid decarboxylase KdcA set out in SEQ ID NO: 54 and its derivatives. An exemplary DNA sequence encoding KdcA is set out in SEQ ID NO: 53.

The dehydrogenase catalyzes a reaction to convert 3-hydroxy-propionaldehyde to 3-hydroxypropionyl-CoA. In some embodiments, the dehydrogenase is a propionaldehyde dehydrogenase. Propionaldehyde dehydrogenases include, but are not limited to, a PduP. Amino acid sequences of some PduP propionaldehyde dehydrogenases known in the art are set out in SEQ ID NOs: 60 and 62. Exemplary DNA sequences encoding the PduP propionaldehyde dehydrogenases are respectively set out in SEQ ID NOs: 59 and 61.

The 3-hydroxypropionyl-CoA transferase or thioesterase catalyzes a reaction to convert 3-hydroxypropionyl-CoA to 3-hydroxypropionate. Contemplated thioesterases include, but are not limited to *E. coli* TesB set out in SEQ ID NO: 90, the *C. propionicum*-derived thioesterase including an E324D substitution set out in SEQ ID NO: 92 and the *M. elsdenii*-derived thioesterase including an E325D substitution set out in SEQ ID NO: 94. Exemplary DNA sequences encoding these acryloyl-CoA thioesterases are respectively set out in SEQ ID NOs: 89, 91 (codon-optimized for *E. coli*) and 93 (codon-optimized for *E. coli*).

At least one embodiment relates to a method for producing 3-hydroxypropionate in which the fifth type of microorganism is cultured to produce 3-hydroxypropionate. The fifth type of method for producing acrylate converts homoserine to 2-keto-4-hydroxybutyrate, 2-keto-4-hydroxybutyrate to 3-hydroxy-propionaldehyde, 3-hydroxy-propionaldehyde to 3-hydroxypropionyl-CoA, and 3-hydroxy-propionyl-CoA to 3-hydroxypropionate.

### Producing poly-3 -hydroxypropionate

At least one embodiment relates a microorganism that converts homoserine to poly-3-hydroxypropionate, wherein the microorganism expresses recombinant genes encoding: a deaminase or transaminase, a dehydrogenase or decarboxylase, and a PHA synthase.

The deaminase or transaminase catalyzes a reaction to convert homoserine to 2-keto-4-hydroxybutyrate. In some embodiments, the deaminase or transaminase is an aminotransferase, an L-amino acid oxidase or an L-amino acid dehydrogenase. Aminotransferases include, but are not limited to, a glutamate-oxaloacetate aminotransferase, a glutamate-pyruvate aminotransferase, an L-aspartate:2-oxoglutarate aminotransferase, an L-alanine:2-oxoglutarate aminotransferase. Amino acid sequences of some aminotransferases known in the art are set out in SEQ ID NOs: 2, 4, 6, 8, 10 and 12. Exemplary DNA sequences encoding those aminotransferases are respectively set out in SEQ ID NOs: 1, 3, 5, 7, 9 and 11. Amino acid sequences of some L-amino acid oxidases known in the art are set out in SEQ ID NOs: 14 and 16. Exemplary DNA sequences encoding those L-amino acid oxidases are respectively set out in SEQ ID NOs: 13 and 15. Amino acid sequences of some L-amino acid dehydrogenases known in the art are set out in SEQ ID NOs: 18 and 20. Exemplary DNA sequences encoding those L-amino acid dehydrogenases are set out in SEQ ID NOs: 17 and 19.

The dehydrogenase or decarboxylase catalyzes a reaction to convert 2-keto-4-hydroxybutyrate to 3-hydroxypropionyl-CoA. In some embodiments, the dehydrogenase is a 2-keto acid dehydrogenase (or an alpha keto acid dehydrogenase). Dehydrogenases include, but are not limited to, a pyruvate dehydrogenase, a 2-keto-glutarate dehydrogenase or a branched chain keto acid dehydrogenase. A pyruvate dehydrogenase known in the art is the pyruvate dehydrogenase PDH, the amino acid sequences of the subunits of which are set out in SEQ ID NOs: 30, 32 and 34. Exemplary DNA sequences encoding those subunits are respectively set out in SEQ ID NOs: 29, 31 and 33. A 2-keto-glutarate dehydrogenase known in the art similarly comprises three subunits, the amino acid sequences of which are set out in SEQ ID NOs: 36, 38 and 40. Exemplary DNA sequences encoding those subunits are respectively set out in SEQ ID NOs: 35, 37 and 39. A branched chain keto acid dehydrogenase known in the art is the branched chain keto acid dehydrogenase BKD, the amino acid sequences of the subunits of which are set out in SEQ ID NOs: 22, 24, 26 and 28. Exemplary DNA sequences encoding those subunits are respectively set out in SEQ ID NOs: 21, 23, 25 and 27.

The PHA synthase catalyzes a reaction to convert 3-hydroxypropionyl-CoA to poly-3-hydroxyalkanoate containing 3-hydroxypropionate monomers. The polymer may have a molecule of Coenzyme A (CoA) at the carboxy end. The amino acid sequence of a PHA synthase known in the art is set out in SEQ ID NO: 42. An exemplary DNA sequence encoding the PHA synthase is set out in SEQ ID NO: 41.

At least one embodiment relates to a method for producing poly-3-hydroxypropionate in which the sixth type of microorganism is cultured to produce poly-3-hydroxypropionate. The method converts homoserine to 2-keto-4-hydroxybutyrate, 2-keto-4-hydroxybutyrate to 3-hydroxypropionyl-CoA and 3-hydroxypropionyl-CoA to poly-3-hydroxypropionate.

At least one embodiment relates to a microorganism that converts homoserine to poly-3-hydroxypropionate, wherein the microorganism expresses recombinant genes encoding: a deaminase or transaminase, a decarboxylase, a dehydrogenase and a PHA synthase.

The deaminase or transaminase catalyzes a reaction to convert homoserine to 2-keto-4-hydroxybutyrate. In some embodiments, the deaminase or transaminase is an aminotransferase, an L-amino acid oxidase or an L-amino acid dehydrogenase. Aminotransferases include, but are not limited to, a glutamate-oxaloacetate aminotransferase, a glutamate-pyruvate aminotransferase, an L-aspartate:2-oxoglutarate aminotransferase, an L-alanine:2-oxoglutarate aminotransferase. Amino acid sequences of some aminotransferases known in the art are set out in SEQ ID NOs: 2, 4, 6, 8, 10 and 12. Exemplary DNA sequences encoding those aminotransferases are respectively set out in SEQ ID NOs: 1, 3, 5, 7, 9 and 11. Amino acid sequences of some L-amino acid oxidases known in the art are set out in SEQ ID NOs: 14 and 16. Exemplary DNA sequences encoding those L-amino acid oxidases are respectively set out in SEQ ID NOs: 13 and 15. Amino acid sequences of some L-amino acid dehydrogenases known in the art are set out in SEQ ID NOs: 18 and 20. Exemplary DNA sequences encoding those L-amino acid dehydrogenases are set out in SEQ ID NOs: 17 and 19.

The decarboxylase catalyzes a reaction to convert 2-keto-4-hydroxybutyrate to 3-hydroxy-propionaldehyde. In some embodiments, the decarboxylase is a 2-keto acid decarboxylase. The 2-keto acid decarboxylases include, but are not limited to, the 2-keto acid decarboxylase KdcA set out in SEQ ID NO: 54 and its derivatives. An exemplary DNA sequence encoding KdcA is set out in SEQ ID NO: 53..

The dehydrogenase catalyzes a reaction to convert 3-hydroxy-propionaldehyde to 3-hydroxypropionyl-CoA. In some embodiments, the dehydrogenase is a propionaldehyde dehydrogenase. Propionaldehyde dehydrogenases include, but are not limited to, a PduP. Amino acid sequences encoding of PduP propionaldehyde dehydrogenases known in the art are set out in SEQ ID NOs: 60 and 62. Exemplary DNA sequences encoding the PduP propionaldehyde dehydrogenases are respectively set out in SEQ ID NOs: 59 and 61.

The PHA synthase catalyzes a reaction to convert 3-hydroxypropionyl-CoA to poly-3-hydroxyalkanoate containing 3-hydroxypropionate monomers. The polymer may have a molecule of Coenzyme A (CoA) at the carboxy end. The amino acid sequence of a PHA synthase known in the art is set out in SEQ ID NO: 42. An exemplary DNA sequence encoding the PHA synthase is set out in SEQ ID NO: 41.

At least one embodiment relates to method for producing poly-3-hydroxypropionate in which the seventh type of microorganism is cultured to produce poly-3-hydroxypropionate. The method converts homoserine to 2-keto-4-hydroxybutyrate, 2-keto-4-hydroxybutyrate to 3-hydroxy-propionaldehyde, 3-hydroxy-propionaldehyde to 3-hydroxypropionyl-CoA and then 3-hydroxy-propionyl-CoA to poly-3-hydroxypropionate.

### Producing 3-hydroxypropionyl-CoA

At least one embodiment relates to a microorganism that converts homoserine to 3-hydroxypropionyl-CoA, wherein the microorganism expresses recombinant genes encoding: a deaminase or transaminase, and a dehydrogenase or decarboxylase.

The deaminase or transaminase catalyzes a reaction to convert homoserine to 2-keto-4-hydroxybutyrate. In some embodiments, the deaminase or transaminase is an aminotransferase, an L-amino acid oxidase or an L-amino acid dehydrogenase. Aminotransferases include, but are not limited to, a glutamate-oxaloacetate aminotransferase, a glutamate-pyruvate aminotransferase, an L-aspartate:2-oxoglutarate aminotransferase, an L-alanine:2-oxoglutarate aminotransferase. Amino acid sequences of some aminotransferases known in the art are set out in SEQ ID NOs: 2, 4, 6, 8, 10 and 12. Exemplary DNA sequences encoding those aminotransferases are respectively set out in SEQ ID NOs: 1, 3, 5, 7, 9 and 11. Amino acid sequences of some L-amino acid oxidases known in the art are set out in SEQ ID NOs: 14 and 16. Exemplary DNA sequences encoding those L-amino acid oxidases are respectively set out in SEQ ID NOs: 13 and 15. Amino acid sequences of some L-amino acid dehydrogenases known in the art are set out in SEQ ID NOs: 18 and 20. Exemplary DNA sequences encoding those L-amino acid dehydrogenases are set out in SEQ ID NOs: 17 and 19.

The dehydrogenase or decarboxylase catalyzes a reaction to convert 2-keto-4-hydroxybutyrate to 3-hydroxypropionyl-CoA. In some embodiments, the dehydrogenase is a 2-keto acid dehydrogenase (or an alpha keto acid dehydrogenase). Dehydrogenases include, but are not limited to, a pyruvate dehydrogenase, a 2-keto-glutarate dehydrogenase or a branched chain keto acid dehydrogenase. A pyruvate dehydrogenase known in the art is the pyruvate dehydrogenase PDH, the amino acid sequences of the subunits of which are set out in SEQ ID NOs: 30, 32 and 34. Exemplary DNA sequences encoding those subunits are respectively set out in SEQ ID NOs: 29, 31 and 33. A 2-keto-glutarate dehydrogenase known in the art similarly comprises three subunits, the amino acid sequences of which are set out in SEQ ID NOs: 36, 38 and 40. Exemplary DNA sequences encoding those subunits are respectively set out in SEQ ID NOs: 35, 37 and 39. A branched chain keto acid dehydrogenase known in the art is the branched chain keto acid dehydrogenase BKD, the amino acid sequences of the subunits of which are set out in SEQ ID NOs: 22, 24, 26 and 28. Exemplary DNA sequences encoding those subunits are respectively set out in SEQ ID NOs: 21, 23, 25 and 27.

At least one embodiment relates to a method for producing 3-hydroxypropionyl-CoA in which the eighth type of microorganism is cultured to produce 3-hydroxypropionyl-CoA. The eighth type of method converts homoserine to 2-keto-4-hydroxybutyrate and then converts 2-keto-4-hydroxybutyrate to 3-hydroxypropionyl-CoA.

At least one embodiment relates to a microorganism that converts homoserine to 3-hydroxypropionyl-CoA, wherein the microorganism expresses recombinant genes encoding: a deaminase or transaminase, a decarboxylase, and a dehydrogenase.

The deaminase or transaminase catalyzes a reaction to convert homoserine to 2-keto-4-hydroxybutyrate. In some embodiments, the deaminase or transaminase is an aminotransferase, an L-amino acid oxidase or an L-amino acid dehydrogenase. Aminotransferases include, but are not limited to, a glutamate-oxaloacetate aminotransferase, a glutamate-pyruvate aminotransferase, an L-aspartate:2-oxoglutarate aminotransferase, an L-alanine:2-oxoglutarate aminotransferase. Amino acid sequences of some aminotransferases known in the art are set out in SEQ ID NOs: 2, 4, 6, 8, 10 and 12. Exemplary DNA sequences encoding those aminotransferases are respectively set out in SEQ ID NOs: 1, 3, 5, 7, 9 and 11. Amino acid sequences of some L-amino acid oxidases known in the art are set out in SEQ ID NOs: 14 and 16. Exemplary DNA sequences encoding those L-amino acid oxidases are respectively set out in SEQ ID NOs: 13 and 15. Amino acid sequences of some L-amino acid dehydrogenases known in the art are set out in SEQ ID NOs: 18 and 20. Exemplary DNA sequences encoding those L-amino acid dehydrogenases are set out in SEQ ID NOs: 17 and 19.

The decarboxylase catalyzes a reaction to convert 2-keto-4-hydroxybutyrate to 3-hydroxy-propionaldehyde. In some embodiments, the decarboxylase is a 2-keto acid decarboxylase. The 2-keto acid decarboxylases include, but are not limited to, the 2-keto acid decarboxylase KdcA set out in SEQ ID NO: 54 and its derivatives. An exemplary DNA sequence encoding KdcA is set out in SEQ ID NO: 53.

The dehydrogenase catalyzes a reaction to convert 3-hydroxy-propionaldehyde to 3-hydroxypropionyl-CoA. In some embodiments, the dehydrogenase is a propionaldehyde dehydrogenase. Propionaldehyde dehydrogenases include, but are not limited to, a PduP. Amino acid sequences encoding of PduP propionaldehyde dehydrogenases known in the art are set out in SEQ ID NOs: 60 and 62. Exemplary DNA sequences encoding the PduP propionaldehyde dehydrogenases are respectively set out in SEQ ID NOs: 59 and 61.

At least one embodiment relates to a method for producing 3-hydroxypropionyl-CoA in which the ninth type of microorganism is cultured to produce 3-hydroxypropionyl-CoA. The method converts homoserine to 2-keto-4-hydroxybutyrate, 2-keto-4-hydroxybutyrate to 3-hydroxy-propionaldehyde, and 3-hydroxy-propionaldehyde to 3-hydroxypropionyl-CoA.

### Producing 1,3-propanediol

At least one embodiment relates to a microorganism that converts homoserine to 1,3-propanediol, wherein the microorganism expresses recombinant genes encoding: a deaminase or transaminase, a decarboxylase and a 1,3-propanediol dehydrogenase or aldehyde reductase.

The deaminase or transaminase catalyzes a reaction to convert homoserine to 2-keto-4-hydroxybutyrate. In some embodiments, the deaminase or transaminase is an aminotransferase, an L-amino acid oxidase or an L-amino acid dehydrogenase. Aminotransferases include, but are not limited to, a glutamate-oxaloacetate aminotransferase, a glutamate-pyruvate aminotransferase, an L-aspartate:2-oxoglutarate aminotransferase, an L-alanine:2-oxoglutarate aminotransferase. Amino acid sequences of some aminotransferases known in the art are set out in SEQ ID NOs: 2, 4, 6, 8, 10 and 12. Exemplary DNA sequences encoding those aminotransferases are respectively set out in SEQ ID NOs: 1, 3, 5, 7, 9 and 11. Amino acid sequences of some L-amino acid oxidases known in the art are set out in SEQ ID NOs: 14 and 16. Exemplary DNA sequences encoding those L-amino acid oxidases are respectively set out in SEQ ID NOs: 13 and 15. Amino acid sequences of some L-amino acid dehydrogenases known in the art are set out in SEQ ID NOs: 18 and 20. Exemplary DNA sequences encoding those L-amino acid dehydrogenases are set out in SEQ ID NOs: 17 and 19.

The decarboxylase catalyzes a reaction to convert 2-keto-4-hydroxybutyrate to 3-hydroxy-propionaldehyde. In some embodiments, the decarboxylase is a 2-keto acid decarboxylase. The 2-keto acid decarboxylases include, but are not limited to, the 2-keto acid decarboxylase KdcA set out in SEQ ID NO: 54 and its derivatives. An exemplary DNA sequence encoding KdcA is set out in SEQ ID NO: 53.

The 1,3-propanediol dehydrogenase or aldehyde reductase catalyzes a reaction to convert 3-hydroxypropionaldehyde to 1,3-propanediol. Amino acid sequences of some 1,3-propanediol dehydrogenases know in the art are set out in SEQ ID NOs: 64, 66, 68, 70 and 72. Exemplary DNA sequence encoding the 1,3-propanediol dehydrogenases are respectively set out in SEQ ID NOs: 63, 65, 67, 69 and 71.

At least one embodiment relates to a method for producing 1,3-propanediol in which the tenth type of microorganism is cultured to produce 1,3-propanediol. The method converts homoserine to 2-keto-4-hydroxybutyrate, 2-keto-4-hydroxybutyrate to 3-hydroxy-propionaldehyde and then 3-hydroxy-propionaldehyde to 1,3-propanediol.

### Increasing the carbon flow to homoserine

At least one embodiment relates to microorganisms that include further genetic modifications in order to increase the carbon flow to homoserine which, in turn, increases the production of acrylate or other products of the invention. The microorganisms exhibit one or more of the following characteristics. In some embodiments, the microorganism exhibits increased carbon flow to oxaloacetate in comparison to a corresponding wild-type microorganism. The microorganism expresses a recombinant gene encoding, for example, phosphoenolpyruvate carboxylase or pyruvate carboxylase (or both). The phosphoenolpyruvate caroxylases include, but are not limited to, the phosphoenolpyruvate carboxylase set out in SEQ ID NO: 84. An exemplary DNA sequence encoding the phosphoenolpyruvate carboxylase is set out in SEQ ID NO: 83. The pyruvate carboxylases include, but are not limited to, the pyruvate carboxylases set out in SEQ ID NOs: 86 and 88. Exemplary DNA sequences encoding the pyruvate carboxylases are set out in SEQ ID NO: 85 and 87. In some embodiments, the microorganism exhibits reduced aspartate kinase feedback inhibition in comparison to a corresponding wild-type microorganism. The microorganism expresses one or more of the genes encoding the polypeptides including, but not limited to, S345F ThrA (SEQ ID NO: 76), T352I LysC (SEQ ID NO: 78) and MetL (SEQ ID NO: 74). Exemplary coding sequences encoding the polypeptides are respectively set out in SEQ ID NO: 75, SEQ ID NO: 77 and SEQ ID NO: 73. In some embodiments, the microorganism exhibits reduced *lysA* gene expression or diaminopimelate decarboxylase activity in comparison to a corresponding wild-type microorganism. In some embodiments, the microorganism exhibits reduced *dapA* expression or dihydropicolinate synthase activity in comparison to a corresponding wild type organism. An exemplary DNA sequence of a *lysA* coding sequence known in the art is set out in SEQ ID NO: 113. It encodes the amino acid sequence set out in SEQ ID NO: 114. An exemplary DNA sequence of a *dapA* coding sequence known in the art is set out in SEQ ID NO: 115. It encodes the amino acid sequence set out in SEQ ID NO: 116. In some embodiments, the microorganism exhibits reduced *metA* gene expression or homoserine succinyltransferase activity in comparison to a corresponding wild-type microorganism. An exemplary DNA sequence of a *metA* coding sequence known in the art is set out in SEQ ID NO: 79. It encodes the amino acid sequence set out in SEQ ID NO: 80. In some embodiments, the microorganism exhibits reduced *thrB* gene expression or homoserine kinase activity in comparison to a corresponding wild-type microorganism. An exemplary DNA sequence of a *thrB* coding sequence known in the art is set out in SEQ ID NO: 81. It encodes the amino acid sequence set out in SEQ ID NO: 82. In some embodiments, the microorganism does not express an *eda* gene. An exemplary DNA sequence of an *eda* coding sequence known in the art is set out in SEQ ID NO: 43. It encodes the amino acid sequence set out in SEQ ID NO: 44. At least one embodiment relates to methods of culturing the further modified microorganisms to produce products of the invention.

### Thioesterases

At least one embodiment relates to a thioesterase that hydrolyzes an intermediate of a metabolic pathway described herein to produce a desired end product. In this regard, a microorganism of the invention expresses a recombinant gene comprising a nucleic acid sequence encoding a thioesterase with activity against Coenzyme A (CoA) attached to a two-, three- or four-carbon chain, such as a three- or four-carbon chain comprising a double bond (*e.g*., a three- or four-carbon chain comprising a double bond between C2 and C3). In some embodiments, the thioesterase hydrolyzes acryloyl-CoA to form acrylic acid. Alternatively (or in addition), in some embodiments the thioesterase hydrolyzes crotonoyl-CoA to form crotonic acid. In at least one embodiment, the thioesterases are predicated with activity against substrates with short carbon chains (*e.g*., less than four carbons in the main chain) comprising double bonds. While thioesterases have been identified that hydrolyze saturated short carbon chains, it would not have been expected that the identified thioesterases would act upon an unsaturated carbon chain. Thioesterases would be expected to exhibit a high degree of substrate specificity with respect to short carbon chains to avoid hydrolysis of acetyl-CoA, which is critical to fatty synthesis. Unexpectedly, thioesterases that hydrolyze CoA intermediates attached to short, unsaturated carbon chains were identified and successfully produced (or overproduced) in host cells. Exemplary thioesterases include, but are not limited to, TesB from *E. coli* and homologs thereof from different organisms. In this regard, the host cell optionally comprises a polynucleotide comprising a nucleic acid sequence encoding an amino acid sequence with at least 80% homology (*e.g*., 85%, 90%, 95%, 99%, or 100% homology) to the amino acid sequence set forth in SEQ ID NO: 90 (TesB), and encoding a polypeptide having thioesterase activity (*i.e*., the polypeptide hydrolyzes thioesters bonds). An exemplary DNA sequence encoding the TesB amino acid sequence is set out in SEQ ID NO: 89. The amino acid sequences of other known thioesterases are set out in SEQ ID NO: 96, 98, 100, 102, 104, 106 and 108. Exemplary codon-optimized (for *E.coli*) DNA sequences encoding the thioesterases are respectively set out in SEQ ID NOs: 95, 97, 99, 101, 103, 105 and 107.

Engineered thioesterases also are appropriate for use in the invention. For example, mutation(s) within the active site of a CoA transferase confers thioesterase activity to the enzyme while substantially reducing (if not eliminating) transferase activity. Use of a thioesterase is, in various aspects, superior to use of a CoA transferase by releasing energy associated with the CoA bond. The energy release drives the acrylic acid or crotonic acid pathway to completion. An exemplary method of modifying a CoA transferase to obtain thioesterase activity comprises substituting the amino acid serving as the catalytic carboxylate with an alternate amino acid. CoA transferases suitable for modification and use in the context of the invention include, but are not limited to, acetyl-CoA transferases, propionyl-CoA transferases, and butyryl-CoA transferases. In one aspect, the thioesterase of the invention comprises the amino acid sequence of a propionyl-CoA transferase wherein the catalytic glutamate residue is replaced with an alternate amino acid, such as aspartate. Exemplary propionyl-CoA transferases suitable for mutation include propionyl-CoA transferases from *C. propionicum* and *M. elsdenii.* Glutamate residue 324 and glutamate residue 325 are the catalytic carboxylates in *C. propionicum* propionyl-CoA transferase and *M. elsdenii* propionyl-CoA transferase, respectively. As the catalytic carboxylate is conserved among CoA transferases, the catalytic amino acid residue in propionate CoA transferases from other sources is identified by sequence alignment with, *e.g*., the amino acid sequence of *C. propionicum* propionyl-CoA transferase. Similarly, the catalytic amino acid residue in other CoA transferases (*e.*g., acetyl-CoA transferase or butyryl-CoA transferases) is identified by sequence alignment with, *e.g*.*,* the amino acid sequence of *C. propionicum* propionyl-CoA transferase. *C. propionicum* propionyl-CoA transferase is an example of a sequence suitable for comparison with other CoA transferases; it will be appreciated that sequences of other CoA transferase sequences can be compared to identify the conserved glutamate catalytic residue for mutation. It will also be appreciated that mutated CoA transferase having thioesterase activity can be generated by altering the nucleic acid sequence of an existing CoA transferase-encoding polynucleotide, or by generating a new polynucleotide based on the coding sequence of a CoA transferase. Thus, in these embodiments, the host cell of the invention comprises a polynucleotide comprising a nucleic acid sequence encoding an amino acid sequence with at least 80% homology (*e.g*., 85%, 90%, 95%, 99%, or 100% homology) to the amino acid sequence set forth in SEQ ID NO: 92 (*C. propionicum*-derived thioesterase including an E324D substitution) or SEQ ID NO: 94 (*M. elsdenii*-derived thioesterase including an E325D substitution) and encoding a polypeptide having thioesterase activity. Exemplary codon-optimized (for *E. coli*) DNA sequences encoding the two thioesterases are respectively set out in SEQ ID NOs: 91 and 93. Amino acid sequences of other engineered thioesterases are set out in SEQ ID NOs: 109, 110, 111 and 112.

### Isolated Enzymes

In some embodiments, isolated enzymes can be used to catalyze one or more steps described in the aspects of the invention. Advantages may include higher product yields, easier product recovery from a more concentrated solution without cell related impurities, a greater range of possible reaction conditions the use of less expensive reactors.

### Polynucleotides

The polynucleotide(s) encoding one or more enzyme activities for steps in the pathways of the invention may be derived from any source. Depending on the embodiment of the invention, the polynucleotide is isolated from a natural source such as bacteria, algae, fungi, plants, or animals; produced via a semi-synthetic route (*e.g*., the nucleic acid sequence of a polynucleotide is codon optimized for expression in a particular host cell, such as *E. coli*); or synthesized de novo. In certain embodiments, it is advantageous to select an enzyme from a particular source based on, *e.g*., the substrate specificity of the enzyme or the level of enzyme activity in a given host cell. In some embodiments of the invention, the enzyme and corresponding polynucleotide are naturally found in the host cell and over-expression of the polynucleotide is desired. In this regard, in some embodiments, additional copies of the polynucleotide are introduced in the host cell to increase the amount of enzyme. In some embodiments, over-expression of an endogenous polynucleotide may be achieved by upregulating endogenous promoter activity, or operably linking the polynucleotide to a more robust heterologous promoter.

Exogenous enzymes and their corresponding polynucleotides also are suitable for use in the context of the invention, and the features of the biosynthesis pathway or end product can be tailored depending on the particular enzyme used.

The invention contemplates that polynucleotides of the invention may be engineered to include alternative degenerate codons to optimize expression of the polynucleotide in a particular microorganism. For example, a polynucleotide may be engineered to include codons preferred in *E. coli* if the DNA sequence will be expressed in *E. coli.* Methods for codon-optimization are known in the art.

### Enzyme variants

In certain embodiments, the microorganism produces an analog or variant of the polypeptide encoding an enzyme activity. Amino acid sequence variants of the polypeptide include substitution, insertion, or deletion variants, and variants may be substantially homologous or substantially identical to the unmodified polypeptides. In certain embodiments, the variants retain at least some of the biological activity, *e.g*., catalytic activity, of the polypeptide. Other variants include variants of the polypeptide that retain at least about 50%, preferably at least about 75%, more preferably at least about 90%, of the biological activity.

Substitutional variants typically exchange one amino acid for another at one or more sites within the protein. Substitutions of this kind can be conservative, that is, one amino acid is replaced with one of similar shape and charge. Conservative substitutions include, for example, the changes of: alanine to serine; arginine to lysine; asparagine to glutamine; aspartate to glutamate; cysteine to serine; glutamine to asparagine; glutamate to aspartate; isoleucine to leucine or valine; leucine to valine or isoleucine; lysine to arginine; methionine to leucine or isoleucine; phenylalanine to tyrosine, leucine or methionine; serine to threonine; threonine to serine; tryptophan to tyrosine; tyrosine to tryptophan or phenylalanine; and valine to isoleucine or leucine. An example of the nomenclature used herein to indicate a amino acid substitution is "S345F ThrA" wherein the naturally occurring serine occurring at position 345 of the naturally occurring ThrA enzyme which has been substituted with a phenylalanine.

In some instances, the microorganism comprises an analog or variant of the exogenous or over-expressed polynucleotide(s) described herein. Nucleic acid sequence variants include one or more substitutions, insertions, or deletions, and variants may be substantially homologous or substantially identical to the unmodified polynucleotide. Polynucleotide variants or analogs encode mutant enzymes having at least partial activity of the unmodified enzyme. Alternatively, polynucleotide variants or analogs encode the same amino acid sequence as the unmodified polynucleotide. Codon optimized sequences, for example, generally encode the same amino acid sequence as the parent/native sequence but contain codons that are preferentially expressed in a particular host organism.

A polypeptide or polynucleotide "derived from" an organism contains one or more modifications to the naturally-occurring amino acid sequence or nucleotide sequence and exhibits similar, if not better, activity compared to the native enzyme (*e.g*., at least 70%, at least 80%, at least 90%, at least 95%, at least 100%, or at least 110% the level of activity of the native enzyme). For example, enzyme activity is improved in some contexts by directed evolution of a parent/naturally-occurring sequence. Additionally or alternatively, an enzyme coding sequence is mutated to achieve feedback resistance.

### Expression vectors/Transfer into microorganisms

Expression vectors for recombinant genes can be produced in any suitable manner to establish expression of the genes in a microorganism. Expression vectors include, but are not limited to, plasmids and phage. The expression vector can include the exogenous polynucleotide operably linked to expression elements, such as, for example, promoters, enhancers, ribosome binding sites, operators and activating sequences. Such expression elements may be regulatable, for example, inducible (via the addition of an inducer). Alternatively or in addition, the expression vector can include additional copies of a polynucleotide encoding a native gene product operably linked to expression elements. Representative examples of useful heterologous promoters include, but are not limited to: the LTR (long terminal 35 repeat from a retrovirus) or SV40 promoter, the *E. coli lac, tet,* or *trp* promoter, the phage Lambda PL promoter, and other promoters known to control expression of genes in prokaryotic or eukaryotic cells or their viruses. In one aspect, the expression vector also includes appropriate sequences for amplifying expression. The expression vector can comprise elements to facilitate incorporation of polynucleotides into the cellular genome.

Introduction of the expression vector or other polynucleotides into cells can be performed using any suitable method, such as, for example, transformation, electroporation, microinjection, microprojectile bombardment, calcium phosphate precipitation, modified calcium phosphate precipitation, cationic lipid treatment, photoporation, fusion methodologies, receptor mediated transfer, or polybrene precipitation. Alternatively, the expression vector or other polynucleotides can be introduced by infection with a viral vector, by conjugation, by transduction, or by other suitable methods.

### Culture

Microorganisms of the invention comprising recombinant genes are cultured under conditions appropriate for growth of the cells and expression of the gene(s). Microorganisms expressing the polypeptide(s) can be identified by any suitable methods, such as, for example, by PCR screening, screening by Southern blot analysis, or screening for the expression of the protein. In some embodiments, microorganisms that contain the polynucleotide can be selected by including a selectable marker in the DNA construct, with subsequent culturing of microorganisms containing a selectable marker gene, under conditions appropriate for survival of only those cells that express the selectable marker gene. The introduced DNA construct can be further amplified by culturing genetically modified microorganisms under appropriate conditions (*e.g*., culturing genetically modified microorganisms containing an amplifiable marker gene in the presence of a concentration of a drug at which only microorganisms containing multiple copies of the amplifiable marker gene can survive).

In some embodiments, the microorganisms (such as genetically modified bacterial cells) have an optimal temperature for growth, such as, for example, a lower temperature than normally encountered for growth and/or fermentation. In addition, in certain embodiments, cells of the invention exhibit a decline in growth at higher temperatures as compared to normal growth and/or fermentation temperatures as typically found in cells of the type.

Any cell culture condition appropriate for growing a microorganism and synthesizing a product of interest is suitable for use in the inventive method.

### Recovery

The methods of the invention optionally comprise a step of product recovery. Recovery of acrylate, 3-hydroxypropionyl-CoA, 3-hydroxypropionate, poly-3-hydroxypropionate or 1,3-propanediol can be carried out by methods known in the art. For example, acrylate can be recovered by distillation methods, extraction methods, crystallization methods, or combinations thereof; 3-hydroxypropionate can be recovered as described in U.S. Published Patent Application No. 2011/038364 or International Publication No. WO 2011/0125118; polyhydroxyalkanoates can be recovered as described in Yu and Chen, Biotechnol Prog, 22(2): 547-553 (2006); and 1,3 propanediol can be recovered as described in U.S. Patent No. 6428992 or Cho et al., Process Biotechnology, 41(3): 739-744 (2006).

### EXAMPLES

The following examples further describe and demonstrate embodiments within the scope of the present invention. The examples are given solely for the purpose of illustration and are not to be construed as limiting the present invention. Examples 1 to 6 describe the construction of different plasmids for heterologous expression of proteins in *E. coli*; Examples 7 and 8 describe the transformation and culture of *E. coli* strains; Examples 9 and 10 describe the purification of several proteins; Example 12 describes a method for quantification of acyl-CoA molecules; Examples 11 and 13 to 16 describe the in vitro reconstitution of the enzymatic activity of several proteins described in the present invention; Example 17 describe the production of 3-hydroxypropionic acid in engineered *E. coli.*

### Example 1: Expression vectors for aminotransferase genes

*E. coli* expression vectors were constructed for production of recombinant aminotransferases. A common cloning strategy was established utilizing the pET30a vector (Novagen, EMD Chemicals, Gibbstown, NJ, catalog #69909-30) for expression of proteins linked to an N-terminal hexahistidine tag under the T7 promoter. Modifications to the pET30a vector were made by replacing the DNA sequence between the *Sph*I and *Xho*I sites with a synthesized DNA sequence (SEQ ID NO: 117) (GenScript, Piscataway, NJ). In this resulting vector, designated pET30a-BB, the *Xba*I site in the *lac* operator was removed and the region encoding for the thrombin, S-tag and enterokinase sites was replaced for a sequence encoding for a Factor Xa recognition site. Furthermore, the multiple cloning site was modified to include *Eco*RV, *Eco*RI, *Bam*HI, *Sac*I, and *Pst*I sites.

Several aminotransferase genes were codon-optimized for expression in *E. coli.* To facilitate cloning, the common restriction sites: *Avr*II; *Bam*HI; *Bgl*II; *Bst*BI; *Eag*I; *Eco*RI; *Eco*RV; *Hind*III; *Kpn*I; *Nco*I; *Nhe*I; *Not*I; *Nsp*V; *Pst*I; *Pvu*II; *Sac*I; *Sal*I; *Sap*I; *Sfu*I; *Spe*I; *Xba*I; *Xho*I were also removed from the gene sequences. In addition, the 5' prefix sequence (SEQ ID NO: 118) was added immediately upstream of the start codon and a *SpeI, NotI* and *PstI* restriction site 3' suffix sequence (SEQ ID NO: 119) was added immediately downstream of the stop codon. The optimized sequences were synthesized (GenScript, Piscataway, NJ) and cloned into the pET30a-BB vector at the *Kpn*I and *Pst*I sites. The resulting plasmids and the encoded proteins are described in Table 1.

**Table 1. List of plasmids encoding for different aminotransferases**

| **Plasmid** | **Enzyme Key** | **Species and Protein (DNA SEQ ID NO:)** | **Accession# (Amino Acid SEQ ID NO:)** |
|---|---|---|---|
| pET30a-BB *Pf* AT | *Pf* AT | *Pseudomonas fluorescens* branched-chain amino acid aminotransferase (SEQ ID NO: 122) | YP_002873519.1 (SEQ ID NO: 8) |
| *pET30a-BB Ec AT* | *Ec* AT | *E. coli* valine-pyruvate aminotransferase (SEQ ID NO: 123) | NP_416793.1 (SEQ ID NO: 9) |
| pET30a-BB *Rn* AT | *Rn* AT | *Rattus norvegicus* Alanine aminotransferase (SEQ ID NO: 121) | BAA01185.1 (SEQ ID NO: 4) |
| pET30a-BB *Ss* AT | *Ss* AT | *Sus scrofa* aspartate aminotransferase, cytoplasmic (SEQ ID NO: 120) | NP_999092.1 (SEQ ID NO: 2) |

### Example 2: Expression vector for branched-chain 2-keto acid decarboxylase (KdcA)

An *E. coli* expression vector was constructed for production of a recombinant branched-chain 2-keto acid decarboxylase (KdcA). A *Lactococcus lactis* branched-chain 2-keto acid decarboxylase gene was codon-optimized for expression in *E. coli,* and the common restriction sites: *Avr*II; *Bam*HI; *Bgl*II; *Bst*BI; *Eag*I; *Eco*RI; *Eco*RV; *Hind*III; *Kpn*I; *Nco*I; *Nhe*I; *Not*I; *Nsp*V; *Pst*I; *Pvu*II; *Sac*I; *Sal*I; *Sap*I; *Sfu*I; *Spe*I; *Xba*I; *Xho*I were removed to facilitate cloning. Furthermore, additional *Eco*RI, *Not*I, *Xba*I restriction sites and a ribosomal binding site (RBS) 5' to the ATG start codon, and *Spe*I, *Not*I and *Pst*I restriction sites 3' to the stop codon were included into the sequence. The optimized sequence (SEQ ID NO: 124) was synthesized (GenScript, Piscataway, NJ) and cloned into the pET30a-BB vector at the *EcoRI* and *PstI* sites. The resulting expression vector encoding N-terminal histidine tagged KdcA (SEQ ID NO: 54) was designated pET30a-BB *Ll* KDCA.

### Example 3: Expression vector for coenzyme-A acylating propionaldehyde dehydrogenase (PduP)

An *E. coli* expression vector was constructed for production of a recombinant coenzyme-A acylating propionaldehyde dehydrogenase (PduP). A *Salmonella enterica* coenzyme-A acylating propionaldehyde dehydrogenase gene was codon-optimized for expression in *E. coli,* and the common restriction sites: *Avr*II; *Bam*HI; *Bgl*II; *Bst*BI; *Eag*I; *Eco*RI; *Eco*RV; *Hind*III; *Kpn*I; *Nco*I; *Nhe*I; *Not*I; *Nsp*V; *Pst*I; *Pvu*II; *Sac*I; *Sa*II; *Sap*I; *Sfu*I; *Spe*I; *Xba*I; *Xho*I were removed to facilitate cloning. Furthermore, additional *Eco*RI, *Not*I, *Xba*I restriction sites and a ribosomal binding site (RBS) 5' to the ATG start codon, and *Spe*I, *Not*I and *Pst*I restriction sites 3' to the stop codon were included into the sequence. The optimized sequence (SEQ ID NO: 125) was synthesized (GenScript, Piscataway, NJ) and cloned into the pET30a-BB vector at the *Eco*RI and *Pst*I sites. The resulting expression vector, designated pET30a-BB *Se* PDUP, encodes N-terminal histidine tagged version of PduP (SEQ ID NO: 60).

### Example 4: Expression vector for poly(3-hydroxybutyrate) polymerase (PhaC or PHA synthase)

An *E. coli* expression vector was constructed for production of a recombinant poly(3-hydroxybutyrate) polymerase. A *Cupriavidus necator* poly (3-hydroxybutyrate) polymerase (*phaC*) gene was codon-optimized for expression in *E. coli,* and the common restriction sites: *Avr*II; *Bam*HI; *Bgl*II; *Bst*BI; *Eag*I; *Eco*RI; *Eco*RV; *Hind*III; *Kpn*I; *Nco*I; *Nhe*I; *Not*I; *Nsp*V; *Pst*I; *Pvu*II; *Sac*I; *Sal*I; *Sap*I; *Sfu*I; *Spe*I; *Xba*I; *Xho*I were removed to facilitate cloning. Furthermore, additional *Eco*RI, *Not*I, *Xba*I restriction sites and a ribosomal binding site (RBS) 5' to the ATG start codon, and *Spe*I, *Not*I and *Pst*I restriction sites 3' to the stop codon were included into the sequence. The optimized sequence (SEQ ID NO: 126) was synthesized (GenScript, Piscataway, NJ) and cloned into the pET30a-BB vector at the *Eco*RI and *Pst*I sites. The resulting expression vector, designated pET30a-BB *Cn* PHAS, encodes N-terminal histidine tagged version of PHA synthase (SEQ ID NO: 42).

### Example 5: Expression vector for 3-hydroxypropionyl-CoA dehydratase

An *E. coli* expression vector was constructed for production of a recombinant 3-hydroxypropionyl-CoA dehydratase. A *Metallosphaera sedula* 3-hydroxypropionyl-CoA dehydratase gene was codon-optimized for expression in *E. coli,* and the common restriction sites: *Avr*II; *Bam*HI; *Bgl*II; *Bst*BI; *Eag*I; *Eco*RI; *Eco*RV; *Hind*III; *Kpn*I; *Nco*I; *Nhe*I; *Not*I; *Nsp*V; *Pst*I; *Pvu*II; *Sac*I; *Sal*I; *Sap*I; *Sfu*I; *Spe*I; *Xba*I; *Xho*I were removed to facilitate cloning. Furthermore, additional *Eco*RI, *Not*I, *Xba*I restriction sites and a ribosomal binding site (RBS) 5' to the ATG start codon, and *Spe*I, *Not*I and *Pst*I restriction sites 3' to the stop codon were included into the sequence. The optimized sequence (SEQ ID NO: 127) was synthesized (GenScript, Piscataway, NJ) and cloned into the pET30a-BB vector at the *EcoRI* and *PstI* sites. The resulting expression vector, designated pET30a-BB *Ms* 3HP-CD, encodes N-terminal histidine tagged version of the dehydratase (SEQ ID NO: 48).

### Example 6: Expression vectors for acyl-CoA thioesterase

*E. coli* expression vectors were constructed for production of recombinant short to medium-chain acyl-CoA thioesterases. Thioesterase genes from different organisms were codon-optimized for expression in *E. coli,* and the common restriction sites: *Bam*HI, *Bgl*II, *Bst*BI, *Eco*RI, *Hind*III, *Kpn*I, *Pst*I, *Nco*I, *Not*I, *Sac*I, *Sal*I, *Xba*I, and *Xho*I were removed to facilitate cloning. Furthermore, additional *Bam*HI and *Xba*I restriction sites 5' to the ATG start codon, and *Sac*I and *Hind*III restriction sites 3' to the stop codon were included into the sequence. The optimized sequences were synthesized (GenScript, Piscataway, NJ or GeneArt, Invitrogen, Carlsbad, CA) and cloned into the pET30a vector at the *Bam*HI and *Sac*I sites. The resulting plasmids and the encoded proteins are described in Table 2.

**Table 2. List of plasmids encoding for different thioesterases**

| Plasmid | Enzyme Key | Species / Protein | Accession # |
|---|---|---|---|
| pET30a-*Sc* Acot8 | ScACOT8 | *Saccharomyces cerevisiae* peroxisomal acyl-CoA thioesterase (SEQ ID NO: 96) | NP_012553 |
| pET30a-*Mus* Acot8 | MusACOT8 | *Mus musculus* acyl-CoA thioesterase 8 (SEQ ID NO: 98) | AAL35333 |
| pET30a-*Rn* Acot12 | RnACOT12 | *R. norvegicus* acyl-CoA thioesterase 12 (SEQ ID NO: 100) | NP_570103 |
| pET30a-*Ec* TesB | EcTesB | *E. coli* acyl-CoA thioesterase II (TesB) (SEQ ID NO: 90) | NP_286194 |
| pET30a-*Bs* SrfD | BsSrfD | *Bacillus subtilis* surfactin synthetase (SrfAD) (SEQ ID NO: 102) | NP_388234 |
| pET30a-*Cp* T | CpT | *C. propionicum* propionate CoA-transferase | CAB77207 |
| pET30a-Cp TT | CpTT | *C. propionicum* propionate CoA-transferase (with E324D mutation) (SEQ ID NO: 92) | Similar to CAB77207 |
| pET30a-Me T | MeT | *M. elsdenii* coenzyme A-transferase | Similar to CCC72964 except for T271A and K517R |
| pET30a-*Me* TT | MeTT | *M. elsdenii* coenzyme A-transferase (with E325D mutation) (SEQ ID NO: 94) | Similar to CCC72964 except for T271A, K517R, and E325D |
| pET30a-*Hi* YbgC | HiYbgC | *Haemophilus influenzae* thioesterase (YbgC) (SEQ ID NO: 108) | YP_248101 |

### Example 7: Transformation of E.coli

The recombinant plasmids were then used to transform chemically competent One Shot BL21 (DE3) pLysS *E*. *coli* cells (Invitrogen, Carlsbad, CA). Individual vials of cells were thawed on ice and gently mixed with 10 ng of plasmid DNA. The vials were incubated on ice for 30 min. The vials were briefly incubated at 42°C for 30 sec and quickly replaced back on ice for an additional 2 min. An aliquot of 250 µL of 37°C SOC medium was added and the vials were secured horizontally on a shaking incubator platform and incubated for 1 h at 37°C, 225 rpm. Aliquots of 20 µL and 200 µL cells were plated onto LB agar plates supplemented with the appropriate antiobiotics (50 µg/mL kanamycin; 34 µg/mL chloramphenicol) to select for cells carrying the recombinant and pLysS plasmids respectively, followed by incubation overnight at 37°C. Single colony isolates were isolated, cultured in 5 mL of selective LB broth and recombinant plasmids were isolated using a QIAPrep® Spin Miniprep kit (Qiagen, Valencia, CA) spin plasmid miniprep kit. Plasmid DNAs were characterized by gel electrophoresis of restriction digests with *Afl*III*.*

### Example 8: Culture of E. coli strains and expression of recombinant proteins

Aliquots of LB broth (15 mL), supplemented with the appropriate antibiotics (34 µg/mL chloramphenicol; 50 µg/mL kanamycin) were inoculated with different *E*. *coli* strains from frozen glycerol stocks. Cultures were incubated overnight at 25°C with 250 rpm shaking. LB broth (150 ml, containing 34 µg/mL chloramphenicol, 50 µg/mL kanamycin; equilibrated to 25° C) in 1 to 2.8 L fluted Erlenmeyer flasks was inoculated from the overnight cultures at an optical density (OD) at 600 nm of ∼0.1. Cultures were continued at 25°C with 250 rpm shaking and optical density was monitored until A₆₀₀ of -0.4. Production of recombinant proteins was induced by addition of 1M IPTG (Teknova, Hollister, CA; 1 mM final concentration). Cultures were further incubated for 24 h at 25°C with 250 rpm shaking before harvesting by centrifugation. The cell pellets were stored at -80° C until used.

### Example 9: Recombinant protein isolation

His-tagged recombinant proteins were isolated by immobilized metal affinity chromatography (IMAC) utilizing nickel-nitrilotriacetic acid coupled Sepharose CL-6B resin (Ni-NTA, Qiagen, Valencia, CA) as follows. Cell pellets were thawed on ice and suspended in 20 mL of binding buffer (20 mM sodium phosphate, 500 mM NaCl, 20 mM imidazole, pH 7.4) supplemented with 1 mg/mL lysozyme and 1 pellet of Complete EDTA-free protease inhibitor (Roche Applied Science, Indianapolis, IN) . Samples were incubated at 4°C with 30 rpm rotation for 30 min followed by French-pressing (1000 psi). Cell debris was pelleted by centrifugation for 1 h at 15,000 × g and 4°C. The supernatant was transferred to a 5 mL column bed of Ni-NTA resin equilibrated with binding buffer. The Ni-NTA resin was resuspended in the supernatant and incubated for 60 min with slow rocker mixing at 4°C. The unbound material was removed by gravity flow and the resin was washed by gravity flow with 20 column volume (CV, 100 mL) of binding buffer followed by 10 CV (50 mL) of rinse buffer (20 mM sodium phosphate, 500 mM NaCl, 100 mM imidazole, pH 7.4). Bound proteins were eluted by gravity-flow in 10 CV (50 mL) of elution buffer (20 mM sodium phosphate, 500 mM NaCl, 500 mM imidazole, pH 7.4) and collected in fractions. Elution aliquots were assayed for protein content by SDS-PAGE analysis, pooled, and concentrated with Amicon Ultra-15 centrifugal filter devices (EMD Millipore, Billerica, MA) with 30 kDa nominal molecular weight cut-off. The concentrated protein isolates were desalted and eluted into 3.5 mL of storage buffer (50 mM HEPES, 300 mM NaCl, 20% glycerol, pH 7.3) using PD-10 desalting columns (GE Healthcare Biosciences, Pittsburgh, PA).

### Example 10: Recombinant thioesterases isolation

His-tagged recombinant thioesterases were isolated by IMAC utilizing sepharose based magnetic beads with nickel ions (His Mag Sepharose Ni) as follows. Cell pellets were thawed at room temperature and suspended in 1.7 mL of 1X BugBuster (primary amine free; with 1µl/mL Benzonase nuclease; Novagen # 70923-3 and 70750-3 respectively). Samples were incubated at room temperature with 60 rpm rotation for 30 min. Cell debris was pelleted by centrifugation for 10 min at 14,000 rpm. The supernatants were transferred to His Mag Sepharose Ni (GE Healthcare Biosciences, Piscataway, NJ # 28-9799-17) beads equilibrated according to kit instructions in binding buffer (20 mM sodium phosphate, 500 mM NaCl, 20 mM imidazole, pH 7.4). The beads were suspended in the supernatant and incubated for 60 min with slow end-over-end mixing. The beads were then washed for a total of 5 times in 800 µl of binding buffer and slow end-over-end mixing for -3-5 min with each wash. The recombinant thioesterases were eluted from the beads in 300 µL of elution buffer (20 mM sodium phosphate, 500 mM NaCl, 500 mM imidazole, pH 7.4) by slow end-over-end mixing for 5 min.

### Example 11: In vitro reconsitution of aminotransferases and liquid chromatography coupled to mass spectrometry (LC-MS) analysis

The activities of purified recombinant aminotransferases (Example 9) were tested by LC-MS analysis of expected products. In separate reactions, each enzyme was added at 0.27 mg/mL final concentration to reaction buffer (20 mM potassium phosphate, 500 mM sodium chloride, pH 8 ). L-Homoserine (Sigma, St. Louis, MO; catalog # H6515) or a different amino acid substrate (Sigma, St. Louis, MO) was added at 1 mM final concentration. Secondary substrates were either α-ketoglutaric acid (disodium salt, dehydrate; Sigma, St. Louis, MO; catalog # 75892) or pyruvate (Sigma, St. Louis, MO; catalog # P2256), each at 1mM final concentration. Pyroxidal 5'-phosphate hydrate (Sigma, St. Louis, MO; catalog # P9255) was added at 50 µM final concentration. The reactions were incubated overnight at room temperature. After incubation, each solution was filtered using Amicon Ultra centrifugal filter devices (EMD Millipore, Billerica, MA) with 3 kDa nominal molecular weight cut-off that had been prewashed with ultra pure water. The filtrates were collected and stored at -20 °C until LC-MS analysis.

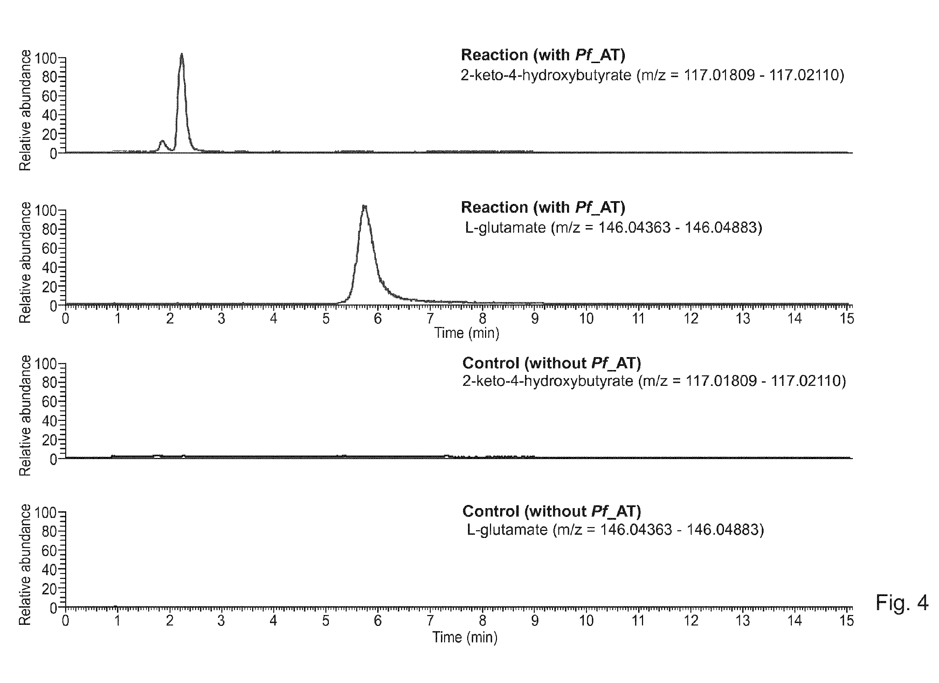

Aliquots of reaction mixture were diluted 50-100 × and analyzed by high performance liquid chromatography coupled to mass spectrometry (LC-MS) in negative mode, using an electrospray ionization (ESI) Fourier transform orbital trapping MS (Exactive Model; Thermo Fisher, San Jose,CA) at 50,000 resolution. Separations were performed using a ZIC-pHILIC column (2.1 × 100 mm, 5 µm polymer, Sequant, EMD Millipore, Catalog # 1504620001; Darmstadt, Germany) and a mobile phase of 2 mM ammonium formate in 85% acetonitrile / 15% water at a flow rate of 200 µL/min. The LC-MS analysis indicated that every tested enzyme (Table 1) produced the expected product when combined with its ideal substrate and all enzymes produced 2-keto-4-hydroxybutyrate when combined with L-homoserine (Figure 4).

### Spectrophotometric assays with aminotransferases

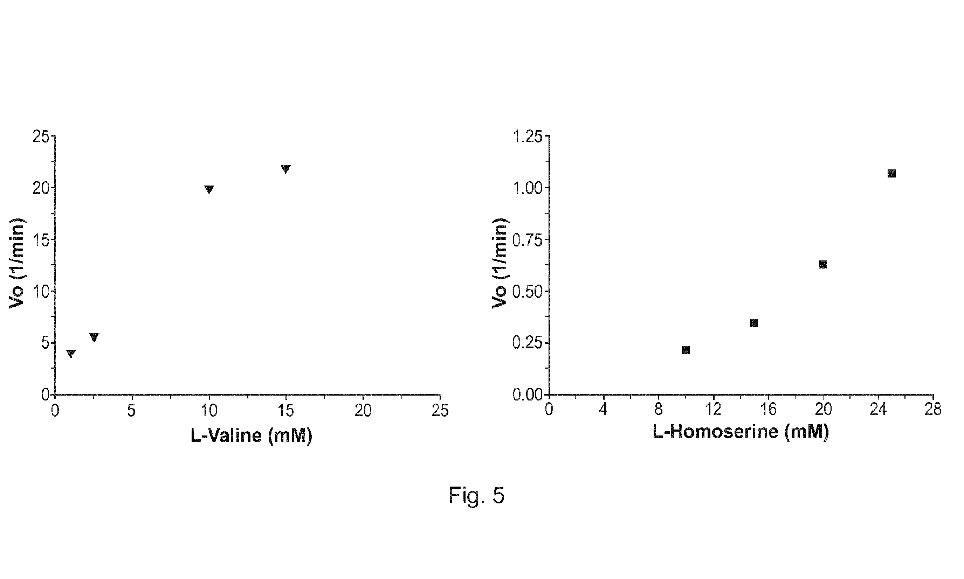

To further confirm the enzymatic activity of the aminotransferases, the purified recombinant proteins were assayed spectrophotometrically in a series of coupled enzyme reactions. In separate reactions, *Pf* AT aminotransferase was added at 0.27 mg/ml final concentration to 100 mM potassium phosphate buffer (pH 8.0; Sigma, St. Louis, MO). L-Homoserine (Sigma, St. Louis, MO; catalog # H6515) or L-Valine (Sigma, St. Louis, MO; catalog # V0500) was added as a substrate at 10 - 25 mM final concentration. The aminotransferase reaction was coupled with a dehdrogenase reaction in order to generate reduced β-nicotinamide adenine dinucleotide (NADH) which can be detected spectrophotometrically. β-Nicotinamide adenine dinucleotide (NAD⁺; Sigma, St. Louis, MO; catalog # N8410) was added at 3 mM final concentration. Pyroxidal 5'-phosphate hydrate (Sigma, St. Louis, MO; catalog # P9255) was added at 50 µM final concentration. α-Ketoglutaric acid, disodium salt, dehydrate (Sigma, St. Louis, MO; catalog # 75892) was added as a secondary substrate at 1 mM final concentration. L-Glutamic dehydrogenase from bovine liver (Sigma, St. Louis, MO; catalog # G2626) was added at 10 U/mL. Each reaction was added to a 1 mL quartz cuvette and the formation of NADH was followed over time at 340 nm in a spectrophotometer. As expected, the initial rate of conversion of L-homoserine was dependent on its concentration (Figure 5). Saturation of the enzyme with L-homoserine was not achieved even when high concentrations were used.

### Example 12: Acyl-CoA levels as a measurement of enzymatic activities: Liquid chromatography coupled to mass spectrometry (LC-MS).

### E. coli culture sample preparation for acyl-CoA levels analysis

A stable-labeled (deuterium) internal standard-containing master mix is prepared, comprising d₃-3-hydroxymethylglutaryl-CoA (200 µL of 50 µg/mL stock in 10 mL of 15% trichloroacetic acid). An aliquot (500 µl) of the master mix is added to a 2-mL tube. Silicone oil (AR200; Sigma, St. Louis, MO; catalog # 85419; 800 µl) is layered onto the master mix. An aliquot of *E*. *coli* culture (800 µl) is layered gently on top of the silicone oil. The sample is subject to centrifugation at 20,000 × g for 5 min at 4°C in an Eppendorf 5417C centrifuge. An aliquot (300 µL) of the master mix-containing layer is transferred to an empty tube and frozen on dry ice for 30 min.

### Measurement of acyl-CoA levels

The acyl-CoA content of samples was determined using LC-MS/MS. Individual acyl-CoA standards were purchased from Sigma (St. Louis, MO) and prepared as 500 µg/mL stocks in methanol. Acryloyl-CoA was synthesized and prepared similarly. The analytes were pooled, and standards with all of the analytes were prepared by dilution with 15% trichloroacetic acid. Standards for regression were prepared by transferring 500 µL of the working standards to an autosampler vial containing 10 µL of the 50 µg/mL internal standard. Sample peak areas (or heights) were normalized to the stable-labeled internal standard (d₃-3-hydroxymethylglutaryl-CoA). Samples were assayed by LC-MS/MS on a Sciex API5000 mass spectrometer in positive ion Turbo Ion Spray. Separation was carried out by reversed-phase high performance liquid chromatography (HPLC) using a Phenomenex Onyx Monolithic C18 column (2 x 100 mm) and mobile phases A (5 mM ammonium acetate, 5 mM dimethylbutylamine, and 6.5 mM acetic acid) and B (0.1 % formic acid in acetonitrile), with the gradient described in table 3 at a flow rate of 0.6 mL/min.

**Table 3. Composition of mobile phase during LC-MS/MS analysis**

| Time | Mobile Phase A (%) | Mobile Phase B (%) |
|---|---|---|
| 0 min | 97.5 | 2.5 |
| 1.0 min | 97.5 | 2.5 |
| 2.5 min | 91.0 | 9.0 |
| 5.5 min | 45 | 55 |
| 6.0 min | 45 | 55 |
| 6.1 min | 97.5 | 2.5 |
| 7.5 min | - | - |
| 9.5 min | End Run | |

The conditions on the mass spectrometer were: DP 160, CUR 30, GS1 65, GS2 65, IS 4500, CAD 7, TEMP 650 C. The transitions used for the multiple reaction monitoring (MRM) are described in table 4.

**Table 4. Description of parameters for quantification of different acyl-CoA molecules**

| Compound | Precursor Ion¹ | Product Ion¹ | Collision Energy | CXP |
|---|---|---|---|---|
| 3-Hydroxypropionyl-CoA² | 840.3 | 333.2 | 45 | 13 |
| n-Propionyl-CoA | 824.3 | 317.2 | 41 | 32 |
| Succinyl-CoA | 868.2 | 361.1 | 49 | 38 |
| Isobutyryl-CoA | 838.3 | 331.2 | 43 | 21 |
| Lactoyl-CoA | 840.3 | 333.2 | 45 | 38 |
| Acryloyl-CoA | 822.4 | 315.4 | 45 | 36 |
| Coenzyme A | 768.3 | 261.2 | 45 | 34 |
| Isovaleryl-CoA | 852.2 | 345.2 | 45 | 34 |
| Malonyl-CoA | 854.2 | 347.2 | 41 | 36 |
| Acetyl-CoA | 810.3 | 303.2 | 43 | 30 |
| d₃-3-Hydroxymethylglutaryl-CoA | 915.2 | 408.2 | 49 | 13 |

| | | | | |
|---|---|---|---|---|
| ¹ Energies, in volts, for the MS/MS analysis ²Quantified based on *n*-propionyl-CoA response | | | | |

### Example 13: In vitro production of 3-hydroxypropionyl-COA with 2-keto acid decarboxylases or dehydrogenases

In a first assay, D-homoserine (2 mM; Acros, Geel, Belgium; catalog # 348362500) was incubated with D-amino acid oxidase (1 U/mL; Sigma, St. Louis, MO; catalog # A5222) and bovine liver catalase (600 U/mL; Sigma, St. Louis, MO; catalog # C40) in the presence of HEPES buffer (50 mM, pH 7.3). After incubation at room temperature for 2 - 4 h, coenzyme A (2 mM), β-NAD⁺- (2 mM), thiamine pyrophosphate (0.2 mM), and MgCl₂ (2 mM) were added to the solution and the components were further incubated with or without commercial porcine heart α-ketoglutarate dehydrogenase (1.0 mg/mL; Sigma, St. Louis, MO; catalog # K1502).

In a second assay, D-homoserine (2 mM; Acros, Geel, Belgium; catalog # 348362500) was incubated with D-amino acid oxidase (1 U/mL; Sigma, St. Louis, MO; catalog # A5222) and bovine liver catalase (600 U/mL; Sigma, St. Louis, MO; catalog # C40) in the presence of HEPES buffer (50 mM, pH 7.3). After incubation at room temperature for 2 - 4 h, coenzyme A (2 mM), β-NAD⁺ (2 mM), thiamine pyrophosphate (0.2 mM), and MgCl₂ (2 mM) were added to the solution and the components were further incubated with or without purified 2-keto acid decarboxylase KdcA (1.8 µm) and propionaldehyde dehydrogenase PduP (1.8 µm).

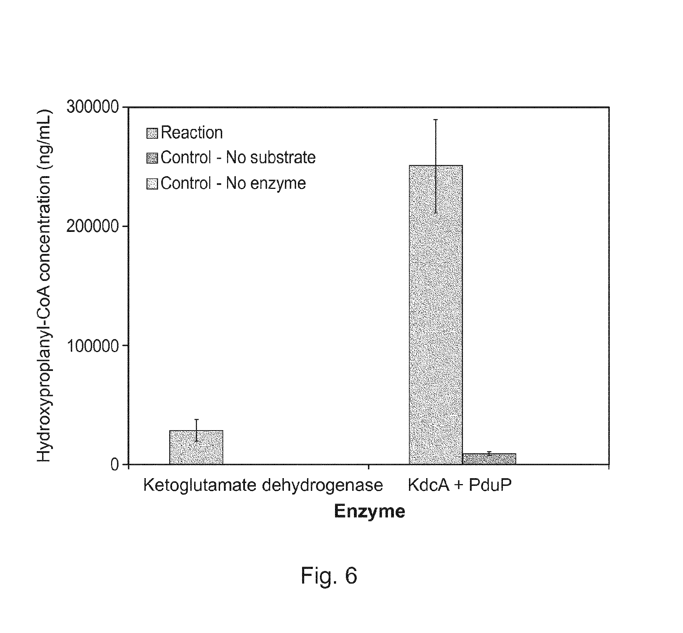

The samples were incubated at room temperature overnight, followed by LC-MS analysis to determine concentrations of 3-hydroxypropionyl-CoA as described in example 12. Only when the dehydrogenases (and decarboxylase) were present, the product was detected in significant amounts (Figure 6).

### Example 14: In vitro production of 3-hydroxypropionyl-CoA from acryloyl-CoA with 3-hydroxypropionyl-CoA dehydratase

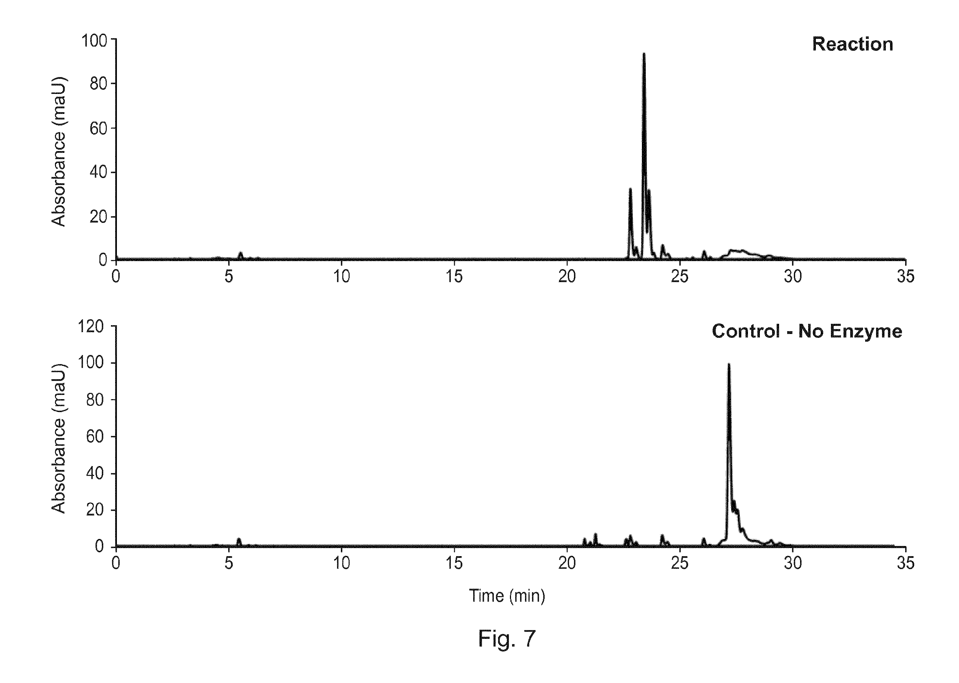

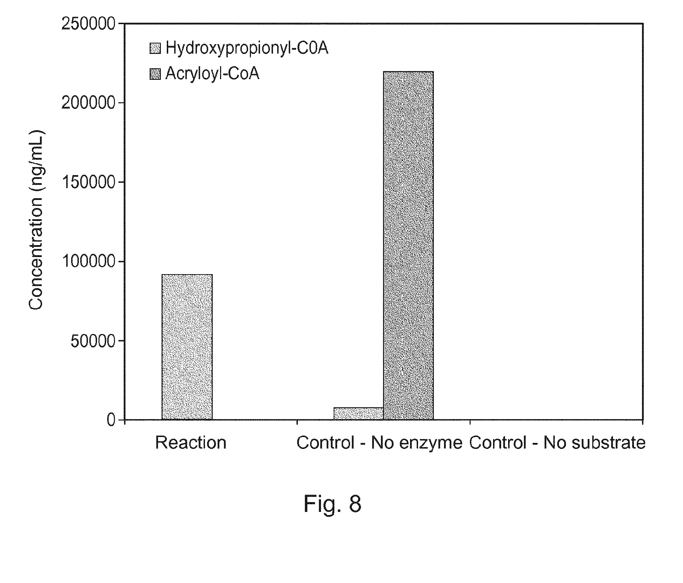

Acryloyl-CoA (1 mM) was incubated with or without 3-hydroxypropionyl-CoA dehydratase (20 µM) in the presence of HEPES buffer (50 mM, pH 7.3). After incubation at room temperature for 2 - 4 h, aliquots were analyzed by high performance liquid chromatography (HPLC) using an Agilent 1100 system (Agilent, Santa Clara, CA) monitoring absorbance at 254 nm and a Waters Atlantis T3 column (Waters, Milford, MA; catalog # 186003748). Mobile phases were 0.1 % phosphoric acid in water (A) and 0.1 % phosphoric acid in 80% acetonitrile / 20% water (B). Analytes were eluted isocratically at 2% B in A over 12 min, followed by a linear gradient from 2% to 35% B in A over 18 min. The HPLC analysis indicates consumption of acryloyl-CoA and formation of a different absorbing molecule (Figure 7). The identity of the reaction product, 3-hydroxypropionyl-CoA, was confirmed by LC-MS analysis as described in example 12 (Figure 8).

### Example 15: In vitro reconstitution of PHA synthase

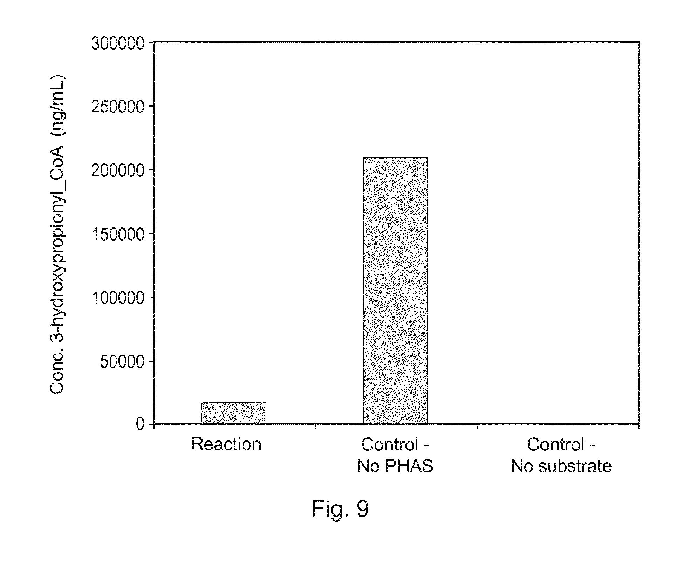

A solution of 3-hydroxypropionic acid (5 mM; Aldrich, St. Louis, MO; catalog # AMS000335), coenzyme A (2 mM), ATP (6 mM), MgCl₂ (2 mM), and HEPES buffer (50 mM, pH 7.3) was incubated with acetyl-CoA synthetase (5 U/mL; Sigma, St. Louis, MO; catalog # A1765 ) and with or without purified PHA synthase (1 µM). After incubation at room temperature for 2 - 4 h, aliquots were analyzed by LC-MS as described in example 12 to determine concentrations of 3-hydroxypropionyl-CoA. When PHA synthase was present, the concentration of 3-hydroxypropionyl-CoA considerably decreased compared with a sample with no enzyme (Figure 9).

### Example 16: Thioesterase activity assay: Ellman's reagent

To measure relative thioesterase enzyme activity, Ellman's reagent, also known as DTNB (5,5'-dithiobis-(2-nitrobenzoic acid)), was used. The assay buffer was 50 mM KCl, 10 mM HEPES (pH 7.4). A 10mM Ellman's reagent stock solution was prepared in ethanol. An acryloyl-CoA substrate stock solution was prepared to 10 mM in assay buffer.

For each enzyme and substrate tested, the reaction was as follows: a 10mM Ellman's reagent stock solution was diluted to a 50 µM final concentration in assay buffer. Acryloyl-CoA stock solution was added to provide a 90 µM final concentration. The Ellman's reagent/acryloyl-CoA mixture (95 µL per well) was added to a 96-well polystyrene untreated microtiter plate. Equivalent reactions with no substrate were prepared as controls. Purified enzyme was serially diluted 1:3 in assay buffer in a separate plate, and 5 µL was added to a reaction well. Thioesterase activity was assessed at 60 min by measuring the optical density (OD) at 412 nm on a plate reader. Relative enzyme activities were calculated by subtracting OD (412 nm) of substrate-free controls from OD (412 nm) of substrate-containing samples.

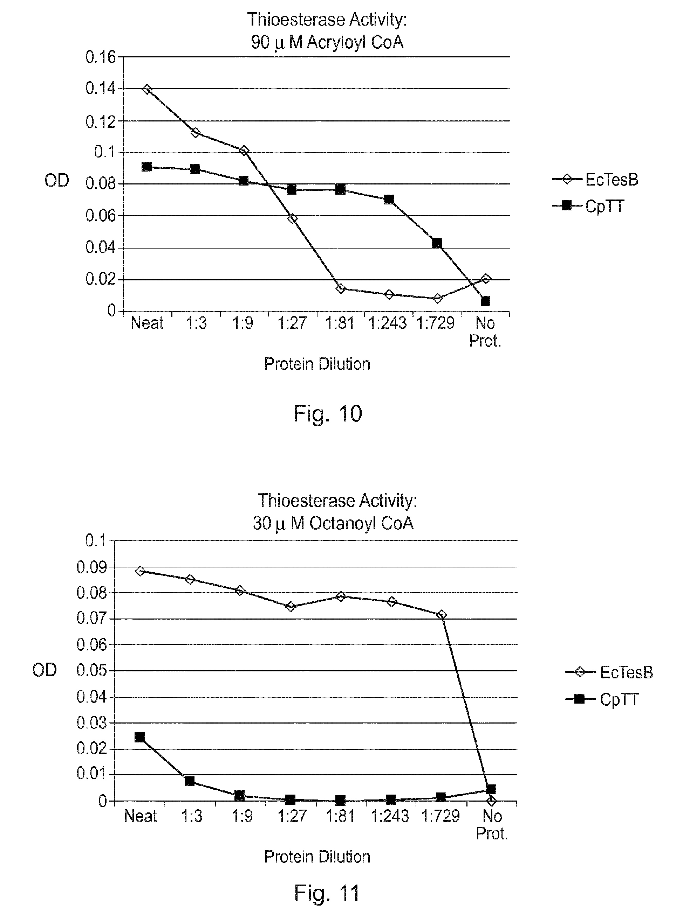

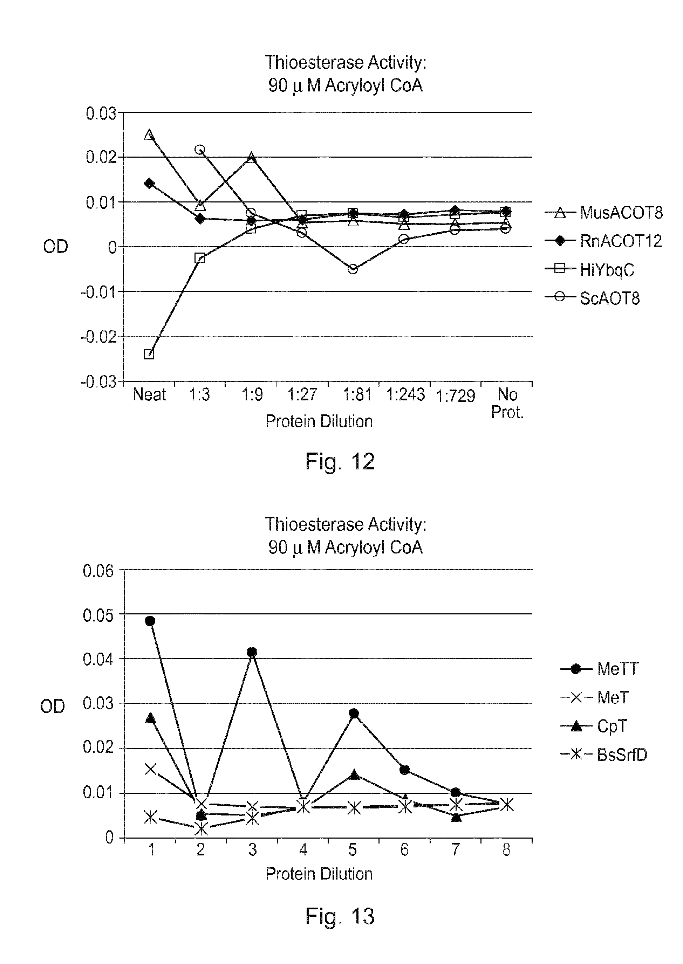

Two thioesterases, EcTesB and CpTT, each showed hydrolysis activity against the acryloyl-CoA substrate, with the activity increasing with increasing amounts of thioesterase (Figure 10). EcTesB was also active against other substrates (Figure 11). EcTesB hydrolyzed octanoyl-CoA, even at relatively low amounts of EcTesB. In contrast, CpTT only showed an increase in octanyol-CoA hydrolysis with the highest amounts of thioesterase (Figure 11). The other thioesterases showed little or no thioesterase activity against acryloyl-CoA (Figures 10 and 11), yet their apparent hydrolysis of octanoyl-CoA suggested that the recombinant enyzmes were active (Figures 12 and 13). To confirm that the thioesterases were active on the coenzyme A substrate tested, samples were analyzed using liquid chromatography coupled to mass spectrometry (LC-MS) as described in example 12.

### Monitoring of substrate and product by LC-MS

EcTesB and CpTT showed acryloyl-CoA thioesterase activity in the assay based on generation of a free sulfhydryl from the acryloyl-CoA. As a further test of this thioesterase activity, it is useful to observe the disappearance of substrate and appearance of product. Therefore, LC-MS was used to monitor substrate and product amounts in assays with these enzymes as described in Example 12. The amount of EcTesB correlates with the increase in acryloyl-CoA hydrolysis, as indicated by both the detection of Coenzyme A by Ellman's reagent and by the disappearance of acryloyl-CoA (Table 1). As the enzyme is diluted, the thioesterase activity levels decline, as indicated by each assay. These results support EcTesB's role as a thioesterase that is active on acryloyl-CoA.

**Table 5. Relative enzyme activity and acryloyl-CoA quantitation of TesB thioesterase samples. The activity (OD at 412 nm) refers to the assay based on color change in the presence of Ellman's reagent. The acryloyl-CoA measurements were based on LC/MS.**

| **TesB Dilution** | **Activity OD(412nm)** | **Acryloyl CoA (ng)**** |
|---|---|---|
| Neat | 0.140 | <200 |
| 1:3 | 0.113 | 508 |
| 1:9 | 0.101 | 14600 |
| 1:27 | 0.058 | 39400 |

| | | |
|---|---|---|
| ** Values above 5000 are extrapolated estimates | | |

EcTesB and CpTT each show acryloyl-CoA hydrolysis activity by two different assays (Table 6). Each enzyme causes in increase in coenzyme A, as detected with Ellman's reagent. Each enzyme also causes a changing profile in the LC-MS analysis, with the thioesterases causing a decrease in acryloyl-CoA and an increase in coenzyme A (Table 6).

**Table 6. Coenzyme A and acryloyl-CoA quantitation of thioesterase samples. The activity (or at 412 nm) refers to the assay based on color change in the presence of Ellman's reagent. The acryloyl-CoA and coenzyme A measurements were based on LC-MS analysis.**

| **Sample Name** | **Activity OD(412nm)** | **CoA (ng)** | **Acryloyl CoA (ng)**** |
|---|---|---|---|
| EcTesB | 0.2157 | 28100 | 756 |
| CpTT | 0.0992 | 13600 | 1430 |
| no enzyme | 0.051 | 259 | 79000 |

### Example 17: Production of 3-hydroxypropionic acid in engineered E. coli

This example demonstrates increased production of 3-hydroxypropionic acid in *E. coli* host cells which can then be converted to poly-3-hydroxypropionic acid or acylic acid. *E. coli* strains were established to overexpress *P. fluorescens* branched-chain amino acid aminotransferase (*Pf* AT) set out in SEQ ID NO: 8, *L. lactis* branched-chain 2-keto acid decarboxylase (KdcA) set out in SEQ ID NO: 54, *S. enterica* coenzyme-A acylating propionaldehyde dehydrogenase (PduP) set out in SEQ ID NO: 60, and in some instances *C*. *necator* Poly(3-hydroxybutyrate) polymerase (PhaC) set out in SEQ ID NO: 42.

In this example, *P. fluorescens* branched-chain amino acid aminotransferase (SEQ ID NO: 8) promoted the conversion of L-homoserine to 2-keto-4-hydroxybutyrate. The *L. lactis* branched-chain 2-keto acid decarboxylase (KdcA, set out in SEQ ID NO: 540 catalyzed the conversion of 2-keto-4-hydroxybutyrate to 3-hydroxy-propionaldehyde. The *S*. *enteric* coenzyme-A acylating propionaldehyde dehydrogenase (PduP, set out in SEQ ID NO: 60) catalyzed the conversion of 3-hydroxy-propionaldehyde to 3-hydroxypropionyl-CoA. A thioesterase catalyzed the conversion of 3-hydroxypropionyl-CoA to 3-hydroxypropionate. Alternative, the *C*. *necator* Poly (3-hydroxybutyrate) polymerase (PhaC, set out in SEQ ID NO: 42) can catalyze the conversion of 3-hydroxypropionyl-CoA to poly-3-hydroxypropionate.

### Plasmid construction

An *E. coli* expression vector was constructed for overexpression of a recombinant *P*. *fluorescens* branched-chain amino acid aminotransferase *(Pf* AT) and *C*. *necator* Poly (3-hydroxybutyrate) polymerase (PhaC). The codon optimized *C*. *necator* Poly (3-hydroxybutyrate) polymerase *(phaC)* from pET30a-BB *Cn* PHAS (Example 4) was cloned into pET30a-BB *Pf* AT (Example 1) by double digestion of pET30a-BB *Cn* PHAS with restriction enzymes *Xba*I and *Pst*I*.* The *Cn* PHAS fragment was band isolated, purified using a QIAquick Gel Extraction Kit (Qiagen, Carlsbad, CA) and ligated (Fast-Link Epicentre Biotechnologies, Madison, WI) with S*pe*I/P*st*I-digested pET30a-BB *Pf* AT vector. The ligation mix was used to transform OneShot Top10™ *E. coli* cells (Invitrogen, Carlsbad, CA). Individual vials of cells were thawed on ice and gently mixed with 2 µL of ligation mix. The vials were incubated on ice for 30 min. The vials were briefly incubated at 42°C for 30 sec and quickly replaced back on ice for an additional 2 min. 250 µL of 37°C SOC medium was added and the vials were secured horizontally on a shaking incubator platform and incubated for 1 h at 37°C and 225 rpm. Aliquots of 20 µL and 200 µL cells were plated onto LB agar supplemented with kanamycin (50 µg/mL). Single colony isolates were isolated and cultured in 5 mL of LB broth with kanamycin (50 µg/mL). The recombinant plasmid was isolated using a Qiagen Plasmid Mini Kit and characterized by gel electrophoresis of restriction digests with *Afl*III*.* The resulting plasmid was designated pET30a-BB *Pf AT_Cn* PHAS. An *E. coli* expression vector was constructed for overexpression of a recombinant S. *enterica* coenzyme-A acylating propionaldehyde dehydrogenase (PduP) and *L*. *lactis* branched-chain 2-keto acid decarboxylase (KdcA). The codon optimized *L. lactis* branched-chain 2-keto acid decarboxylase *(kdcA)* from pET30a-BB *Ll* KDCA (Example 2) was cloned into pET30a-BB Se PDUP (Example 3) by double digestion of pET30a-BB *Ll* KDCA with restriction enzymes *Xba*I and *Pst*I*.* The *Ll* KDCA fragment was band isolated, purified using a QIAquick Gel Extraction Kit (Qiagen, Carlsbad, CA) and ligated (Fast-Link Epicentre Biotechnologies, Madison, WI) with SpeI/PstI-digested pET30a-BB *Se* PDUP vector. The ligation mix was used to transform OneShot Top10™ *E. coli* cells (Invitrogen, Carlsbad, CA). Individual vials of cells were thawed on ice and gently mixed with 2 µL of ligation mix. The vials were incubated on ice for 30 min. The vials were briefly incubated at 42°C for 30 sec and quickly replaced back on ice for an additional 2 min. 250 µL of 37°C SOC medium was added and the vials were secured horizontally on a shaking incubator platform and incubated for 1 h at 37°C and 225 rpm. Aliquots of 20 µL and 200 µL cells were plated onto LB agar supplemented with kanamycin (50 µg/mL). Single colony isolates were isolated and cultured in 5 mL of LB broth with kanamycin (50 µg/mL) and the recombinant plasmid was isolated using a Qiagen Plasmid Mini Kit and characterized by gel electrophoresis of restriction digests with *AflIII.* The resulting plasmid was designated pET30a-BB *Se* PDUP_*Ll* KDCA. To facilitate cotransformation with pET30a-BB *Pf* AT or pET30a-BB *Pf AT_Cn* PHAS, the codon optimized *S. enterica* coenzyme-A acylating propionaldehyde dehydrogenase *(pduP)* and *L. lactis* Branched-chain 2-keto acid decarboxylase *(kdcA)* gene pair was subcloned from pET30a-BB *Se* PDUP_*Ll* KDCA into the pCDFDuet-1 vector (Novagen, EMD Chemicals, Gibbstown, NJ; catalog # 71340-3) by double digestion of pET30a-BB *Se* PDUP_*Ll* KDCA with restriction enzymes *EcoRI* and *PstI.* The *Se* PDUP_*Ll* KDCA fragment was band isolated, purified using a QIAquick Gel Extraction Kit (Qiagen, Carlsbad, CA) and ligated (Fast-Link Epicentre Biotechnologies, Madison, WI) with *Eco*RI/*Pst*I-digested pCDFDuet-1. The ligation mix was used to transform OneShot Top10™ *E. coli* cells (Invitrogen, Carlsbad, CA). Individual vials of cells were thawed on ice and gently mixed with 2 µL of ligation mix. The vials were incubated on ice for 30 min. The vials were briefly incubated at 42°C for 30 sec and quickly replaced back on ice for an additional 2 min. 250 µL of 37°C SOC medium was added and the vials were secured horizontally on a shaking incubator platform and incubated for 1 h at 37°C and 225 rpm. Aliquots of 20 µL and 200 µL cells were plated onto LB agar supplemented with spectinomycin (50 µg/mL). Single colony isolates were isolated, cultured in 5 mL of LB broth with spectinomycin (50 µg/mL) and the recombinant plasmid was isolated using a Qiagen Plasmid Mini kit and characterized by gel electrophoresis of restriction digests with *Afl*III*.* The resulting plasmid was designated pCDFDuet-1 *Se* PDUP_*Ll* KDCA.

### Co-transformation of E. coli

The recombinant plasmids and empty parent vectors were used to co-transform chemically competent BL21 (DE3) pLysS *E*. *coli* cells (Invitrogen, Carlsbad, CA) in the following combinations:
pET30a-BB *Pf AT_Cn* PHAS and pCDFDuet-1 *Se* PDUP_*Ll* KDCA
pET30a-BB *Pf AT* and *pCDFDuet-1 Se* PDUP_*Ll* KDCA
pET30a-BB and pCDFDuet-1

Individual vials of cells were thawed on ice and gently mixed with 50 ng of plasmid DNA. The vials were incubated on ice for 30 min. The vials were briefly incubated at 42°C for 30 sec and quickly replaced back on ice for an additional 2 min. 250 µL of 37°C SOC medium was added and the vials were secured horizontally on a shaking incubator platform and incubated for 1 h at 37°C and 225 rpm. Aliquots of 20 µL and 200 µL cells were plated onto LB agar supplemented with the appropriate antibiotics (50 µg/mL kanamycin; 50 µg/mL spectinomycin; 34 µg/mL chloramphenicol) to select for cells carrying the recombinant pET30a-BB, pCDFDuet-1 and pLysS plasmids respectively and incubated overnight at 37°C. Single colony isolates were isolated, cultured in 5 mL of selective LB broth and the recombinant plasmids were isolated using a QIAPrep® Spin Miniprep Kit (Qiagen, Valencia, CA) and characterized by gel electrophoresis of restriction digests with *Afl*III*.*

### Strain culture

Single colony forming units of *E*. *coli* BL21 (DE3) pLysS cells co-transformed with the described plasmids were used to inoculated aliquots of minimal M9 broth (25 mL) supplemented with the appropriate antibiotics (34 µg/mL chloramphenicol, 50 µg/mL kanamycin, and 50 µg/mL spectinomycin). The cultures were incubated overnight at 37°C with shaking at 250 rpm and used to inoculated fresh minimal M9 media (50 mL) supplemented with the same antibiotics. After overnight incubation under similar conditions, aliquots of cultures were used to inoculate a new set of M9 broths (50 mL) with antibiotics (34 µg/mL chloramphenicol, 50 µg/mL kanamycin, and 50 µg/mL spectinomycin) and supplemented with or without L-homoserine (1 g/L; Sigma, St. Louis, MO), followed by incubation at 25°C with shaking at 250 rpm. When OD₆₀₀ of about 0.2 was reached, protein expression was induced by addition of 50 µL of 1M IPTG (1 mM final concentration; Teknova, Hollister, CA), followed by incubation for 17 h at 25°C with 250 rpm shaking. Cells were harvested by centrifugation and supernatants were filtered through Acrodisc Syringe Filters (0.2 µm HT Tuffryn membrane; low protein binding; Pall Corporation, Ann Arbor, MI) and frozen on dry ice prior to storage at -80° C until analysis.

| Minimal M9 Media Component | 1X Base Recipe |
|---|---|
| Na₂HPO₄ | 6 g/L |
| KH₂PO₄ | 3 g/L |
| NaCl | 0.5 g/L |
| NH₄Cl | 1 g/L |
| CaCl₂ * 2H₂O | 0.1 mM |
| MgSO₄ | 1 mM |
| Dextrose | 80 mM |
| Thiamine | 1 mg/L |
| Chloramphenicol | 34 µg/mL |
| Kanamycin | 50 µg/mL |
| Spectinomycin | 50 µg/mL |

### Detection of 3-hydroxypropionic acid by engineered E. coli

An internal standard solution of 100 µg/mL of ¹³C₃-labelled lactic acid in 1:1 MeOH:H₂O was prepared. External standard solutions were prepared at 3-hydroxypropanoic acid concentrations of 1 µg/mL, 2.5 µg/mL, 5 µg/mL, 10 µg/mL and 25 µg/mL in 1:1 MeOH:H₂O. 900 µL of filtered supernatant or external standard was added to 100 µL of the internal standard solution. These solutions were subjected to ion exclusion liquid chromatography (LC) separations and mass spectrometry (MS) detection. The LC separation conditions were as follows: 10 µL of sample/standard were injected onto a Thermo Fisher Dionex ICE-AS1 (4x250 mm) column (with guard) running an isocratic mobile phase of 1 mM heptafluorbutyric acid at a flow rate of 0.15 mL/min. 20 mM NH₄OH in MeCN at 0.15 mL/min was teed into the column effluent. The MS detection conditions were as follows: A Sciex API-4000 MS was run in negative ion mode and monitored the m/z 89 to 59 (unit resolution) transition of 3-hydroxypropanoic acid and the m/z 92 to 45 (unit resolution) transition of ¹³C₃-labelled lactic acid. The dwell time used was 300 ms, the declustering potential was set at -38, the entrance potential was set at -10, the collision gas was set at 12, the curtain gas was set at 15, the ion source gas 1 was set at 55, the ion source gas 2 was set at 55, the ionspray voltage was set at - 3500, the temperature was set at 650, the interface heater was on. For 3-hydroxypropanoic acid, the collision energy was set at -16 and the collision set exit potential was set at -9. For ¹³C₃-labelled lactic acid, the collision energy was set at -18 and the collision set exit potential was set at -16. The results of the analysis are shown in Table 7. The data evidenced that increased levels of 3-hydroxypropanoic acid were produced when *Pf* AT, KdcA, and PduP were overexpressed and L-homoserine was supplemented to the culture media. Endogenous L-homoserine and *E*. *coli* proteins likely supported production of small amounts of 3-hydroxypropionic acid when no exogenous homoserine was added to the culture medium and/or when empty pET30a-BB and pCDF Duet-1 vectors were present.

**Table 7. Production of 3-hydroxypropionic acid by engineered E. coli**

| Plasmids | Addition of L-homoserine? | Concentration of 3-hydroxypropionic acid (µg/mL) |
|---|---|---|
| pET30a-BB *Pf*_AT::*Cn*_PHAS and | | |
| pCDF Duet-1 *Se_*PDUP::*Ll_*KDCA | No | 0.08 |
| pET30a-BB *Pf_*AT and | | |
| pCDF Duet-1 *Se_*PDUP::*Ll_*KDCA | No | 0.08 |
| pET30a-BB and | | |
| pCDF Duet-1 | No | 0.15 |
| pET30a-BB *Pf_AT::Cn_*PHAS and | | |
| pCDF Duet-1 *Se_*PDUP::*Ll_*KDCA | Yes | 2.00 |
| pET30a-BB *PF*_AT and | | |
| pCDF Duet-1 *Se_*PDUP::*Ll_*KDCA | Yes | 4.13 |
| pET30a-BB and | | |
| pCDF Duet-1 | Yes | 0.73 |

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

**1.** A micro-organism that is able to convert homoserine to 3-hydroxypropionaldehyde, wherein the micro-organism expresses a protein capable of converting homoserine to 2-keto-4-hydroxybutyrate wherein the protein is selected from the group consisting of: aminotransferases, an amino acid oxidases, and amino acid dehydrogenases.

**2.** The micro-organism of claim 1, wherein the protein is an aminotransferase and wherein the aminotransferase has the amino acid sequence as set out in SEQ ID NOs: 2, 4, 6, 8, 10 or 12, or a polypeptide having at least 60% amino acid homology thereto, preferably at least 80% amino acid homology thereto.

**2.** The micro-organism of claim 1, wherein the protein is an aminotransferase and wherein the aminotransferase is the pig heart glutamate-oxaloacetate aminotransferase set out in SEQ ID NO: 2 or a polypeptide having at least 60% amino acid homology thereto, preferably at least 80% amino acid homology thereto.

**3.** The micro-organism of claim 1, wherein the protein is selected from the group consisting of: glutamate-pyruvate aminotransferase; L-aspartate:2-oxoglutarate aminotransferase; L-alanine:2-oxoglutarate aminotransferase; L-amino acid oxidase; L-amino acid dehydrogenase.

**4.** The micro-organism of any of the preceding claims, wherein the micro-organism expresses a protein capable of converting 2-keto-4-hydroxybutyrate to 3-hydroxypropionaldehyde.

**5.** The micro-organism of claim 4, wherein the protein capable of converting 2-keto-4-hydroxybutyrate to 3-hydroxypropionaldehyde is 2-keto acid decarboxylase.

**6.** The micro-organism of claims 4, wherein the protein capable of converting 2-keto-4-hydroxybutyrate to 3-hydroxypropionaldehyde is selected from the group consisting of 2-keto acid decarboxylase KdcA set out in SEQ ID NO: 54, or a polypeptide having at least 60% amino acid homology thereto, preferably at least 80% amino acid homology thereto.

**7.** The micro-organism of any of the preceding claims, wherein the micro-organism is able to convert homoserine to 2-keto-4-hydroxybutyrate, 2-keto-4-hydroxybutyrate to 3-hydroxypropionaldehyde, and 3-hydroxy-propionaldehyde to 3-hydroxypropionate.

**8.** The micro-organism of claim 7, wherein the micro-organism expresses a protein capable of converting 3-hydroxy-propionaldehyde to 3-hydroxypropionate and wherein the protein capable of converting 3-hydroxy-propionaldehyde to 3-hydroxypropionate is a dehydrogenase, preferably an aldehyde dehydrogenase.

**9.** The micro-organism of claim 8, wherein the aldehyde dehydrogenase is as set out in SEQ ID NOs: 56 or 58, or a polypeptide having at least 60% amino acid homology thereto, preferably at least 80% amino acid homology thereto.

**10.** The micro-organism of any of the preceding claims, wherein the micro-organism is able to convert 3-hydroxy-propionaldehyde to 3-hydroxypropionyl-CoA and 3-hydroxypropionyl-CoA to 3-hydroxypropionate; and wherein the the micro-organism expresses a protein capable of converting 3-hydroxy-propionaldehyde to 3-hydroxypropionyl-CoA; and wherein the micro-organism expresses a protein capable of converting 3-hydroxypropionyl-CoA to 3-hydroxypropionate; and wherein the protein capable of converting 3-hydroxy-propionaldehyde to 3-hydroxypropionyl-CoA is a propionaldehyde dehydrogenase (pduP); and wherein the protein capable of converting 3-hydroxypropionyl-CoA to 3-hydroxypropionate is a 3-hydroxypropionyl-CoA transferase or thioesterase.

**11.** The micro-organism of claim 10, wherein the protein capable of converting 3-hydroxypropionyl-CoA to 3-hydroxypropionate is as set out in SEQ ID NOs: 90, 92, or 94, or a polypeptide having at least 60% amino acid homology thereto, preferably at least 80% amino acid homology thereto.

**12.** The micro-organism of any of the preceding claims, wherein the micro-organism expresses:
- the transferase of SEQ ID NO: 8, or a polypeptide having at least 60% amino acid homology thereto, preferably at least 80% amino acid homology thereto; and
- the decarboxylase of SEQ ID NO: 54, or a polypeptide having at least 60% amino acid homology thereto, preferably at least 80% amino acid homology thereto; and
- the dehydrogenase of SEQ ID NO: 60, or a polypeptide having at least 60% amino acid homology thereto, preferably at least 80% amino acid homology thereto.

**13.** A method for producing 3-hydroxypropionate or 3-hydroxypropionic acid, comprising culturing a micro-organism of any of the preceding claims in media comprising L-homoserine.

**14.** A process for producing acrylate comprising:
(a) culturing a micro-organism of any of claims 1 to 12 in media comprising L-homoserine;
(b) isolating 3-hydroxypropionate or 3-hydroxypropionic acid from the micro-organism;
(c) converting 3-hydroxypropionate or 3-hydroxypropionic acid into acrylate.

**15.** Use of a micro-organism according to any of claims 1 to 12 for producing 3-hydroxypropionate or 3-hydroxypropionic acid.
